(19) **Europäisches Patentamt / European Patent Office / Office européen des brevets**

(11) **EP 4 119 577 A2**

(12) # EUROPEAN PATENT APPLICATION

(43) Date of publication:
**18.01.2023 Bulletin 2023/03**

(21) Application number: **22177615.6**

(22) Date of filing: **18.06.2013**

(51) International Patent Classification (IPC):
**C07K 16/18** (2006.01)    **C07K 16/40** (2006.01)
**A61K 39/00** (2006.01)    **A61K 45/06** (2006.01)

(52) Cooperative Patent Classification (CPC):
**C07K 16/40; A61K 45/06; A61P 1/00; A61P 1/16;
A61P 7/00; A61P 7/02; A61P 11/00; A61P 11/06;
A61P 13/12; A61P 17/02; A61P 19/02;
A61P 27/02; A61P 29/00; A61P 31/00;
A61P 31/04;** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **18.06.2012 US 201261661167 P**

(62) Document number(s) of the earlier application(s) in
accordance with Art. 76 EPC:
**21152111.7 / 3 878 865
13807326.7 / 2 861 246**

(71) Applicants:
• **Omeros Corporation
Seattle, WA 98119 (US)**
• **University of Leicester
Leicester LE1 7RH (GB)**

(72) Inventors:
• **Schwaeble, Hans-Wilhelm
Mountsorrel, LE12 7AF (GB)**

• **Demopulos, Gregory A.
Seattle, 98040 (US)**
• **Dudler, Thomas
Bellevue, 98005 (US)**
• **Gray, Patrick
Seattle, 98122 (US)**

(74) Representative: **Avidity IP
Broers Building
Hauser Forum
21 JJ Thomson Avenue
Cambridge CB3 0FA (GB)**

Remarks:
•The complete document including Reference
Table(s) and the Sequence Listing(s) can be
downloaded from the EPO website
•This application was filed on 07.06.2022 as a
divisional application to the application mentioned
under INID code 62.

(54) **COMPOSITIONS AND METHODS OF INHIBITING MASP-1 AND/OR MASP-2 AND/OR MASP-3 FOR THE TREATMENT OF VARIOUS DISEASES AND DISORDERS**

(57)    In one aspect, the invention provides methods and compositions for inhibiting MASP-3-dependent complement activation in a subject suffering from or at risk for developing, a disease or disorder selected from the group consisting of paroxysmal nocturnal hemoglobinuria, age-related macular degeneration, arthritis, disseminated intravascular coagulation, thrombotic microangiopathy, asthma, dense deposit disease, pauci-immune necrotizing crescentic glomerulonephritis, traumatic brain injury, aspiration pneumonia, endophthalmitis, neuromyelitis optica and Behcet's disease by administering to the subject a composition comprising an amount of a MASP-3 inhibitory agent in an amount effective to inhibit MASP-3-dependent complement activation. In some embodiments, the subject is administered a MASP-2 inhibitory agent and a MASP-1 inhibitory agent, a MASP-2 inhibitory agent and a MASP-3 inhibitory agent administered, a MASP-3 inhibitory agent and a MASP-1 inhibitory agent, or a MASP-1 inhibitory agent, a MASP-2 inhibitory agent and a MASP-3 inhibitory agent.

EP 4 119 577 A2

(52) Cooperative Patent Classification (CPC): (Cont.)
**A61P 31/10; A61P 31/12; A61P 33/00;**
**A61P 33/14; A61P 35/00; A61P 37/06;**
**A61P 39/02; A61P 43/00; C07K 16/18;**
A61K 2039/505; C07K 2317/21; C07K 2317/31;
C07K 2317/33; C07K 2317/622; C07K 2317/76;
C07K 2317/92

**Description**

CROSS-REFERENCE TO RELATED APPLICATION

This application claims the benefit of Application No 61/661,167, filed June 18, 2012.

STATEMENT REGARDING SEQUENCE LISTING

The sequence listing associated with this application is provided in text format in lieu of a paper copy and is hereby incorporated by reference into the specification. The name of the text file containing the sequence listing is MP_1_0176_PCT_SequenceListingasFiled_20130614.txt and is being submitted via EFS-Web with the filing of the specification.

BACKGROUND

The complement system provides an early acting mechanism to initiate, amplify and orchestrate the immune response to microbial infection and other acute insults (M.K. Liszewski and J.P. Atkinson, 1993, in Fundamental Immunology, Third Edition, edited by W.E. Paul, Raven Press, Ltd., New York), in humans and other vertebrates. While complement activation provides a valuable first-line defense against potential pathogens, the activities of complement that promote a protective immune response can also represent a potential threat to the host (K.R. Kalli, et al., Springer Semin. Immunopathol. 15:417-431, 1994; B.P. Morgan, Eur. J. Clinical Investig. 24:219-228, 1994). For example, C3 and C5 proteolytic products recruit and activate neutrophils. While indispensable for host defense, activated neutrophils are indiscriminate in their release of destructive enzymes and may cause organ damage. In addition, complement activation may cause the deposition of lytic complement components on nearby host cells as well as on microbial targets, resulting in host cell lysis.

The complement system has also been implicated in the pathogenesis of numerous acute and chronic disease states, including: myocardial infarction, stroke, ARDS, reperfusion injury, septic shock, capillary leakage following thermal burns, postcardiopulmonary bypass inflammation, transplant rejection, rheumatoid arthritis, multiple sclerosis, myasthenia gravis, and Alzheimer's disease. In almost all of these conditions, complement is not the cause but is one of several factors involved in pathogenesis. Nevertheless, complement activation may be a major pathological mechanism and represents an effective point for clinical control in many of these disease states. The growing recognition of the importance of complement-mediated tissue injury in a variety of disease states underscores the need for effective complement inhibitory drugs. To date, Eculizumab (Solaris®), an antibody against C5, is the only complement-targeting drug that has been approved for human use. Yet, C5 is one of several effector molecules located "downstream" in the complement system, and blockade of C5 does not inhibit activation of the complement system. Therefore, an inhibitor of the initiation steps of complement activation would have significant advantages over a "downstream" complement inhibitor.

Currently, it is widely accepted that the complement system can be activated through three distinct pathways: the classical pathway, the lectin pathway, and the alternative pathway. The classical pathway is usually triggered by a complex composed of host antibodies bound to a foreign particle (*i.e.*, an antigen) and thus requires prior exposure to an antigen for the generation of a specific antibody response. Since activation of the classical pathway depends on a prior adaptive immune response by the host, the classical pathway is part of the acquired immune system. In contrast, both the lectin and alternative pathways are independent of adaptive immunity and are part of the innate immune system.

The activation of the complement system results in the sequential activation of serine protease zymogens. The first step in activation of the classical pathway is the binding of a specific recognition molecule, C1q, to antigen-bound IgG and IgM molecules. C1q is associated with the C1r and C1s serine protease proenzymes as a complex called C1. Upon binding of C1q to an immune complex, autoproteolytic cleavage of the Arg-Ile site of C1r is followed by C1r-mediated cleavage and activation of C1s, which thereby acquires the ability to cleave C4 and C2. C4 is cleaved into two fragments, designated C4a and C4b, and, similarly, C2 is cleaved into C2a and C2b. C4b fragments are able to form covalent bonds with adjacent hydroxyl or amino groups and generate the C3 convertase (C4b2a) through noncovalent interaction with the C2a fragment of activated C2. C3 convertase (C4b2a) activates C3 by proteolytic cleavage into C3a and C3b subcomponents leading to generation of the C5 convertase (C4b2a3b), which, by cleaving C5 leads to the formation of the membrane attack complex (C5b combined with C6, C7, C8 and C-9, also referred to as "MAC") that can disrupt cellular membranes resulting in cell lysis. The activated forms of C3 and C4 (C3b and C4b) are covalently deposited on the foreign target surfaces, which are recognized by complement receptors on multiple phagocytes.

Independently, the first step in activation of the complement system through the lectin pathway is also the binding of specific recognition molecules, which is followed by the activation of associated serine protease proenzymes. However, rather than the binding of immune complexes by C1q, the recognition molecules in the lectin pathway comprise a group of carbohydrate-binding proteins (mannan-binding lectin (MBL), H-ficolin, M-ficolin, L-ficolin and C-type lectin CL-11), collectively referred to as lectins. See J. Lu et al., Biochim. Biophys. Acta 1572:387-400, (2002); Holmskov et al., Annu. Rev. Immunol. 21:547-578 (2003); Teh et al., Immunology 101:225-232 (2000)). See also J. Luet et al., Biochim Biophys Acta 1572:387-400 (2002); Holmskov et al, Annu Rev Immunol 21:547-578 (2003); Teh et al., Immunology 101:225-232 (2000); Hansen et al, J. Immunol 185(10):6096-6104 (2010).

Ikeda et al. first demonstrated that, like C1q, MBL could activate the complement system upon binding to yeast mannan-coated erythrocytes in a C4-dependent manner (Ikeda et al., J. Biol. Chem. 262:7451-7454, (1987)). MBL, a member of the collectin protein family, is a calcium-dependent lectin that binds carbohydrates with 3-and 4-hydroxy groups oriented in the equatorial plane of the pyranose ring. Prominent ligands for MBL are thus D-mannose and N-acetyl-D-glucosamine, while carbohydrates not fitting this steric requirement have undetectable affinity for MBL (Weis et al., Nature 360:127-134, (1992)). The interaction between MBL and monovalent sugars is extremely weak, with dissociation constants typically in the single-digit millimolar range. MBL achieves tight, specific binding to glycan ligands by avidity, i.e., by interacting simultaneously with multiple monosaccharide residues located in close proximity to each other (Lee et al., Archiv. Biochem. Biophys. 299:129-136, (1992)). MBL recognizes the carbohydrate patterns that commonly decorate microorganisms such as bacteria, yeast, parasites and certain viruses. In contrast, MBL does not recognize D-galactose and sialic acid, the penultimate and ultimate sugars that usually decorate "mature" complex glycoconjugates present on mammalian plasma and cell surface glycoproteins. This binding specificity is thought to promote recognition of "foreign" surfaces and help protect from "self-activation." However, MBL does bind with high affinity to clusters of high-mannose "precursor" glycans on N-linked glycoproteins and glycolipids sequestered in the endoplasmic reticulum and Golgi of mammalian cells (Maynard et al., J. Biol. Chem. 257:3788-3794, (1982)). In addition, it has been shown that MBL can bind the polynucleotides, DNA and RNA, which may be exposed on necrotic and apoptotic cells (Palaniyar et al., Ann. N.Y. Acad. Sci., 1010:467-470 (2003); Nakamura et al., J. Leuk. Biol. 86:737-748 (2009)). Therefore, damaged cells are potential targets for lectin pathway activation via MBL binding.

The ficolins possess a different type of lectin domain than MBL, called the fibrinogen-like domain. Ficolins bind sugar residues in a $Ca^{++}$-independent manner. In humans, three kinds of ficolins (L-ficolin, M-ficolin and H-ficolin) have been identified. The two serum ficolins, L-ficolin and H-ficolin, have in common a specificity for N-acetyl-D-glucosamine; however, H-ficolin also binds N-acetyl-D-galactosamine. The difference in sugar specificity of L-ficolin, H-ficolin, CL-11, and MBL means that the different lectins may be complementary and target different, though overlapping, glycoconjugates. This concept is supported by the recent report that, of the known lectins in the lectin pathway, only L-ficolin binds specifically to lipoteichoic acid, a cell wall glycoconjugate found on all Gram-positive bacteria (Lynch et al., J. Immunol. 172:1198-1202, (2004)). In addition to acetylated sugar moieties, the ficolins can also bind acetylated amino acids and polypeptides (Thomsen et al., Mol. Immunol. 48(4):369-81 (2011)). The collectins (i.e., MBL) and the ficolins bear no significant similarity in amino acid sequence. However, the two groups of proteins have similar domain organizations and, like C1q, assemble into oligomeric structures, which maximize the possibility of multisite binding.

The serum concentrations of MBL are highly variable in healthy populations and this is genetically controlled by polymorphisms/mutations in both the promoter and coding regions of the MBL gene. As an acute phase protein, the expression of MBL is further upregulated during inflammation. L-ficolin is present in serum at concentrations similar to those of MBL. Therefore, the L-ficolin branch of the lectin pathway is potentially comparable to the MBL arm in strength. MBL and ficolins can also function as opsonins, which allow phagocytes to target MBL- and ficolin-decorated surfaces (see Jack et al., J Leukoc Biol., 77(3):328-36 (2004), Matsushita and Fujita, Immunobiology, 205(4-5):490-7 (2002), Aoyagi et al., J Immunol, 174(1):418-25(2005). This opsonization requires the interaction of these proteins with phagocyte receptors (Kuhlman et al., J. Exp. Med. 169:1733, (1989); Matsushita et al., J. Biol. Chem. 271:2448-54, (1996)), the identity of which has not been established.

Human MBL forms a specific and high-affinity interaction through its collagen-like domain with unique C1r/C1s-like serine proteases, termed MBL-associated serine proteases (MASPs). To date, three MASPs have been described. First, a single enzyme "MASP" was identified and characterized as the enzyme responsible for the initiation of the complement cascade (i.e., cleaving C2 and C4) (Matsushita et al., J Exp Med 176(6):1497-1502 (1992); Ji et al., J. Immunol. 150:571-578, (1993)). It was subsequently determined that the MASP activity was, in fact, a mixture of two proteases: MASP-1 and MASP-2 (Thiel et al., Nature 386:506-510, (1997)). However, it was demonstrated that the MBL-MASP-2 complex alone is sufficient for complement activation (Vorup-Jensen et al., J. Immunol. 165:2093-2100, (2000)). Furthermore, only MASP-2 cleaved C2 and C4 at high rates (Ambrus et al., J. Immunol. 170:1374-1382, (2003)). Therefore, MASP-2 is the protease responsible for activating C4 and C2 to generate the C3 convertase, C4b2a. This is a significant difference from the C1 complex of the classical pathway, where the coordinated action of two specific serine proteases (C1r and C1s) leads to the activation of the complement system. In addition, a third novel protease, MASP-3, has been isolated (Dahl, M.R., et al., Immunity 15:127-35, 2001). MASP-1 and MASP-3 are alternatively spliced products of the same gene.

MASPs share identical domain organizations with those of C1r and C1s, the enzymatic components of the C1 complex (Sim et al., Biochem. Soc. Trans. 28:545, (2000)). These domains include an N-terminal C1r/C1s/sea urchin VEGF/bone morphogenic protein (CUB) domain, an epidermal growth factor-like domain, a second CUB domain, a tandem of complement control protein domains, and a serine protease domain. As in the C1 proteases, activation of MASP-2 occurs through cleavage of an Arg-Ile bond adjacent to the serine protease domain, which splits the enzyme into disulfide-linked A and B chains, the latter consisting of the serine protease domain.

MBL can also associate with an alternatively spliced form of MASP-2, known as MBL-associated protein of 19 kDa (MAp19) or small MBL-associated protein (sMAP), which lacks the catalytic activity of MASP-2. (Stover, J. Immunol. 162:3481-90, (1999); Takahashi et al., Int. Immunol. 11:859-863, (1999)). MAp19 comprises the first two domains of MASP-2, followed by an extra sequence of four unique amino acids. The function of Map19 is unclear (Degn et al., J Immunol. Methods, 2011). The MASP-1 and MASP-2 genes are located on human chromosomes 3 and 1, respectively (Schwaeble et al., Immunobiology 205:455-466, (2002)).

Several lines of evidence suggest that there are different MBL-MASP complexes and a large fraction of the MASPs in serum is not complexed with MBL (Thiel, et al., J. Immunol. 165:878-887, (2000)). Both H- and L-ficolin bind to all MASPs and activate the lectin complement pathway, as does MBL (Dahl et al., Immunity 15:127-35, (2001); Matsushita et al., J. Immunol. 168:3502-3506, (2002)). Both the lectin and classical pathways form a common C3 convertase (C4b2a) and the two pathways converge at this step.

The lectin pathway is widely thought to have a major role in host defense against infection in the naive host. Strong evidence for the involvement of MBL in host defense comes from analysis of patients with decreased serum levels of functional MBL (Kilpatrick, Biochim. Biophys. Acta 1572:401-413, (2002)). Such patients display susceptibility to recurrent bacterial and fungal infections. These symptoms are usually evident early in life, during an apparent window of vulnerability as maternally derived antibody titer wanes, but before a full repertoire of antibody responses develops. This syndrome often results from mutations at several sites in the collagenous portion of MBL, which interfere with proper formation of MBL oligomers. However, since MBL can function as an opsonin independent of complement, it is not known to what extent the increased susceptibility to infection is due to impaired complement activation.

In contrast to the classical and lectin pathways, no initiators of the alternative pathway have previously been found to fulfill the recognition functions that C1q and lectins perform in the other two pathways. Currently it is widely accepted that the alternative pathway spontaneously undergoes a low level of turnover activation, which can be readily amplified on foreign or other abnormal surfaces (bacteria, yeast, virally infected cells, or damaged tissue) that lack the proper molecular elements that keep spontaneous complement activation in check. There are four plasma proteins directly involved in the activation of the alternative pathway: C3, factors B and D, and properdin.

Although there is extensive evidence implicating both the classical and alternative complement pathways in the pathogenesis of non-infectious human diseases, the role of the lectin pathway is just beginning to be evaluated. Recent studies provide evidence that activation of the lectin pathway can be responsible for complement activation and related inflammation in ischemia/reperfusion injury. Collard et al. (2000) reported that cultured endothelial cells subjected to oxidative stress bind MBL and show deposition of C3 upon exposure to human serum (Collard et al., Am. J. Pathol. 156:1549-1556, (2000)). In addition, treatment of human sera with blocking anti-MBL monoclonal antibodies inhibited MBL binding and complement activation. These findings were extended to a rat model of myocardial ischemia-reperfusion in which rats treated with a blocking antibody directed against rat MBL showed significantly less myocardial damage upon occlusion of a coronary artery than rats treated with a control antibody (Jordan et al., Circulation 104:1413-1418, (2001)). The molecular mechanism of MBL binding to the vascular endothelium after oxidative stress is unclear; a recent study suggests that activation of the lectin pathway after oxidative stress may be mediated by MBL binding to vascular endothelial cytokeratins, and not to glycoconjugates (Collard et al., Am. J. Pathol. 159:1045-1054, (2001)). Other studies have implicated the classical and alternative pathways in the pathogenesis of ischemia/reperfusion injury and the role of the lectin pathway in this disease remains controversial (Riedermann, N.C., et al., Am. J. Pathol. 162:363-367, 2003).

Recent studies have shown that MASP-1 and MASP-3 convert the alternative pathway activation enzyme factor D from its zymogen form into its enzymatically active form (see Takahashi M. et al., J Exp Med 207(1):29-37 (2010); Iwaki et al., J. Immunol. 187:3751-58 (2011)). The physiological importance of this process is underlined by the absence of alternative pathway functional activity in plasma of MASP-1/3-deficient mice. Proteolytic generation of C3b from native C3 is required for the alternative pathway to function. Since the alternative pathway C3 convertase (C3bBb) contains C3b as an essential subunit, the question regarding the origin of the first C3b via the alternative pathway has presented a puzzling problem and has stimulated considerable research.

C3 belongs to a family of proteins (along with C4 and $\alpha$-2 macroglobulin) that contain a rare posttranslational modification known as a thioester bond. The thioester group is composed of a glutamine whose terminal carbonyl group forms a covalent thioester linkage with the sulfhydryl group of a cysteine three amino acids away. This bond is unstable and the electrophilic glutamyl-thioester can react with nucleophilic moieties such as hydroxyl or amino groups and thus form a covalent bond with other molecules. The thioester bond is reasonably stable when sequestered within a hydrophobic pocket of intact C3. However, proteolytic cleavage of C3 to C3a and C3b results in exposure of the highly reactive thioester bond on C3b and, following nucleophilic attack by adjacent moieties comprising hydroxyl or amino groups, C3b becomes covalently linked to a target. In addition to its well-documented role in covalent attachment of C3b to complement targets, the C3 thioester is also thought to have a pivotal role in triggering the alternative pathway. According to the widely accepted "tick-over theory", the alternative pathway is initiated by the generation of a fluid-phase convertase, iC3Bb, which is formed from C3 with hydrolyzed thioester (iC3; C3($H_2$O)) and factor B (Lachmann, P.J., et al., Springer Semin. Immunopathol. 7:143-162, (1984)). The C3b-like C3($H_2$O) is generated from native C3 by a slow spontaneous hydrolysis of the internal thioester in the protein (Pangburn, M.K., et al., J. Exp. Med. 154:856-867, 1981). Through the activity of the C3($H_2$O)Bb convertase, C3b molecules are deposited on the target surface thereby initiating the alternative pathway.

Prior to the instant discovery described herein, very little was known about the initiators of activation of the alternative pathway. Activators were thought to include yeast cell walls (zymosan), many pure polysaccharides, rabbit erythrocytes, certain immunoglobulins, viruses, fungi, bacteria, animal tumor cells, parasites, and damaged cells. The only feature common to these activators is the presence of carbohydrate, but the complexity and variety of carbohydrate structures has made it difficult to establish the shared molecular determinants which are recognized. It has been widely accepted that alternative pathway activation is controlled through the fine balance between inhibitory regulatory components of this pathway, such as factor H, factor I, DAF, and CR1, and properdin, the latter of which is the only positive regulator of the alternative pathway (see Schwaeble W.J. and Reid K.B., Immunol Today 20(1):17-21 (1999)).

In addition to the apparently unregulated activation mechanism described above, the alternative pathway can also provide a powerful amplification loop for the lectin/classical pathway C3 convertase (C4b2a) since any C3b generated can participate with factor B in forming additional alternative pathway C3 convertase (C3bBb). The alternative pathway C3 convertase is stabilized by the binding of properdin. Properdin extends the alternative pathway C3 convertase half-life six to ten fold. Addition of C3b to the alternative pathway C3 convertase leads to the formation of the alternative pathway C5 convertase.

All three pathways (i.e., the classical, lectin and alternative) have been thought to converge at C5, which is cleaved to form products with multiple proinflammatory effects. The converged pathway has been referred to as the terminal complement pathway. C5a is the most potent anaphylatoxin, inducing alterations in smooth muscle and vascular tone, as well as vascular permeability. It is also a powerful chemotaxin and activator of both neutrophils and monocytes. C5a-mediated cellular activation can significantly amplify inflammatory responses by inducing the release of multiple additional inflammatory mediators, including cytokines, hydrolytic enzymes, arachidonic acid metabolites, and reactive oxygen species. C5 cleavage leads to the formation of C5b-9, also known as the membrane attack complex (MAC). There is now strong evidence that sublytic MAC deposition may play an important role in inflammation in addition to its role as a lytic pore-forming complex.

In addition to its essential role in immune defense, the complement system contributes to tissue damage in many clinical conditions. Thus, there is a pressing need to develop therapeutically effective complement inhibitors to prevent these adverse effects.

SUMMARY

In one aspect, the present invention provides a method of inhibiting MASP-3-dependent complement activation in a subject suffering from paroxysmal nocturnal hemoglobinuria (PNH), age-related macular degeneration (AMD), ischemia-reperfusion injury, arthritis, disseminated intravascular coagulation, thrombotic microangiopathy, asthma, dense deposit disease, pauci-immune necrotizing crescentic glomerulonephritis, traumatic brain injury, aspiration pneumonia, endophthalmitis, neuromyelitis optica or Behcet's disease. The method includes the step of administering to the subject a composition comprising an amount of a MASP-3 inhibitory agent effective to inhibit MASP-3- dependent complement activation. In some embodiments, the method further comprises administering to the subject a composition comprising a MASP-2 inhibitory agent.

In another aspect, the present invention provides a method of inhibiting MASP-2-dependent complement activation in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica or Behcet's disease. The method includes the step of administering to the subject a composition comprising an amount of a MASP-2 inhibitory agent effective to inhibit MASP-2 dependent complement activation. In some embodiments, the MASP-2 inhibitory agent is a MASP-2 antibody or fragment thereof. In some embodiments, the MASP-2 inhibitory agent is a MASP-2 monoclonal antibody, or fragment thereof that specifically binds to a portion of SEQ ID NO:5. In some embodiments, the MASP-2 antibody is a chimeric, humanized or human antibody.

In another aspect, the present invention provides a pharmaceutical composition comprising at least one inhibitory agent, wherein the at least one inhibitory agent comprises a MASP-2 inhibitory agent and a MASP-3 inhibitory agent and a pharmaceutically acceptable carrier.

In another aspect, the present invention provides a pharmaceutical composition comprising a MASP-3 inhibitory agent that binds to a portion of MASP-1 (SEQ ID NO: 10: full-length) and that also binds to a portion of MASP-3 (SEQ ID NO:8) and a pharmaceutical carrier.

In another aspect, the present invention provides a pharmaceutical composition comprising a MASP-3 inhibitory agent that binds to a portion of MASP-2 (SEQ ID NO: 5: full-length) and that also binds to a portion of MASP-3 (SEQ ID NO:8) and a pharmaceutical carrier.

In another aspect, the present invention provides a pharmaceutical composition comprising a MASP-3 inhibitory agent that binds to a portion of MASP-1 (SEQ ID NO: 10: full-length) and that also binds to a portion of MASP-2 (SEQ ID NO:5) and a pharmaceutical carrier.

In another aspect, the present invention provides a pharmaceutical composition comprising a MASP-3 inhibitory agent that binds to a portion of MASP-1 (SEQ ID NO: 10 full length), that binds to a portion of MASP-2 (SEQ ID NO: 5: full-length) and that also binds to a portion of MASP-3 (SEQ ID NO:8) and a pharmaceutical carrier.

In another aspect, the present invention provides a method of manufacturing a medicament for use in inhibiting the effects of MASP-3-dependent complement activation in living subjects in need thereof, comprising combining a therapeutically effective amount of a MASP-3 inhibitory agent in a pharmaceutical carrier. In some embodiments, the method in accordance with this aspect of the invention comprises manufacturing a medicament for use in inhibiting the effects of MASP-3-dependent complement activation in a subject suffering from, or at risk for developing a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy, asthma, dense deposit disease, pauci-immune necrotizing crescentic glomerulonephritis, traumatic brain injury, aspiration pneumonia, endophthalmitis, neuromyelitis optica or Behcet's disease. In some embodiments, the method further comprises combining a therapeutically effective amount of a MASP-2 inhibitory agent into or with the medicament comprising the MASP-3 inhibitor.

In another aspect, the present invention provides a method of manufacturing a medicament for use in inhibiting the effects of MASP-2-dependent complement activation in living subjects in need thereof, comprising combining a therapeutically effective amount of a MASP-2 inhibitory agent in a pharmaceutical carrier. In some embodiments, the method in accordance with this aspect of the invention comprises manufacturing a medicament for use in inhibiting the effects of MASP-2-dependent complement activation in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica or Behcet's disease. In some embodiments, the method further comprises combining a therapeutically effective amount of a MASP-3 inhibitory agent into or with the medicament comprising the MASP-2 inhibitor.

As described herein, the various embodiments of the MASP-3 inhibitory agents and/or the various embodiments of the MASP-2 inhibitory agents can be used in the pharmaceutical compositions of the invention.

As described herein, the pharmaceutical compositions of the invention can be used in accordance with the methods of the invention.

These and other aspects and embodiments of the herein described invention will be evident upon reference to the following detailed description and drawings. All of the U.S. patents, U.S. patent application publications, U.S. patent applications, foreign patents, foreign patent applications and non-patent publications referred to in this specification are incorporated herein by reference in their entirety, as if each was incorporated individually.

DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

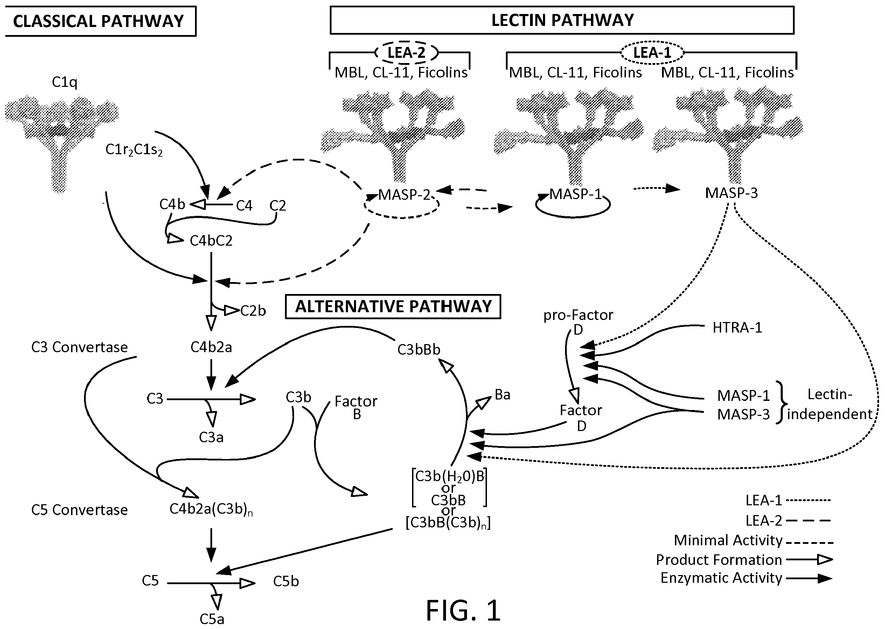

FIGURE 1 illustrates a new understanding of the lectin and alternative pathways;

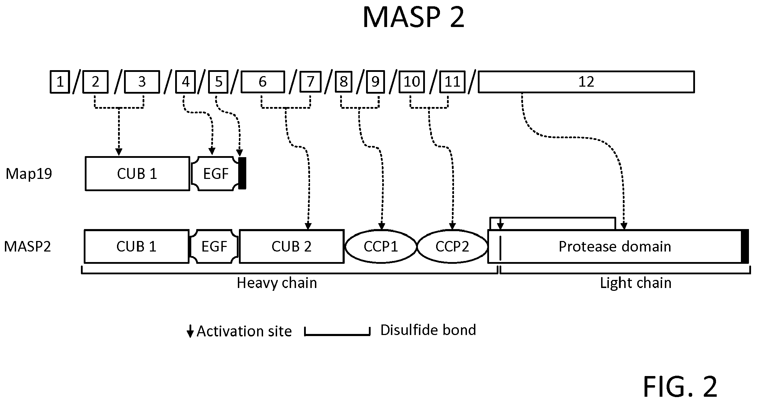

FIGURE 2 is a schematic diagram adapted from Schwaeble et al., Immunobiol 205:455-466 (2002), as modified by Yongqing et al., BBA 1824:253 (2012), illustrating the MASP-2 and MAp19 protein domains and the exons encoding the same;

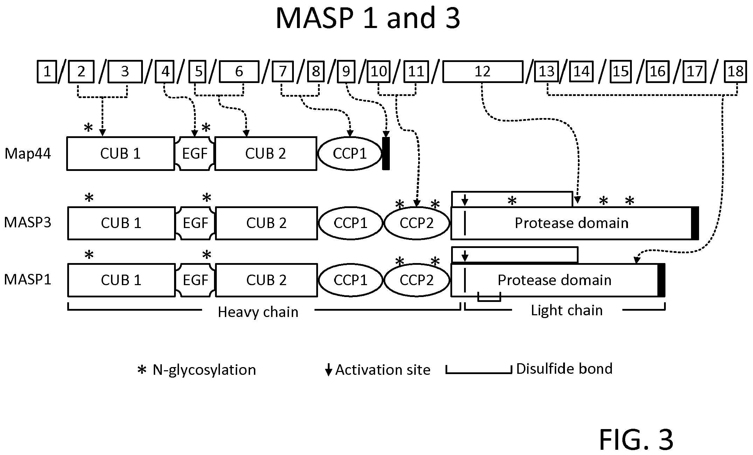

FIGURE 3 is a schematic diagram adapted from Schwaeble et al., Immunobiol 205:455-466 (2002), as modified by Yongqing et al., BBA 1824:253 (2012), illustrating the MASP-1, MASP-3 and MAp44 protein domains and the exons encoding the same;

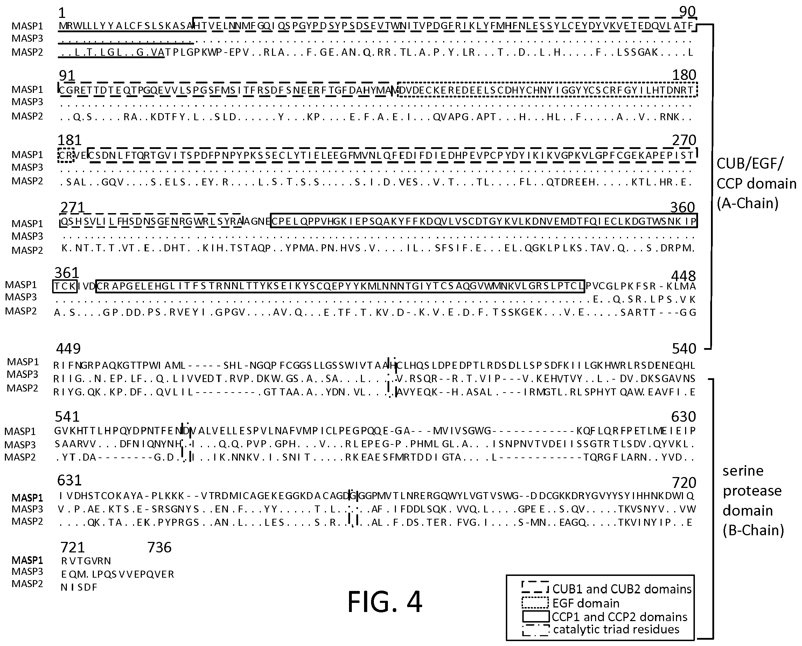

FIGURE 4 shows an alignment of the amino acid sequences of the MASP-1, MASP-2 and MASP-3 proteins and indicates consensus regions therebetween;

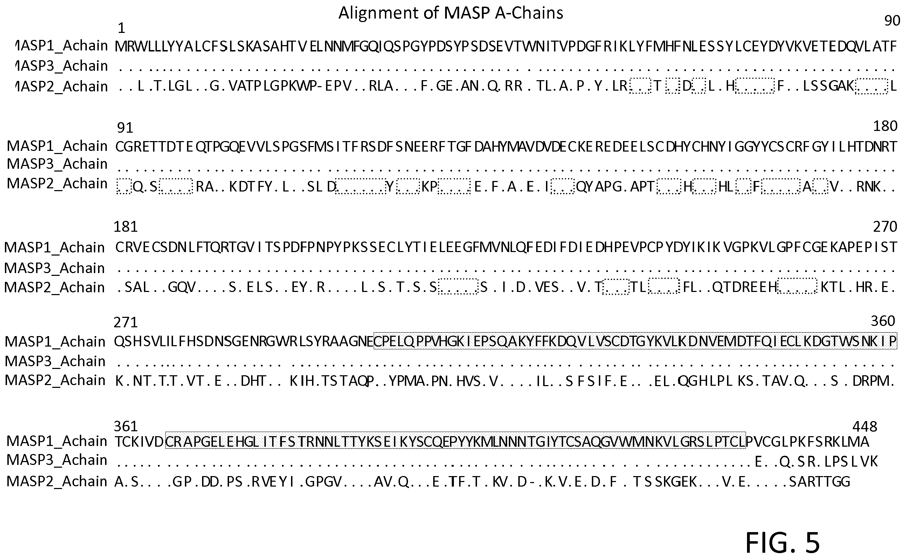

FIGURE 5 shows an alignment of the amino acid sequences of the MASP-1, MASP-2 and MASP-3 Alpha chains;

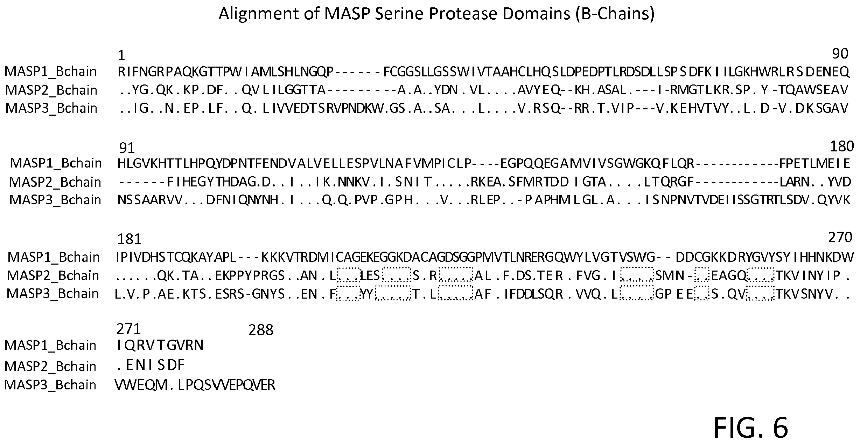

FIGURE 6 shows an alignment of the amino acid sequences of the MASP-1, MASP-2 and MASP-3 Beta Chains;

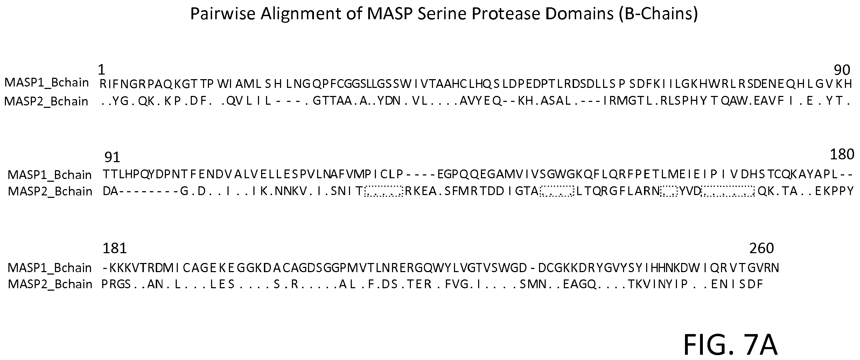

FIGURE 7A shows a pairwise alignment of the amino acid sequences of the MASP-1 and MASP-2 Protease Domains (Beta-chains);

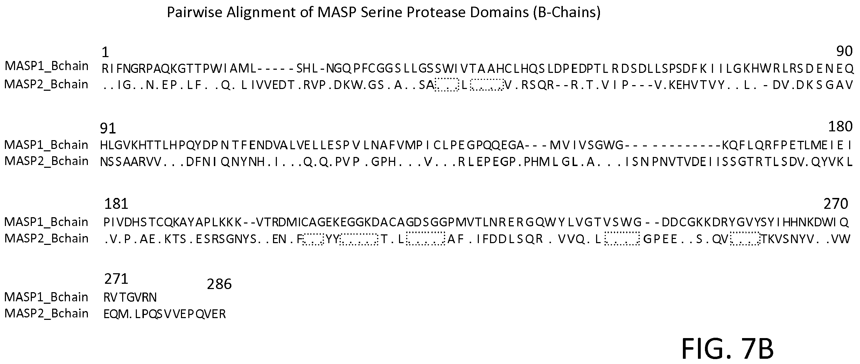

FIGURE 7B shows a pairwise alignment of the amino acid sequences of the MASP-1 and MASP-3 Protease Domains (Beta-chains);

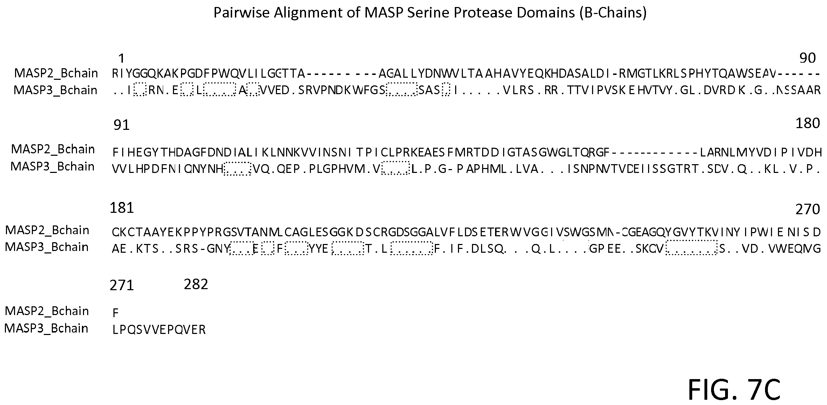

FIGURE 7C shows a pairwise alignment of the amino acid sequences of the MASP-2 and MASP-3 Protease Domains (Beta-chains);

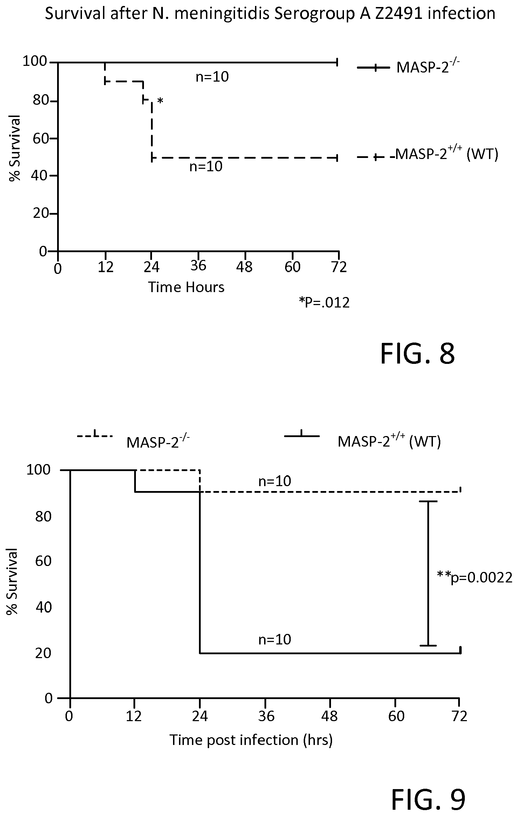

FIGURE 8 is a Kaplan-Meyer plot graphically illustrating the percent survival of MASP-2 KO and WT mice after administration of an infective dose of $2.6 \times 10^7$ cfu of *N. meningitidis* serogroup A Z2491, demonstrating that MASP-2 deficient mice are protected from *N. meningitidis* induced mortality, as described in Example 1;

FIGURE 9 is a Kaplan-Meyer plot graphically illustrating the percent survival of MASP-2 KO and WT mice after administration of an infective dose of $6 \times 10^6$ cfu of *N. meningitidis* serogroup B strain MC58, demonstrating that MASP-2 deficient mice are protected from *N. meningitidis* induced mortality, as described in Example 1;

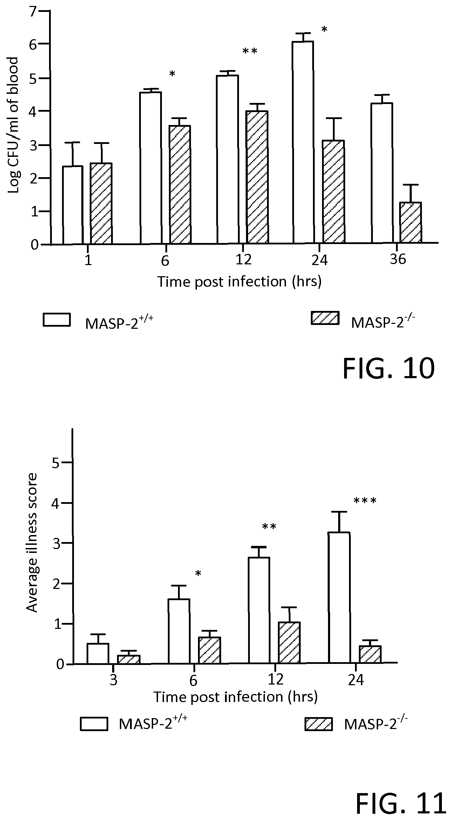

FIGURE 10 graphically illustrates the log cfu/mL of *N. meningitidis* serogroup B strain MC58 per mL of blood recovered from MASP-2 KO and WT mice at different time points after i.p. infection with $6 \times 10^6$ cfu of *N. meningitidis* serogroup B strain MC58 (n=3 at different time points for both groups of mice), demonstrating that although the MASP-2 KO mice were infected with the same dose of *N. meningitidis* serogroup B strain MC58 as the WT mice, the MASP-2 KO mice have enhanced clearance of bacteremia as compared to WT, as described in Example 1;

FIGURE 11 graphically illustrates the average illness score of MASP-2 KO and WT mice at 3, 6, 12 and 24 hours after infection with $6 \times 10^6$ cfu of *N. meningitidis* serogroup B strain MC58, demonstrating that the MASP-2-deficient mice showed much lower illness scores at 6 hours, 12 hours, and 24 hours after infection, as compared to WT mice, as described in Example 1;

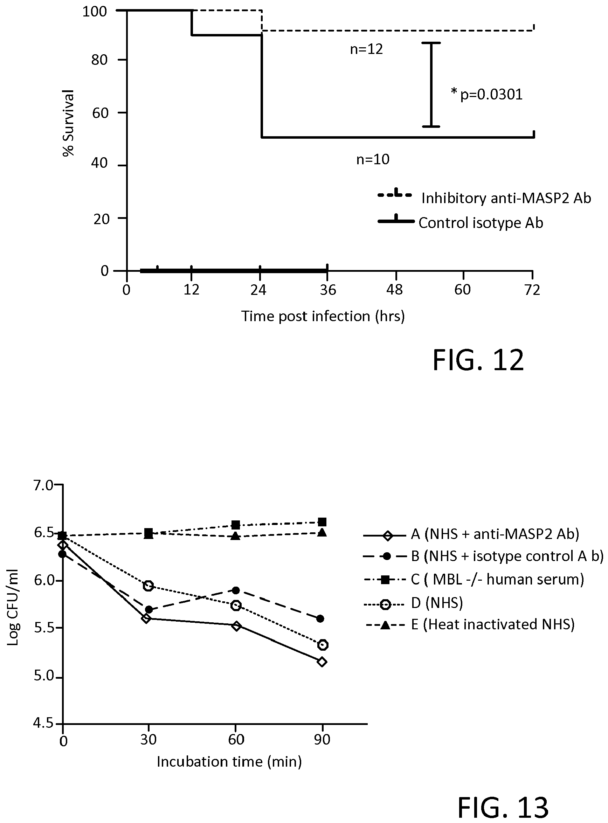

FIGURE 12 is a Kaplan-Meyer plot graphically illustrating the percent survival of mice after administration of an infective dose of $4 \times 10^6$ cfu of *N. meningitidis* serogroup B strain MC58, followed by administration 3 hours post-infection of either inhibitory MASP-2 antibody (1 mg/kg) or control isotype antibody, demonstrating that MASP-2 antibody is effective to treat and improve survival in subjects infected with N. *meningitidis,* as described in Example 2;

FIGURE 13 graphically illustrates the log cfu/mL of viable counts of *N. meningitidis* serogroup B strain MC58 recovered at different time points in the human sera samples shown in TABLE 5 taken at various time points after incubation with *N. meningitidis* serogroup B strain MC58, as described in Example 3;

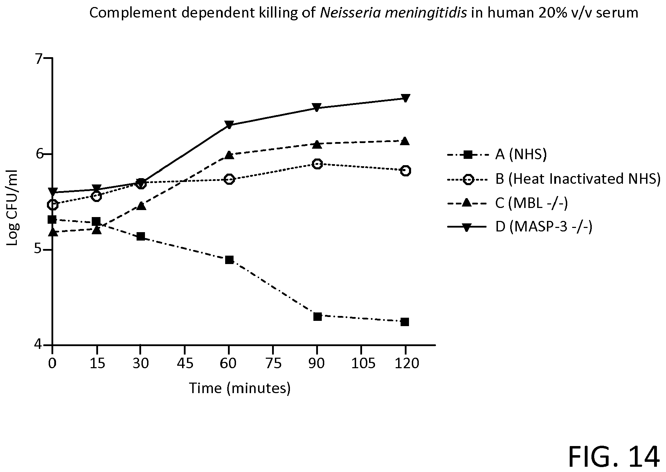

FIGURE 14 graphically illustrates the log cfu/mL of viable counts of *N. meningitidis* serogroup B-MC58 recovered at different time points in the human sera samples shown in TABLE 7, showing that complement-dependent killing of *N. meningitidis* in human 20% (v/v) serum is MASP-3 and MBL-dependent, as described in Example 3;

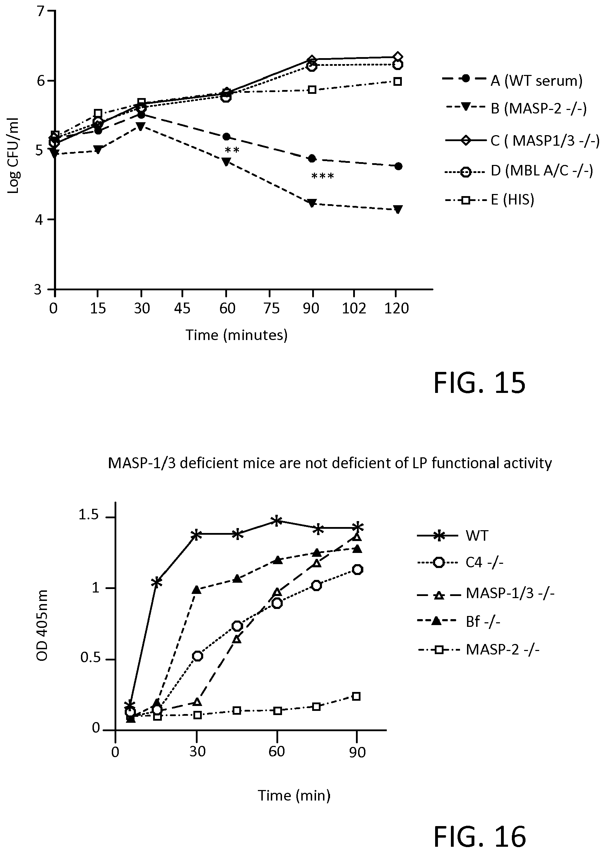

FIGURE 15 graphically illustrates the log cfu/mL of viable counts of *N. meningitidis* serogroup B-MC58 recovered at different time points in the mouse sera samples shown in TABLE 9, showing that the MASP-2 -/- knockout mouse (referred to as "MASP-2 -/-") serum has a higher level of bactericidal activity for N. meningitidis than WT mouse serum, whereas in contrast, the MASP-1/3 -/- mouse serum does not have any bactericidal activity, as described in Example 3;

FIGURE 16 graphically illustrates the kinetics of C3 activation under lectin pathway-specific conditions (1% plasma) in WT, C4-/-, MASP-1/3-/-, Factor B-/- and MASP-2-/- mouse sera, as described in Example 4;

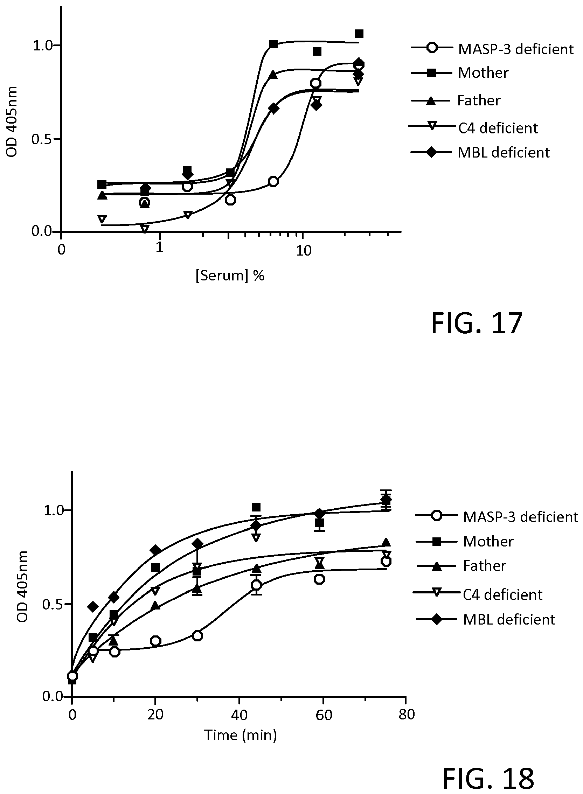

FIGURE 17 graphically illustrates the level of alternative pathway-driven (AP-driven) C3b deposition on zymosan-coated microtiter plates under "traditional" alternative pathway-specific (AP-specific) conditions (*i.e.* BBS/EGTA/Mg$^{++}$ without Ca$^{++}$) as a function of serum concentration in serum samples obtained from MASP-3-deficient, C4-deficient and MBL-deficient human subjects, as described in Example 4;

FIGURE 18 graphically illustrates the level of AP-driven C3b deposition on zymosan-coated microtiter plates under "traditional" AP-specific conditions (*i.e.*, BBS/EGTA/Mg$^{++}$ without Ca$^{++}$) as a function of time in 10% human serum samples obtained from MASP-3-deficient, C4-deficient and MBL-deficient human subjects, as described in Example 4;

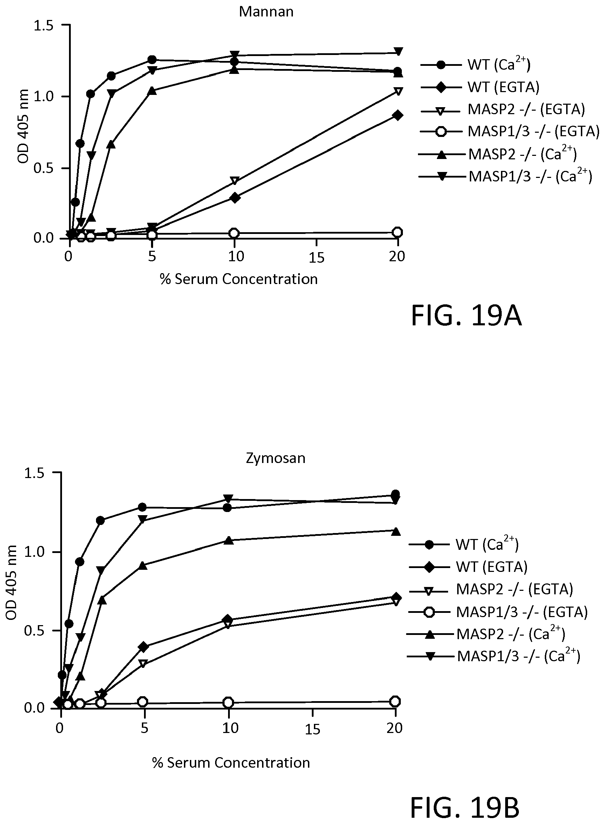

FIGURE 19A graphically illustrates the level of C3b deposition on mannan-coated microtiter plates as a function of serum concentration in serum samples obtained from WT, MASP-2-deficient, and MASP-1/3-deficient mice under "traditional" AP-specific conditions (*i.e.* BBS/EGTA/Mg$^{++}$ without Ca$^{++}$) or under physiological conditions allowing both the lectin pathway and the alternative pathway (AP) to function (BBS/Mg$^{++}$/Ca$^{++}$), as described in Example 4;

FIGURE 19B graphically illustrates the level of C3b deposition on zymosan-coated microtiter plates as a function of serum concentration in serum samples obtained from WT, MASP-2-deficient, and MASP-1/3-deficient mice under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), as described in Example 4;

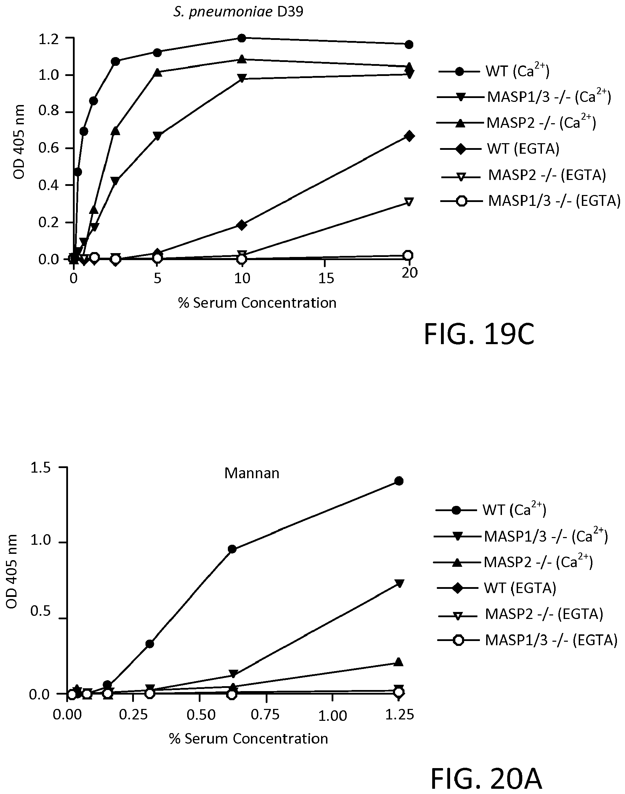

FIGURE 19C graphically illustrates the level of C3b deposition on *S. pneumoniae* D39-coated microtiter plates as a function of serum concentration in serum samples obtained from WT, MASP-2-deficient, and MASP-1/3-deficient mice under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), as described in Example 4;

FIGURE 20A graphically illustrates the results of a C3b deposition assay in highly diluted sera carried out on mannan-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), using serum concentrations ranging from 0 % up to 1.25%, as described in Example 4;

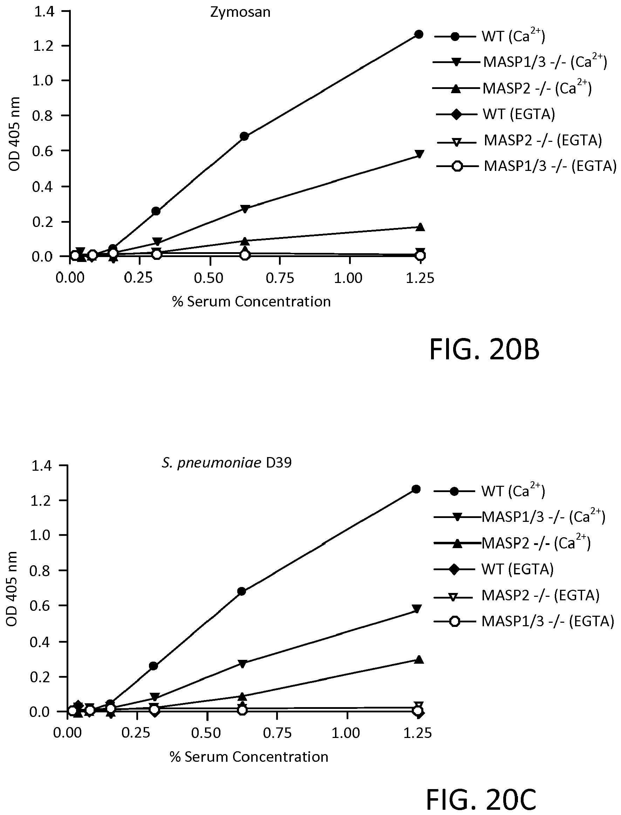

FIGURE 20B graphically illustrates the results of a C3b deposition assay carried out on zymosan-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), using serum concentrations ranging from 0 % up to 1.25%, as described in Example 4;

FIGURE 20C graphically illustrates the results of a C3b deposition assay carried out on *S. pneumoniae* D39-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), using serum concentrations ranging from 0 % up to 1.25%, as described in Example 4;

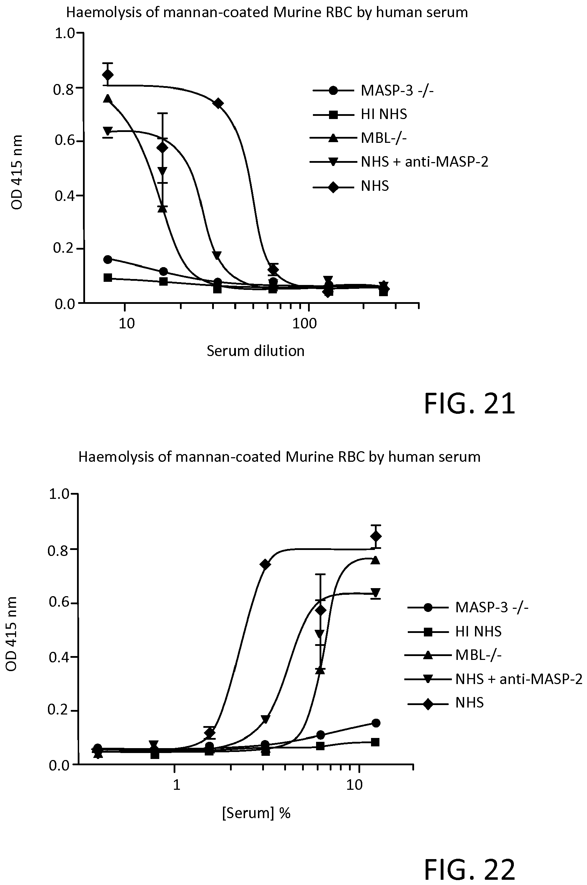

FIGURE 21 graphically illustrates the level of hemolysis (as measured by hemoglobin release of lysed mouse erythrocytes (Crry/C3-/-) into the supernatant measured by photometry) of mannan-coated murine erythrocytes by human serum under physiological conditions (*i.e.,* in the presence of Ca$^{++}$) over a range of serum dilutions in serum from MASP-3-/-, heat inactivated normal human serum (HI NHS), MBL-/-, NHS + MASP-2 monoclonal antibody and NHS control, as described in Example 5;

FIGURE 22 graphically illustrates the level of hemolysis (as measured by hemoglobin release of lysed mouse erythrocytes (Crry/C3-/-) into the supernatant measured by photometry) of mannan-coated murine erythrocytes by human serum under physiological conditions (*i.e.,* in the presence of Ca$^{++}$) over a range of serum concentration in serum from MASP-3-/-, heat inactivated (HI) NHS, MBL-/-, NHS + MASP-2 monoclonal antibody and NHS control, as described in Example 5;

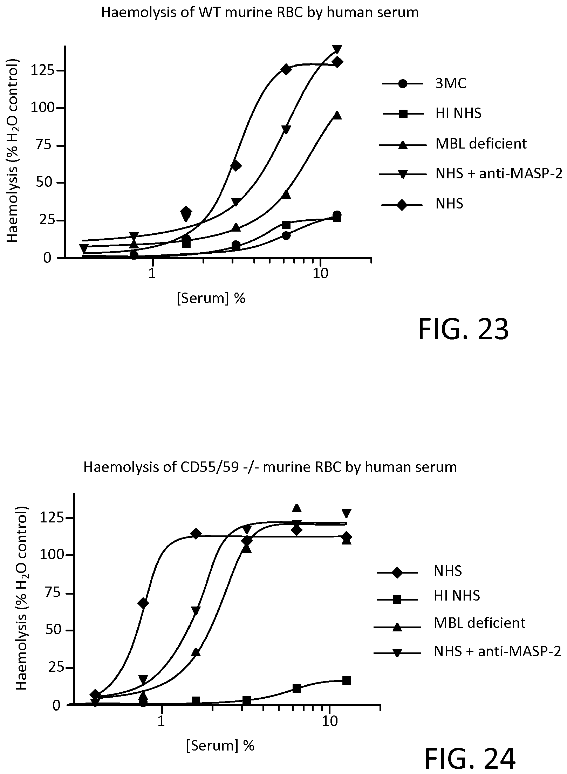

FIGURE 23 graphically illustrates the level of hemolysis (as measured by hemoglobin release of lysed WT mouse erythrocytes into the supernatant measured by photometry) of non-coated murine erythrocytes by human serum under physiological conditions (*i.e.,* in the presence of Ca$^{++}$) over a range of serum concentrations in serum from 3MC (MASP-3-/-), heat inactivated (HI) NHS, MBL-/-, NHS + MASP-2 monoclonal antibody and NHS control, as described in Example 5;

FIGURE 24 graphically illustrates hemolysis (as measured by hemoglobin release of lysed mouse erythrocytes (CD55/59-/-) into the supernatant measured by photometry) of non-coated murine erythrocytes by human serum under physiological conditions (*i.e.,* in the presence of Ca$^{++}$) over a range of serum concentrations in serum from heat inactivated (HI) NHS, MBL-/-, NHS + MASP-2 monoclonal antibody and NHS control, as described in Example 5;

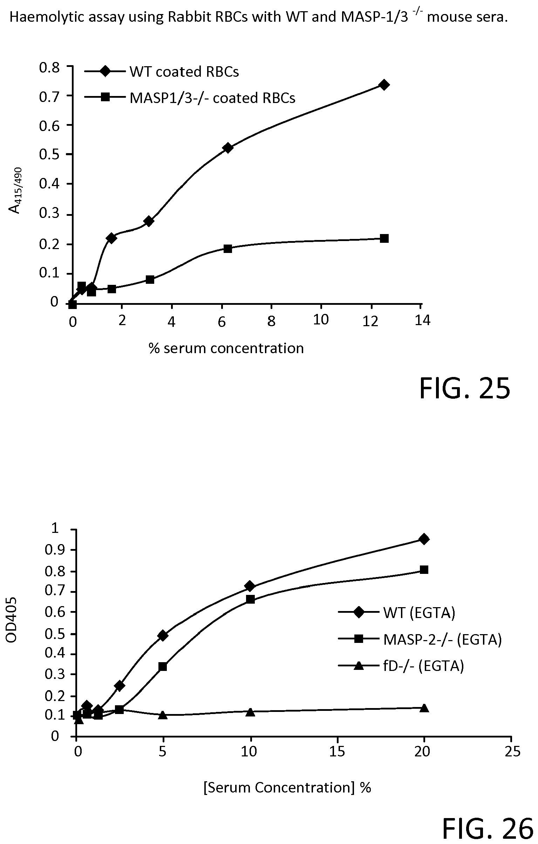

FIGURE 25 graphically illustrates hemolysis (as measured by hemoglobin release of lysed rabbit erythrocytes into the supernatant measured by photometry) of mannan-coated rabbit erythrocytes by MASP-1/3-/- mouse serum and WT control mouse serum under physiological conditions (*i.e.,* in the presence of Ca$^{++}$) over a range of serum concentrations, as described in Example 6;

FIGURE 26 graphically illustrates the level of C3b deposition (OD 405 nm) on a zymosan-coated microtiter plate as a function of serum concentration in serum samples from factor D-/-, MASP-2-/- and WT mouse sera in a C3 deposition assay carried out under AP-specific conditions, as described in Example 7;

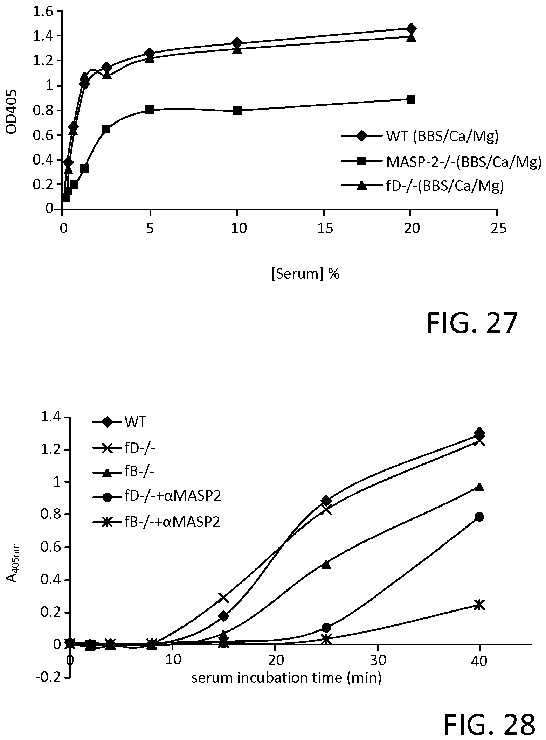

FIGURE 27 graphically illustrates the level of C3b deposition (OD 405 nm) on a zymosan-coated microtiter plate as a function of serum concentration in serum samples from factor D-/-; MASP-2-/- and WT mouse sera in a C3 deposition assay carried out under physiological conditions (in the presence of Ca$^{++}$), as described in Example 7;

FIGURE 28 graphically illustrates the level of C3b deposition (OD 405 nm) on a zymosan-coated microtiter plate as a function of serum incubation time (minutes) in mouse serum samples obtained from factor D-/-; factor B-/-; plus and minus MASP-2 monoclonal antibody in a C3b deposition assay carried out under physiological conditions (in the presence of Ca$^{++}$), as described in Example 7;

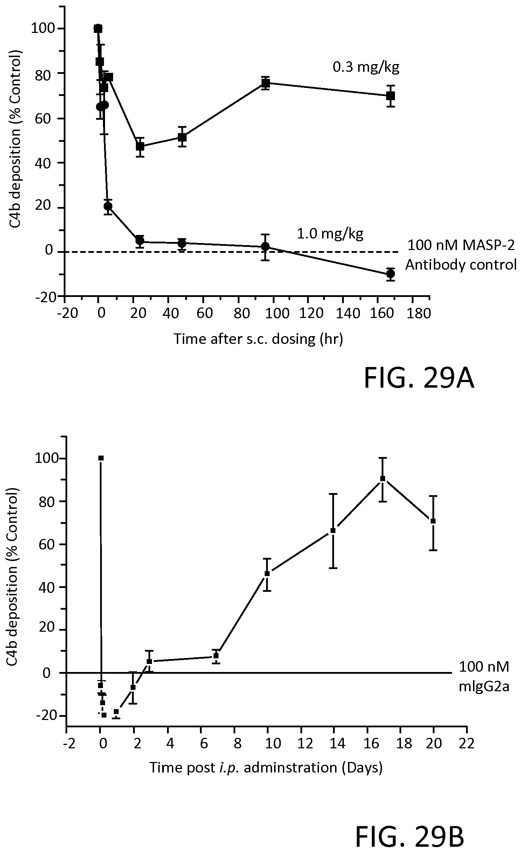

FIGURE 29A graphically illustrates lectin pathway specific C4b deposition on a zymosan-coated microtiter plate, measured *ex vivo* in undiluted serum samples taken from mice (n=3 mice/group) at various time points after subcutaneous dosing of either 0.3 mg/kg or 1.0 mg/kg of the mouse MASP-2 MoAb, as described in Example 13;

FIGURE 29B graphically illustrates the time course of lectin pathway recovery for three weeks following a single intraperitoneal administration of mouse MASP-2 MoAb at 0.6 mg/kg in mice, as described in Example 13;

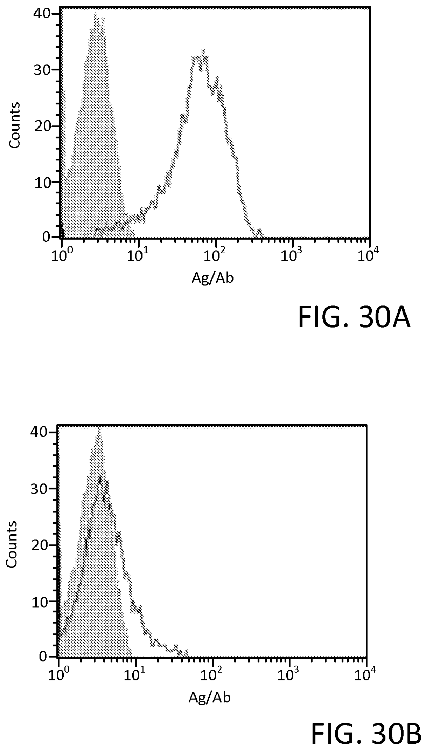

FIGURE 30A is a FACS histogram of MASP-3 antigen/antibody binding for clone M3J5, as described in Example 15;

FIGURE 30B is a FACS histogram of MASP-3 antigen/antibody binding for clone M3M1, as described in Example 15;

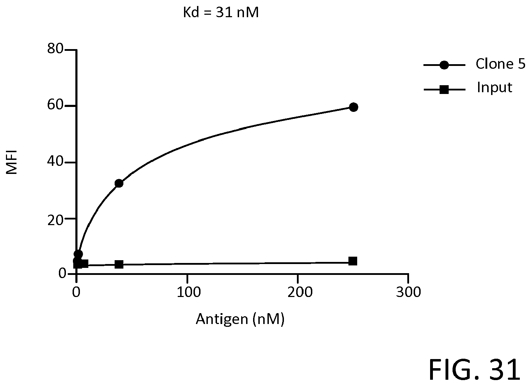

FIGURE 31 graphically illustrates a saturation binding curve of clone M3J5 (Clone 5) for the MASP-3 antigen, as described in Example 15;

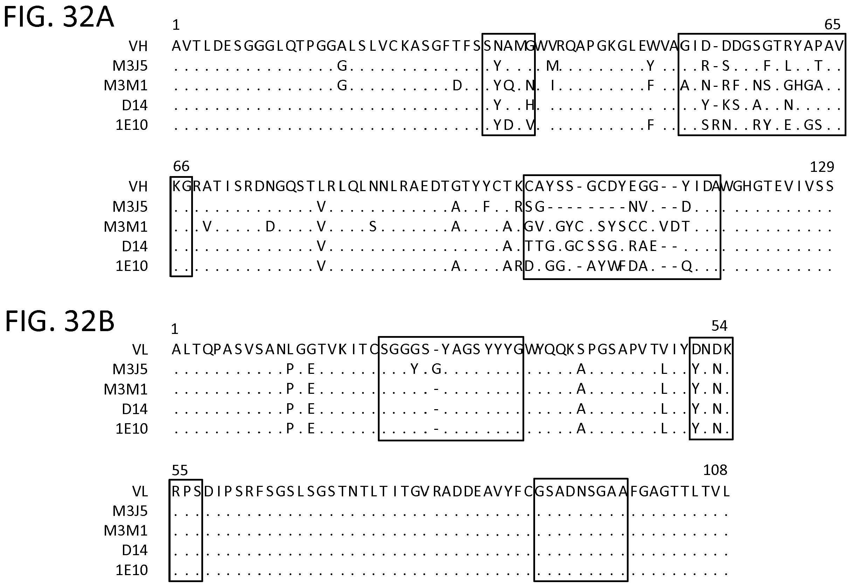

FIGURE 32A is an amino acid sequence alignment of the VH regions of M3J5, M3M1, D14 and 1E10 to the chicken DT40 VH sequence, wherein dots represent amino acid identity with the DT40 sequence and dashes indicate spaces introduced to maximize the alignment, as described in Example 15;

FIGURE 32B is an amino acid sequence alignment of the VL regions of M3J5, M3M1, D14 and 1E10 to the chicken DT40 VL sequence, wherein dots represent amino acid identity with the DT40 sequence and dashes indicate spaces introduced to maximize the alignment, as described in Example 15;

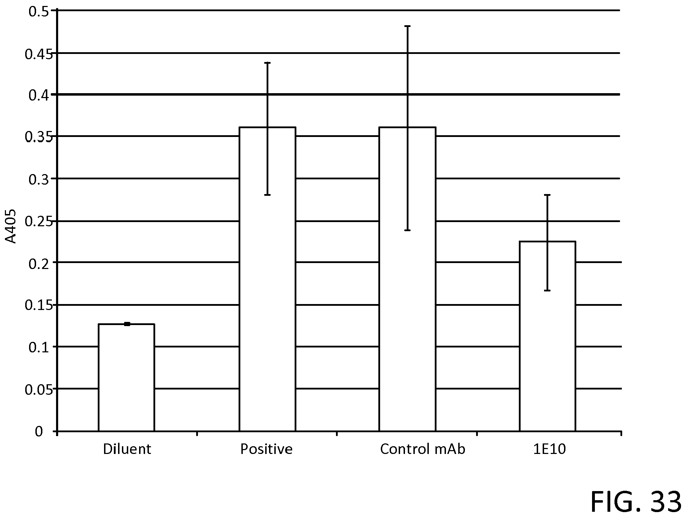

FIGURE 33 is a bar graph showing the inhibitory activity of the mAb1E10 in the Wieslab Complement System Screen, MBL Pathway in comparison to the positive serum provided with the assay kit, as well as an isotype control antibody, demonstrating that mAb1E10 partial inhibits LEA-2-dependent activation, (via inhibition of MASP-1-dependent activation of MASP-2), whereas the isotype control antibody does not, as described in Example 15;

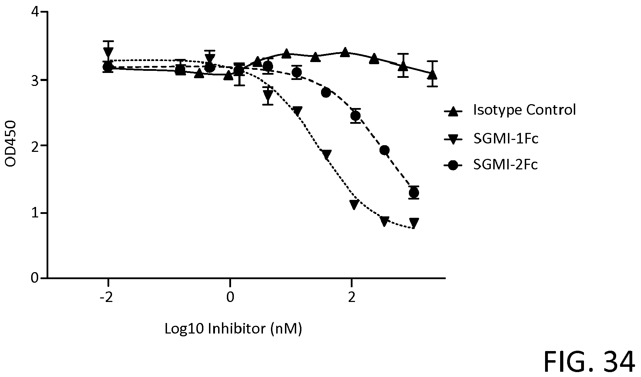

FIGURE 34 graphically illustrates the level of C3b deposition for 1% normal human serum plus isotype control, SGMI-1Fc or SGMI-2Fc over a concentration range of 0.15 to 1000 nM, demonstrating that both SGMI-1Fc and SGMI-2Fc inhibited C3b deposition from normal serum in mannan-coated ELISA wells, with $IC_{50}$ values of approximately 27nM and 300nM, respectively, as described in Example 16;

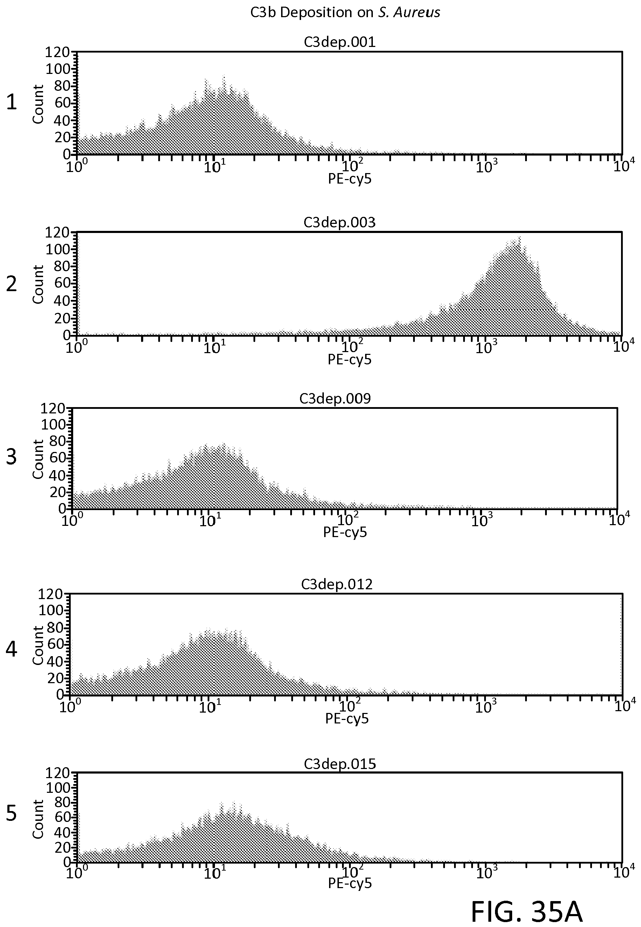

FIGURE 35A provides the results of flow cytometry analysis for C3b deposition on heat-killed *Staphylococcus aureus,* demonstrating that in normal human serum in the presence of EDTA, which is known to inactivate the lectin and alternative pathways, no C3b deposition was observed (panel 1), in normal human serum treated with $Mg^{++}$/EGTA, alternative pathway-driven C3b deposition is observed (panel 2), and as shown in panel 3, 4 and 5, in factor B-depleted, factor D-depleted and properdin (factor P)-depleted serum, respectively, no alternative pathway driven C3b deposition is observed, as described in Example 17;

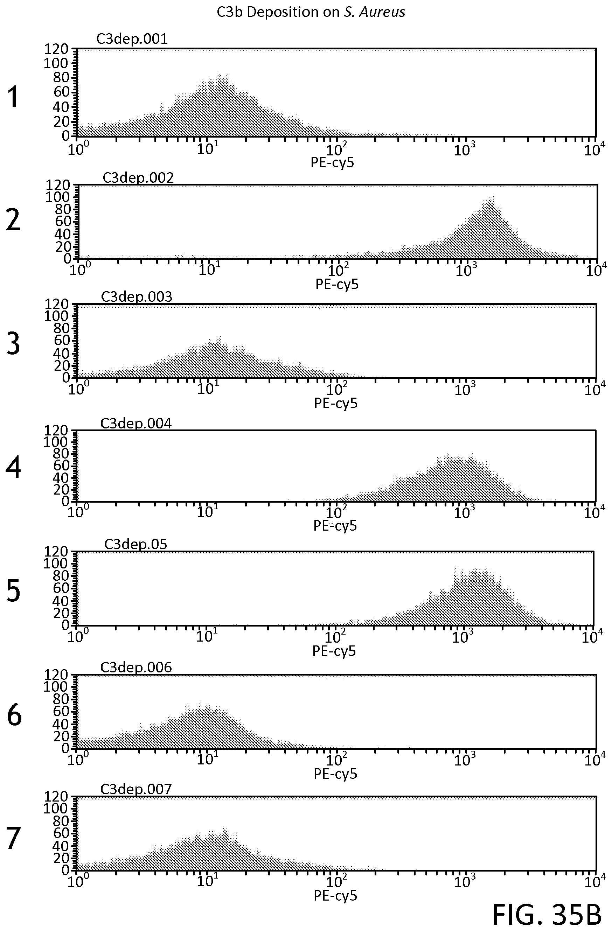

FIGURE 35B provides the results of flow cytometry analysis for C3b deposition on heat-killed *S. aureus,* demonstrating that, as in EDTA-treated normal serum (panel 1), AP-driven C3b deposition is absent in 3MC serum in the presence of $Mg^{++}$/EGTA (panel 3), whereas panels 4 and 5 show that active full length rMASP-3 (panel 4) and active rMASP-3 (CCP1-CCP2-SP) (panel 5) both restore AP-driven C3b deposition in 3MC serum to levels observed in normal serum treated with $Mg^{++}$/EGTA (panel 2), neither inactive rMASP-3 (S679A) (panel 6) nor wild type rMASP-1 (panel 7) can restore AP-driven C3b deposition in 3MC serum, as described in Example 17;

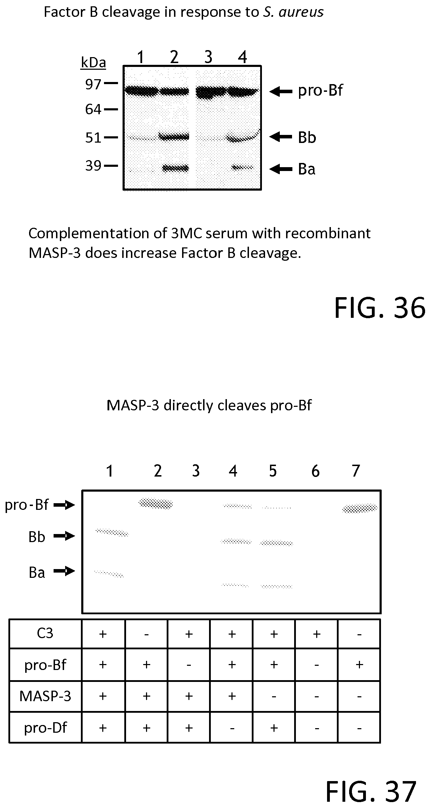

FIGURE 36 shows the results of a Western blot analysis to determine factor B cleavage in response to *S. aureus* in 3MC serum in the presence or absence of rMASP-3, demonstrating that the normal human serum in the presence of EDTA (negative control, lane 1) demonstrates very little Factor B cleavage relative to normal human serum in the presence of $Mg^{++}$/EGTA, shown in lane 2 (positive control), as further shown in lane 3, 3MC serum demonstrates very little Factor B cleavage in the presence of $Mg^{++}$/EGTA. However, as shown in lane 4, Factor B cleavage is restored by the addition and preincubation of full-length, recombinant MASP-3 protein to the 3MC serum, as described in Example 17;

FIGURE 37 shows Comassie staining of a protein gel in which Factor B cleavage is analyzed, demonstrating that Factor B cleavage is most optimal in the presence of C3, MASP-3 and pro-factor D (lane 1), and as shown in lanes 4 and 5, either MASP-3 or pro-factor D alone are able to mediate Factor B cleavage, as long as C3 is present, as described in Example 17;

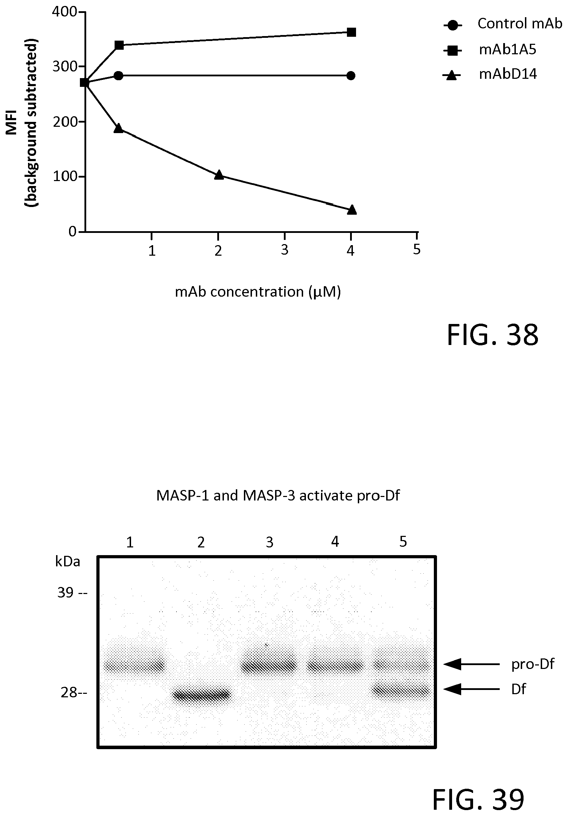

FIGURE 38 graphically illustrates the mean fluorescent intensities (MFI) of C3b staining of *S. aureus* obtained from mAbD14 (which binds MASP-3), mAb1A5 (negative control antibody) and an isotype control antibody plotted as a function of mAb concentration in 3MC serum in the presence of rMASP-3, demonstrating that mAbD14 inhibits MASP-3-dependent C3b deposition in a concentration-dependent manner, as described in Example 17;

FIGURE 39 shows Western blot analysis of pro-factor D substrate cleavage, wherein compared to pro-factor D alone (lane 1) or the inactive full length recombinant MASP-3 (S679A; lane 3) or MASP-1 (S646A; lane 4), full length wild type recombinant MASP-3 (lane2) and MASP-1 (lane 5) either completely or partially cleave pro-factor D to generate mature factor D, as described in Example 18;

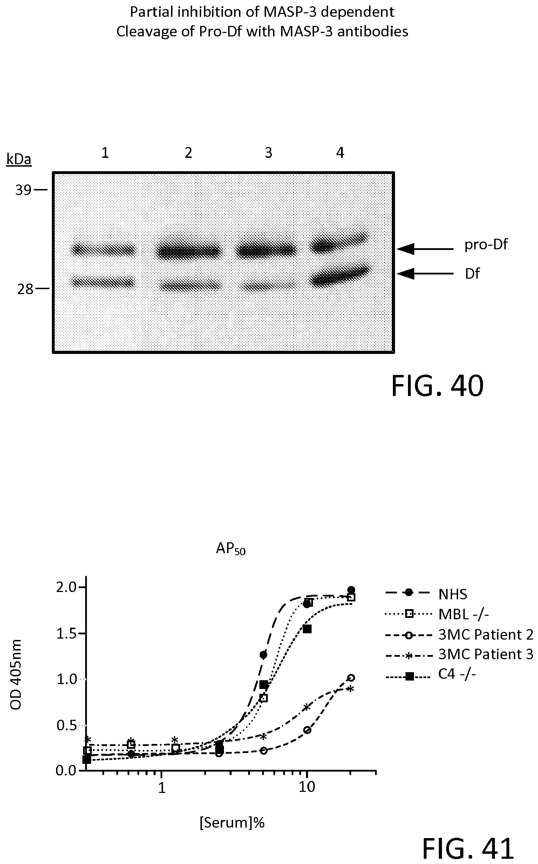

FIGURE 40 is a Western blot showing the inhibitory activity of the MASP-3 binding mAbs D14 (lane 2) and M3M1 (lane 3) on MASP-3-dependent pro-factor D cleavage in comparison to a control reaction containing only MASP-3 and pro-factor D (no mAb, lane 1), as well as a control reaction containing a mAb obtained from the DTLacO library that binds MASP-1, but not MASP-3 (lane 4), as described in Example 18;

FIGURE 41 graphically illustrates the level of AP-driven C3b deposition on zymosan-coated microtiter plates as a function of serum concentration in serum samples obtained from MASP-3-deficient (3MC), C4-deficient and MBL-deficient subjects, demonstrating that MASP-3-deficient sera from Patient 2 and Patient 3 have residual AP activity at high serum concentrations (25%, 12.5%, 6.25% serum concentrations), but a significantly higher $AP_{50}$ (i.e., 8.2% and 12.3% of serum needed to achieve 50% of maximum C3 deposition), as described in Example 19;

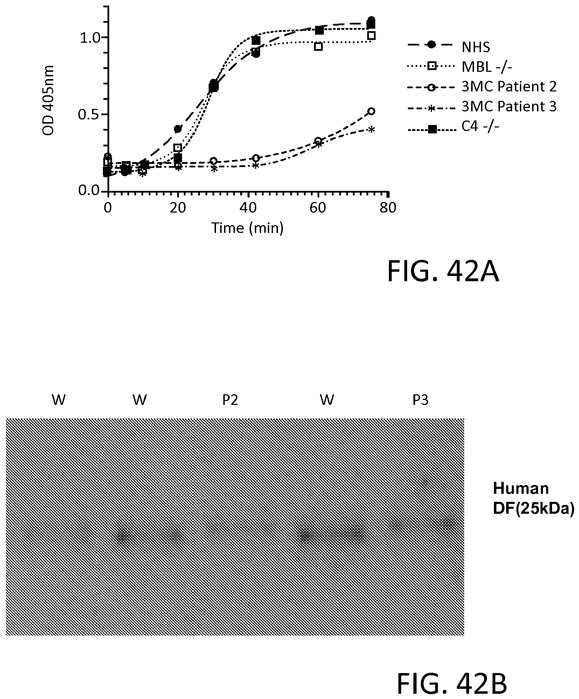

FIGURE 42A graphically illustrates the level of AP-driven C3b deposition on zymosan-coated microtiter plates under "traditional" AP-specific conditions (i.e., BBS/EGTA/$Mg^{++}$ without $Ca^{++}$) as a function of time in 10% human serum samples obtained from MASP-3 deficient, C4-deficient and MBL-deficient human subjects, as described in Example 19;

FIGURE 42B shows a western blot with plasma obtained from 3MC patient #2 (MASP-3 (-/-), MASP-1 (+/+)), 3MC patient #3 (MASP-3 (-/-), MASP-1 (-/-)), and sera from normal donors (W), wherein human pro-factor D (25,040 Da) and/or mature factor D (24,405 Da) was detected with a human factor D-specific antibody, as described in Example 19;

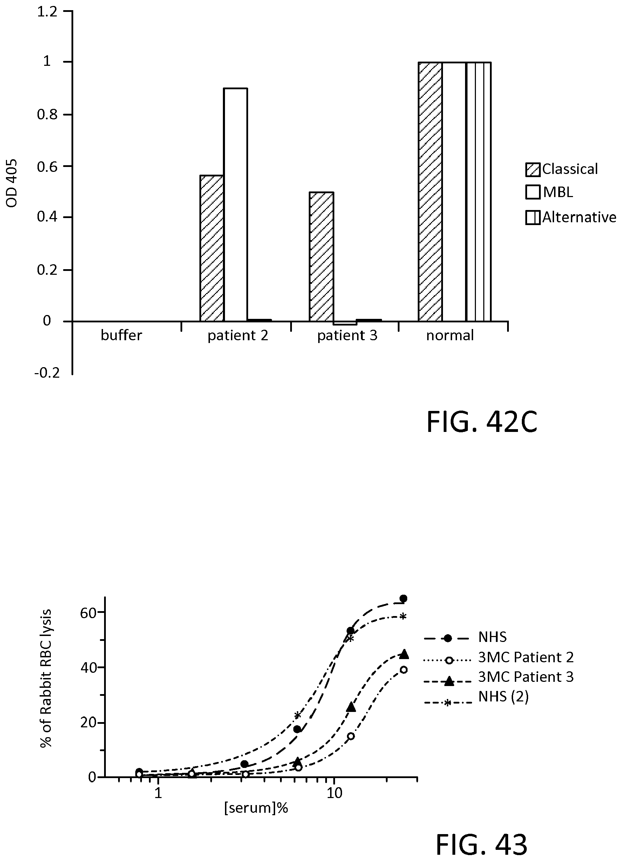

FIGURE 42C graphically illustrates the results of the Weislab classical, lectin and alternative pathway assays with plasma obtained from 3MC patient #2, 3MC patient #3, and normal human serum, as described in Example 19;

FIGURE 43 graphically illustrates the percent hemolysis (as measured by hemoglobin release of lysed rabbit erythrocytes into the supernatant measured by photometry) of mannan-coated rabbit erythrocytes over a range of serum concentrations in serum from two normal human subjects (NHS) and from two 3MC patients (Patient 2 and Patient 3), measured in the absence of $Ca^{++}$, demonstrating that MASP-3 deficiency reduces the percentage of complement-mediated lysis of mannan-coated erythrocytes as compared to normal human serum, as described in Example 19;

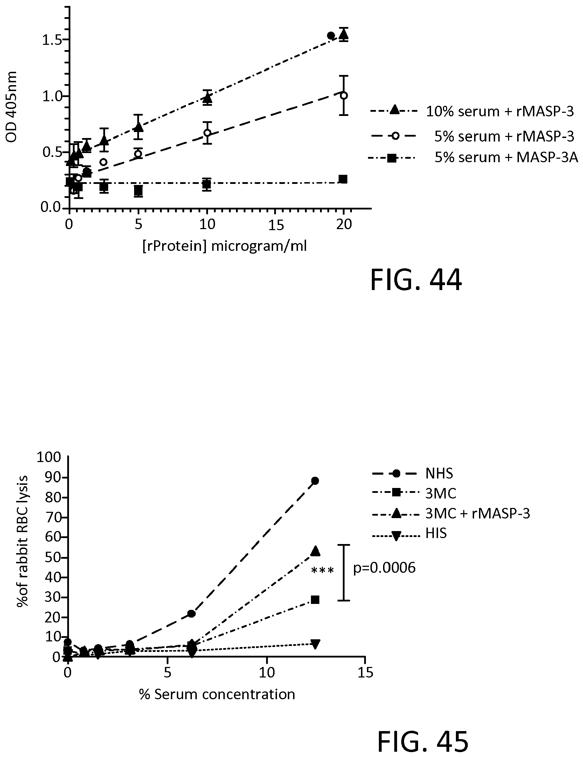

FIGURE 44 graphically illustrates the level of AP-driven C3b deposition on zymosan-coated microtiter plates as a function of the concentration of recombinant full length MASP-3 protein added to serum samples obtained from human 3MC Patient 2 (MASP-3$^{-/-}$), demonstrating that, compared to the negative control inactive recombinant MASP-3 (MASP-3A; S679A), active recombinant MASP-3 protein reconstitutes AP-driven C3b deposition on zymosan-coated plates in a concentration-dependent manner, as described in Example 19;

FIGURE 45 graphically illustrates the percent hemolysis (as measured by hemoglobin release of lysed rabbit erythrocytes into the supernatant measured by photometry) of mannan-coated rabbit erythrocytes over a range of serum concentrations in (1) normal human serum (NHS); (2) 3MC patient serum; (3) 3MC patient serum plus active full length recombinant MASP-3 (20 µg/ml); and (4) heat-inactivated human serum (HIS), measured in the absence of $Ca^{++}$, demonstrating that the percent lysis of rabbit erythrocytes is significantly increased in 3MC serum containing rMASP-3 as compared to the percent lysis in 3MC serum without recombinant MASP-3 (p=0.0006), as described in Example 19;

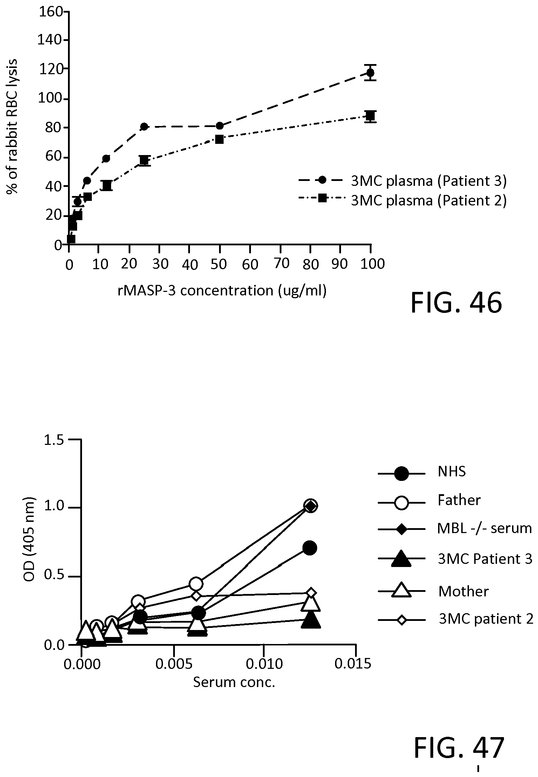

FIGURE 46 graphically illustrates the percentage of rabbit erythrocyte lysis in 7% human serum from 3MC Patient 2 and from 3MC Patient 3 containing active recombinant MASP-3 at a concentration range of 0 to 110 µg/ml (in BBS/ $Mg^{++}$/EGTA, demonstrating that the percentage of rabbit erythrocyte lysis increases with the amount of recombinant MASP-3 in a concentration-dependent manner, as described in Example 19; and

FIGURE 47 graphically illustrates the level of LEA-2-driven C3b deposition on Mannan-coated ELISA plates as a function of the concentration of human serum diluted in BBS buffer, for serum from a normal human subject (NHS), from two 3MC patients (Patient 2 and Patient 3), from the parents of Patient 3 and from a MBL-deficient subject.

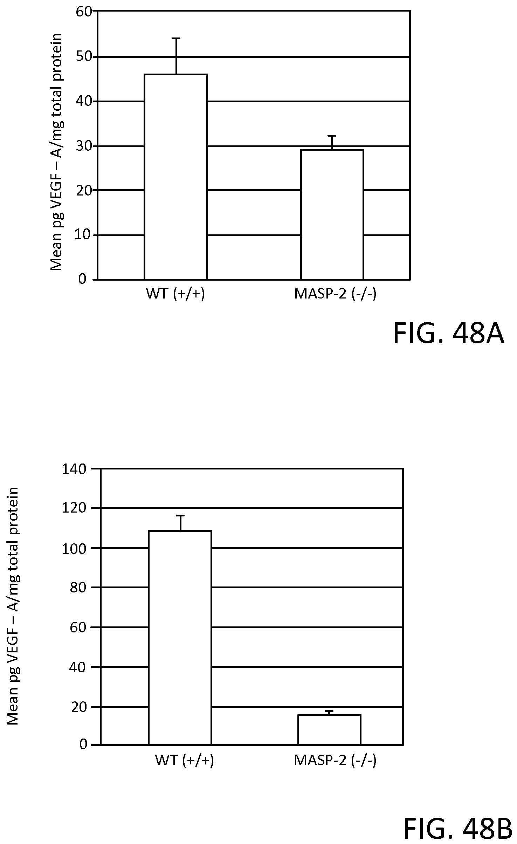

FIGURE 48A presents results showing the baseline VEGF protein levels in RPE-choroid complex isolated from wild type (WT) (+/+) and MASP-2 (-/-) mice, as described in Example 20;

FIGURE 48B presents results showing the VEGF protein levels in RPE-choroid complex at day 3 in (WT) (+/+) and MASP-2 (-/-) mice following laser-induced injury in a macular degeneration model, as described in Example 20;

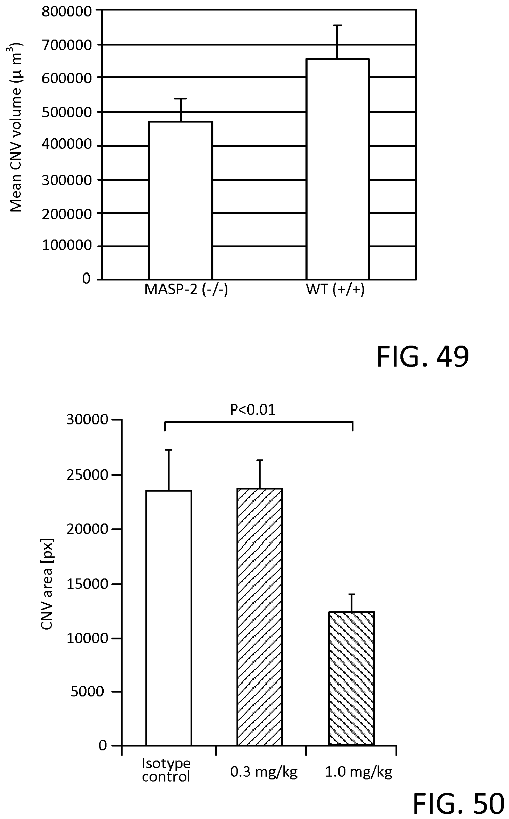

FIGURE 49 presents results showing the mean choroidal neovascularization (CNV) volume at day 7 following laser induced-injury in (WT) (+/+) and MASP-2 (-/-) mice, as described in Example 20;

FIGURE 50 graphically illustrates the mean choroidal neovascularization (CNV) area at day 7 following laser-induced injury in WT (+/+) mice pre-treated with a single ip injection of 0.3 mg/kg or 1.0 mg/kg mouse MASP-2 monoclonal antibody; as described in Example 21;

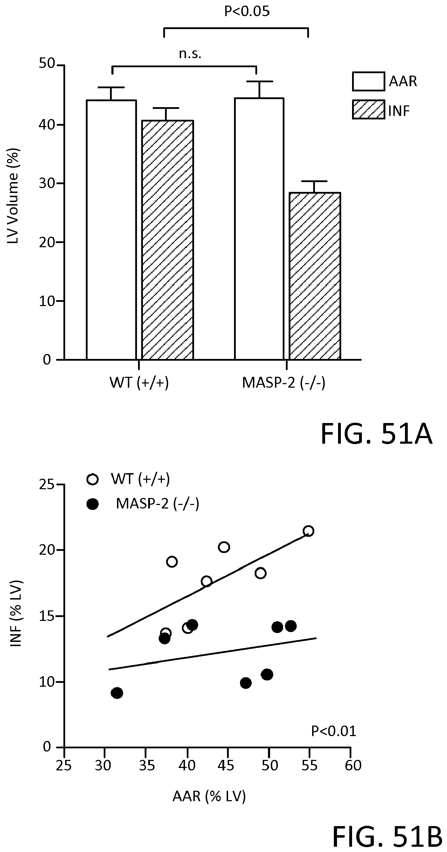

FIGURE 51A presents results demonstrating the infarct size for (WT) (+/+) and reduced infarct size in MASP-2 (-/-) mice after injury in a coronary artery occlusion and reperfusion model, as described in Example 22;

FIGURE 51B presents results showing the distribution of the individual animals tested in the coronary artery occlusion and reperfusion model, as described in Example 22;

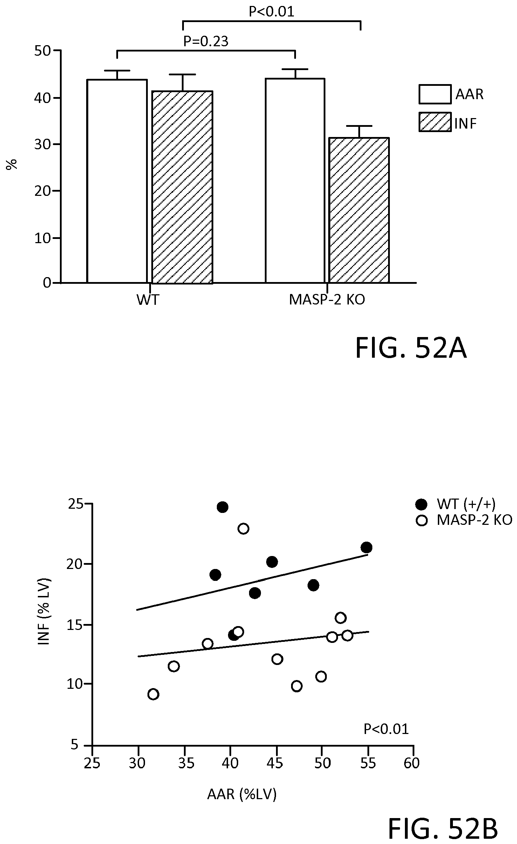

FIGURE 52A graphically illustrates the mean area-at-risk (AAR) and infarct volumes (INF) as a percentage of total myocardial volumes in WT (+/+) and MASP-2 (-/-) mice after undergoing left anterior descending coronary artery occlusion and reperfusion, as described in Example 23;

FIGURE 52B graphically illustrates the relationship between infarct volume (INF) plotted against the mean area-at-risk (AAR) as a percentage of left ventricle myocardial volume in WT (+/+) and MASP-2 (-/-) mice after undergoing artery occlusion and reperfusion, as described in Example 23;

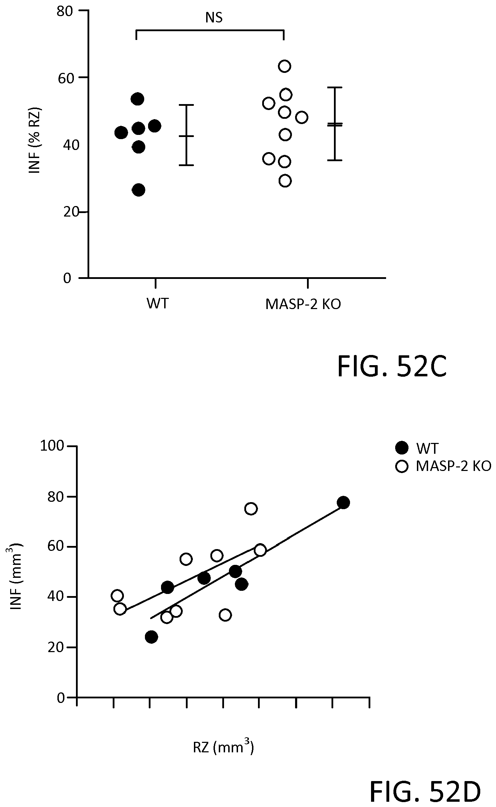

FIGURE 52C graphically illustrates the infarct volume (INF) in the buffer-perfused hearts of WT (+/+) and MASP-2 (-/-) mice prepared in accordance with the Langendorff isolated-perfused mouse heart model, in which global ischemia and reperfusion was carried out in the absence of serum, as described in Example 23;

FIGURE 52D graphically illustrates the relationship between infarct volume (INF) and risk zone (RZ) in the buffer-perfused hearts of WT (+/+) and MASP-2 (-/-) mice prepared in accordance with the Langendorff isolated-perfused mouse heart model, as described in Example 23;

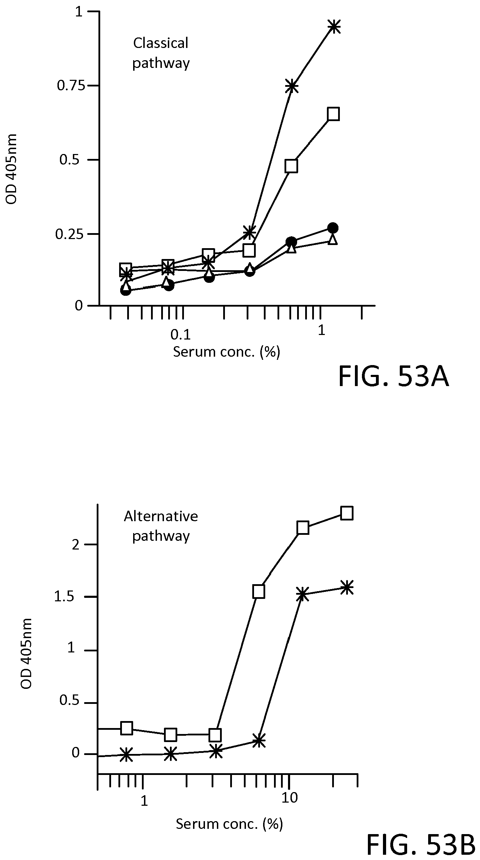

FIGURE 53A graphically illustrates the results of a C3b deposition assay on immune complex-coated plates, wherein the symbol "*" symbol indicates serum from WT (MASP-2 (+/+)); the symbol "●" indicates serum from WT (C1q depleted); the symbol "□" indicates serum from MASP-2 (-/-); and the symbol "Δ" indicates serum from MASP-2 (-/-) (C1q depleted), demonstrating that MASP-2 (-/-) mice retain a functional classical pathway, as described in Example 24;

FIGURE 53B graphically illustrates the results of a C3b deposition assay on zymosan-coated plates, wherein the symbol "*" symbol indicates serum from WT (MASP-2 (+/+)), and the symbol "□" indicates serum from MASP-2 (-/-); demonstrating that MASP-2 (-/-) mice retain a functional alternative pathway, as described in Example 24;

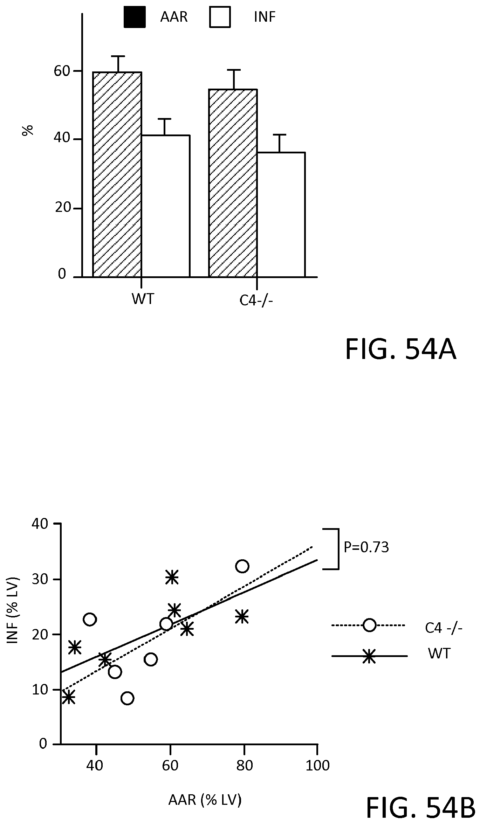

FIGURE 54A graphically illustrates myocardial ischemia/reperfusion injury (MIRI)-induced tissue loss following ligation of the left anterior descending branch of the coronary artery (LAD) and reperfusion in C4 (-/-) mice (n=6) and matching WT littermate controls (n=7), showing area at risk (AAR) and infarct size (INF) as described in Example 24;

FIGURE 54B graphically illustrates infarct size (INF) as a function of area at risk (AAR) in C4 (-/-) and WT mice treated as describe in FIGURE 42A, demonstrating that C4 (-/-) mice are as susceptible to MIRI as WT controls (dashed line), as described in Example 24;

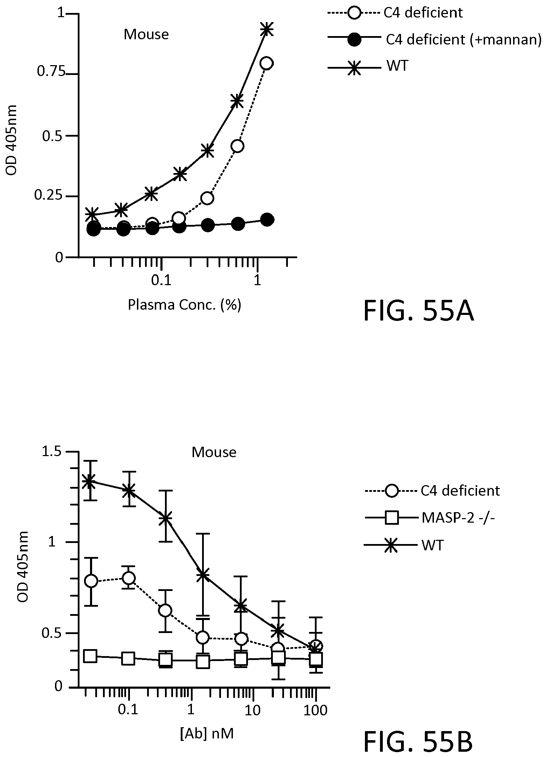

FIGURE 55A graphically illustrates the results of a C3b deposition assay using serum from WT mice, C4 (-/-) mice and serum from C4 (-/-) mice pre-incubated with mannan, as described in Example 24;

FIGURE 55B graphically illustrates the results of a C3b deposition assay using serum from WT, C4 (-/-), and MASP-2 (-/-) mice mixed with various concentrations of a murine MASP-2 mAb (mAbM11), as described in Example 24;

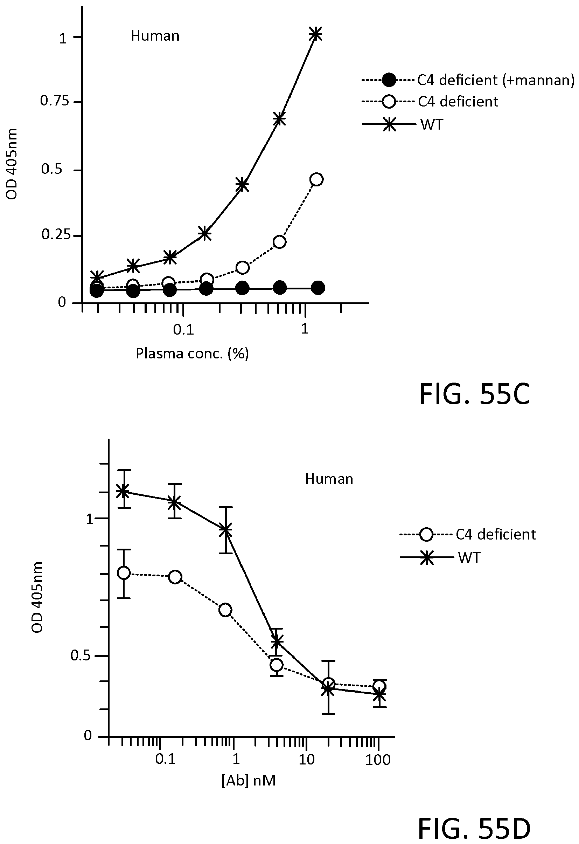

FIGURE 55C graphically illustrates the results of a C3b deposition assay using human serum from WT (C4-sufficient) and C4-deficient subjects, and serum from C4 deficient subjects pre-incubated with mannan, as described in Example 24;

FIGURE 55D graphically illustrates the results of a C3b deposition assay using human serum from WT (C4-sufficient) and C4-deficient subjects mixed with a human MASP-2 mAb (mAbH3), as described in Example 24;

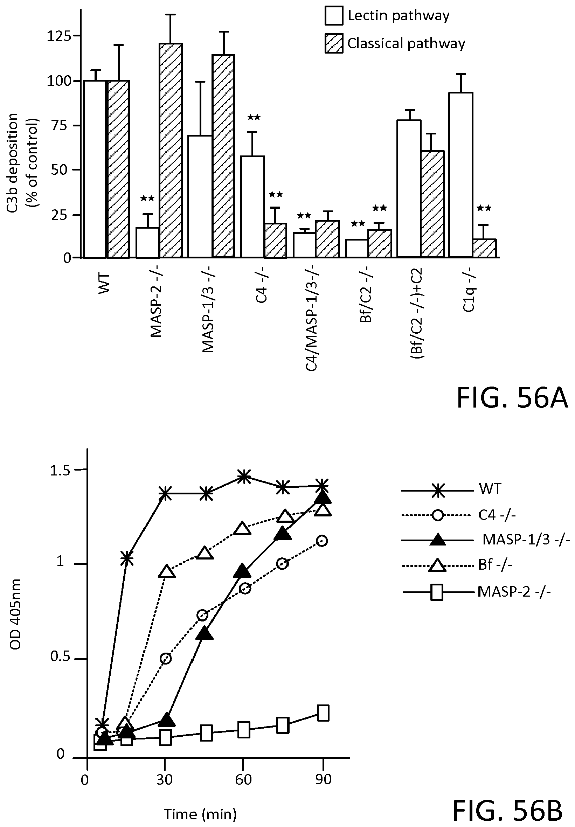

FIGURE 56A graphically illustrates a comparative analysis of C3 convertase activity in plasma from various complement-deficient mouse strains tested either under lectin activation pathway-specific assay conditions, or under classical activation pathway-specific assay conditions, as described in Example 24;

FIGURE 56B graphically illustrates the time-resolved kinetics of C3 convertase activity in plasma from various complement-deficient mouse strains tested under lectin activation pathway-specific conditions, as described in Example 24;

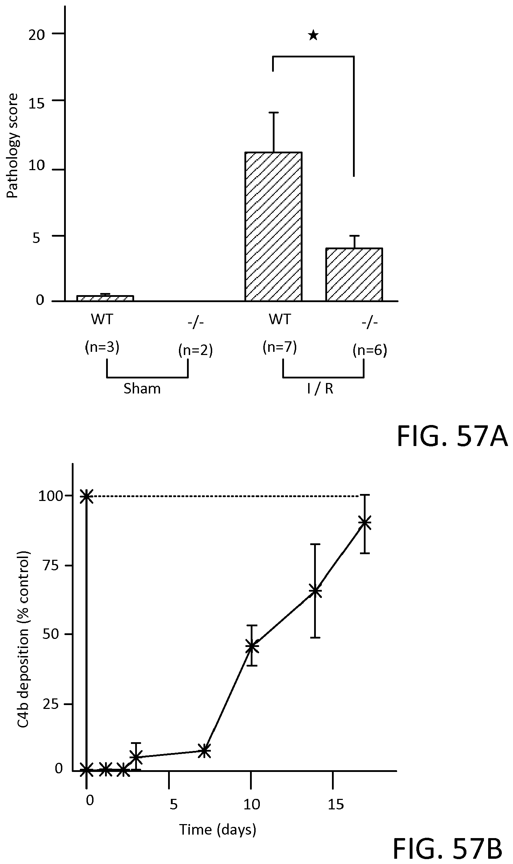

FIGURE 57A graphically illustrates the degree of tissue damage in WT and MASP-2 (-/-) mice after induction of transient ischemia/reperfusion injury in the gastrointestinal tract (GIRI), demonstrating that MASP-2 (-/-) mice have a significant degree of protection as compared to WT controls, as described in Example 25;

FIGURE 57B graphically illustrates the results of a C4b deposition assay carried out using serum obtained from mice (n=3) over time after an intraperitoneal single dose bolus injection of a recombinant murine MASP-2 antibody (mAbM11), demonstrating *in vivo* ablation of lectin pathway functional activity, as described in Example 25;

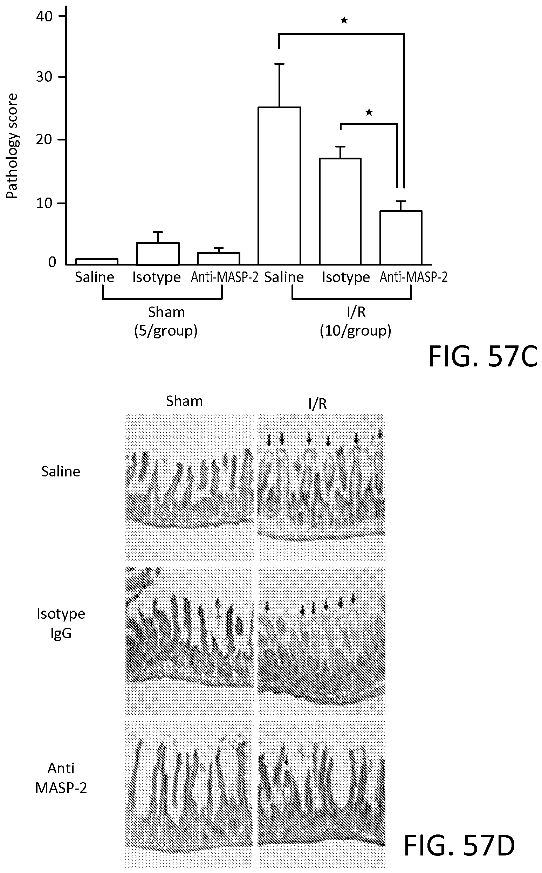

FIGURE 57C graphically illustrates the effect of a MASP-2 mAb treatment on the severity of GIRI pathology, demonstrating that mice dosed with the a murine MASP-2 mAb (mAbM11) 24 hours before being subjected to transient ischemia/reperfusion injury in the gastrointestinal tract (GIRI) had significantly reduced tissue damage as compared to mice dosed with saline (*$p < 0.05$ when comparing animals treated with either the MASP-2 inhibitory antibody mAbM11 or an irrelevant isotype control antibody), as described in Example 25;

FIGURE 57D shows histological presentation of GIRI-mediated pathology of the small intestine in mice pre-treated with a single dose intraperitoneal injection of saline, an isotope control antibody, or a recombinant murine MASP-2 antibody (mAbM11) 12 hours prior to induction of GIRI, as described in Example 25;

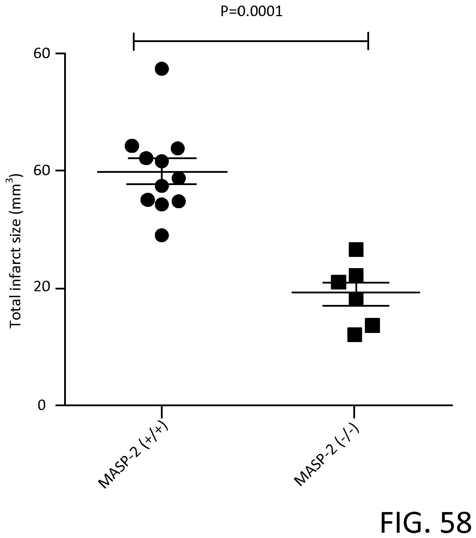

FIGURE 58 graphically illustrates the cerebral infarct volume in WT (MASP-2 (+/+)) and MASP-2 (-/-) mice following 30 minutes ischemia and 24 hours reperfusion, as described in Example 26;

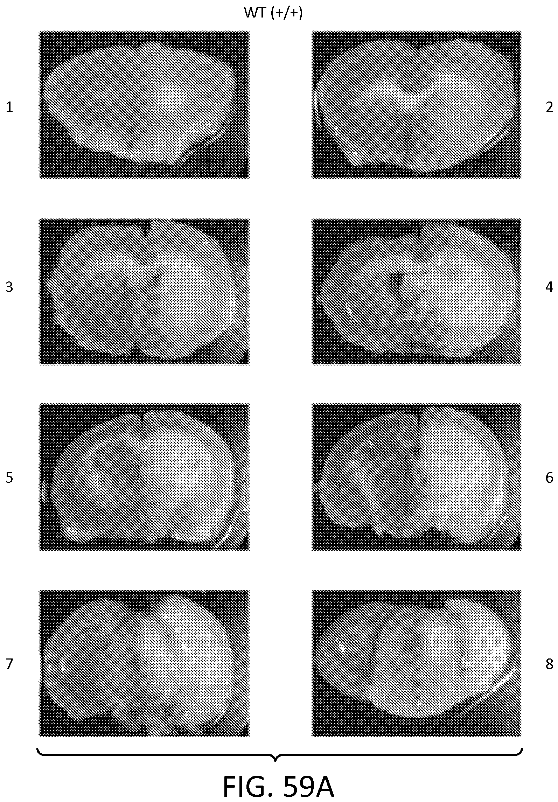

FIGURE 59A shows a series of photographs of stained brain sections from a WT (MASP-2 +/+) mouse after 30 minutes ischemia and 24 hours reperfusion. Panels 1-8 of FIGURE 52A show the different section areas of the brain corresponding to Bregma 1-8, respectively, in relation to the exit of the acoustic nerve (Bregma 0), as described in Example 26;

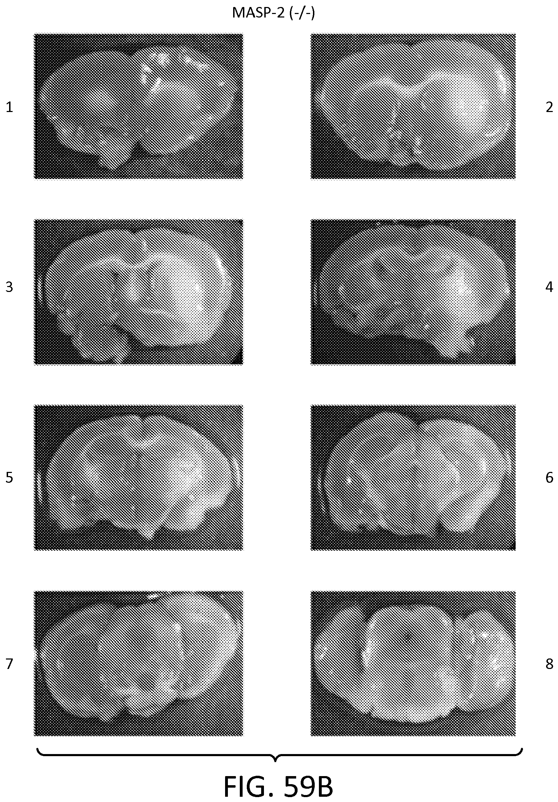

FIGURE 59B shows a series of photographs of stained brain sections from a MASP-2 (-/-) mouse after 30 minutes ischemia and 24 hours reperfusion. Panels 1-8 of FIGURE 52B show the different sections areas of the brain corresponding to Bregma 1-8, respectively, in relation to the exit of the acoustic nerve (Bregma 0), as described in Example 26;

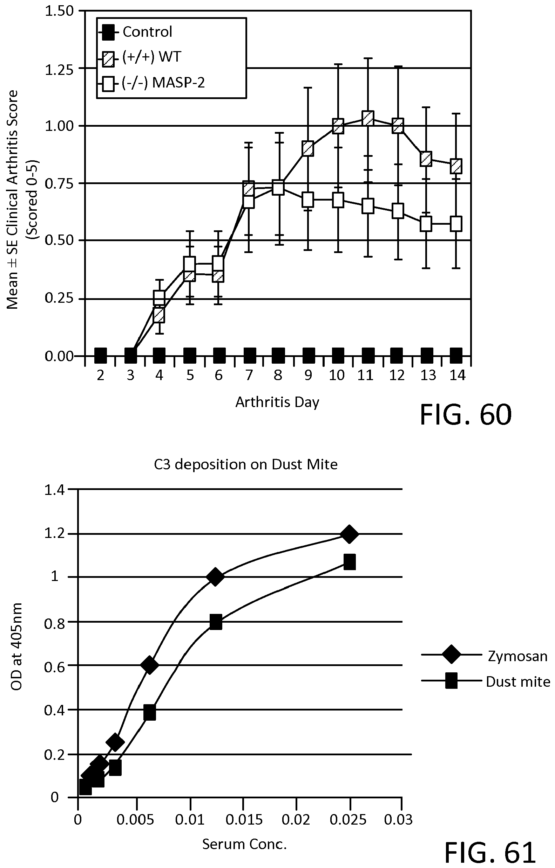

FIGURE 60 presents results showing the mean clinical arthritis score of (WT) (+/+) and MASP-2 (-/-) mice over time following Col2 mAb- induced rheumatoid arthritis, as described in Example 27;

FIGURE 61 graphically illustrates the results of the C3 deposition assay on serum samples obtained from WT mice in the presence of house dust mite or zymosan, as described in Example 28;

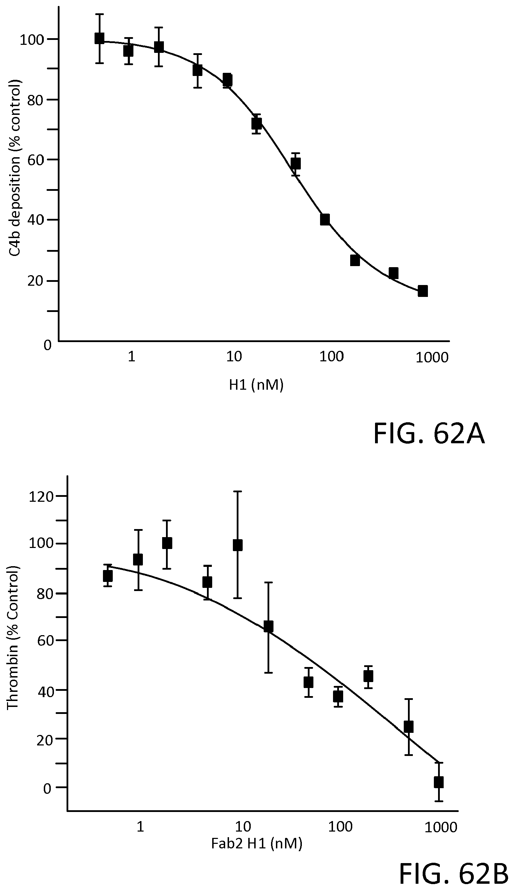

FIGURES 62A and 62B present dose response curves for the inhibition of C4b deposition (FIG. 62A) and the inhibition of thrombin activation following the administration of a MASP-2 Fab2 antibody (HI) in normal rat serum, as described in Example 29;

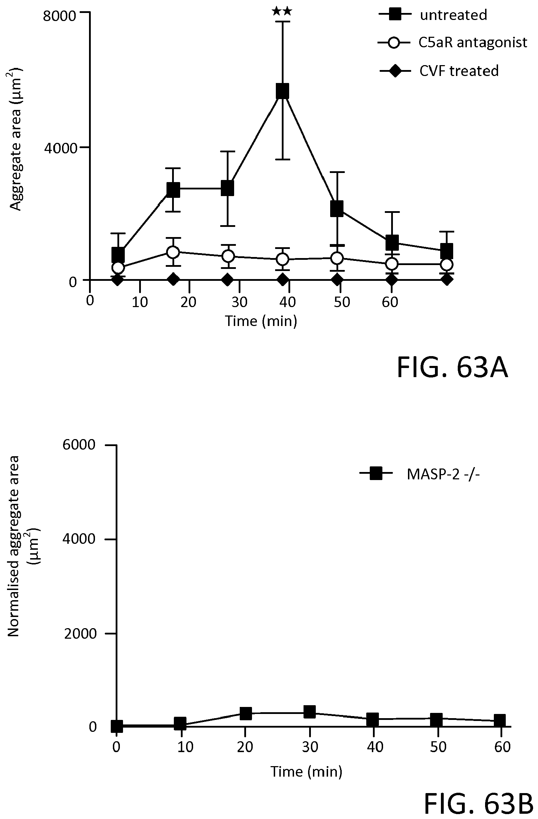

FIGURES 63A and 63B present measured platelet aggregation (expressed as aggregate area) in MASP-2 (-/-) mice (FIG. 63B) as compared to platelet aggregation in untreated wild-type mice and wild-type mice in which the complement pathway is inhibited by depletory agent cobra venom factor (CVF) and a terminal pathway inhibitor (C5aR antagonist) (FIGURE 63A) in a localized Schwartzman reaction model of disseminated intravascular coagulation, as described in Example 30;

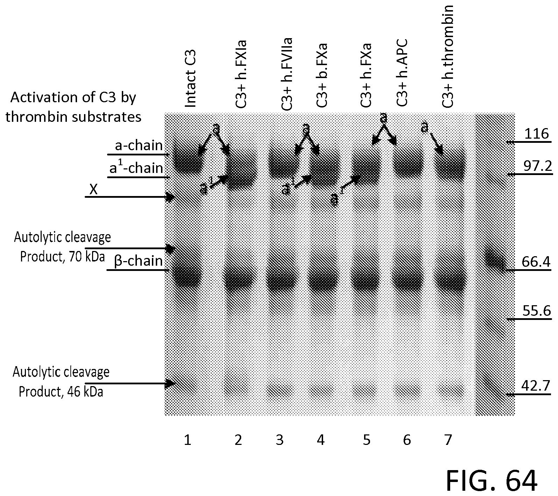

FIGURE 64 illustrates the results of a Western blot analysis showing activation of human C3, shown by the presence of the a' chain, by thrombin substrates FXIa and FXa, as described in Example 31;

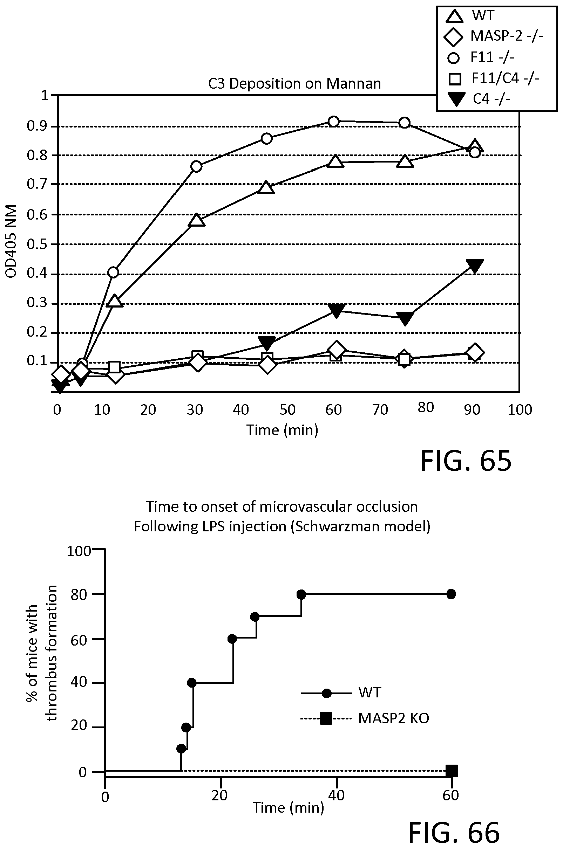

FIGURE 65 graphically illustrates the results of a C3b deposition assay on serum samples obtained from WT, MASP-2 (-/-), F11(-/-), F11(-/-)/C4 (-/-) and C4 (-/-) mice, demonstrating that there is a functional lectin pathway even in the complete absence of C4, or F11, while mice with combined F11-(-/-)/C4 (-/-)-deficiency lack a functional lectin pathway, as described in Example 31;

FIGURE 66 graphically illustrates the time to onset of microvascular occlusion following LPS injection in MASP-2 -/- and WT mice, showing the percentage of mice with thrombus formation measured over 60 minutes, demonstrating that thrombus formation is detected after 15 minutes in WT mice, with up to 80% of the WT mice demonstrating thrombus formation at 60 minutes; in contrast, none of the MASP-2 -/- mice showed any thrombus formation during the 60-minute period (log rank: p=0.0005), as described in Example 32;

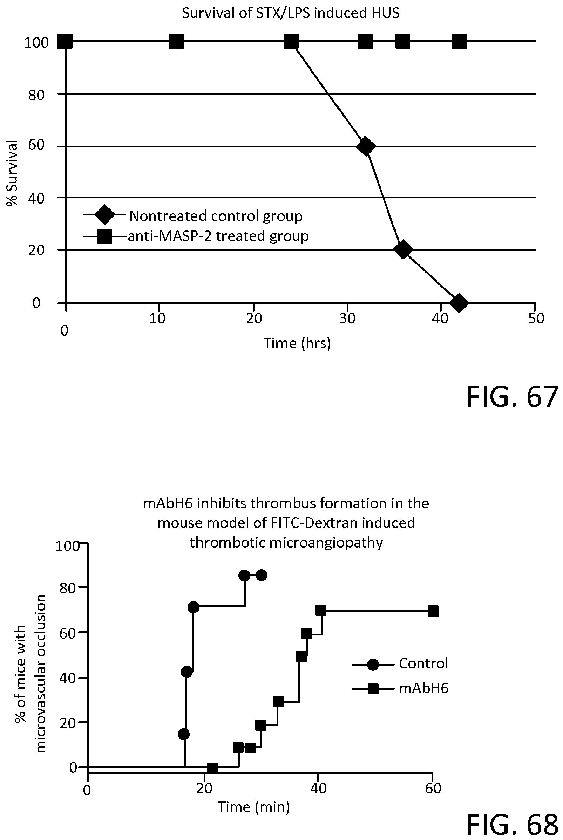

FIGURE 67 graphically illustrates the percent survival of saline-treated control mice (n=5) and MASP-2 antibody-treated mice (n=5) in the STX/LPS-induced model of HUS over time (hours), demonstrating that all of the control mice died by 42 hours, whereas, in contrast, 100 % of the MASP-2 antibody-treated mice survived throughout the time course of the experiment, as described in Example 33;

FIGURE 68 graphically illustrates, as a function of time after injury induction, the percentage of mice with microvascular occlusion in the FITC/Dextran UV model after treatment with isotype control, or human MASP-2 antibody mAbH6 (10mg/kg) dosed at 16 hours and 1 hour prior to injection of FITC/Dextran, as described in Example 34;

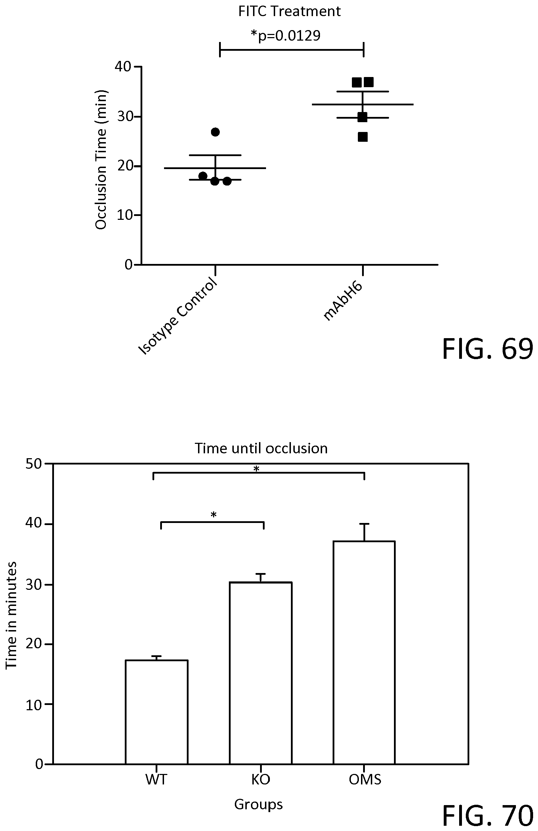

FIGURE 69 graphically illustrates the occlusion time in minutes for mice treated with the human MASP-2 antibody (mAbH6) and the isotype control antibody, wherein the data are reported as scatter-dots with mean values (horizontal bars) and standard error bars (vertical bars). The statistical test used for analysis was the unpaired t test; wherein the symbol "*" indicates p=0.0129, as described in Example 34; and

FIGURE 70 graphically illustrates the time until occlusion in minutes for wild-type mice, MASP-2 KO mice, and wild-type mice pre-treated with human MASP-2 antibody (mAbH6) administered i.p. at 10mg/kg 16 hours before, and again 1 hour prior to the induction of thrombosis in the FITC-dextran/light induced endothelial cell injury model of thrombosis with low light intensity (800-1500), as described in Example 34.

DESCRIPTION OF SEQUENCE LISTING

SEQ ID NO:1 human MAp19 cDNA
SEQ ID NO:2 human MAp19 protein (with leader)
SEQ ID NO:3 human MAp19 protein (mature)
SEQ ID NO:4 human MASP-2 cDNA
SEQ ID NO:5 human MASP-2 protein (with leader)
SEQ ID NO:6 human MASP-2 protein (mature)
SEQ ID NO:7 human MASP-3 cDNA
SEQ ID NO:8 human MASP-3 protein (w/leader)
SEQ ID NO:9 human MASP-1 cDNA
SEQ ID NO:10 human MASP-1 protein (w/leader)
SEQ ID NO:11 human MAp44 protein (w/leader)
SEQ ID NO:12 rat MASP-2 cDNA
SEQ ID NO:13 rat MASP-2 protein (with leader)
SEQ ID NO:14 DNA encoding 17D20_dc35VH21N11VL (OMS646) heavy chain variable region (VH) (without signal peptide)
SEQ ID NO:15 17D20_dc35VH21N11VL (OMS646) heavy chain variable region (VH) polypeptide
SEQ ID NO:16 17N16mc heavy chain variable region (VH) polypeptide
SEQ ID NO:17 17D20_dc21N11VL (OMS644) light chain variable region (VL) polypeptide
SEQ ID NO:18 DNA encoding 17N16_dc17N9 (OMS641) light chain variable region (VL) (without signal peptide)
SEQ ID NO:19 17N16_dc17N9 (OMS641) light chain variable region (VL) polypeptide
SEQ ID NO:20: scFv daughter clone 17N16m_d17N9 full length polypeptide
SEQ ID NO:21: scFv daughter clone 17D20m_d3521N11 full length polypeptide
SEQ ID NO:22: scFv daughter clone 17N16m_d17N9 DNA encoding full length polypeptide (without signal peptide)
SEQ ID NO:23: scFv daughter clone 17D20m_d3521N11 DNA encoding full length polypeptide (without signal peptide)
SEQ ID NO:24: parent DTLacO heavy chain variable region (VH) polypeptideSEQ ID NO:25: MASP-3 specific clone M3J5 heavy chain variable region (VH) polypeptide
SEQ ID NO:26: MASP-3 specific clone M3M1 heavy chain variable region (VH) polypeptide
SEQ ID NO:27: parent DTLacO light chain variable region (VL) polypeptide
SEQ ID NO:28: MASP-3 specific clone M3J5 light chain variable region (VL) polypeptide
SEQ ID NO:29: MASP-3 specific clone M3M1 light chain variable region (VL) polypeptide

SEQ ID NO:30: MASP-3 clone D14 heavy chain variable region (VH) polypeptide
SEQ ID NO:31: MASP-3 clone D14 light chain variable region (VL) polypeptide
SEQ ID NO:32: MASP-1 clone 1E10 heavy chain variable region (VH) polypeptide
SEQ ID NO:33: MASP-1 clone 1E10 light chain variable region (VL) polypeptide
SEQ ID NO:34 SGMI-1 peptide
SEQ ID NO:35 SGMI-2 peptide
SEQ ID NO:36 human IgG1-Fc polypeptide;
SEQ ID NO:37 peptide linker #1 (12aa);
SEQ ID NO:38: peptide linker #2 (10aa);
SEQ ID NO:39: nucleic acid encoding polypeptide fusion comprising the human IL-2-signal sequence, SGMI-1, linker#1, and human IgG1-Fc;
SEQ ID NO:40: mature polypeptide fusion comprising SGMI-1, linker#1 and human IgG1-Fc (SGMI-1Fc);
SEQ ID NO:41: nucleic acid encoding polypeptide fusion comprising the human IL-2-signal sequence, SGMI-2, linker#1 and human IgG1-Fc;
SEQ ID NO:42: mature polypeptide fusion comprising SGMI-2, linker#1 and human IgG1-Fc (SGMI-2Fc).

DETAILED DESCRIPTION

**I. DEFINITIONS**

Unless specifically defined herein, all terms used herein have the same meaning as would be understood by those of ordinary skill in the art of the present invention. The following definitions are provided in order to provide clarity with respect to the terms as they are used in the specification and claims to describe the present invention.

As used herein, the lectin pathway effector arm 1 ("LEA-1") refers to lectin-dependent activation of factor B and factor D by MASP-3.

As used herein, the lectin pathway effector arm 2 ("LEA-2") refers to MASP-2-dependent complement activation.

As used herein, the term "MASP-3-dependent complement activation" comprises two components: (i) lectin MASP-3-dependent activation of factor B and factor D, encompassed in LEA-1-mediated complement activation, occurs in the presence of $Ca^{++}$, commonly leading to the conversion of C3bB to C3bBb and of pro-factor D to factor D; and (ii) lectin-independent conversion of factor B and factor D, which can occur in the absence of $Ca^{++}$, commonly leading to the conversion of C3bB to C3bBb and of pro-factor D to factor D. LEA-1-mediated complement activation and lectin-independent conversion of factor B and factor D have been determined to cause opsonization and/or lysis. While not wishing to be bound by any particular theory, it is believed that only when multiple C3b molecules associate and bind in close proximity, the C3bBb C3 convertase changes its substrate specificity and cleaves C5 as the alternative pathway C5 convertase termed C3bBb(C3b)n.

As used herein, the term "MASP-2-dependent complement activation", also referred to herein as LEA-2-mediated complement activation, comprises MASP-2 lectin-dependent activation, which occurs in the presence of $Ca^{++}$, leading to the formation of the lectin pathway C3 convertase C4b2a and upon accumulation of the C3 cleavage product C3b subsequently to the C5 convertase C4b2a(C3b)n, which has been determined to cause opsonization and/or lysis.

As used herein, the term "traditional understanding of the alternative pathway" also referred to as the "traditional alternative pathway" refers to the alternative pathway prior to the instant discovery described herein, *i.e.,* complement activation that is triggered, for example, by zymosan from fungal and yeast cell walls, lipopolysaccharide (LPS) from Gram negative outer membranes, and rabbit erythrocytes, as well as from many pure polysaccharides, viruses, bacteria, animal tumor cells, parasites and damaged cells, and which has traditionally been thought to arise from spontaneous proteolytic generation of C3b from complement factor C3. As used herein, activation of the "traditional alternative pathway", also referred to herein as the "alternative pathway", is measured in $Mg^{++}$/EGTA buffer *(i.e.,* in the absence of $Ca^{++}$).

As used herein, the term "lectin pathway" refers to complement activation that occurs via the specific binding of serum and non-serum carbohydrate-binding proteins including mannan-binding lectin (MBL), CL-11 and the ficolins (H-ficolin, M-ficolin, or L-ficolin). As described herein, the inventors have discovered that the lectin pathway is driven by the two effector arms, lectin pathway effector arm 1 (LEA-1), which is now known to be MASP-3-dependent, and lectin pathway effector arm 2 (LEA-2), which is MASP-2-dependent. As used herein, activation of the lectin pathways are assessed using $Ca^{++}$ containing buffers.

As used herein, the term "classical pathway" refers to complement activation that is triggered by antibody bound to a foreign particle and requires binding of the recognition molecule C1q.

As used herein, the term "HTRA-1" refers to the serine peptidase High-temperature requirement serine protease A1.

As used herein, the term "MASP-3 inhibitory agent" refers to any agent that directly or indirectly inhibits MASP-3-dependent complement activation, including agents that bind to or directly interact with MASP-3, including MASP-3 antibodies and MASP-3 binding fragments thereof, natural and synthetic peptides, competitive substrates, small-molecules, expression inhibitors and isolated natural inhibitors, and also encompasses peptides that compete with MASP-3 for binding to another recognition molecule (e.g., MBL, CL-11, H-ficolin, M-ficolin, or L-ficolin) in the lectin pathway. In one embodiment, the MASP-3 inhibitory agent is specific to MASP-3, and does not bind to MASP-1 or MASP-2. An inhibitory agent that directly inhibits MASP-3 can be referred to as a direct MASP-3 inhibitory agent (e.g., a MASP-3 antibody), while an inhibitory agent that indirectly inhibits MASP-3 can be referred to as an indirect MASP-3 inhibitory agent (e.g., a MASP-1 antibody that inhibits MASP-3 activation). An example of a direct MASP-3 inhibitory agent is a MASP-3 specific inhibitory agent, such as a MASP-3 inhibitory agent that specifically binds to a portion of MASP-3 (SEQ ID NO:8) with a binding affinity of at least 10 times greater than to other components in the complement system. In one embodiment, a MASP-3 inhibitory agent indirectly inhibits MASP-3 activity, such as, for example, an inhibitor of MASP-3 activation, including an inhibitor of MASP-1-mediated MASP-3 activation (e.g., a MASP-1 antibody or MASP-1 binding fragments thereof, natural and synthetic peptides, small-molecules, expression inhibitors and isolated natural inhibitors, and also encompasses peptides that compete with MASP-1 for binding to MASP-3). In another embodiment, a MASP-3 inhibitory agent inhibits MASP-3-mediated maturation of factor D. In another embodiment, a MASP-3 inhibitory agent inhibits MASP-3-mediated activation of factor B. MASP-3 inhibitory agents useful in the method of the invention may reduce MASP-3-dependent complement activation by greater than 10%, such as greater than 20%, greater than 50%, or greater than 90%. In one embodiment, the MASP-3 inhibitory agent reduces MASP-3-dependent complement activation by greater than 90% (i.e., resulting in MASP-3 complement activation of only 10% or less). It is expected that MASP-3 inhibition will block, in full or in part, both LEA-1-related lysis and opsonization and lectin-independent conversion of factor B and factor D-related lysis and opsonization.

As used herein, the term "MASP-1 inhibitory agent" refers to any agent that binds to or directly interacts with MASP-1 and inhibits at least one of (i) MASP-3-dependent complement activation and/or (ii) MASP-2-dependent complement activation and/or (iii) lectin-independent or lectin-dependent MASP-1-mediated maturation of factor D, wherein the lectin-dependent MASP-1 maturation of factor D involves direct activation of factor D, including MASP-1 antibodies and MASP-1 binding fragments thereof, natural and synthetic peptides, small-molecules, expression inhibitors and isolated natural inhibitors, and also encompasses peptides that compete with MASP-1 for binding to another recognition molecule (e.g., MBL, CL-11, H-ficolin, M-ficolin, or L-ficolin) in the lectin pathway. In one embodiment, MASP-1 inhibitory agents useful in the method of the invention reduce MASP-3-dependent complement activation by greater than 10%, such as greater than 20%, greater than 50%, or greater than 90%. In one embodiment, the MASP-1 inhibitory agent reduces MASP-3-dependent complement activation by greater than 90% (i.e., resulting in MASP-3 complement activation of only 10% or less). In another embodiment, MASP-1 inhibitory agents useful in the method of the invention reduce MASP-2-dependent complement activation by greater than 10%, such as greater than 20%, greater than 50%, or greater than 90%. In one embodiment, the MASP-1 inhibitory agent reduces MASP-2-dependent complement activation by greater than 90% (i.e., resulting in MASP-2 complement activation of only 10% or less).

In another embodiment, MASP-1 inhibitory agents useful in the method of the invention reduce both MASP-3-dependent complement activation (LEA-1), lectin-independent conversion of factor B and factor D, and MASP-2-dependent complement activation (LEA-2) by greater than 10%, such as greater than 20%, greater than 50%, or greater than 90%. In one embodiment, the MASP-1 inhibitory agent reduces MASP-3-dependent complement activation (LEA-1), lectin-independent conversion of factor B and factor D, and MASP-2-dependent complement activation (LEA-2) by greater than 90% (i.e., resulting in MASP-3 complement activation of only 10% or less and MASP-2 complement activation of only 10% or less).

An example of a direct MASP-1 inhibitory agent is a MASP-1-specific inhibitory agent, such as a MASP-1 inhibitory agent that specifically binds to a portion of MASP-1 (SEQ ID NO:10) with a binding affinity of at least 10 times greater than to other components in the complement system. In many instances, given that MASP-1 can activate MASP-3, and given the MASP-1 can activate MASP-2, inhibition of MASP-1 would be expected to be effective in inhibiting MASP-3 and/or MASP-2. In some instances, however, inhibition of either MASP-1 or MASP-3 or MASP-2 may be a preferred embodiment relative to inhibition of the other MASP targets. For example, in the setting of *Staphylococcus aureus (S. aureus)* infection, MASP-3 has been shown to be activated and is responsible for *S. aureus* opsonization in the absence of MASP-1 (see Iwaki D. et al., J Immunol 187(7):3751-8 (2011)). Therefore, in the treatment of paroxysmal nocturnal hemoglobinuria (PNH), for example, it might be advantageous to directly inhibit MASP-1 rather than MASP-3, thereby reducing the potential susceptibility to *S. aureus* during LEA-1-inhibitory treatment of PNH

As used herein, the term "MASP-2 inhibitory agent" refers to any agent that binds to or directly interacts with MASP-2 and inhibits at least one of (i) MASP-2-dependent complement activation and/or (ii) MASP-1-dependent complement activation, including MASP-2 antibodies and MASP-2 binding fragments thereof, natural and synthetic peptides, small-molecules, expression inhibitors and isolated natural inhibitors, and also encompasses peptides that compete with MASP-2 for binding to another recognition molecule (e.g., MBL, CL-11, H-ficolin, M-ficolin, or L-ficolin) in the lectin pathway. MASP-2 inhibitory agents useful in the method of the invention may reduce MASP-2-dependent complement activation by greater than 10%, such as greater than 20%, greater than 50%, or greater than 90%. In one embodiment, the MASP-2 inhibitory agent reduces MASP-2-dependent complement activation by greater than 90% (i.e., resulting in MASP-2 complement activation of only 10% or less). An example of a direct MASP-2 inhibitory agent is a MASP-2-specific inhibitory agent, such as a MASP-2 inhibitory agent that specifically binds to a portion of MASP-2 (SEQ ID NO:5) with a binding affinity of at least 10 times greater than to other components in the complement system.

As used herein, the term "antibody" encompasses antibodies and antibody fragments thereof, derived from any antibody-producing mammal (e.g., mouse, rat, rabbit, and primate including human), or from a hybridoma, phage selection, recombinant expression or transgenic animals (or other methods of producing antibodies or antibody fragments"), that specifically bind to a target polypeptide, such as, for example, MASP-1, MASP-2 or MASP-3 polypeptides or portions thereof. It is not intended that the term "antibody" limited as regards to the source of the antibody or the manner in which it is made (e.g., by hybridoma, phage selection, recombinant expression, transgenic animal, peptide synthesis, etc). Exemplary antibodies include polyclonal, monoclonal and recombinant antibodies; pan-specific, multispecific antibodies (e.g., bispecific antibodies, trispecific antibodies); humanized antibodies; murine antibodies; chimeric, mouse-human, mouse-primate, primate-human monoclonal antibodies; and anti-idiotype antibodies, and may be any intact antibody or fragment thereof. As used herein, the term "antibody" encompasses not only intact polyclonal or monoclonal antibodies, but also fragments thereof (such as dAb, Fab, Fab', F(ab')$_2$, Fv), single chain (ScFv), synthetic variants thereof, naturally occurring variants, fusion proteins comprising an antibody portion with an antigen-binding fragment of the required specificity, humanized antibodies, chimeric antibodies, and any other modified configuration of the immunoglobulin molecule that comprises an antigen-binding site or fragment (epitope recognition site) of the required specificity.

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific for the target antigen. The term "monoclonal antibody" encompasses not only intact monoclonal antibodies and full-length monoclonal antibodies, but also fragments thereof (such as Fab, Fab', F(ab')$_2$, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. It is not intended to be limited as regards the source of the antibody or the manner in which it is made (*e.g.,* by hybridoma, phage selection, recombinant expression, transgenic animals, etc.). The term includes whole immunoglobulins as well as the fragments etc. described above under the definition of "antibody".

As used herein, the term "antibody fragment" refers to a portion derived from or related to a full-length antibody, such as, for example, a MASP-1, MASP-2 or MASP-3 antibody, generally including the antigen binding or variable region thereof. Illustrative examples of antibody fragments include Fab, Fab', F(ab)$_2$, F(ab')$_2$ and Fv fragments, scFv fragments, diabodies, linear antibodies, single-chain antibody molecules and multispecific antibodies formed from antibody fragments.

As used herein, a "single-chain Fv" or "scFv" antibody fragment comprises the $V_H$ and $V_L$ domains of an antibody, wherein these domains are present in a single polypeptide chain. Generally, the Fv polypeptide further comprises a polypeptide linker between the $V_H$ and $V_L$ domains, which enables the scFv to form the desired structure for antigen binding.

As used herein, a "chimeric antibody" is a recombinant protein that contains the variable domains and complementarity-determining regions derived from a non-human species (e.g., rodent) antibody, while the remainder of the antibody molecule is derived from a human antibody.

As used herein, a "humanized antibody" is a chimeric antibody that comprises a minimal sequence that conforms to specific complementarity-determining regions derived from non-human immunoglobulin that is transplanted into a human antibody framework. Humanized antibodies are typically recombinant proteins in which only the antibody complementarity-determining regions are of non-human origin (including antibodies generated from phage display or yeast).

As used herein, the term "mannan-binding lectin" ("MBL") is equivalent to mannan-binding protein ("MBP").

As used herein, the "membrane attack complex" ("MAC") refers to a complex of the terminal five complement components (C5b combined with C6, C7, C8 and C9) that inserts into and disrupts membranes (also referred to as C5b-9).

As used herein, "a subject" includes all mammals, including without limitation humans, non-human primates, dogs, cats, horses, sheep, goats, cows, rabbits, pigs and rodents.

As used herein, the amino acid residues are abbreviated as follows: alanine (Ala;A), asparagine (Asn;N), aspartic acid (Asp;D), arginine (Arg;R), cysteine (Cys;C), glutamic acid (Glu;E), glutamine (Gln;Q), glycine (Gly;G), histidine (His;H), isoleucine (Ile;I), leucine (Leu;L), lysine (Lys;K), methionine (Met;M), phenylalanine (Phe;F), proline (Pro;P), serine (Ser;S), threonine (Thr;T), tryptophan (Trp;W), tyrosine (Tyr;Y), and valine (Val;V).

In the broadest sense, the naturally occurring amino acids can be divided into groups based upon the chemical characteristic of the side chain of the respective amino acids. By "hydrophobic" amino acid is meant either Ile, Leu, Met, Phe, Trp, Tyr, Val, Ala, Cys or Pro. By "hydrophilic" amino acid is meant either Gly, Asn, Gln, Ser, Thr, Asp, Glu, Lys, Arg or His. This grouping of amino acids can be further subclassed as follows. By "uncharged hydrophilic" amino acid is meant either Ser, Thr, Asn or Gln. By "acidic" amino acid is meant either Glu or Asp. By "basic" amino acid is meant either Lys, Arg or His.

As used herein the term "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine.

The term "oligonucleotide" as used herein refers to an oligomer or polymer of ribonucleic acid (RNA) or deoxyribonucleic acid (DNA) or mimetics thereof. This term also covers those oligonucleobases composed of naturally-occurring nucleotides, sugars and covalent internucleoside (backbone) linkages as well as oligonucleotides having non-naturally-occurring modifications.

As used herein, an "epitope" refers to the site on a protein (e.g., a human MASP-3 protein) that is bound by an antibody. "Overlapping epitopes" include at least one (e.g., two, three, four, five, or six) common amino acid residue(s), including linear and non-linear epitopes.

As used herein, the terms "polypeptide," "peptide," and "protein" are used interchangeably and mean any peptide-linked chain of amino acids, regardless of length or post-translational modification. The MASP proteins (MASP-1, MASP-2 or MASP-3) described herein can contain or be wild-type proteins or can be variants that have not more than 50 (e.g., not more than one, two, three, four, five, six, seven, eight, nine, ten, 12, 15, 20, 25, 30, 35, 40, or 50) conservative amino acid substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The human MASP-1 protein (set forth as SEQ ID NO:10), human MASP-2 protein (set forth as SEQ ID NO:5) and human MASP-3 protein (set forth as SEQ ID NO:8) described herein also include "peptide fragments" of the proteins, which are shorter than full-length and/or immature (pre-pro) MASP proteins, including peptide fragments of a MASP protein include terminal as well internal deletion variants of the protein. Deletion variants can lack one, two, three, four, five, six, seven, eight, nine, ten, 11, 12, 13, 14, 15, 16, 17, 18, 19, or 20 amino acid segments (of two or more amino acids) or noncontiguous single amino acids. In some embodiments, the human MASP-1 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-1 protein having the amino acid sequence set forth in SEQ ID NO: 10.

In some embodiments, the human MASP-3 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-3 protein having the amino acid sequence set forth in SEQ ID NO: 8.

In some embodiments, the human MASP-2 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-2 protein having the amino acid sequence set forth in SEQ ID NO: 5.

In some embodiments, peptide fragments can be at least 6 (e.g., at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 250, 300, 350, 400, 450, 500, or 600 or more) amino acid residues in length (e.g., at least 6 contiguous amino acid residues in any one of SEQ ID NOS: 5, 8 or 10). In some embodiments, an antigenic peptide fragment of a human MASP protein is fewer than 500 (e.g., fewer than 450, 400, 350, 325, 300, 275, 250, 225, 200, 190, 180, 170, 160, 150, 140, 130, 120, 110, 100, 95, 90, 85, 80, 75, 70, 65, 60, 55, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, or 6) amino acid residues in length (e.g., fewer than 500 contiguous amino acid residues in any one of SEQ ID NOS: 5, 8 or 10).

In some embodiments, in the context of generating an antibody that binds MASP-1, MASP-2 and/or MASP-3, the peptide fragments are antigenic and retain at least 10% (e.g., at least 10%, at least 15%, at least 20%, at least 25%, at least 30%, at least 35%, at least 40%, at least 50%, at least 55%, at least 60%, at least 70%, at least 80%, at least 90%, at least 95%, at least 98%, at least 99%, at least 99.5%, or 100% or more) of the ability of the full-length protein to induce an antigenic response in a mammal (see below under "Methods for Producing an Antibody").

Percent (%) amino acid sequence identity is defined as the percentage of amino acids in a candidate sequence that are identical to the amino acids in a reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

In representative embodiments, the human MASP-1 protein (SEQ ID NO:10) is encoded by the cDNA sequence set forth as SEQ ID NO:9; the human MASP-2 protein (SEQ ID NO:5) is encoded by the cDNA sequence set forth as SEQ ID NO:4; and the human MASP-3 protein (SEQ ID NO:8) is encoded by the cDNA sequence set forth as SEQ ID NO:7. Those skilled in the art will recognize that the cDNA sequences disclosed in SEQ ID NO:9, SEQ ID NO:4 and SEQ ID NO:7 represent a single allele of human MASP-1, MASP-2 and MASP-3, respectively, and that allelic variation and alternative splicing are expected to occur. Allelic variants of the nucleotide sequences shown in SEQ ID NO:9, SEQ ID NO:4 and SEQ ID NO:7, including those containing silent mutations and those in which mutations result in amino acid sequence changes, are within the scope of the present invention. Allelic variants of the MASP-1, MASP-2 or MASP-3 sequence can be cloned by probing cDNA or genomic libraries from different individuals according to standard procedures, or may be identified by homology comparison search (e.g., BLAST searching) of databases containing such information.

## II. THE LECTIN PATHWAY: A NEW UNDERSTANDING

### i. Overview: the Lectin pathway has been redefined

As described herein, the inventors have made the surprising discovery that the lectin pathway of complement has two effector arms to activate complement, both driven by lectin pathway activation complexes formed of carbohydrate recognition components (MBL, CL-11 and ficolins): i) the effector arm formed by the lectin pathway-associated serine proteases MASP-1 and MASP-3, referred to herein as "lectin pathway effector arm 1" or "LEA-1"; and (ii) the MASP-2 driven activation effector arm, referred to herein as "lectin pathway effector arm 2", or "LEA-2". Both LEA-1 and LEA-2 can effect lysis and/or opsonization.

It has also been determined that lectin-independent conversion of factor B by MASP-3 and lectin-independent conversion of factor D by HTRA-1, MASP-1 and MASP-3, which both can occur in the absence of $Ca^{++}$, commonly lead to the conversion of C3bB to C3bBb and of pro-factor D to factor D. Therefore, inhibiting MASP-3 can inhibit both LEA-1 and the lectin-independent activation of factor B and/or factor D, which can result in the inhibition of lysis and/or opsonization.

**FIGURE 1** illustrates this new understanding of the pathways of complement activation. As shown in **FIGURE 1,** LEA-1 is driven by lectin-bound MASP-3, which can activate the zymogen of factor D to its active form and/or cleave the C3b- or $C3b(H_2 0)$-bound factor B, leading to conversion of the C3bB zymogen complex into its enzymatically active form C3bBb. Activated factor D, generated by MASP-3, can also convert the C3bB or $C3b(H_2 0)$ zymogen complexes into their enzymatically active form. MASP-1 is capable of rapid self-activation, whereas MASP-3 is not. In many cases, MASP-1 is the activator of MASP-3.

While in many examples lectins (i.e., MBL, CL-11 or ficolins) can direct activity to cellular surfaces, **FIGURE 1** also outlines the lectin-independent functions of MASP-3, MASP-1, and HTRA-1 in factor B activation and/or factor D maturation. As with the lectin-associated form of MASP-3 in LEA-1, the lectin-independent form of MASP-3 is capable of mediating conversion of C3bB or $C3b(H_2 0)$ to C3bBb (see also **FIGURES 36** and 37) and pro-factor D to factor D (see **FIGURE 39**). MASP-1 (see also **FIGURE 39**) and the non-MASP-related protein HTRA-1 can also activate factor D (Stanton et al., Evidence That the HTRA1 Interactome Influences Susceptibility to Age-Related Macular Degeneration, presented at The Association for Research in Vision and Ophthalmology 2011 conference on May 4, 2011) in a manner in which no lectin component is required.

Thus, MASP-1 (via LEA-1 and lectin-independent forms), MASP-3 (via LEA-1 and lectin-independent forms), and HTRA-1 (lectin-independent only) are capable of either direct or indirect activation at one or more points along a MASP-3 - factor D - factor B axis. In doing so, they generate C3bBb, the C3 convertase of the alternative pathway, and they stimulate the production and deposition of C3b on microbial surfaces. C3b deposition plays a critical role in opsonization, labeling the surfaces of microbes for destruction by host phagocytic cells, such as macrophages. As an example herein (**FIGURE 35**), MASP-3 is critical for opsonization of *S. aureus*. C3b deposition occurs rapidly on *S. aureus* exposed to human serum in a MASP-3-dependent fashion (**FIGURE 35**).

The contributions of LEA-1 and the lectin-independent functions of MASP-3, MASP-1, or HTRA-1 are not limited to opsonization, however. As diagrammed in **FIGURE 1,** these three components can also cause cell lysis by indirect or direct activation of factor B, and the production of C3b. These components form complexes that generate the alternative pathway C5 convertase, $C3bBb(C3b)_n$. As described further herein, the requirement for MASP-3 and MBL, but not MASP-2 (and, therefore, not LEA-2 in this example), in the lysis of *N. meningitidis* (see **FIGURES 13, 14** and **15**) demonstrates a role for LEA-1 in lysis. In summary, the opsonization results obtained from the *S. aureus* studies and the lysis results observed in the *N. meningitidis* studies support the role of LEA-1 in both processes (as diagrammed in **FIGURE 1**). Furthermore, these studies demonstrate that both opsonization and lysis can result from the conversion of C3bB or $C3b(H_2 0)$ and/or of pro-factor D to factor D; therefore, both processes can be outcomes of the lectin-independent roles of MASP-3, MASP-1, or HTRA-1. Thus, the model developed by the inventors in **FIGURE 1** supports the use of inhibitors of principally MASP-3, but also MASP-1 and/or HTRA-1, to block opsonization and/or lysis and to treat pathologies caused by dysregulation of these processes.

## 1. Lectin Pathway Effector Arm (LEA-1)

The first effector arm of the lectin pathway, LEA-1, is formed by the lectin pathway-associated serine proteases MASP-1 and MASP-3. As described herein, the inventors have now shown that, in the absence of MASP-3 and in the presence of MASP-1, the alternative pathway is not effectively activated on surface structures. These results demonstrate that MASP-3 plays a previously undisclosed role in initiating the alternative pathway, and this is confirmed using the MASP-3-deficient 3MC serum obtained from patients with the rare 3MC autosomal recessive disorder (Rooryck C, et al., Nat Genet. 43(3):197-203 (2011)) with mutations that render the serine protease domain of MASP-3 dysfunctional. Based on these novel findings, it is expected that complement activation involving the alternative pathway, as conventionally defined, is MASP-3-dependent. In fact, MASP-3, and its activation of LEA-1, may represent the hitherto elusive initiator of the alternative pathway.

As further described in Examples 1-4 herein, in MASP-2-deficient sera, the inventors observed a higher activity of lectin-dependent alternative pathway activation resulting in a higher bactericidal activity (i.e., lytic activity) against *N. meningitidis.* While not wishing to be bound by any particular theory, it is believed that in absence of MASP-2, MASP-1-bearing carbohydrate recognition complexes are more likely to bind close to MASP-3-bearing carbohydrate recognition complexes to activate MASP-3. It is known that, in many instances, activation of MASP-3 is dependent on MASP-1 activity, as MASP-3 is not an auto-activating enzyme and very often requires the activity of MASP-1 to be converted from its zymogen form into its enzymatically active form. MASP-1 (like MASP-2) is an auto-activating enzyme, while MASP-3 does not auto-activate and, in many instances, needs the enzymatic activity of MASP-1 to be converted into its enzymatically active form. *See,* Zundel S, et al., J Immunol., 172(7):4342-50 (2004). In absence of MASP-2, all lectin pathway recognition complexes are either loaded with MASP-1 or MASP-3. Therefore, the absence of MASP-2 facilitates the MASP-1-mediated conversion of MASP-3 into its enzymatically active form. Once MASP-3 is activated, activated MASP-3 initiates alternative pathway activation, now referred to as "LEA-1" activation, through a MASP-3-mediated conversion of C3bB to C3bBb and/or conversion of pro-factor D to factor D. C3bBb, also referred to as the alternative pathway C3 convertase, cleaves additional C3 molecules yielding deposition of opsonic C3b molecules. If several C3b fragments bind in close proximity to the C3bBb convertase complex, this results in the formation of the alternative pathway C5 convertase C3bBb(C3b)n, which promotes formation of MAC. Additionally, C3b molecules deposited on the surface form new sites for factor B binding, which can now be cleaved by factor D and/or MASP-3 to form additional sites where alternative pathway C3 and C5 convertase complexes can be formed. This latter process is needed for effective lysis and does not require lectins once the initial C3b deposition has occurred. A recent publication (Iwaki D. et al., J Immunol 187(7):3751-8 (2011)) as well as data generated from the inventors (**FIGURE 37**) demonstrate that the alternative pathway C3 convertase zymogen complex C3bB is converted into its enzymatically active form by activated MASP-3. The inventors now have discovered that the MASP-3-mediated cleavage of factor B represents a subcomponent of the newly described LEA-1, which promotes lectin-dependent formation of the alternative pathway C3 convertase C3bBb.

## 2. Lectin Pathway Effector Arm (LEA-2)

The second effector arm of the lectin pathway, LEA-2, is formed by the lectin pathway-associated serine protease MASP-2. MASP-2 is activated upon binding of the recognition components to their respective pattern, and may also be activated by MASP-1, and subsequently cleaves the complement component C4 into C4a and C4b. After the binding of the cleavage product C4b to plasma C2, C4b-bound C2 becomes substrate of a second MASP-2-mediated cleavage step which converts C4b-bound C2 into the enzymatically active complex C4bC2a and a small C2b cleavage fragment. C4b2a is the C3-converting C3 convertase of the lectin pathway, converting the abundant plasma component C3 into C3a and C3b. C3b binds to any surface in close proximity via a thioester bond. If several C3b fragments bind in close proximity to the C3 convertase complex C4b2a, this convertase alters its specificity to convert C5 into C5b and C5a, forming the C5 convertase complex C4b2a(C3b)n. While this C5 convertase can initiate formation of MAC, this process is thought to be insufficiently effective to promote lysis on its own. Rather, the initial C3b opsonins produced by LEA-2 form the nucleus for the formation of new alternative pathway C3 convertase and C5 convertase sites, which ultimately lead to abundant MAC formation and lysis. This latter event is mediated by factor D activation of factor B associated with LEA-2-formed C3b, and hence is dependent on LEA-1 by virtue of the essential role for MASP-1 in the maturation of factor D. There is also a MASP-2-dependent C4-bypass activation route to activate C3 in the absence of C4, which plays an important role in the pathophysiology of ischemia-reperfusion injury, since C4-deficient mice are not protected from ischemia-reperfusion injury while MASP-2-deficient mice are (Schwaeble et al., PNAS, 2011 supra). LEA-2 is also tied to the coagulation pathway, involving the cleavage of prothrombin to thrombin (common pathway) and also the cleavage of factor XII (Hageman factor) to convert into its enzymatically active form XIIa. Factor XIIa in turn cleaves factor XI to XIa (intrinsic pathway). The intrinsic pathway activation of the clotting cascade leads to fibrin formation, which is of critical importance for thrombus formation.

**FIGURE 1** illustrates the new understanding of the lectin pathway and alternative pathway based on the results provided herein. **FIGURE 1** delineates the role of LEA-2 in both opsonization and lysis. While MASP-2 is the initiator of "downstream" C3b deposition (and resultant opsonization) in multiple lectin-dependent settings physiologically (**FIGURES 20A, 20B, 20C**), it also plays a role in lysis of serum-sensitive bacteria. As illustrated in **FIGURE 1,** the proposed molecular mechanism responsible for the increased bactericidal activity of MASP-2-deficient or MASP-2-depleted serum/plasma for serum-sensitive pathogens such as *N. meningitidis* is that, for the lysis of bacteria, lectin pathway recognition complexes associated with MASP-1 and MASP-3 have to bind in close proximity to each other on the bacterial surface, thereby allowing MASP-1 to cleave MASP-3. In contrast to MASP-1 and MASP-2, MASP-3 is not an auto-activating enzyme, but, in many instances, requires activation/cleavage by MASP-1 to be converted into its enzymatically active form.

As further shown in **FIGURE 1,** activated MASP-3 can then cleave C3b-bound factor B on the pathogen surface to initiate the alternative activation cascade by formation of the enzymatically active alternative pathway C3 and C5 convertases C3bBb and C3bBb(C3b)n, respectively. MASP-2-bearing lectin-pathway activation complexes have no part in the activation of MASP-3 and, in the absence of or after depletion of MASP-2, all-lectin pathway activation complexes will either be loaded with MASP-1 or MASP-3. Therefore, in the absence of MASP-2, the likelihood is markedly increased that on the microbial surface MASP-1- and MASP-3-bearing lectin-pathway activation complexes will come to sit in close proximity to each other, leading to more MASP-3 being activated and thereby leading to a higher rate of MASP-3-mediated cleavage of C3b-bound factor B to form the alternative pathway C3 and C5 convertases C3bBb and C3bBb(C3b)n on the microbial surface. This leads to the activation of the terminal activation cascades C5b-C9 that forms the Membrane Attack Complex, composed of surface-bound C5b associated with C6, C5bC6 associated with C7, C5bC6C7 associated with C8, and C5bC6C7C8, leading to the polymerization of C9 that inserts into the bacterial surface structure and forms a pore in the bacterial wall, which will lead to osmolytic killing of the complement-targeted bacterium.

The core of this novel concept is that the data provided herein clearly show that the lectin pathway activation complexes drive the following two distinct activation routes, as illustrated in **FIGURE 1:**

i) LEA-1: A MASP-3-dependent activation route that initiates and drives activation of complement by generating the alternative pathway convertase C3bBb through initial cleavage and activation of factor B on activator surfaces, which will then catalyze C3b deposition and formation of the alternative pathway convertase C3bBb. The MASP-3-driven activation route plays an essential role in the opsonization and lysis of microbes and drives the alternative pathway on the surface of bacteria, leading to optimal rates of activation to generate membrane attack complexes; and

ii) LEA-2: A MASP-2-dependent activation route leading to the formation of the lectin pathway C3 convertase C4b2a and, upon accumulation of the C3 cleavage product C3b, subsequently to the C5 convertase C4b2a(C3b)n. In the absence of complement C4, MASP-2 can form an alternative C3 convertase complex which involves C2 and clotting factor XI.

In addition to its role in lysis, the MASP-2-driven activation route plays an important role in bacterial opsonization leading to microbes being coated with covalently bound C3b and cleavage products thereof (i.e., iC3b and C3dg), which will be targeted for the uptake and killing by C3 receptor-bearing phagocytes, such as granulocytes, macrophages, monocytes, microglia cells and the reticuloendothelial system. This is the most effective route of clearance of bacteria and microorganisms that are resistant to complement lysis. These include most of the gram-positive bacteria.

In addition to LEA-1 and LEA-2, there is the potential for lectin-independent activation of factor D by MASP-3, MASP-1 and/or HTRA-1, and there is also the potential for lectin-independent activation of factor B by MASP-3.

While not wishing to be bound by any particular theory, it is believed that each of (i) LEA-1, (ii) LEA-2 and (iii) lectin-independent activation of factor B and/or factor D lead to opsonization and/or the formation of MAC with resultant lysis.

ii. Background of MASP-1, MASP-2 and MASP-3

Three mannan-binding lectin-associated serine proteases (MASP-1, MASP-2 and MASP-3) are presently known to be associated in human serum with the mannan-binding lectin (MBL). Mannan-binding lectin is also called 'mannose-binding protein' or 'mannose-binding lectin' in the recent literature. The MBL-MASP complex plays an important role in innate immunity by virtue of the binding of MBL to carbohydrate structures present on a wide variety of microorganisms. The interaction of MBL with specific arrays of carbohydrate structures brings about the activation of the MASP proenzymes which, in turn, activate complement by cleaving the complement components C4 and C2 to form the C3 convertase C4b2b (Kawasaki et al., J. Biochem 106:483-489 (1989); Matsushita & Fujita, J. Exp Med. 176:1497-1502 (1992); Ji et al., J. Immunol 150:571-578 (1993)).

The MBL-MASP proenzyme complex was, until recently, considered to contain only one type of protease (MASP-1), but it is now clear that there are two other distinct proteases (i.e., MASP-2 and MASP-3) associated with MBL (Thiel et al., Nature 386:506-510 (1997); Dahl et al., Immunity 15:127-135 (2001)), as well as an additional serum protein of 19 kDa, referred to as "MAp19" or "sMAP" (Stover et al., J. Immunol 162:3481-3490 (1999); Stover et al., J. Immunol 163:6848-6859 (1999); Takahashi et al., Int. Immunol 11:859-63 (1999)).

MAp19 is an alternatively spliced gene product of the structural gene for MASP-2 and lacks the four C-terminal domains of MASP-2, including the serine endopeptidase domain. The abundantly expressed truncated mRNA transcript encoding MAp19 is generated by an alternative splicing/polyadenylation event of the MASP-2 gene. By a similar mechanism, the MASP-1/3 gene gives rise to three major gene products, the two serine proteases MASP-1 and MASP-3 and a truncated gene product of 44 kDa referred to as "MAp44" (Degn et al., J. Immunol 183(11):7371-8 (2009); Skjoedt et al., J Biol Chem 285:8234-43 (2010)).

MASP-1 was first described as the P-100 protease component of the serum Ra-reactive factor, which is now recognized as being a complex composed of MBL plus MASP (Matsushita et al., Collectins and Innate Immunity, (1996); Ji et al., J Immunol 150:571-578 (1993). The ability of an MBL-associated endopeptidase within the MBL-MASPs complex to act on the complement components C4 and C2 in a manner apparently identical to that of the C1s enzyme within the $C1q\text{-}(Clr)_2\text{-}(Cls)_2$ complex of the classical pathway of complement suggests that there is a MBL-MASPs complex which is functionally analogous to the $C1q\text{-}(C1r)_2\text{-}(Cls)_2$ complex. The $C1q\text{-}(C1r)_2\text{-}(C1s)_2$ complex is activated by the interaction of C1q with the Fc regions of antibody IgG or IgM present in immune complexes. This brings about the autoactivation of the C1r proenzyme which, in turn, activates the C1s proenzyme which then acts on complement components C4 and C2.

The stoichiometry of the MBL-MASPs complex differs from the one found for the $C1q\text{-}(C1r)_2\text{-}(C1s)_2$ complex in that different MBL oligomers appear to associate with different proportions of MASP-1/MAp19 or MASP-2/MASP-3 (Dahl et al., Immunity 15:127-135 (2001). The majority of MASPs and MAp19 found in serum are not complexed with MBL (Thiel et al., J Immunol 165:878-887 (2000)) and may associate in part with ficolins, a recently described group of lectins having a fibrinogen-like domain able to bind to N-acetylglucosamine residues on microbial surfaces (Le et al., FEBS Lett 425:367 (1998); Sugimoto et al., J. Biol Chem 273:20721 (1998)). Among these, human L-ficolin, H-ficolin and M-ficolin associate with MASPs as well as with MAp19 and may activate the lectin pathway upon binding to the specific carbohydrate structures recognized by ficolins (Matsushita et al., J Immunol 164:2281-2284 (2000); Matsushita et al., J Immunol 168:3502-3506 (2002)). In addition to the ficolins and MBL, an MBL-like lectin collectin, called CL-11, has been identified as a lectin pathway recognition molecule (Hansen et al. J Immunol 185:6096-6104 (2010); Schwaeble et al. PNAS 108:7523-7528 (2011)). There is overwhelming evidence underlining the physiological importance of these alternative carbohydrate recognition molecules and it is therefore important to understand that MBL is not the only recognition component of the lectin activation pathway and that MBL deficiency is not to be mistaken for lectin-pathway deficiency. The existence of possibly an array of alternative carbohydrate-recognition complexes structurally related to MBL may broaden the spectrum of microbial structures that initiate a direct response of the innate immune system via activation of complement.

All lectin pathway recognition molecules are characterized by a specific MASPs-binding motif within their collagen-homologous stalk region (Wallis et al. J. Biol Chem 279:14065-14073 (2004)). The MASP-binding site in MBLs, CL-11 and ficolins is characterized by a distinct motif within this domain: Hyp-Gly-Lys-Xaa-Gly-Pro, where Hyp is hydroxyproline and Xaa is generally an aliphatic residue. Point mutations in this sequence disrupt MASP binding.

## 1. Respective structures, sequences, chromosomal localization and splice variants

**FIGURE 2** is a schematic diagram illustrating the domain structure of the MASP-2 polypeptide (SEQ ID NO:5) and MAp19 polypeptide (SEQ ID NO:2) and the exons encoding the same. **FIGURE 3** is a schematic diagram illustrating the domain structure of the MASP-1 polypeptide (SEQ ID NO:10), MASP-3 polypeptide (SEQ ID NO:8) and MAp44 polypeptide (SEQ ID NO:11) and the exons encoding the same. As shown in **FIGURES 2 and 3,** the serine proteases MASP-1, MASP-2 and MASP-3 consist of six distinct domains arranged as found in C1r and C1s; *i.e.,* (I) an N-terminal C1r/C1s/sea urchin VEGF/bone morphogenic protein (or CUBI) domain; (II) an epidermal growth factor (EGF)-like domain; (III) a second CUB domain (CUBII); (IV and V) two complement control protein (CCP1 and CCP2) domains; and (VI) a serine protease (SP) domain.

The cDNA-derived amino acid sequences of human and mouse MASP-1 (Sato et al., Int Immunol 6:665-669 (1994); Takada et al., Biochem Biophys Res Commun 196:1003-1009 (1993); Takayama et al., J. Immunol 152:2308-2316 (1994)), human, mouse, and rat MASP-2 (Thiel et al., Nature 386:506-510 (1997); Endo et al., J Immunol 161:4924-30 (1998); Stover et al., J. Immunol 162:3481-3490 (1999); Stover et al., J. Immunol 163:6848-6859 (1999)), as well as human MASP-3 (Dahl et al., Immunity 15:127-135 (2001)) indicate that these proteases are serine peptidases having the characteristic triad of His, Asp and Ser residues within their putative catalytic domains (Genbank Accession numbers: human MASP-1: BAA04477.1; mouse MASP-1: BAA03944; rat MASP-1: AJ457084; Human MASP-3:AAK84071; mouse MASP-3: AB049755, as accessed on Genbank on 2/15/2012, each of which is hereby incorporated herein by reference).

As further shown in **FIGURES 2** and **3,** upon conversion of the zymogen to the active form, the heavy chain (alpha, or A chain) and light chain (beta, or B chain) are split to yield a disulphide-linked A-chain and a smaller B-chain representing the serine protease domain. The single-chain proenzyme MASP-1 is activated (like proenzyme C1r and C1s) by cleavage of an Arg-Ile bond located between the second CCP domain (domain V) and the serine protease domain (domain VI). Proenzymes MASP-2 and MASP-3 are considered to be activated in a similar fashion to that of MASP-1. Each MASP protein forms homodimers and is individually associated with MBL and the ficolins in a Ca++-dependent manner.

**2. MASP-1/3**

The human MASP-1 polypeptide (SEQ ID NO:10) and MASP-3 polypeptide (SEQ ID NO:8) arise from one structural gene (Dahl et al., Immunity 15:127-135 (2001), which has been mapped to the 3q27-28 region of the long arm of chromosome 3 (Takada et al., Genomics 25:757-759 (1995)). The MASP-3 and MASP-1 mRNA transcripts are generated from the primary transcript by an alternative splicing/polyadenylation process. The MASP-3 translation product is composed of an alpha chain, which is common to both MASP-1 and MASP-3, and a beta chain (the serine protease domain), which is unique to MASP-3. As shown in **FIGURE 3,** the human MASP-1 gene encompasses 18 exons. The human MASP-1 cDNA (set forth as SEQ ID NO:9) is encoded by exons 2, 3, 4, 5, 6, 7, 8, 10, 11, 13, 14, 15, 16, 17 and 18. As further shown in **FIGURE 3,** the human MASP 3 gene encompasses twelve exons. The human MASP-3 cDNA (set forth as SEQ ID NO:7) is encoded by exons 2, 3, 4, 5, 6, 7, 8, 10, 11 and 12. An alternative splice results in a protein termed MBL-associated protein 44 ("MAp44)," (set forth as SEQ ID NO:11), arising from exons 2, 3, 4, 5, 6, 7, 8 and 9.

The human MASP-1 polypeptide (SEQ ID NO: 10 from Genbank BAA04477.1) has 699 amino acid residues, which includes a leader peptide of 19 residues. When the leader peptide is omitted, the calculated molecular mass of MASP-1 is 76,976 Da. As shown in **FIGURE 3,** the MASP-1 amino acid sequence contains four N-linked glycosylation sites. The domains of the human MASP-1 protein (with reference to SEQ ID NO:10) are shown in **FIGURE 3** and include an N-terminal C1r/C1s/sea urchin VEFG/bone morphogenic protein (CUBI) domain (aa 25-137 of SEQ ID NO:10), an epidermal growth factor-like domain (aa 139-181 of SEQ ID NO:10), a second CUB domain (CUBII) (aa 185-296 of SEQ ID NO:10), as well as a tandem of complement control protein (CCP1 aa 301-363 and CCP2 aa 367-432 of SEQ ID NO: 10) domains and a serine protease domain (aa 449-694 of SEQ ID NO:10).

The human MASP-3 polypeptide (SEQ ID NO:8, from Genbank AAK84071) has 728 amino acid residues, which includes a leader peptide of 19 residues. When the leader peptides are omitted, the calculated molecular mass of MASP-3 is 81,873 Da. As shown in **FIGURE 3,** there are seven N -linked glycosylation sites in MASP-3. The domains of the human MASP-3 protein (with reference to SEQ ID NO:8) are shown in **FIGURE 3** and include an N-terminal C1r/C1s/sea urchin VEGF/bone morphogenic protein (CUBI) domain (aa 25-137 of SEQ ID NO:8), an epidermal growth factor-like domain (aa 139-181 of SEQ ID NO:8), a second CUB domain (CUBII) (aa 185-296 of SEQ ID NO:8), as well as a tandem of complement control protein (CCP1 aa 301-363 and CCP2 aa 367-432 of SEQ ID NO:8) domains and a serine protease domain (aa 450-711 of SEQ ID NO:8).

The MASP-3 translation product is composed of an alpha chain (heavy chain), containing the CUB-1-EGF-CUB-2-CCP-1-CCP-2 domains (alpha chain: aa 1-448 of SEQ ID NO:8) which is common to both MASP-1 and MASP-3, and a light chain (beta chain: aa 449-728 of SEQ ID NO:8), containing the serine protease domain, which is unique to MASP-3 and MASP-1.

**3. MASP-2**

The human MASP-2 gene is located on chromosome 1p36.3-2 (Stover et al., Cytogenet and Cell Genet 84:148-149 (1999) and encompasses twelve exons, as shown in **FIGURE 2.** MASP-2 (SEQ ID NO:5) and MAp19 (SEQ ID NO:2) are encoded by transcripts of a single structural gene generated by alternative splicing/polyadenylation (Stover et al., Genes and Immunity 2:119-127 (2001)). The human MASP-2 cDNA (SEQ ID NO:4) is encoded by exons 2, 3, 4, 6, 7, 8, 9, 10, 11 and 12. The 20 kDa protein termed MBL-associated protein 19 ("MAp19", also referred to as "sMAP") (SEQ ID NO:2), encoded by (SEQ ID NO:1) arises from exons 2, 3, 4 and 5. MAp19 is a nonenzymatic protein containing the N-terminal CUB1-EGF region of MASP-2 with four additional residues (EQSL) derived from exon 5 as shown in **FIGURE 2.**

The MASP-2 polypeptide (SEQ ID NO:5) has 686 amino acid residues, which includes a leader peptide of 15 residues that is cleaved off after secretion, resulting in the mature form of human MASP-2 (SEQ ID NO:6). As shown in **FIGURE 2,** the MASP-2 amino acid sequence does not contain any N-linked glycosylation sites. The MASP-2 polypeptide exhibits a molecular structure similar to MASP-1, MASP-3, and C1r and C1s, the proteases of the C1 complement system. The domains of the human MASP-2 protein (numbered with reference to SEQ ID NO:5) are shown in **FIGURE 2** and include an N-terminal C1r/C1s/sea urchin VEGF/bone morphogenic protein (CUBI) domain (aa 24-136 of SEQ ID NO:5), an epidermal growth factor-like domain (aa 138-180 of SEQ ID NO:5), a second CUB domain (CUBII) (aa 184-295 of SEQ ID NO:5), as well as a tandem of complement control protein (CCP1 aa 300-359 and CCP2 aa 364-431 of SEQ ID NO:5) domains and a serine protease domain (aa 445-682 of SEQ ID NO:5).

As shown in **FIGURE 2,** the MASP-2 polypeptide has an alpha chain (heavy chain) containing the CUB-1-EGF-CUB-2-CCP-1-CCP-2 domains (alpha chain: aa 1-443 of SEQ ID NO:5) and a beta chain (light chain) containing the serine protease domain (beta chain: aa 444-686). The CUB-1, EGF and CUB-2 domains are required for dimerization and the CUB-1, EGF, CUB-2 and CCP-1 domains contain the binding site for MBP. As described in Wallis et al., J. Biol Chem 279:14065-14073 (2004), each MASP-2 dimer binds to two MBL subunits.

**4. Comparison of MASP-1, MASP-2 and MASP-3 amino acid sequences**

**FIGURE 4** is an amino acid alignment of the protein sequences of MASP-1 (SEQ ID NO:10), MASP-2 (SEQ ID NO:6) and MASP-3 (SEQ ID NO:8), showing the CUBI, EGF, CUBII, CCP1, CCP2 domains and conserved catalytic triad residues (H, D, S) in the serine protease (SP) domains. The symbol "." indicates an identical amino acid sequence.

**FIGURE 5** is an amino acid alignment of the alpha chain sequences, including the CUBI-EGF-CUBII-CCP1-CCP2, of MASP-1 (alpha chain: aa 1-447 of SEQ ID NO:10) MASP-2 (alpha chain: aa 1-443 of SEQ ID NO:5) and MASP-3 (alpha chain: aa 1-448 of SEQ ID NO:8). There are numerous patches of identity in the CUBI, EGF, and CUBII domains, as indicated by dotted boxes in **FIGURE 5.** The CCP1 and CCP2 domains are indicated by the dark shaded boxes. The overall percent identity between the alpha chains of human MASP1/3 and human MASP-2 is provided below in TABLE 1.

**FIGURE 6** is an amino acid alignment of the beta chain sequences (including the serine protease domains) of MASP-1 (beta chain: aa 448-699 of SEQ ID NO:10), MASP-2 (beta chain: aa 444-686 of SEQ ID NO:5) and MASP-3 (beta chain: aa 449-728 of SEQ ID NO:8). **FIGURE 7A** shows a pairwise amino acid alignment between the beta chain sequences of MASP-1 (beta chain: aa 448-699 of SEQ ID NO:10) and MASP-2 (beta chain: aa 444-686 of SEQ ID NO:5). **FIGURE 7B** shows a pairwise amino acid alignment between the beta chain sequences of MASP-1 (beta chain: aa 448-699 of SEQ ID NO:10) and MASP-3 (beta chain: aa 449-728 of SEQ ID NO:8). **FIGURE 7C** shows a pairwise amino acid alignment between the beta chain sequences of MASP-2 (beta chain: aa 444-686 of SEQ ID NO:5) and MASP-3 (beta chain: aa 449-728 of SEQ ID NO:8). The regions of identity in **FIGURES 5-7** are shown as dotted boxes surrounding the identical amino acids (shown as "." Symbol).

The percent identity between the alpha and beta chains of the human MASP-1, MASP-2 and MASP-3 proteins is provided in **TABLE 1** below.

**TABLE 1:** Percent Identity between human MASP proteins

|  | % Identity Between A Chains | | | % Identity Between B Chains | | |
|---|---|---|---|---|---|---|
|  | MASP-1 | MASP-2 | MASP-3 | MASP-1 | MASP-2 | MASP-3 |
| MASP-1 | 100% | 45.6% | 98% | 100% | 27% | 27% |
| MASP-2 | 45.6% | 100% | 45.4% | 27% | 100% | 28.6% |
| MASP-3 | 98% | 45.4% | 100% | 27% | 28.6% | 100% |

With regard to the alpha chains (heavy chains), as indicated above in **TABLE 1,** the MASP-1 and MASP-3 alpha chains are identical (except for the 15 amino acid sequence at 3' end). The overall % identity between the MASP-2 and MASP-3 alpha chain is 45.4%, with numerous patches of identity in the CUBI-EGF-CUBII domains, as shown in **FIGURE 5.**

With regard to the beta chains (light chains), the overall percent identity between the three beta chains is low, in the range of 27% to 28%. However, although overall identity between the three B-chains is low, there are numerous patches of identity, as shown in **FIGURE** 6. As further shown in **FIGURES 7A-C,** identical patches of sequence are more broadly distributed between MASP-2 and 3 than between 1 and 2 or 1 and 3.

All the cysteine residues present in MASP-2, MASP-3, C1r and C1s align with equivalent residues in MASP-1; however, MASP-1 has two cysteine residues (at positions 465 and 481 in the L chain) that are not found in the MASP-2, MASP-3, C1r and C1s. These two cysteine residues in MASP-1 are in the expected positions used to form the 'histidine-loop' disulfide bridge as found in trypsin and chymotrypsin. This suggests that MASP-2, MASP-3, C1r, and C1s may have evolved, by gene duplication and divergence, from MASP-1 (Nonaka & Miyazawa, Genome Biology 3 Reviews 1001.1-1001.5 (2001)).

**5. Respective biological functions/activities, including relevant human genetic data**

The role of the MBL/Ficolin-MASPs complexes in innate immunity is mediated via the calcium-dependent binding of the C-type lectin domains (present in the MBL molecule) or via the binding of the fibrinogen-like domains (present in the ficolin molecule) to carbohydrate structures found on yeast, bacteria, viruses, and fungi. This recognition phase brings about the activation of the proenzyme MASP-2, which then mimics the action of the activated C1s within the C1q-(C1r)$_2$-(C1s)$_2$ complex by cleaving C4 and C2 to form the C3 convertase C4b2b. This allows deposition of C4b and C3b on target pathogens and thus promotes killing and clearance through phagocytosis.

Evidence in the recent literature suggests that the lectin pathway activation complex only requires the activity of MASP-2 to cleave C4 and C2: i) the reconstitution of a minimal lectin-pathway activation complex using recombinant MBL and recombinantly expressed MASP-2 appears to be sufficient to effectively cleave both C4 and C2 *in vitro* (Vorup-Jensen et al., J. Immunol 165:2093-2100 (2000); Rossi et al., J Biol Chem 276:40880-40887 (2001); Ambrus et al., J Immunol 170:1374-1382 (2003); Gál et al, J Biol Chem 280:33435-33444 (2005)); while ii) the serum of mice with a gene-targeted deficiency of MASP-2 is devoid of any lectin pathway functional activity (Schwaeble et al., PNAS 108:7523-7528 (2011)). Recently, a genetically determined deficiency of MASP-2 was described (Stengaard-Pedersen et al., New Eng. J. Med. 349:554-560, (2003)). The mutation of a single nucleotide leads to an Asp-Gly exchange in the CUB1 domain and renders MASP-2 incapable of binding to MBL.

In addition, the functional characterization of sera of mice deficient of both MASP-1 and MASP-3 shows that lectin pathway activity is slower, but not absent when comparing sera of wild-type and MASP-1/MASP-3 knockout (MASP-1/3$^{-/-}$) mice under physiological conditions (Takahashi et al., J. Immunol 180:6132-6138 (2008); Schwaeble et al., PNAS (2011)). These studies suggest that in contrast to the classical pathway effector endopeptidase C1s, activation of MASP-2 does not essentially involve or require the activity of any of the other MBL-associated serine endopeptidases (i.e., MASP-1 or MASP-3) and that the proteolytic activity of MASP-2 suffices to translate binding of the carbohydrate recognition molecules of the lectin pathway (*i.e.,* MBL, ficolins or CL-11) into complement activation. However, more recent studies have demonstrated that while MASP-2 does have the capacity to autoactivate, the catalytic rate of MASP-1 activation of the MASP-2 zymogen exceeds that of MASP-2 cleavage of its own zymogen form by about 85,000 fold (Héja et al., PNAS 106:10498-503 (2011); Megyeri et al., J. Biol. Chem. 288(13):8922-34 (2013)). Therefore, it is likely that the primary activator of MASP-2 in physiological settings is MASP-1. As judged by the size of the fragments of C4 generated, and the functional C3 convertase activity generated, it seems likely that the activated MASP-2 cleaves C4 and C2 in an identical manner to that carried out by activated C1s, *i.e*,. at a single arginyl bond (Arg76 Ala77) within the alpha-chain of C4 and at a single arginyl bond (Arg223 Lys224) within the proenzyme chain of C2. It has also been reported that the mouse MASP (in the form of the mouse MBL-MASP complex designated Ra-reactive factor) can, unlike C1s, cleave the alpha-chain of complement component C3 to yield the biologically active fragments C3a and C3b (Ogata et al, J. Immunol 154:2351-2357 (1995)). If this were to take place in the human system, it would require the cleavage of a single arginyl bond (Arg77 Ser78) within the alpha-chain of C3. Activated MASP-2, like activated C1s, is unable to cleave complement component C5. The proteolytic activities of MASP-1 and MASP-2 are inhibited by C1-Inhibitor (Matsushita et al., J Immunol 165:2637-2642 (2000) whereas C1-Inhibitor does not react with MASP-3 (Dahl et al., Immunity 15:127-135 (2001); Zundel et al., J Immunol 172:4342-4350 (2004)).

The biological functions of MASP-1 and MASP-3 have been slow to emerge. The substrate specificity and the physiological role of MASP-1 have been a subject of debate since its discovery. Numerous potential substrates have been identified during the recent years. It was suggested that MASP-1 can cleave native C3 slowly and this direct cleavage of C3 may initiate the complement cascade perhaps with the contribution of the alternative pathway (Matsushita et al., J Immunol 165:2637-2642 (2000)). Later it was shown that recombinant MASP-1 cleaves the inactive (thioester hydrolyzed) form of C3 which is unproductive in terms of initiating the complement cascade (Ambrus et al., J Immunol 170:1374-1382 (2003)). The lack of lectin pathway activity in the serum dilutions of MASP-2-deficient mice unequivocally proved that the MASP-1-driven C3-bypass mechanism does not exist (Schwaeble et al., PNAS 108:7523-7528 (2011)). The complement components that are cleaved by MASP-1 with considerable efficiency are C2 (Rossi et al., J Biol Chem 276:40880-40887 (2001); Ambrus et al., J Immunol 170:1374-1382 (2003)) and the zymogen form of factor D (Takahashi et al., J Exp Med 207:29-37 (2010)). As for the ability of MASP-1 to cleave C2, it is plausible therefore that MASP-1 can augment the C3-convertase (C4b2a)-forming ability of MASP-2 via C2 cleavage. This suggestion is supported by the observation that the activity of the lectin pathway is diminished in MASP-1-depleted human serum and in the serum of MASP-1/3-deficient mice (Takahashi et al., J Immunol 180:6132-6138 (2008)), which observation also suggests that MASP-1 has a role in activating MASP-2. Moreover, while every C4b deposited by MBL-MASPs complex can form C4b2a convertase, only one out of four C4b deposited by the classical pathway C1 complex can do the same (Rawal et al., J Biol Chem 283 (12):7853-63 (2008)).

MASP-1 also cleaves MASP-2 and MASP-3 (Megyeri M., et al, J Biol Chem. 2013 Mar 29;288(13):8922-34). Recent experiments suggest that although MASP-2 can autoactivate, MASP-1 is the primary activator of zymogen MASP-2. The activation of MASP-2 was delayed in the serum of MASP-1 knockout mice (Takahashi et al., J Immunol 180: 6132-6138 (2008)) and a similar result was obtained when the activity of MASP-1 was blocked by a specific inhibitor in normal human serum (Kocsis et al., J Immunol 185(7):4169-78 (2010)). Moreover, Degn et al. (J. Immunol. 189(8): 3957-69 (2012)) found MASP-1 to be critical for MASP-2 activation and subsequent C4 cleavage in human serum. The catalytic rate for the conversion of zymogen MASP-2 to active MASP-2 is more than 85,000-fold greater than the rate by which MASP-2 can autoactivate (Megyeri et al., J. Biol. Chem. 288:8922-8934 (2013); Héja et al., J. Biol. Chem. 287(24):20290-300 (2012); Héja et al., PNAS 109:10498-503 (2012)).

Recent discoveries have also linked MASP-1 to the alternative pathway. MASP-1 can convert zymogen factor D into its enzymatically active form **(FIGURE 39**; Takahashi et al., J Exp Med 207:29-37 (2010)). Furthermore, MASP-1 activates the zymogen form of MASP-3 (Megyeri et al., J. Biol. Chem. 288:8922-8934 (2013); Degn et al. J. Immunol. 189(8): 3957-69 (2012)), which itself can activate zymogen factor D **(FIGURE 39)** as well as cleave factor B, another essential component of the alternative pathway, to its active form (Iwaki et al., J. Immunol. 187:3751-58 (2011)). The conversion of pro-factor D and pro-factor B, however, is likely to be independent of the activation state of LEA-2 and may occur through non-complex-bound MASP-1.

Several lines of evidence indicate that MASP-1 is a thrombin-like enzyme and is important in activation of the coagulation pathway. MASP-1 can cleave several substrates of thrombin including fibrinogen (Hajela K. et al., Immunobiology 205(4-5):467-75 (2002)), factor XIII (Krarup et al., Biochim Biophys Acta 1784(9):1294-1300 (2008)) and protease-activated receptor 4 (PAR4) (Megyeri et al., J Immunol 183(5):3409-16 (2009)). Moreover, antithrombin in the presence of heparin is a more efficient inhibitor of MASP-1 than C1-inhibitor (Dobó et al., J Immunol 183:1207-1214 (2009)). The connection between the complement and the coagulation pathway is also underlined by the observation that MASP-2 is able to activate prothrombin (Krarup A. et al., PLoS One 2(7):e623 (2007)). Limited coagulation represents an ancient type of innate immunity when the spreading of invading pathogens is prevented by the fibrin clot. The releasing fibrinopeptide B has proinflammatory activity. The MASP-1-mediated cleavage of PAR4 activates the endothelial cells-initiating inflammatory reaction (Megyeri et al., J Immunol 183(5):3409-16 (2009)).

MASP-3 has no proteolytic activity towards C4, C2 or C3 substrates. Conversely, MASP-3 was reported to act as an inhibitor of the lectin pathway (Dahl et al., Immunity 15:127-135 (2001)). This conclusion may have come about because in contrast to MASP-1 and MASP-2, MASP-3 is not an autoactivating enzyme (Zundel S. et al., J Immunol 172:4342-4350 (2004); Megyeri et al., J. Biol. Chem. 288:8922-8934 (2013).

Recently, evidence for possible physiological functions of MASP-1 and MASP-3 emerged from transgenic mouse studies using a mouse strain with a combined MASP-1 and MASP-3 deficiency. While MASP-1/3-knockout mice have a functional lectin pathway (Schwaeble et al., PNAS 108:7523-7528 (2011)), they appear to lack alternative pathway activity (Takahashi et al., JEM 207(1):29-37 (2010)). Lack of alternative pathway activity appears to be due to a processing defect of complement factor D, which is necessary for alternative pathway activity. In MASP-1/3 knockout mice, all factor D is circulating as a proteolytically inactive pro-form, whereas in the serum of normal mice, substantially all of factor D is in the active form. Biochemical analysis suggested that MASP-1 may be able to convert complement factor D from its zymogen form into its enzymatically active form **(FIGURE 39**; Takahashi et al., JEM 207(1):29-37 (2010)). MASP-3 also cleaves pro-factor D zymogen and produce active factor D *in vitro* **(FIGURE 39**; Takahashi et al., JEM207(1):29-37 (2010)). Factor D is present as an active enzyme in circulation in normal individuals, and MASP-1 and MASP-3, as well as HTRA-1, may be responsible for this activation. Furthermore, mice with combined MBL and ficolin deficiencies still produce normal levels of factor D and have a fully functional alternative pathway. Thus, these physiological functions of MASP-1 and MASP-3 do not necessarily involve lectins, and are thus unrelated to the lectin pathway. Recombinant mouse and human MASP-3 also appear to cleave factor B and support C3 deposition on *S. aureus in vitro* (**FIGURE 36**; Iwaki D. et al., J Immunol 187(7):3751-8 (2011)).

An unexpected physiological role for MASP-3 has emerged from recent studies of patients with 3MC syndrome (previously designated the Carnevale, Mingarelli, Malpuech, and Michels syndrome; OMIM # 257920). These patients display severe developmental abnormalities, including cleft palate, cleft lip, cranial malformations and mental retardation. Genetic analysis identified 3MC patients that were homozygous for a dysfunctional MASP-3 gene (Rooryck et al., Nat Genet. 43(3):197-203 (2011)). Another group of 3MC patients was found to be homozygous for a mutation in the MASP-1 gene that leads to the absence of functional MASP-1 and MASP-3 proteins. Yet another group of 3MC patients lacked a functional CL-11 gene. (Rooryck et al., Nat Genet. 43(3):197-203 (2011)). Thus, the CL-11 MASP-3 axis appears to play a role during embryonic development. The molecular mechanisms of this developmental pathway are unclear. It is unlikely, however, to be mediated by a conventional complement-driven process since individuals with deficiencies of common complement components C3 do not develop this syndrome. Thus, prior to the discovery of the instant inventors, as described herein, a functional role for MASP-3 in lectin-dependent complement activation was previously not established.

The structures of the catalytic fragment of MASP-1 and MASP-2 have been determined by X-ray crystallography. Structural comparison of MASP-1 protease domain with those of other complement proteases revealed the basis of its relaxed substrate specificity (Dobó et al., J. Immunol 183:1207-1214 (2009)). While the accessibility of the substrate binding groove of MASP-2 is restricted by surface loops (Harmat et al., J Mol Biol 342:1533-1546 (2004)), MASP-1 has an open substrate binding pocket which resembles that of trypsin rather than other complement proteases. A thrombin-like property of the MASP-1 structure is the unusually large 60 amino acid loop (loop B) which may interact with substrates. Another interesting feature of the MASP-1 structure is the internal salt bridge between the S1 Asp189 and Arg224. A similar salt bridge can be found in the substrate binding pocket of factor D, which can regulate its protease activity. C1s and MASP-2 have almost identical substrate specificities. Surprisingly, some of the eight surface loops of MASP-2, which determine the substrate specificities, have quite different conformations compared to those of C1s. This means that the two functionally related enzymes interact with the same substrates in a different manner. The structure of zymogen MASP-2 shows an inactive protease domain with disrupted oxyanion hole and substrate binding pocket (Gál et al., J Biol Chem 280:33435-33444 (2005)). Surprisingly, zymogen MASP-2 shows considerable activity on a large protein substrate, C4. It is likely that the structure of zymogen MASP-2 is quite flexible, enabling the transition between the inactive and the active forms. This flexibility, which is reflected in the structure, may play a role in the autoactivation process.

Northern blot analysis indicates that liver is the major source of MASP-1 and MASP-2 mRNA. Using a 5' specific cDNA probe for MASP-1, major MASP-1 transcript was seen at 4.8 kb and a minor one at approximately 3.4 kb, both present in human and mouse liver (Stover et al., Genes Immunity 4:374-84 (2003)). MASP-2 mRNA (2.6 kb) and MAp19 mRNA (1.0 kb) are abundantly expressed in liver tissue. MASP-3 is expressed in the liver, and also in many other tissues, including neuronal tissue (Lynch N. J. et al., J Immunol 174:4998-5006 (2005)).

A patient with a history of infections and chronic inflammatory disease was found to have a mutated form of MASP-2 that fails to form an active MBL-MASP complex (Stengaard-Pedersen et al., N Engl J Med 349:554-560 (2003)). Some investigators have determined that deficiency of MBL leads to a tendency to frequent infections in childhood (Super et al., Lancet 2:1236-1239 (1989); Garred et al., Lancet 346:941-943 (1995) and a decreased resistance to HIV infection (Nielsen et al., Clin Exp Immunol 100:219-222 (1995); Garred et al., Mol Immunol 33 (suppl 1):8 (1996)). However, other studies have not demonstrated a significant correlation of low MBL levels with increased infections (Egli et al., PLoS One. 8(1):e51983(2013); Ruskamp et al., J Infect Dis. 198(11):1707-13 (2008); Israëls et al., Arch Dis Child Fetal Neonatal Ed. 95(6):F452-61 (2010)). While the literature is mixed, deficiency, or non-utilization, of MASP may have an adverse effect on an individual's ability to mount immediate, non-antibody-dependent defense against certain pathogens.

iii. Supporting data for the new understanding, underscoring traditional assay conditions that are devoid of $Ca^{++}$ and results obtained using a more physiological set of conditions that include $Ca^{++}$.

Several independent lines of strong experimental evidence are provided herein pointing to the conclusion that the lectin pathway activation route of complement activates complement via two independent effector mechanisms: i) LEA-2: a MASP-2-driven path that mediates complement-driven opsonisation, chemotaxis (Schwaeble et al., PNAS 108:7523-7528 (2011)), and cell lysis, and ii) LEA-1: a novel MASP-3-dependent activation route that initiates complement activation by generating the alternative pathway convertase C3bBb through cleavage and activation of factor B on activator surfaces, which will then catalyze C3b deposition and formation of the alternative pathway convertase C3bBb, which can result in cell lysis as well as microbial opsonization. In addition, as described herein, separate lectin-independent activation of factor B and/or factor D by MASP-1, MASP-3, or HTRA-1, or a combination of any the three, can also lead to complement activation via the alternative pathway.

A lectin pathway-dependent MASP-3-driven activation of the alternative pathway appears to contribute to the well-established factor D-mediated cleavage of C3b-bound factor B to achieve optimal activation rates for complement-dependent lysis through the terminal activation cascade to lyse bacterial cells through the formation of C5b-9 membrane attack complexes (MAC) on the cellular surface (**FIGURES 14-15**). This rate-limited event appears to require optimal coordination as it is defective in the absence of MASP-3 functional activity as well as in the absence of factor D functional activity. As described in Examples 1-4 herein, the inventors discovered this MASP-3-dependent lectin pathway function when studying the phenotype of MASP-2 deficiency and MASP-2 inhibition in experimental mouse models of *N. menigitidis* infection. Gene-targeted, MASP-2-deficient mice and wild-type mice treated with antibody-based MASP-2 inhibitors were highly resistant to experimental *N. meningitidis* infection (see **FIGURES 8-12**). When the infectious dose was adjusted to give approximately 60% mortality in the wild-type littermates, all of the MASP-2-deficient or MASP-2-depleted mice cleared the infection and survived (see **FIGURE 8** and **FIGURE 12**). This extremely high degree of resistance was reflected in a significant increase of serum bactericidal activity in MASP-2-deficient or MASP-2-depleted mouse serum. Further experiments showed that this bactericidal activity was dependent on alternative pathway-driven bacterial lysis. Mouse sera deficient of factor B, or factor D, or C3 showed no bactericidal activity towards *N. meningitidis,* indicating that the alternative pathway is essential for driving the terminal activation cascade. A surprising result was that mouse sera deficient of MBL-A and MBL-C (both being the lectin-pathway recognition molecules that recognize *N. meningitidis*) as well as mouse sera deficient of the lectin pathway-associated serine proteases MASP-1 and MASP-3 had lost all bacteriolytic activity towards *N. meningitidis* (**FIGURE 15**). A recent paper (Takahashi M. et al., JEM 207: 29-37 (2010)) and work presented herein (**FIGURE 39**) demonstrate that MASP-1 can convert the zymogen form of factor D into its enzymatically active form and may in part explain the loss of lytic activity through the absence of enzymatically active factor D in these sera. This does not explain the lack of bactericidal activity in MBL-deficient mice since these mice have normal enzymatically active factor D (Banda et al., Mol Imunol 49(1-2):281-9 (2011)). Remarkably, when testing human sera from patients with the rare 3MC autosomal recessive disorder (Rooryck C, et al., Nat Genet. 43(3):197-203) with mutations that render the serine protease domain of MASP-3 dysfunctional, no bactericidal activity against *N. meningitidis* was detectable (n.b.: These sera have MASP-1 and factor D, but no MASP-3).

The hypothesis that human serum requires lectin pathway-mediated MASP-3-dependent activity to develop bactericidal activity is further supported by the observation that MBL-deficient human sera also fail to lyse *N. meningitidis* (**FIGURES 13-14**). MBL is the only human lectin-pathway recognition molecule that binds to this pathogen. Since MASP-3 does not auto-activate, the inventors hypothesize that the higher bacteriolytic activity in MASP-2-deficient sera could be explained by a favored activation of MASP-3 through MASP-1 since, in the absence of MASP-2, all lectin-pathway activation complexes that bind to the bacterial surface will be loaded with either MASP-1 or MASP-3. Since activated MASP-3 cleaves both factor D (**FIGURE 39**) and factor B to generate their respective enzymatically active forms *in vitro* (**FIGURE 37** and Iwaki D., et al., J. Immunol. 187(7):3751-3758 (2011)), the most likely function of MASP-3 is to facilitate the formation of the alternative pathway C3 convertase (i.e., C3bBb).

While the data for the lectin-dependent role are compelling, multiple experiments suggest that MASP-3 and MASP-1 are not necessarily obligated to function in a complex with lectin molecules. Experiments such as that shown in **FIGURE 35B** demonstrate the ability of MASP-3 to activate the alternative pathway (as demonstrated by C3b deposition on *S. aureus*) under conditions (i.e., the presence of EGTA) in which complexes with lectin would not be present. **FIGURE 35A** demonstrates that deposition under these conditions is dependent upon factor B, factor D, and factor P, all critical components of the alternative pathway. Addtionally, factor D activation by MASP-3 and MASP-1 (**FIGURE 39**), and factor B activation by MASP-3 (**FIGURE 37**) can occur *in vitro* in the absence of lectin. Finally, hemolysis studies of mouse erythrocytes in the presence of human serum demonstrate a clear role for both MBL and MASP-3 for cell lysis. However, the deficiency of MBL does not completely reproduce the severity of the deficiency of MASP-3, in contrast to what would be expected if all functional MASP-3 were complexed with MBL. Thus, the inventors do not wish to be constrained by the notion that all of the roles for MASP-3 (and MASP-1) demonstrated herein can be attributed solely to function associated with lectin.

The identification of the two effector arms of the lectin pathway, as well as the possible lectin-independent functions of MASP-1, MASP-3, and HTRA-1, represent novel opportunities for therapeutic interventions to effectively treat defined human pathologies caused by excessive complement activation in the presence of microbial pathogens or altered host cells or metabolic deposits. As described herein, the inventors have now discovered that in the absence of MASP-3 and in the presence of MASP-1, the alternative pathway is not activated on surface structures (see **FIGURES 17-18**, **35B**, **41-42**, **45-46**). Since the alternative pathway is important in driving the rate-limiting events leading to bacterial lysis as well as cell lysis (Mathieson PW, et al., J Exp Med 177(6): 1827-3 (1993)), our results demonstrate that activated MASP-3 plays an important role in the lytic activity of complement. As shown in **FIGURES 14-15, 21-23, 43-44,** and **46-47,** in serum of 3MC patients lacking MASP-3 but not MASP-1, the lytic terminal activation cascade of complement is defective. The data shown in **FIGURES 14** and **15** demonstrate a loss of bacteriolytic activity in absence of MASP-3 and/or MASP-1/MASP-3 functional activity. Likewise, the loss of hemolytic activity in MASP-3-deficient human serum (**FIGURES 21-23, 43-44** and **46-47**), coupled with the ability to reconstitute hemolysis by adding recombinant MASP-3 (**FIGURES 46-47**), strongly supports the conclusion that activation of the alternative pathway on target surfaces (which is essential to drive complement-mediated lysis) depends on the presence of activated MASP-3. Based on the new understanding of the lectin pathway detailed above, alternative pathway activation of target surfaces is thus dependent upon LEA-1, and/or lectin-independent activation of factor B and/or factor D, which is also mediated by MASP-3, and therefore, agents that block MASP-3-dependent complement activation will prevent alternative pathway activation on target surfaces.

The disclosure of the essential role of MASP-3-dependent initiation of alternative pathway activation implies that the alternative pathway is not an independent, stand-alone pathway of complement activation as described in essentially all current medical textbooks and recent review articles on complement. The current and widely held scientific view is that the alternative pathway is activated on the surface of certain particulate targets (microbes, zymosan, and rabbit erythrocytes) through the amplification of spontaneous "tick-over" C3 activation. However, the absence of any alternative pathway activation in sera of MASP-1 and MASP-3 double-deficient mice and human 3MC patient serum on both zymosan-coated plates and two different bacteria (*N. meningitidis* and *S. aureus*), and the reduction of hemolysis of erythrocytes in MASP-3-deficient sera from human and mouse indicate that initiation of alternative pathway activation on these surfaces requires functional MASP-3. The required role for MASP-3 may be either lectin-dependent or - independent, and leads to formation of the alternative pathway C3 convertase and C5 convertase complexes, i.e. C3bBb and C3bBb(C3b)n, respectively. Thus, the inventors here disclose the existence of a previously elusive initiation routes for the alternative pathway. This initiation route is dependent upon (i) LEA-1, a newly discovered activation arm of the lectin pathway, and/or (ii) lectin-independent roles of the proteins MASP-3, MASP-1, and HTRA-1.

**III. THE ROLE OF MASP-2 AND MASP-3 IN PAROXYSMAL NOCTURNAL HEMOGLOBINURIA AND THERAPEUTIC METHODS USING MASP-2 AND MASP-3 INHIBITORY AGENTS**

i. Overview of PNH

Paroxysmal nocturnal hemoglobinuria (PNH), sometimes also referred to as Marchiafava-Micheli syndrome, is an acquired, potentially life-threatening disease of the blood. PNH may develop on its own, referred to as "primary PNH" or in the context of other bone marrow disorders such as aplastic anemia, referred to as "secondary PNH." The majority of cases are primary PNH PNH is characterized by complement-induced destruction of red blood cells (hemolysis), low red blood cell counts (anemia), thrombosis and bone marrow failure. Laboratory findings in PNH show changes consistent with intravascular hemolytic anemia: low hemoglobin, raised lactate dehydrogenase, raised reticulocyte counts (immature red cells released by the bone marrow to replace the destroyed cells), raised bilirubin (a breakdown product of hemoglobin), in the absence of autoreactive RBC-binding antibodies as a possible cause.

The hallmark of PNH is the chronic complement-mediated hemolysis caused by the unregulated activation of terminal complement components, including the membrane attack complex, on the surface of circulating RBCs. PNH RBCs are subject to uncontrolled complement activation and hemolysis due to the absence of the complement regulators CD55 and CD59 on their surface (Lindorfer, M.A., et al., Blood 115(11):2283-91 (2010), Risitano, et al., Mini-Reviews in Medicinal Chemistry, 11:528-535 (2011)). CD55 and CD59 are abundantly expressed on normal RBCs and control complement activation. CD55 acts as a negative regulator of the alternative pathway, inhibiting the assembly of the alternative pathway C3 convertase (C3bBb) complex and accelerating the decay of preformed convertase, thus blocking the formation of the membrane attack complex (MAC). CD59 inhibits the complement membrane attack complex directly by binding the C5b678 complex and preventing C9 from binding and polymerizing.

While hemolysis and anemia are the dominant clinical features of PNH, the disease is a complex hematologic disorder that further includes thrombosis and bone marrow failure as part of the clinical findings (Risitano et al, Mini Reviews in Med Chem11:528-535 (2011)). At the molecular level, PNH is caused by the abnormal clonal expansion of hematopoietic stem cells lacking a functional PIG A gene. PIG A is an X-linked gene encoding a glycosyl-phosphatidyl inositol transferase required for the stable surface expression of GPI-anchored class A glycoproteins, including CD55 and CD59. For reasons that are presently under investigation, hematopoietic stem cells with a dysfunctional PIG A gene that arise as the result of spontaneous somatic mutations can undergo clonal expansion to the point where their progeny constitute a significant portion of the peripheral hematopoietic cell pool. While both erythrocyte and lymphocyte progeny of the mutant stem cell clone lack CD55 and CD59, only the RBCs undergo overt lysis after they enter the circulation.

Current treatment for PNH includes blood transfusion for anemia, anticoagulation for thrombosis and the use of the monoclonal antibody eculizumab (Soliris®), which protects blood cells against immune destruction by inhibiting the complement system (Hillmen P. et al., N. Engl. J. Med. 350(6):552-559 (2004)). Eculizumab (Soliris®) is a humanized monoclonal antibody that targets the complement component C5, blocking its cleavage by C5 convertases, thereby preventing the production of C5a and the assembly of MAC. Treatment of PNH patients with eculizumab has resulted in a reduction of intravascular hemolysis, as measured by lactate dehydrogenase (LDH), leading to hemoglobin stabilization and transfusion independence in about half of the patients (Risitano et al, Mini-Reviews in Medicinal Chemistry, 11(6) (2011)). While nearly all patients undergoing therapy with eculizumab achieve normal or almost normal LDH levels (due to control of intravascular hemolysis), only about one third of the patients reach a hemoglobin value about 11gr/dL, and the remaining patients on eculizumab continue to exhibit moderate to severe (i.e., transfusion-dependent) anemia, in about equal proportions (Risitano A.M. et al., Blood 113:4094-100 (2009)). As described in Risitano et al., Mini-Reviews in Medicinal Chemistry 11 :528-535 (2011), it was demonstrated that PNH patients on eculizumab contained large amounts of C3 fragments bound to their PNH erythrocytes (while untreated patients did not). This finding lead to the recognition that in Soliris treated PNH patients, PNH RBCs that are no longer hemolyzed due to C5 blockade now can accumulate copious amounts of membrane-bound C3 fragments, which operate as opsonins, resulting in their entrapment in the reticuloendothelial cells through specific C3 receptors and subsequent extravascular hemolysis. Thus, while preventing intravascular hemolysis and the resulting sequelae, eculizumab therapy simply diverts the disposition of these RBCs from intravascular to extravascular hemolysis, resulting in substantial residual untreated anemia in many patients (Risitano A.M. et al., Blood 113:4094-100 (2009)). Therefore, therapeutic strategies in addition to the use of eculizumab are needed for those patients developing C3-fragment-mediated extravascular hemolysis, because they continue to require red cell transfusions. Such C3 fragment targeting approaches have demonstrated utility in experimental systems (Lindorfer et al., Blood 115:2283-91, 2010).

ii. Complement-initiating mechanisms in PNH

The causal link between defective surface expression of the negative complement regulators CD55 and CD59 in PNH, combined with the effectiveness of eculizumab in preventing intravascular hemolysis, clearly define PNH as a condition mediated by the complement system. While this paradigm is widely accepted, the nature of the events initiating complement activation, and the complement activation pathway(s) involved remain unresolved. Because CD55 and CD59 negatively regulate the terminal amplification steps in the complement cascade common to all complement initiation pathways, deficiency of these molecules will lead to exaggerated formation and membrane integration of membrane attack complexes, regardless of whether complement activation is initiated by the lectin pathway, by the classical pathway or by spontaneous turnover of the alternative pathway. Thus, in PNH patients, any complement activation events that lead to C3b deposition on the RBC surface can trigger subsequent amplification and pathological hemolysis (intravascular and/or extravascular) and precipitate a hemolytic crisis. A clear mechanistic understanding of the molecular events triggering hemolytic crisis in PNH patients has remained elusive. Because no complement initiating event is overtly evident in PNH patients undergoing a hemolytic crisis, the prevailing view is that complement activation in PNH may occur spontaneously owing to low level "tick-over" activation of the alternative pathway, which is subsequently magnified by inappropriate control of terminal complement activation due to lack of CD55 and CD59.

However, it is important to note that in its natural history, PNH usually develops or is exacerbated after certain events, such as an infection or an injury (Risitano, Biologics 2:205-222 (2008)), which have been shown to trigger complement activation. This complement activation response is not dependent on prior immunity of the host towards the inciting pathogen, and hence likely does not involve the classical pathway. Rather, it appears that this complement activation response is initiated by lectin binding to foreign or "altered self' carbohydrate patterns expressed on the surface of microbial agents or damaged host tissue. Thus, the events precipitating hemolytic crisis in PNH are tightly linked to complement activation initiated via lectins. This makes it very likely that lectin activation pathways provide the initiating trigger that ultimately leads to hemolysis in PNH patients.

Using well-defined pathogens that activate complement via lectins as experimental models to dissect the activation cascades at the molecular level, we demonstrate that, depending on the inciting microbe, complement activation can be initiated by either LEA-2 or LEA-1, leading to opsonization and/or lysis. This same principle of dual responses (i.e., opsonization and/or lysis) to lectin initiation events will likely also apply to other types of infectious agents, or to complement activation by lectins following tissue injury to the host, or other lectin-driven complement activation events that may precipitate PNH On the basis of this duality in the lectin pathway, we infer that LEA-2- and/or LEA-1-initiated complement activation in PNH patients promotes opsonization and/or lysis of RBCs with C3b and subsequent extravascular and intravascular hemolysis. Therefore, in the setting of PNH, inhibition of both LEA-1 and LEA-2 would be expected to address both intravascular and extravascular hemolysis, providing a significant advantage over the C5 inhibitor eculizumab.

It has been determined that exposure to *S. pneumoniae* preferentially triggers lectin-dependent activation of LEA-2, which leads to opsonization of this microbe with C3b. Since *S. pneumonia* is resistant to MAC-mediated lysis, its clearance from circulation occurs through opsonisation with C3b. This opsonization and subsequent removal from circulation is LEA-2-dependent, as indicated by compromised bacterial control in MASP-2-deficient mice and in mice treated with MASP-2 monoclonal antibodies (PLOS Pathog., 8: e1002793. (2012)).

In exploring the role of LEA-2 in the innate host responses to microbial agents, we tested additional pathogens. A dramatically different outcome was observed when *Neisseria meningitidis* was studied as a model organism. *N. meningitidis* also activates complement via lectins, and complement activation is required for containment of *N. meningitidis* infections in the naive host. However, LEA-2 plays no host protective functional role in this response: As shown in **FIGURES 8** and **9**, blockade of LEA-2 through genetic ablation of MASP-2 does not reduce survival following infection with *N. meningitidis.* To the contrary, LEA-2 blockade by MASP-2 ablation significantly improved survival (**FIGURES 8** and **9**) as well as illness scores (**FIGURE 11**) in these studies. LEA-2 blockade by administration of MASP-2 antibody yielded the same result (**FIGURE 12**), eliminating secondary or compensatory effects in the knockout-mouse strain as a possible cause. These favorable outcomes in LEA-2-ablated animals were associated with a more rapid elimination of *N. meningitidis* from the blood (**FIGURE 10**). Also, as described herein, incubation of *N. meningitidis* with normal human serum killed *N. meningitidis* (**FIGURE 13**). Addition of a functional monoclonal antibody specific for human MASP-2 that blocks LEA-2, but not administration of an isotype control monoclonal antibody, may enhance this killing response. Yet, this process depends on lectins and at least a partially functional complement system, as MBL-deficient human serum or heat-inactivated human serum was unable to kill *N. meningitidis* (**FIGURE 13**). Collectively, these novel findings suggest that *N. meningitidis* infections in the presence of a functional complement system are controlled by a lectin-dependent but LEA-2-independent pathway of complement activation.

The hypothesis that LEA-1 may be the complement pathway responsible for lectin-dependent killing of *N. meningitidis* was tested using a serum specimen from a 3MC patient. This patient was homozygous for a nonsense mutation in exon 12 of the MASP-1/3 gene. As a result, this patient lacked a functional MASP-3 protein, but was otherwise complement sufficient (exon 12 is specific for the MASP-3 transcript; the mutation has no effect on MASP-1 function or expression levels) (see Nat Genet 43(3):197-203 (2011)). Normal human serum efficiently kills *N. meningitidis,* but heat-inactivated serum deficient in MBL (one of the recognition molecules for the Lectin pathway) and MASP-3-deficient serum were unable to kill *N. meningitidis* (**FIGURE 14**). Thus, LEA-1 appears to mediate *N. meningitidis* killing. This finding was confirmed using serum samples from knockout mouse strains. While complement containing normal mouse serum readily killed *N. meningitidis,* MBL-deficient or MASP-1/3-deficient mouse serum was as ineffective as heat-inactivated serum that lacks functional complement **(FIGURE 15)**. Conversely, MASP-2-deficient serum exhibited efficient killing of *N. meningitidis.*

These findings provide evidence for a hitherto unknown duality in the lectin pathway by revealing the existence of separate LEA-2 and LEA-1 pathways of lectin-dependent complement activation. In the examples detailed above, LEA-2 and LEA-1 are non-redundant and mediate distinct, functional outcomes. The data suggest that certain types of lectin pathway activators (including, but not limited to *S. pneumonia*) preferentially initiate complement activation via LEA-2 leading to opsonization, while others (exemplified by *N. meningitidis*) preferentially initiate complement activation via LEA-1 and promote cytolytic processes. The data do not, however, necessarily limit LEA-2 to opsonization and LEA-1 to cytolytic processes, as both pathways in other settings can mediate opsonization and/or lysis.

In the context of lectin-dependent complement activation by *N. meningitidis,* LEA-2 and LEA-1 arms appear to compete with each other, as blockade of LEA-2 enhanced LEA-1-dependent lytic destruction of the organism *in vitro* (**FIGURE 15**). As detailed above, this finding can be explained by the increased likelihood of lectin MASP-1 complexes residing in close proximity to lectin MASP-3 complexes in the absence of MASP-2, which will enhance LEA-1 activation and thus promote more effective lysis of *N. meningitides.* Because lysis of *N. meningitidis* is the main protective mechanism in the naive host, blockade of LEA-2 *in vivo* increases *N. meningitidis* clearance and leads to enhanced killing.

While the examples discussed above illustrate opposing effects of LEA-2 and LEA-1 with respect to outcomes following infection with *N. meningitidis,* there may be other settings where both LEA-2 and LEA-1 may synergize to produce a certain outcome. As detailed below, in other situations of pathological complement activation via lectins such as those present in PNH, LEA-2- and LEA-1-driven complement activation may cooperate in a synergistic manner to contribute to the overall pathology of PNH In addition, as described herein, MASP-3 also contributes to the lectin-independent conversion of factor B and factor D, which can occur in the absence of $Ca^{++}$, commonly leading to the conversion of C3bB to C3bBb and of pro-factor D to factor D, which may further contribute to the pathology of PNH

### iii. Biology and expected functional activity in PNH

This section describes the inhibitory effects of LEA-2 and LEA-1 blockade on hemolysis in an *in vitro* model of PNH. The findings support the utility of LEA-2-blocking agents (including, but not limited to, antibodies that bind to and block the function of MASP-2) and LEA-1-blocking agents (including, but not limited to, antibodies that bind to and block the function of MASP-1-mediated activation of MASP-3, MASP-3, or both) to treat subjects suffering from one or more aspects of PNH, and also the use of inhibitors of LEA-2 and/or LEA-1, and/or MASP-3-dependent, lectin-independent complement activation (including MASP-2 inhibitors, MASP-3 inhibitors, and dual- or bispecific MASP-2/MASP-3 or MASP-1/MASP-2 inhibitors, and pan-specific MASP-1/MASP-2/MASP-3 inhibitors) to ameliorate the effects of C3-fragment-mediated extravascular hemolysis in PNH patients undergoing therapy with a C5-inhibitor such as eculizumab.

### iv. MASP-2 inhibitors to block opsonization and extravascular hemolysis of PNH RBCs through the reticuloendothelial system

As detailed above, PNH patients become anemic owing to two distinct mechanisms of RBC clearance from circulation: intravascular hemolysis via activation of the membrane attack complex (MAC), and extravascular hemolysis following opsonization with C3b and subsequent clearance following complement receptor binding and uptake by the reticuloendothelial system. The intravascular hemolysis is largely prevented when a patient is treated with eculizumab. Because eculizumab blocks the terminal lytic effector mechanism that occurs downstream of both the complement-initiating activation event as well as the ensuing opsonization, eculizumab does not block extravascular hemolysis (Risitano A.M. et al., Blood 113:4094-100 (2009)). Instead, RBCs that would have undergone hemolysis in untreated PNH patients now can accumulate activated C3b proteins on their surface, which augments uptake by the reticuloendothelial system and enhances their extravascular hemolysis. Thus, eculizumab treatment effectively diverts RBC disposition from intravascular hemolysis to potential extravascular hemolysis. As a result, some eculizumab-treated PNH patients remain anemic. It follows that agents that block complement activation upstream and prevent the opsonization of PNH RBCs may be particularly suitable to block the extravascular hemolysis occasionally seen with eculizumab.

The microbial data presented here suggest that LEA-2 is often the dominant route for lectin-dependent opsonization. Furthermore, when lectin-dependent opsonization (measured as C3b deposition) was assessed on three prototypic lectin activation surfaces (mannan, **FIGURE 19A**; zymosan, **FIGURE 19B,** and *S. pneumonia;* **FIGURE 19C),** LEA-2 appears to be the dominant route for lectin-dependent opsonization under physiologic conditions (i.e., in the presence of $Ca^{++}$ wherein all complement pathways are operational). Under these experimental conditions, MASP-2-deficient serum (which lacks LEA-2) is substantially less effective in opsonizing the test surfaces than WT serum. MASP-1/3-deficient serum (which lacks LEA-1) is also compromised, though this effect is much less pronounced as compared to serum lacking LEA-2. The relative magnitude of the contributions of LEA-2 and LEA-1 to lectin-driven opsonization is further illustrated in **FIGURES 20A** - **20C.** While the alternative pathway of complement has been reported to support opsonization of lectin activating surfaces in the absence of lectin pathway or classical pathway (Selander et al., J Clin Invest 116(5):1425-1434 (2006)), the alternative pathway in isolation (measured under $Ca^{++}$-free assay conditions) appears substantially less effective than the LEA-2- and LEA-1-initiated processes described herein. By extrapolation, these data suggest that opsonization of PNH RBCs may also be preferentially initiated by LEA-2 and, to a lesser extent, by LEA-1 (possibly amplified by the alternative pathway amplification loop), rather than the result of lectin-independent alternative pathway activation. Therefore, LEA-2 inhibitors may be expected to be most effective at limiting opsonization and preventing extravascular hemolysis in PNH However, recognition of the fact that lectins other than MBL, such as ficolins, bind to non-carbohydrate structures such as acetylated proteins, and that MASP-3 preferentially associates with H-ficolin (Skjoedt et al., Immunobiol. 215:921-931, 2010), leaves open the possibility of a significant role for LEA-1 in PNH-associated RBC opsonization as well. Therefore, LEA-1 inhibitors are expected to have additional anti-opsonization effects, and the combination of LEA-1 and LEA-2 inhibitors is expected to be optimal and mediate the most robust treatment benefit in limiting opsonization and extravascular hemolysis in PNH patients. This concept is further supported by opsonization data shown in **FIGURE 28**: factor D-deficient mouse serum (which lacks the ability to activate the alternative pathway in fluid phase but has a functional classical pathway as well as functional LEA-1 and LEA-2 pathways) shows no deficit in opsonization compared to WT serum. Factor B-deficient serum, which lacks LEA-1, shows reduced opsonization while factor D-deficient serum treated with MASP-2 monoclonal antibody to block LEA-2-mediated complement activation yields a more robust suppression of opsonization **(FIGURE 28)**. Importantly, addition of MASP-2 monoclonal antibody to factor B-deficient serum suppressed opsonization more effectively than either MASP-2 blockade or factor D blockade alone. Thus, LEA-2 and LEA-1 act additively or synergistically to promote opsonization, and a crossreactive or bispecific LEA-1/LEA-2 inhibitor is expected to be most effective at blocking opsonization and extravascular hemolysis in PNH

v. Role of MASP-3 inhibitors in PNH

Using an *in vitro* model of PNH, we demonstrated that complement activation and the resulting hemolysis in PNH are indeed initiated by LEA-2 and/or LEA-1 activation, and that it is not an independent function of the alternative pathway. These studies used mannan-sensitized RBCs of various mouse stains, including RBCs from Crry-deficient mice (an important negative regulator of the terminal complement pathway in mice) as well as RBCs from CD55/CD59-deficient mice, which lack the same complement regulators that are absent in PNH patients). When mannan-sensitized Crry-deficient RBCs were exposed to complement-sufficient human serum, the RBCs effectively hemolysed at a serum concentration of 3% **(FIGURE 21 and 22)** while complement-deficient serum (HI: heat-inactivated) was not hemolytic. Remarkably, complement-sufficient serum where LEA-2 was blocked by addition of MASP-2 antibody had reduced hemolytic activity, and 6% serum was needed for effective hemolysis. Similar observations were made when CD55/CD59-deficient RBCs were tested **(FIGURE 24)**. Complement-sufficient human serum supplemented with MASP-2 monoclonal antibody (i.e., serum where LEA-2 is suppressed) was about two-fold less effective than untreated serum in supporting hemolysis. Furthermore, higher concentrations of LEA-2-blocked serum (i.e., treated with antiMASP-2 monoclonal antibody) were needed to promote effective hemolysis of untreated WT RBCs compared to untreated serum **(FIGURE 23)**.
Even more surprisingly, serum from a 3MC patient homozygous for a dysfunctional MASP-3 protein (and hence lacking LEA-1) was completely unable to hemolyze mannan-sensitized Crry-deficient RBCs **(FIGURE 22** and **FIGURE 23)**. A similar outcome was observed when unsensitized normal RBCs were used: As shown in **FIGURE 23,** LEA-1-defective serum isolated from a 3MC patient was completely ineffective at mediating hemolysis. Collectively, these data indicate that whereas LEA-2 contributes significantly to the intravascular hemolysis response, LEA-1 is the predominant complement-initiating pathway leading to hemolysis. Thus, while LEA-2 blocking agents are expected to significantly reduce intravascular hemolysis of RBCs in PNH patients, LEA-1 blocking agents are expected to have a more profound effect and largely eliminate complement-driven hemolysis.
It should be noted that the serum of the LEA-1-deficient 3MC patient used in this study possessed a diminished but functional alternative pathway when tested under conventional alternative pathway assay conditions **(FIGURE 17)**. This finding suggests that LEA-1 makes a greater contribution to hemolysis than alternative pathway activity as conventionally defined in this experimental setting of PNH. By inference, it is implied that LEA-1-blocking agents will be at least as effective as agents blocking other aspects of the alternative pathway in preventing or treating intravascular hemolysis in PNH patients.

vi. Role of MASP-2 inhibitors in PNH

The data presented herein suggest the following pathogenic mechanisms for anemia in PNH: intravascular hemolysis due to unregulated activation of terminal complement components and lysis of RBC by formation of MAC, which is initiated predominantly, though not exclusively, by LEA-1, and extravascular hemolysis caused by opsonization of RBCs by C3b, which appears to be initiated predominately by LEA-2. While a discernible role for LEA-2 in initiating complement activation and promoting MAC formation and hemolysis is apparent, this process appears substantially less effective than LEA-1-initiated complement activation leading to hemolysis. Thus, LEA-2-blocking agents are expected to significantly reduce intravascular hemolysis in PNH patients, though this therapeutic activity is expected to be only partial. By comparison, a more substantial reduction in intravascular hemolysis in PNH patients is expected for LEA-1-blocking agents.

Extravascular hemolysis, a less dramatic, yet equally important mechanism of RBC destruction that leads to anemia in PNH, is primarily the result of opsonization by C3b, which appears to be predominantly mediated by LEA-2. Thus, LEA-2-blocking agents may be expected to preferentially block RBC opsonization and the ensuing extravascular hemolysis in PNH. This unique therapeutic activity of LEA-2-blocking agents is expected to provide a significant treatment benefit to all PNH patients as no treatment currently exists for those PNH patients who experience this pathogenic process.

vii. LEA-2 inhibitors as adjunct treatment to LEA-1 inhibitors or terminal complement blocking agents

The data presented herein detail two pathogenic mechanisms for RBC clearance and anemia in PNH which can be targeted, separately or in combination, by distinct classes of therapeutic agents: the intravascular hemolysis initiated predominantly, though not exclusively, by LEA-1 and thus expected to be effectively prevented by a LEA-1-blocking agent, and extravascular hemolysis due to C3b opsonization driven predominantly by LEA-2, and thus effectively prevented by a LEA-2-blocking agent.

It is well documented that both intravascular and extravascular mechanisms of hemolysis lead to anemia in PNH patients (Risitano et al., Blood 113:4094-4100 (2009)). Therefore, it is expected that a LEA-1-blocking agent that prevents intravascular hemolysis in combination with a LEA-2 blocking agent that primarily prevents extravascular hemolysis will be more effective than either agent alone in preventing the anemia that develops in PNH patients. In fact, the combination of LEA-1- and LEA-2-blocking agents is expected to prevent all relevant mechanisms of complement initiation in PNH and thus block all symptoms of anemia in PNH

It is also known that C5-blocking agents (such as eculizumab) effectively block intravascular hemolysis but do not interfere with opsonization. This leaves some anti-C5-treated PNH patients with substantial residual anemia due to extravascular hemolysis mediated by LEA-2 that remains untreated. Therefore, it is expected that a C5-blocking agent (such as eculizumab) that prevents intravascular hemolysis in combination with a LEA-2 blocking agent that reduces extravascular hemolysis will be more effective than either agent alone in preventing the anemia that develops in PNH patients.

Other agents that block the terminal amplification loop of the complement system leading to C5 activation and MAC deposition (including, but not limited to agents that block properdin, factor B or factor D or enhance the inhibitory activity of factor I, factor H or other complement inhibitory factors) are also expected to inhibit intravascular hemolysis. However, these agents are not expected to interfere with LEA-2-mediated opsonization in PNH patients. This leaves some PNH patients treated with such agents with substantial residual anemia due to extravascular hemolysis mediated by LEA-2 that remains untreated. Therefore, it is expected that treatment with such agents that prevent intravascular hemolysis in combination with a LEA-2-blocking agent that minimizes extravascular hemolysis will be more effective than either agent alone in preventing the anemia that develops in PNH patients. In fact, the combination of such agents and a LEA-2 blocking agent is expected to prevent all relevant mechanisms of RBC destruction in PNH and thus block all symptoms of anemia in PNH

viii. Use of LEA-1 and LEA-2 multiple, bispecific or pan-specific antibodies to treat PNH

As detailed above, the use of a combination of pharmacologic agents that individually block LEA-1 and LEA-2, and thus in combination block all complement activation events that mediate the intravascular as well as the extravascular hemolysis, is expected to provide the best clinical outcome for PNH patients. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity will effectively block intravascular as well as the extravascular hemolysis and prevent anemia in PNH. Such an entity may comprise or consist of a bispecific antibody where one antigen-combining site specifically recognizes MASP-1 and blocks LEA-1 and diminishes LEA-2 and the second antigen-combining site specifically recognizes MASP-2 and further blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 and diminishes LEA-2 while the second antigen-combining site specifically recognized MASP-2 and further blocks LEA-2. Based on the similarities in the overall protein sequence and architecture, it can also be envisioned that a conventional antibody with two identical binding sites can be developed that specifically binds to MASP-1 and to MASP-2 and to MASP-3 in a functional manner, thus achieving functional blockade of LEA-1 and LEA-2. Such an antibody with pan-MASP inhibitory activity is expected to block both the intravascular as well as the extravascular hemolysis and thus effectively treat the anemia in PNH patients.

## IV. THE ROLE OF MASP-2 AND MASP-3 IN AGE-RELATED MACULAR DEGENERATION AND THERAPEUTIC METHODS USING MASP-2 AND MASP-3 INHIBITORY AGENTS

Age-related macular degeneration (AMD) is the leading cause of visual impairment and blindness in the elderly and accounts for up to 50% of cases of blindness in developed countries. The prevalence of AMD is around 3% in adults and increases with age such that almost two-thirds of the population over 80 years of age will have some signs. It is estimated that over 1.75 million individuals in the United States have advanced AMD and the prevalence is increasing as the population ages and is expected to reach almost 3 million by 2020 (Friedman, D.S., et al., Arch. Ophthalmol. 122:564-572, 2004). AMD is an abnormality of the retinal pigment epithelium (RPE) that results in degeneration of the photoreceptors of the overlying central retina, or macula, and loss of central vision. Early and intermediate forms of AMD are characterized by progressive deposits of drusen, a yellowish material containing lipid, protein, lipoprotein, and cellular debris, in the subretinal space adjacent to the RPE, along with pigmentary irregularities in the retina. Advanced AMD consists of two clinical subtypes: non-neovascular geographic atrophic ('dry') AMD and neovascular exudative ('wet') AMD. Although dry AMD accounts for 80-90% of advanced AMD, the majority of sudden and severe vision loss occurs in patients with wet AMD It is not known whether the two types of AMD represent differing phenotypes arising from similar pathologies or two distinct conditions. Currently no therapy has been approved by the United States Food and Drug Administration (FDA) to treat dry AMD FDA-approved treatment options for wet AMD include intravitreal injections of anti-angiogenic drugs (ranibizumab, pegaptanib sodium, aflibercept), laser therapy, photodynamic laser therapy, and implantable telescope.

The etiology and pathophysiology of AMD are complex and incompletely understood. Several lines of evidence support the role of dysregulation of the complement system in the pathogenesis of AMD Gene association studies have identified multiple genetic loci associated with AMD, including genes coding for a range of complement proteins, factors, and regulators. The strongest association is with polymorphisms in the complement factor H (CFH) gene, with the Y402H variant homozygotes having approximately 6-fold and heterozygotes approximately 2.5-fold increased risk for developing AMD compared to the non-risk genotype (Khandhadia, S., et al., Immunobiol. 217:127-146, 2012). Mutations in other complement pathway encoding genes have also been associated with increased or decreased risk of AMD, including complement factor B (CFB), C2, C3, factor I, and CFH-related proteins 1 and 3 (Khandhadia et al.). Immunohistochemical and proteomic studies in donor eyes from AMD patients showed that proteins of the complement cascade to be increased and localized in drusen (Issa, P.C., et al., Graefes. Arch. Clin. Exp. Ophthalmol. 249:163-174, 2011). Furthermore, AMD patients have increased systemic complement activation as measured in peripheral blood (Issa et al., 2011, supra).

The alternative pathway of complement appears to be more relevant than the classical pathway in the pathogenesis of AMD. C1q, the essential recognition component for activation of the classical pathway, was not detected in drusen by immunohistochemical analyses (Mullins et al., FASEB J. 14:835-846, 2000; Johnson et al., Exp. Eye Res. 70:441-449, 2000). Genetic association studies have implicated CFH and CFB genes. These proteins are involved in the alternative pathway amplification loop, with CFH being a fluid phase inhibitor and CFB being an activating protease component of the alternative pathway. The Y402H variant of CFH affects interaction with ligand binding, including binding with C-reactive protein, heparin, M protein, and glycosaminoglycans. This altered binding to ligands may reduce binding to cell surfaces, which in turn may lead to reduced factor I mediated degradation of C3b activation fragment and impaired regulation of the alternative C3 convertase, resulting in over activation of the alternative pathway (Khandhadia et al., 2012, supra). Variations in the CFB gene are associated with a protective effect for the development of AMD. A functional variant fB32Q had 4 times less binding affinity to C3b than the risk variant fB32R, resulting in a reduction in C3 convertase formation (Montes, T. et al., Proc. Natl. Acad. Sci. U.S.A. 106:4366-4371, 2009).

## Complement-initiating mechanisms in AMD

The human genetic linkage studies discussed above suggest an important role for the complement system in AMD pathogenesis. Furthermore, complement activation products are abundantly present in drusen (Issa, P.C., et al., Graefes. Arch. Clin. Exp. Ophthalmol. 249:163-174, 2011), a hallmark pathologic lesion in both wet and dry AMD However, the nature of the events initiating complement activation, and the complement activation pathway(s) involved remain incompletely understood.

It is important to note that drusen deposits are composed of cellular debris and oxidative waste products originating from the retina that accumulate beneath the RPE as the eye ages. In addition, oxidative stress appears to play an important role (Cai et al; Front Biosci., 17:1976-95, 2012), and has been shown to cause complement activation in RPE (J Biol Chem., 284(25):16939-47, 2009). It is widely appreciated that both oxidative stress and cellular or tissue injury activate the complement system lectins. For example, Collard et al. have demonstrated that endothelial cells exposed to oxidative stress trigger abundant complement deposition mediated by lectins (Collard CD et al., Mol Immunol., 36(13-14):941-8, 1999; Collard C.D. et al., Am J Pathol., 156(5):1549-56, 2000), and that blockade of lectin binding and lectin-dependent complement activation improves outcomes in experimental models of oxidative stress injury (Collard C.D. et al., Am J Pathol., 156(5):1549-56, 2000). Thus, it appears likely that oxidative waste products present in drusen also activate complement via the lectins. By inference, lectin-dependent complement activation may play a pivotal role in AMD pathogenesis.

The role of the complement system has been evaluated in mouse models of AMD In the light-damage mouse model, an experimental model for oxidative stress-mediated photoreceptor degeneration, knockout mice with an elimination of the classical pathway (C1q$\alpha^{-/-}$ on a C57BL/6 background) had the same sensitivity to light damage compared to wild-type littermates, whereas elimination of complement factor D of the alternative pathway (CFD$^{-/-}$) resulted in protection from light damage (Rohrer, B. et al., Invest. Ophthalmol. Vis. Sci. 48:5282-5289, 2007). In a mouse model of choroidal neovascularization (CNV) induced by laser photocoagulation of the Bruch membrane, knockout mice without complement Factor B (CFB$^{-/-}$) were protected against CNV compared with wild-type mice (Rohrer, B. et al., Invest. Ophthalmol. Vis. Sci. 50:3056-3064, 2009). In the same model, intravenous administration of a recombinant form of complement Factor H targeted to sites of complement activation (CR2-fH) reduced the extent of CNV. This protective effect was observed whether CR2-fH was administered at the time of laser injury or therapeutically (after laser injury). A human therapeutic version of CR2-fH (TT30) was also efficacious in the murine CNV model (Rohrer, B. et al. J. Ocul. Pharmacol. Ther.,28:402-409, 2012). Because fB is activated by LEA-1, and because MASP-1 and MASP-3 contribute to the maturation of factor D, these findings imply that LEA-1 inhibitors may have therapeutic benefit in AMD patients.

Initial experimental studies in a rodent model of AMD using MBL-deficient mice did not support a critical role for the lectin pathway in pathogenic complement activation (Rohrer et al., Mol Immunol. 48:e1-8, 2011). However, MBL is only one of several lectins, and lectins other than MBL may trigger complement activation in AMD. Indeed, our previous work has shown that MASP-2, the rate-limiting serine protease that is critically required for lectin pathway function, plays a critical role in AMD As described in US Patent No. 7,919,094 (assigned to Omeros Corporation), incorporated herein by reference, and in Examples 20 and 21 herein, MASP-2-deficient mice and mice treated with MASP-2 antibody were protected in a mouse model of laser-induced CNV, a validated preclinical model of wet AMD (Ryan et al., Tr Am Opth Soc LXXVII:707-745, 1979). Thus, inhibitors of LEA-2 are expected to effectively prevent CNV and improve outcomes in AMD patients.

Thus, in view of the above, LEA-1 and LEA-2 inhibitors are expected to have independent therapeutic benefit in AMD. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bispecific antibody composed of MASP-1/3 and a MASP-2-specific binding site, or a dual specificity antibody where each binding site can bind to and block MASP-1/3 or MASP-2

In accordance with the foregoing, an aspect of the invention thus provides a method for inhibiting LEA-1-dependent complement activation to treat age-related macular degeneration (wet and dry forms) by administering a composition comprising a therapeutically effective amount of a MASP-1 inhibitory agent, a MASP-3 inhibitory agent, or a combination of a MASP-1/3 inhibitory agent, in a pharmaceutical carrier to a subject suffering from such a condition. The MASP-1, MASP-3, or MASP-1/3 inhibitory composition may be administered locally to the eye, such as by irrigation, intravitreal administration, or application of the composition in the form of a gel, salve or drops. Alternately, the MASP-1, MASP-3, or MASP-1/3 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In one embodiment, the method according to this aspect of the invention further comprises inhibiting LEA-2-dependent complement activation in a subject suffering from age-related macular degeneration, comprising administering a therapeutically effective amount of a MASP-2 inhibitory agent and a MASP-1, MASP-3 or MASP1/3 inhibitory agent to the subject in need thereof. As detailed above, the use of a combination of pharmacologic agents that individually block LEA-1 and LEA-2 is expected to provide an improved therapeutic outcome in AMD patients as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory composition may be administered locally to the eye, such as by irrigation, intravitreal injection or topical application of the composition in the form of a gel, salve or drops. Alternately, the MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual inhibitory agents, or co-administration of separate compositions), or a limited sequence of administrations, for treatment of AMD Alternatively, the composition may be administered at periodic intervals such as daily, biweekly, weekly, every other week, monthly or bimonthly over an extended period of time for treatment of AMD

V. THE ROLE OF MASP-2 AND MASP-3 IN ISCHEMIA REPERFUSION INJURY AND THERAPEUTIC METHODS USING MASP-2 AND MASP-3 INHIBITORY AGENTS

Tissue ischemia is the basis for a wide spectrum of clinical disorders. Although timely restoration of blood flow is essential to preservation of ischemic tissue, it has long been recognized that reperfusion, which can occur either spontaneously or through therapeutic intervention, may lead to additional tissue injury, a phenomenon that has been termed ischemia reperfusion (I/R) injury (Eltzschig, H.K. and Tobias, E., Nat. Med. 17:1391-1401, 2011). I/R injury may affect single organs, such as the heart (acute coronary syndrome), kidney (acute kidney injury), intestine (intestinal I/R), and brain (stroke). I/R injury may also affect multiple organs, such as following major trauma and resuscitation (multiple organ failure), circulatory arrest (hypoxic brain injury, acute kidney injury), peripheral vascular disease, and sickle cell disease (acute chest syndrome, acute kidney injury). Major surgery may be associated with I/R injury, including cardiac surgery (acute heart failure after cardiopulmonary bypass), thoracic surgery (acute lung injury), peripheral vascular surgery (compartment syndrome), vascular surgery (acute kidney injury), and solid organ transplantation (acute graft failure). Currently there are no specific therapies that target I/R injury and there is a need for effective treatments in order to maximize the salvage of tissue in the ischemic zone and improve functional outcome in these common settings.

The pathophysiology of I/R injury is complex and characterized by a robust inflammatory response following reperfusion. Activation of the complement system has been implicated as an important component of I/R injury and inhibition of complement activity has been efficacious in a variety of animal models (Diepenhorst, G.M.P., et al., Ann. Surg. 249:889-899, 2009). The relative importance of the classical, lectin, and alternative pathways in I/R injury is largely unsettled and may differ depending on the organs affected. Recently the availability of knockout mice deficient in specific complement proteins and pathway-specific inhibitors has generated data that implicate the lectin and alternative pathways in I/R injury.

The role of the alternative pathway in gastrointestinal I/R injury was investigated using factor D-deficient (-/-) and heterozygotus (+/-) mice (Stahl, G.L., et al. Am. J. Pathol. 162:449-455, 2003). Following transient gastrointestinal ischemia, intestinal and pulmonary injury were reduced but not prevented in factor D-deficient mice compared with heterozygotus mice, and addition of human factor D to -/- mice restored I/R injury. The same model was evaluated in C1q-deficient and MBL-A/C-deficient mice and the results showed that gastrointestinal I/R injury was independent of C1q and classical pathway activation, but that MBL and lectin pathway activation was required for intestinal injury (Hart, M.L., et al. J. Immunol. 174:6373-6380, 2005). Conversely, the C1q recognition molecule of the classical pathway was responsible for pulmonary injury after intestinal I/R (Hart, M.L., et al. J. Immunol. 174:6373-6380, 2005). One hypothesis is that activation of complement during I/R injury occurs through natural IgM binding to self-antigens present on the surface of ischemic (but not normal) tissue, for example non-muscle myosin heavy chains type II. In a mouse gastrointestinal I/R injury model, immunocomplexes from gut tissue were evaluated for the presence of initiating factors in the classical (C1q), lectin (MBL), or alternative (Factor B) pathways (Lee, H., et al., Mol. Immunol. 47:972-981, 2010). The results showed that C1q and MBL were detected whereas Factor B was not detected in these immunocomplexes, indicating involvement of the classical and lectin pathways but not the alternative pathway. In the same model, Factor B-deficient mice were not protected from local tissue injury, providing additional support for the lack of involvement of the alternative pathway. The role of the lectin pathway in gastrointestinal I/R injury was directly evaluated in MASP-2-deficient mice and the results showed that gastrointestinal injury was reduced in these mice compared with wide-type controls; treatment with MASP-2 monoclonal antibody was similarly protective (Schwaeble, W.J., et al., Proc. Natl. Acad. Sci. 108:7523-7528, 2011), see also Example 23 herein. Taken together, these results provide support for the involvement of the lectin pathway in gastrointestinal I/R injury, with conflicting data regarding involvement of the alternative pathway.

In a mouse myocardial I/R injury model, a pathogenic role was demonstrated for the lectin pathway as MBL-deficient mice were protected from myocardial injury whereas C1q-deficient and C2/fB-deficient mice were not (Walsh, M.C. et al., J. Immunol. 175:541-546, 2005). Protection from myocardial I/R injury was also observed in MASP-2-deficient mice (Schwaeble, W.J., et al., Proc. Natl. Acad. Sci. 108:7523-7528, 2011); see also Examples 22 and 23 herein. Treatment of rats in a myocardial I/R model with monoclonal antibodies against rat MBL resulted in reduced postischemic reperfusion injury (Jordan, J.E., et al., Circulation 104:1413-18, 2001). In a study of myocardial infarction patients treated with angioplasty, MBL deficiency was associated with reduced 90-day mortality compared to MBL-sufficient counterparts (M Trendelenburg et al., Eur Heart J. 31:1181, 2010). Furthermore, myocardial infarction patients that develop cardiac dysfunction after angioplasty have approximately ~ three fold higher MBL levels compared to patients with functional recovery (Haahr-Pedersen S., et al., J Inv Cardiology, 21:13, 2009). MBL antibodies also reduced complement deposition on endothelial cells *in vitro* after oxidative stress indicating a role for the lectin pathway in myocardial I/R injury (Collard, C.D., et al., Am. J. Pathol. 156:1549-56, 2000). In a mouse heterotopic isograft heart transplant model of I/R injury, the role of the alternative pathway was investigated using the pathway-specific fusion protein CR2-fH (Atkinson, C., et al., J. Immunol. 185:7007-7013, 2010). Systemic administration of CR2-fH immediately posttransplantation resulted in a reduction in myocardial I/R injury to an extent comparable to treatment with CR2-Crry, which inhibits all complement pathways, indicating that the alternative pathway is of key importance in this model.

In a mouse model of renal I/R injury, the alternative pathway was implicated as factor B-deficient mice were protected from a decline in renal function and tubular injury, compared with wild-type mice (Thurman, J.M., et al., J. Immunol. 170:1517-1523, 2003). Treatment with an inhibitory monoclonal antibody to factor B prevented complement activation and reduced murine renal I/R injury (Thurman, J.M., et al., J. Am. Soc. Nephrol. 17:707-715, 2006). In a bilateral renal I/R injury model, MBL-A/C-deficient mice were protected from kidney damage compared with wild-type mice and recombinant human MBL reversed the protective effect in MBL-A/C-deficient mice, implicating a role for MBL in this model (Moller-Kristensen, M., et al., Scand. J. Immunol. 61:426-434, 2005). In a rat unilateral renal I/R injury model, inhibition of MBL with a monoclonal antibody to MBL-A preserved renal function after I/R (van der Pol, P., et al., Am. J. Transplant. 12:877-887, 2010). Interestingly, the role of MBL in this model did not appear to involve activation of the terminal complement components, as treatment with a C5 antibody was ineffective in preventing renal injury. Rather, MBL appeared to have a direct toxic effect on tubular cells, as human proximal tubular cells incubated with MBL *in vitro* internalized MBL with subsequent cellular apoptosis. In a swine model of renal I/R, Castellano G. et al., (Am J Pathol, 176(4):1648-59, 2010), tested a C1 inhibitor, which irreversibly inactivates C1r and C1s proteases in the classical pathway and also MASP-1 and MASP-2 proteases in MBL complexes of the lectin pathway, and found that C1 inhibitor reduced complement deposition in peritubular capillaries and glomerulus and reduced tubular damage.

The alternative pathway appears to be involved in experimental traumatic brain injury as factor B-deficient mice had reduced systemic complement activation as measured by serum C5a levels and reduced posttraumatic neuronal cell death compared with wide-type mice (Leinhase, I., et al., BMC Neurosci. 7:55-67, 2006). In human stroke, complement components C1q, C3c, and C4d were detected by immunohistochemical staining in ischemic lesions, suggesting activation via the classical pathway (Pedersen, E.D., et al., Scand. J. Immunol. 69:555-562, 2009). Targeting of the classical pathway in animal models of cerebral ischemia has yielded mixed results, with some studies demonstrating protection while others showing no benefit (Arumugam, T.V., et al., Neuroscience 158:1074-1089, 2009). Experimental and clinical studies have provided strong evidence for lectin pathway involvement. In experimental stroke models, deficiency of either MBL or MASP-2 results in reduced infarct sizes compared to wild-type mice (Cervera A, et al.; PLoS One 3;5(2):e8433, 2010; Osthoff M. et al., PLoS One, 6(6):e21338, 2011, and Example 26 herein). Furthermore, stroke patients with low levels of MBL have a better prognosis compared to their MBL-sufficient counterpart (Osthoff M. et al., PLoS One, 6(6):e21338, 2011).

In a baboon model of cardiopulmonary bypass, treatment with a factor D monoclonal antibody inhibited systemic inflammation as measured by plasma levels of C3a, sC5b-9, and IL-6, and reduced myocardial tissue injury, indicating involvement of the alternative pathway in this model (Undar, A., et al., Ann. Thorac. Surg. 74:355-362, 2002).

Thus, depending on the organ affected by I/R, all three pathways of complement can contribute to pathogenesis and adverse outcomes. Based on the experimental and clinical findings detailed above, LEA-2 inhibitors are expected to be protective in most settings of I/R. Lectin-dependent activation of LEA-1 may cause complement activation via the alternative pathway at least in some settings. In addition, LEA-2-initiated complement activation may be further amplified by the alternative pathway amplification loop and thus exacerbate I/R-related tissue injury. Thus, LEA-1 inhibitors are expected to provide additional or complementary treatment benefits in patients suffering from an ischemia-related condition.

In view of the above, LEA-1 and LEA-2 inhibitors are expected to have independent therapeutic benefits in treating, preventing or reducing the severity of ischemia-reperfusion related conditions. In addition, LEA-1 and LEA-2 inhibitors used together may achieve additional treatment benefits compared to either agent alone. An optimally effective treatment for an I/R-related condition therefore comprises active pharmaceutical ingredients that, alone or in combination, block both LEA-1 and LEA-2. Combined LEA-1 and LEA-2 inhibition may be accomplished by co-administration of a LEA-1 blocking agent and a LEA-2 blocking agent. Preferably, LEA-1 and LEA-2 inhibitory function may be encompassed in a single molecular entity, such as a bispecific antibody composed of MASP-1/3 and a MASP-2-specific binding site, or a dual specificity antibody where each binding site can bind to and block MASP-1/3 or MASP-2.

In accordance with the foregoing, an aspect of the invention thus provides a method for inhibiting LEA-1-dependent complement activation for treating, preventing or reducing the severity of ischemia reperfusion injuries by administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject experiencing ischemic reperfusion. The MASP-1, MASP-3, or MASP-1/3 inhibitory composition may be administered to the subject by intra-arterial, intravenous, intracranial, intramuscular, subcutaneous, or other parenteral administration, and potentially orally for non-peptidergic inhibitors, and most suitably by intra-arterial or intravenous administration. Administration of the LEA-1 inhibitory compositions of the present invention suitably commences immediately after or as soon as possible after an ischemia reperfusion event. In instances where reperfusion occurs in a controlled environment (e.g., following an aortic aneurism repair, organ transplant or reattachment of severed or traumatized limbs or digits), the LEA-1 inhibitory agent may be administered prior to and/or during and/or after reperfusion. Administration may be repeated periodically as determined by a physician for optimal therapeutic effect.

In some embodiments, the methods are used to treat or prevent an ischemia-reperfusion injury associated with at least one of aortic aneurysm repair, cardiopulmonary bypass, vascular reanastomosis in connection with organ transplants and/or extremity/digit replantation, stroke, myocardial infarction, and hemodynamic resuscitation following shock and/or surgical procedures.

In some embodiments, the methods are used to treat or prevent an ischemia-reperfusion injury in a subject that is about to undergo, is undergoing, or has undergone an organ transplant. In some embodiments the methods are used to treat or prevent an ischemica-reperfusion injury in a subject that is about to undergo, is undergoing, or has undergone an organ transplant, provided that the organ transplant is not a kidney transplant.

In one embodiment, the method according to this aspect of the invention further comprises inhibiting LEA-2-dependent complement activation in a subject experiencing ischemic reperfusion, comprising administering a therapeutically effective amount of a MASP-2 inhibitory agent and a MASP-1, MASP-3, or MASP-1/3 inhibitory agent to the subject. As detailed above, the use of a combination of pharmacologic agents that individually block LEA-1 and LEA-2, is expected to provide an improved therapeutic outcome in treating, preventing, or reducing the severity of ischemia reperfusion injuries as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory composition may be administered to a subject in need thereof by intra-arterial, intravenous, intracranial, intramuscular, subcutaneous, or other parenteral administration, and potentially orally for non-peptidergic inhibitors, and most suitably by intra-arterial or intravenous administration. Administration of the MASP-2 inhibitory compositions of the present invention suitably commences immediately after or as soon as possible after an ischemia reperfusion event. In instances where reperfusion occurs in a controlled environment (e.g., following an aortic aneurism repair, organ transplant or reattachment of severed or traumatized limbs or digits), the MASP-2 inhibitory agent may be administered prior to and/or during and/or after reperfusion. Administration may be repeated periodically as determined by a physician for optimal therapeutic effect.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual inhibitory agents, or co-administration of separate compositions), or a limited sequence of administrations, for treatment or prevention of ischemia reperfusion injuries. Alternatively, the composition may be administered at periodic intervals such as daily, biweekly, weekly, every other week, monthly or bimonthly over an extended period of time for treatment of a subject experiencing ischemic reperfusion.

VI. THE ROLE OF MASP-2 AND MASP-3 IN INLAMMATORY AND NON-INFLAMMATORY ARTHRITIDES AND THERAPEUTIC METHODS USING MASP-2 AND MASP-3 INHIBITORY AGENTS

Rheumatoid arthritis (RA) is a chronic inflammatory disease of synovial joints that may also have systemic manifestations. RA affects approximately 1% of the world population, with women being two to three times more likely to be afflicted. Joint inflammation manifests in swelling, pain, and stiffness. As the disease progresses there may be joint erosion and destruction, resulting in impaired range of motion and deformities. Treatment goals in RA include prevention or control of joint damage, prevention of loss of joint function and disease progression, relief of symptoms and improvement in quality of life, and achievement of drug-free remission. Pharmacological treatment of RA includes disease-modifying anti-rheumatic drugs (DMARDs), analgesics, and anti-inflammatory agents (glucocorticoids and non-steroidal anti-inflammatory drugs). DMARDs are the most important treatment because they can induce durable remissions and delay or halt the progression of joint destruction, which is irreversible. Traditional DMARDs include small molecules such as methotrexate, sulfasalazine, hydroxychloroquine, gold salts, leflunomide, D-penicillamine, cyclosporine, and azathioprine. If traditional DMARDs are inadequate to control the disease then several biologic agents targeting inflammatory cells or mediators are available treatment options, such as tumor necrosis factor inhibitors (etanercept, infliximab, adalimumab, certolizumab pegol, and golimumab), cytokine antagonists (anakinra and tocilizumab), rituximab, and abatacept.

Although adaptive immunity is clearly central to RA pathogenesis as evidenced by genetic association with T-cell activation genes and the presence of autoantibodies, innate immune mechanisms have also been implicated (McInnes, I.B. and Schett, G. New Engl. J. Med. 365:2205-2219, 2011). In human RA, synovial fluid levels of the alternative pathway cleavage fragment Bb were several fold higher than samples from patients with crystal-induced arthritis or degenerative joint disease, implicating preferential activation of the alternative pathway in RA patients (Brodeur, J.P., et al., Arthritis Rheum. 34:1531-1537, 1991). In the experimental anti-type II collagen antibody-passive transfer model of arthritis, factor B-deficient mice had decreased inflammation and joint damage compared with wild-type mice, whereas C4-deficient mice had similar disease activity as wild-type mice, indicating the requirement for the alternative pathway and not the classical pathway in this model (Banda, N.K. et al., J. Immunol. 177:1904-1912, 2006). In the same experimental model of collagen antibody-induced arthritis (CAIA), mice with only classical pathway active or only lectin pathway active were not capable of developing arthritis (Banda, N.K. et al., Clin. Exp. Immunol. 159:100-108, 2010). Data from this study suggested that either the classical or lectin pathways were capable of activating low levels of C3 *in vitro.* However, in the absence of the alternative pathway amplification loop, the level of joint deposition of C3 was inadequate to produce clinical disease. A key step in the activation of the alternative pathway is conversion of the zymogen of factor D (pro-factor D) to mature factor D, which is mediated by MASP-1 and/or MASP-3 (Takahashi, M., et al., J. Exp. Med. 207:29-37, 2010) and/or HTRA1 (Stanton et al., Evidence That the HTRA1 Interactome Influences Susceptibility to Age-Related Macular Degeneration, presented at The Association for Research in Vision and Ophthalmology 2011 conference on May 4, 2011). The role of MASP-1/3 was evaluated in murine CAIA and the results showed that MASP-1/3 deficient mice were protected from arthritis compared with wild-type mice (Banda, N.K., et al., J. Immunol. 185:5598-5606, 2010). In MASP-1/3-deficient mice, pro-factor D but not mature factor D was detected in serum during the evolution of CAIA, and the addition of human factor D *in vitro* reconstituted C3 activation and C5a generation using sera from these mice. In contrast, in a murine model of the effector phase of arthritis, C3-deficient mice developed very mild arthritis compared to WT mice while factor B-deficient mice still developed arthritis, indicating independent contribution of both the classical/lectin and alternative pathways (Hietala, M.A. et al., Eur. J. Immunol. 34:1208-1216, 2004). In the K/BxN T cell receptor transgenic mouse model of inflammatory arthritis, mice lacking C4 or C1q developed arthritis similar to wild-type mice whereas mice lacking factor B either did not develop arthritis or had mild arthritis, demonstrating the requirement for the alternative pathway and not the classical pathway in this model (Ji H. et al., Immunity 16:157-168, 2002). In the K/BxN model, mice lacking MBL-A were not protected from serum-induced arthritis, but as the role of MBL-C was not investigated, a potential role for the lectin pathway could not be eliminated (Ji et al., 2002, supra).

Two research groups have independently proposed that lectin-dependent complement activation promotes inflammation in RA patients *via* interaction of MBL with specific IgG glycoforms (Malhotra et al., Nat. Med. 1:237-243, 1995; Cuchacovich et al., J. Rheumatol. 23:44-51, 1996). It is noted that rheumatoid conditions are associated with a marked increase in IgG glycoforms that lack galactose (referred to as IgG0 glycoforms) in the Fc region of the molecule (Rudd et al., Trends Biotechnology 22:524-30, 2004). The percentage of IgG0 glycoforms increases with disease progression of rheumatoid conditions, and returns to normal when patients go into remission. *In vivo*, IgG0 is deposited on synovial tissue and MBL is present at increased levels in synovial fluid in individuals with RA. Aggregated agalactosyl IgG (IgG0) associated with RA can bind MBL and therefore can initiate lectin-dependent complement activation via LEA-1 and/or LEA-2. Furthermore, results from a clinical study looking at allelic variants of MBL in RA patients suggest that MBL may have an inflammatory-enhancing role in the disease (Garred et al., J. Rheumatol. 27:26-34, 2000). Therefore, the lectin-dependent complement activation via LEA-1 and/or LEA-2 may play an important role in the pathogenesis of RA.

Complement activation also plays in important role in juvenile rheumatoid arthritis (Mollnes, T.E., et al., Arthritis Rheum. 29:1359-64, 1986). Similar to adult RA, in juvenile rheumatoid arthritis, elevated serum and synovial fluid levels of alternative pathway complement activation product Bb compared to C4d (a marker for classical or LEA-2 activation), indicate that complement activation is mediated predominantly by LEA-1 (El-Ghobarey, A.F. et al., J. Rheumatology 7:453-460, 1980; Agarwal, A., et al., Rheumatology 39:189-192, 2000).

Similarly, complement activation plays an important role in psoriatic arthritis. Patients with this condition have increased complement activation products in their circulation, and their red blood cells appear to have lower levels of the complement regulator CD59 (Triolo,. Clin Exp Rheumatol., 21(2):225-8, 2003). Complement levels are associated with disease activity, and have a high predictive value to determine treatment outcomes (Chimenti at al., Clin Exp Rheumatol., 30(1):23-30, 2012). In fact, recent studies suggest that the effect of anti-TNF therapy for this condition is attributable to complement modulation (Ballanti et al., Autoimmun Rev., 10(10):617-23, 2011). While the precise role of complement in psoriatic arthritis has not been determined, the presence of C4d and Bb complement activation products in the circulation of these patients suggests an important role in pathogenesis. On the basis of the products observed, it is believed that LEA-1, and possibly also LEA-2 are responsible for pathologic complement activation in these patients.

Osteoarthritis (OA) is the most common form of arthritis, affecting over 25 million people in the United States. OA is characterized by breakdown and eventual loss of joint cartilage, accompanied by new bone formation and synovial proliferation, leading to pain, stiffness, loss of joint function, and disability. Joints that are frequently affected by OA are hands, neck, lower back, knees and hips. The disease is progressive and current treatments are for symptomatic pain relief and do not alter the natural history of disease. The pathogenesis of OA is unclear, but a role for complement has been implicated. In a proteomic and transcriptomic analyses of synovial fluid from patients with OA, several components of complement were aberrantly expressed compared to samples from healthy individuals, including classical (C1s and C4A) and alternative (factor B) pathways, and also C3, C5, C7, and C9 (Wang, Q., et al., Nat. Med. 17:1674-1679, 2011). Moreover, in a mouse model of OA induced by medial meniscectomy, C5-deficient mice had less cartilage loss, osteophyte formation and synovitis than C5-positive mice, and treatment of wild-type mice with CR2-fH, a fusion protein that inhibits the alternative pathway, attenuated the development of OA (Wang et al., 2011 supra).

Ross River virus (RRV) and chikungunya virus (CHIKV) belong to a group of mosquito-borne viruses that can cause acute and persistent arthritis and myositis in humans. In addition to causing endemic disease, these viruses can cause epidemics that involve millions of infected individuals. The arthritis is believed to be initiated by viral replication and induction of host inflammatory response in the joint and the complement system has been invoked as a key component in this process. Synovial fluid from humans with RRV-induced polyarthritis contains higher levels of C3a than synovial fluid from humans with OA (Morrison, T.E., et al., J. Virol. 81:5132-5143, 2007). In a mouse model of RRV infection, C3-deficient mice developed less severe arthritis compared with wild-type mice, implicating the role of complement (Morrison et al., 2007, supra). The specific complement pathway involved was investigated and mice with inactivated lectin pathway (MBL-A$^{-/-}$ and MBL-C$^{-/-}$) had attenuated arthritis compared with wide-type mice. In contrast, mice with inactivated classical pathway (C1q$^{-/-}$) or alternative pathway (factor B$^{-/-}$) developed severe arthritis, indicating that the lectin pathway initiated by MBL had an essential role in this model (Gunn, B.M., et al., PLoS Pathog. 8:e1002586, 2012). Because arthritides involve damage to the joints, the initial joint damage caused by various etiologies may trigger a secondary wave of complement activation via LEA-2. In support of this concept, our previous work has demonstrated that MASP-2 KO mice have reduced joint injury compared to WT mice in the collagen-induced model of RA, as described in Example 27 herein.

In view of the body of evidence detailed above, LEA-1 and LEA-2 inhibitors, alone or in combination, are expected to be therapeutically useful for the treatment of arthritides. An optimally effective treatment for arthritides may therefore comprise active pharmaceutical ingredients that, alone or in combination, can block both LEA-1 and LEA-2. Combined LEA-1 and LEA-2 inhibition may be accomplished by co-administration of an LEA-1 blocking agent and a LEA2 blocking agent. Preferentially, LEA-1 and LEA-2 inhibitory function may be encompassed in a single molecular entity, such as a bispecific antibody composed of MASP-1/3 and a MASP-2-specific binding site, or a dual specificity antibody where each binding site can bind to and block MASP-1/3 or MASP-2.In accordance with the foregoing, an aspect of the invention thus provides a method for inhibiting LEA-1-dependent complement activation for treating, preventing, or reducing the severity of inflammatory or non-inflammatory arthritides, including osteoarthritis, rheumatoid arthritis, juvenile rheumatoid arthritis and psoriatic arthritis, by administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing, inflammatory or non-inflammatory arthritides. The MASP-1, MASP-3, or MASP-1/3 inhibitory composition may be administered to the subject systemically, such as by intra-arterial, intravenous, intramuscular, subcutaneous, or other parenteral administration, or by oral administration. Alternatively, administration may be by local delivery, such as by intra-articular injection. The LEA-1 inhibitory agent may be administered periodically over an extended period of time for treatment or control of a chronic condition, or may be by single or repeated administration in the period before, during and/or following acute trauma or injury, including surgical procedures performed on the joint.

In one embodiment, the method according to this aspect of the invention further comprises inhibiting LEA-2-dependent complement activation in a subject suffering from, or at risk for developing, inflammatory or non-inflammatory arthritides (including osteoarthritis, rheumatoid arthritis, juvenile rheumatoid arthritis and psoriatic arthritis), by administering a therapeutically effective amount of a MASP-2 inhibitory agent and a MASP-1, MASP-3, or MASP1/3 inhibitory agent to the subject. As detailed above, the use of a combination of pharmacologic agents that individually block LEA-1 and LEA-2, is expected to provide an improved therapeutic outcome in treating or preventing arthritides as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory composition may be administered to the subject in need thereof systemically, such as by intra-arterial, intravenous, intramuscular, subcutaneous, or other parenteral administration, or potentially by oral administration for non-peptidergic inhibitors. Alternatively, administration may be by local delivery, such as by intra-articular injection. The MASP-2 inhibitory agent may be administered periodically over an extended period of time for treatment or control of a chronic condition, or may be by single or repeated administration in the period before, during and/or following acute trauma or injury, including surgical procedures performed on the joint.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual-inhibitory agents, or co-administration of separate compositions), or a limited sequence of administrations, for treating, preventing or reducing the severity of inflammatory or non-inflammatory arthritides. Alternatively, the composition may be administered at periodic intervals such as daily, biweekly, weekly, every other week, monthly or bimonthly over an extended period of time for treatment of a subject suffering from inflammatory or non-inflammatory arthritides.

## VII. THE ROLE OF MASP-2 AND MASP-3 IN DISSEMINATED INTRAVASCULAR COAGULATION (DIC) AND THERAPEUTIC METHODS USING MASP-2 AND MASP-3 INHIBITORY AGENTS

Disseminated intravascular coagulation (DIC) is a syndrome of pathologic overstimulation of the coagulation system that can manifest clinically as hemorrhage and/or thrombosis. DIC does not occur as a primary condition but rather in association with a variety of disease processes, including tissue damage (trauma, burns, heat stroke, transfusion reaction, acute transplant rejection), neoplasia, infections, obstetric conditions (placenta previa, amniotic fluid embolism, toxemia of pregnancy), and miscellaneous conditions such as cardiogenic shock, near drowning, fat embolism, aortic aneurysm. Thrombocytopenia is a frequent abnormality in patients in the intensive care unit, with an incidence of 35% to 44%, and DIC is the etiology in about 25% of these cases, i.e., DIC occurs in approximately 10% of critically ill patients (Levi, M. and Opal, S.M. Crit. Care 10:222-231, 2006). The pathophysiology of DIC is that the underlying disease process initiates a physiological coagulation response. However, the prothrombotic substances overwhelm the normal counterbalancing mechanisms such that there is the inappropriate deposition of fibrin and platelets in the microcirculation, leading to organ ischemia, hypofibrinogenemia, and thrombocytopenia. The diagnosis of DIC is based on the clinical presentation in the appropriate underlying illness or process, along with abnormalities in laboratory parameters (prothrombin time, partial thromboplastin time, fibrin degradation products, D-dimer, or platelet count). The primary treatment of DIC is to address the underlying condition that is the responsible trigger. Blood product support in the form of red blood cells, platelets, fresh frozen plasma, and cryoprecipitate may be necessary to treat or prevent clinical complications.

The role of the complement pathways in DIC has been investigated in several studies. Complement activation was evaluated in pediatric patients with meningococcal infection comparing the clinical course in relation to MBL genotype (Sprong, T. et al., Clin. Infect. Dis. 49:1380-1386, 2009). At admission to the hospital, patients with MBL deficiency had lower circulating levels of C3bc, terminal complement complex, C4bc, and C3bBbP than MBL-sufficient patients, indicating lower extent of common complement, terminal complement, and alternative pathway activation. Furthermore, extent of systemic complement activation correlated with disease severity and parameters of DIC and the MBL-deficient patients had a milder clinical course than MBL-sufficient patients. Therefore, although MBL deficiency is a risk factor for susceptibility to infections, MBL deficiency during septic shock may be associated with lower disease severity.

As demonstrated in Examples 1-4 herein, experimental studies have highlighted the important contribution of MBL and MASP-1/3 in innate immune response to *Neisseria menigitidis,* the etiological agent of meningococcal infection. MBL-deficient sera from mice or humans, MASP-3 deficient human sera, or the MASP-1/3 knockout mouse are less effective at activating complement and lysing meningococci *in vitro* compared to wild-type sera. Similarly, naive MASP-1/3 knockout mice are more susceptible to neisserial infection than their wild-type counterparts. Thus, in the absence of adaptive immunity, the LEA-1 pathway contributes to innate-host resistance to neisserial infection. Conversely, LEA-1 augments pathologic complement activation triggering a harmful host response, including DIC.

In a murine model of arterial thrombosis, MBL-null and MASP-1/-3 knockout mice had decreased $FeCl_3$-induced

thrombogenesis compared with wild-type or C2/factor B-null mice, and the defect was reconstituted with recombinant human MBL (La Bonte, L.R., et al., J. Immunol. 188:885-891, 2012). *In vitro,* MBL-null or MASP-1/-3 knockout mouse sera had decreased thrombin substrate cleavage compared with wild-type or C2/factor B-null mouse sera; addition of recombinant human MASP-1 restored thrombin substrate cleavage in MASP-1/-3 knockout mouse sera (La Bonte et al., 2012, supra). These results indicate that MBL/MASP complexes, in particular MASP-1, play a key role in thrombus formation. Thus, LEA-1 may play an important role in pathologic thrombosis, including DIC.

Experimental studies have established an equally important role for LEA-2 in pathologic thrombosis. As described in Example 30 herein, in a mouse model of localized DIC, we have demonstrated that MASP-2 knockout mice are much less susceptible than wild-type mice to LPS-induced microvascular coagulation. *In vitro* studies further demonstrate that LEA-2 provides a molecular link between the complement system and the coagulation system. As described in Examples 29 and 31 herein, MASP-2 has factor Xa-like activity and activates prothrombin through cleavage to form thrombin, which can subsequently clear fibrinogen and promote fibrin clot formation (see also Krarup et al., PLoS One, 18:2(7):e623, 2007).

Separate studies have shown that lectin-MASP complexes can promote clot formation, fibrin deposition and fibrinopeptide release in a MASP-2 dependent process (Gulla et al., Immunology, 129(4):482-95, 2010). Thus, LEA-2 promotes simultaneous lectin-dependent activation of complement and the coagulation system.

*In vitro* studies have further shown that MASP-1 has thrombin-like activity (Presanis J.S., et al., MolImmunol, 40(13):921-9, 2004), and cleaves fibrinogen and factor XIII (Gulla K. C. et Ia., Immunology, 129(4):482-95, 2010), suggesting that LEA-1 may activate coagulation pathways independently or in concert with LEA-2.

The data detailed above suggest that LEA-1 and LEA-2 provide independent links between lectin-dependent complement activation and coagulation. Thus, in view of the above, LEA-1 and LEA-2 inhibitors are expected to have independent therapeutic benefits in treating a subject suffering from disseminated intravascular coagulation. In some embodiments, the subject is suffering from disseminated intravascular coagulation secondary to sepsis, trauma, infection (bacterial, viral, fungal, parasitic), malignancy, transplant rejection, transfusion reaction, obstetric complication, vascular aneurysm, hepatic failure, heat stroke, burn, radiation exposure, shock, or severe toxic reaction (e.g., snake bite, insect bite, transfusion reaction). In some embodiments, the trauma is a neurological trauma. In some embodiments, the infection is a bacterial infection, such as a Neisseria meningitidis infection.

In addition, LEA-1 and LEA-2 inhibitors used together may achieve additional treatment benefits compared to either agent alone. As both LEA-1 and LEA-2 are known to be activated by conditions that lead to DIC (for example infection or trauma), LEA-1- and LEA-2-blocking agents, either separately or in combination, are expected to have therapeutic utility in the treatment of DIC. LEA-1 and LEA-2 blocking agents may prevent different cross-talk mechanisms between complement and coagulation. LEA-1- and LEA-2-blocking agents may thus have complementary, additive or synergistic effects in preventing DIC and other thrombotic disorders.

In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bispecific antibody composed of MASP-1/3 and a MASP-2-specific binding site, or a dual specificity antibody where each binding site and bind to and block MASP-1/3 or MASP-2.

In accordance with the foregoing, an aspect of the invention thus provides a method for inhibiting LEA-1-dependent complement activation for treating, preventing, or reducing the severity of disseminated intravascular coagulation in a subject in need thereof comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject experiencing, or at risk for developing, disseminated intravascular coagulation. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled. For treatment or prevention of DIC secondary to trauma or other acute event, the LEA-1 inhibitory composition may be administered immediately following the traumatic injury or prophylactically prior to, during, immediately following, or within one to seven days or longer, such as within 24 hours to 72 hours, after trauma-inducing injury or situations such as surgery in patients deemed at risk of DIC. In some embodiments, the LEA-1 inhibitory composition may suitably be administered in a fast-acting dosage form, such as by intravenous or intra-arterial delivery of a bolus of a solution containing the LEA-1 inhibitory agent composition.

In one embodiment, the method according to this aspect of the invention further comprises inhibiting LEA-2-dependent complement activation for treating, preventing, or reducing the severity of disseminated intravascular coagulation in a subject in need thereof, comprising administering a therapeutically effective amount of a MASP-2 inhibitory agent and a MASP-1, MASP-3, or MASP-1/3 inhibitory agent to the subject. As detailed above, the use of a combination of pharmacologic agents that individually block LEA-1 and LEA-2 is expected to provide an improved therapeutic outcome in treating or preventing disseminated intravascular coagulation as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject in need thereof systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled. For DIC secondary to trauma or other acute event, the MASP-2 inhibitory composition may be administered immediately following the traumatic injury or prophylactically prior to, during, immediately following, or within one to seven days or longer, such as within 24 hours to 72 hours, after trauma-inducing injury or situations such as surgery in patients deemed at risk of DIC. In some embodiments, the MASP-2 inhibitory composition may suitably be administered in a fast-acting dosage form, such as by intravenous or intra-arterial delivery of a bolus of a solution containing the MASP-2 inhibitory agent composition.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual-inhibitory agents, or co-administration of separate compositions), or a limited sequence of administrations, for treating, preventing, or reducing the severity of disseminated intravascular coagulation in subject in need thereof. Alternatively, the composition may be administered at periodic intervals such as daily, biweekly, weekly, every other week, monthly or bimonthly over an extended period of time for treatment of a subject experiencing, or at risk for developing disseminated intravascular coagulation.

VIII. THE ROLE OF MASP-2 AND MASP-3 IN THROMBOTIC MICROANGIOPATHY (TMA), INCLUDING HEMOLYTIC UREMIC SYNDROME (HUS), ATYPICAL HEMOLYTIC UREMIC SYNDROME (AHUS) AND THROMBOTIC THROMBOCYTOPENIC PURPURA (TTP) AND THERAPEUTIC METHODS USING MASP-2 AND MASP-3 INHIBITORY AGENTS

Thrombotic microangiopathy (TMA) refers to a group of disorders characterized clinically by thrombocytopenia, microangiopathic hemolytic anemia, and variable organ ischemia. The characteristic pathological features of TMA are platelet activation and the formation of microthrombi in the small arterioles and venules. The classic TMAs are hemolytic uremic syndrome (HUS) and thrombotic thrombocytopenic purpura (TTP). HUS is distinguished from TTP by the presence of acute renal failure. HUS occurs in two forms: diarrhea-associated (D+) or typical HUS, and diarrhea negative (D-) or atypical HUS (aHUS).

HUS

D+HUS is associated with a prodromal diarrheal illness usually caused by *Escherichia coli* 0157 or another Shiga-toxin-producing strain of bacteria, accounts for over 90% of the HUS cases in children, and is the most common cause of acute renal failure in children. Although human infection with *Escherichia coli* 0157 is relatively frequent, the percentages of bloody diarrhea that progresses to D+HUS ranged from 3% to 7% in sporadic cases and 20% to 30% in some outbreaks (Zheng, X.L. and Sadler, J.E., Annu. Rev. Pathol. 3:249-277, 2008). HUS usually occurs 4 to 6 days after the onset of diarrhea and approximately two-third of children require dialysis in the acute phase of the disease. Treatment of D+HUS is supportive as no specific treatments have been shown to be effective. The prognosis of D+HUS is favorable, with the majority of patients regaining renal function.

The pathogenesis of D+HUS involves bacteria-produced Shiga toxins that bind to membranes on microvascular endothelial cells, monocytes, and platelets. The microvasculature of the kidney is most often affected. Following binding, the toxin is internalized, leading to release of proinflammatory mediators and eventual cell death. It is thought that endothelial cell damage triggers renal microvascular thrombosis by promoting the activation of the coagulation cascade. There is evidence for activation of the complement system in D+HUS. In children with D+HUS, plasma levels of Bb and SC5b-9 were increased at the time of hospitalization compared to normal controls and, at day 28 after hospital discharge, the plasma levels had normalized (Thurman, J.M. et al., Clin. J. Am. Soc. Nephrol. 4:1920-1924, 2009). Shiga toxin 2 (Stx2) was found to activate human complement in the fluid phase *in vitro,* predominantly via the alternative pathway as activation proceeded in the presence of ethylene glycol tetraacetic acid which blocks the classical pathway (Orth, D. et al., J. Immunol. 182:6394-6400, 2009). Furthermore, Stx2 bound factor H and not factor I, and delayed the cofactor activity of factor H on cell surfaces (Orth et al, 2009, supra). These results suggest that Shiga toxin may cause renal damage through multiple potential mechanisms, including a direct toxic effect, and indirectly through activation of complement or inhibition of complement regulators. Toxic effects on the vascular endothelium are expected to activate complement via LEA-2, as evidenced by the effectiveness of MASP-2 blockade in preventing complement-mediated reperfusion injury in various vascular beds as demonstrated in Examples 21-23 herein, see also Schwaeble, W.J., et al., Proc. Natl. Acad. Sci. 108:7523-7528, 2011.

In a murine model of HUS induced by co-injection of Shiga toxin and lipopolysaccharide, factor B-deficient mice had less thrombocytopenia and were protected from renal impairment compared with wild-type mice, implicating LEA-1-dependent activation of the alternative pathway in microvascular thrombosis (Morigi, M. et al., J. Immunol. 187:172-180, 2011). As described in Example 33 herein, in the same model, administration of MASP-2 antibody was also effective and increased survival following STX challenge, implicating LEA-2-dependent complement pathway in microvascular thrombosis.

Based on the foregoing, LEA-1 and LEA-2 inhibitors are expected to have independent therapeutic benefit in the treatment or prevention of HUS. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bispecific antibody composed of MASP-1/3 and a MASP-2-specific binding site, or a dual-specificity antibody where each binding site can bind to and block MASP-1/3 or MASP-2.

aHUS

Atypical HUS is a rare disease, with an estimated incidence of 2 per million in the United States (Loirat, C. and Fremeaux-Bacchi, V. Orphanet J. Rare Dis. 6:60-90, 2011). Atypical HUS can develop at any age, although the majority of patients have an onset during childhood. Atypical HUS is heterogeneous: some cases are familial, some are recurring, and some are triggered by an infectious illness, typically upper respiratory tract or gastroenteritis. The onset of aHUS is usually sudden and most patients require dialysis at admission. Extra renal manifestations are present in about 20% of patients and may involve the central nervous system, myocardial infarction, distal ischemic gangrene, or multiorgan failure. Treatment of aHUS includes supportive care for organ dysfunction, plasma infusion or plasma exchange, and eculizumab, a humanized monoclonal antibody that targets C5 that was recently approved for use in the United States and European Union. The prognosis in aHUS is not as good as in D+HUS, with approximately 25% mortality during the acute stage and most survivors develop end-stage renal disease.

Atypical HUS has been characterized as a disease of complement dysregulation in that approximately 50% of patients have mutations in genes encoding complement regulatory proteins (Zheng and Sadler, 2008 supra). Most mutations are seen in factor H (FH); other mutations include membrane cofactor protein (MCP), factor I (FI), factor B, and C3. Functional studies showed that the mutations in FH, MCP, and FI lead to loss of function and therefore more complement activation, whereas mutations in factor B are gain of function. The effects of these mutations predominantly affect the alternative pathway. These genetic abnormalities are risk factors rather than the only cause of disease as approximately 50% of family members who carry the mutation do not present with the disease by age 45 (Loirat and Fremeaux-Bacchi, 2011 supra).

Factor H is a complement control protein that protects host tissue from alternative pathway complement attack. FH regulates the alternative pathway amplification loop in three ways: it is a cofactor for FI, which cleaves C3b, it inhibits the formation of the alternative pathway C3 convertase, C3bBb, and it binds to polyanions on cell surfaces and tissue matrices and blocks deposition of C3b (Atkinson, J.P. and Goodship, T.H.J., J. Exp. Med. 6:1245-1248, 2007). The majority of FH mutations in aHUS patients occur in the C-terminal short consensus repeat domains of the protein, which result in defective binding of FH to heparin, C3b, and endothelium, but do not alter plasma C3 regulation which resides among N-terminal domains (Pickering, M.C. et al., J. Exp. Med. 204:1249-1256, 2007). FH-deficient mice have uncontrolled plasma C3 activation and spontaneously develop membranoproliferative glomerulonephritis type II, but not aHUS. However, FH-deficient mice that transgenically expressed a mouse FH protein functionally equivalent to aHUS-associated human FH mutants spontaneously develop a HUS but not membranoproliferative glomerulonephritis type II, providing *in vivo* evidence that defective control of alternative pathway activation in renal endothelium is a key event in the pathogenesis of FH-associated aHUS (Pickering et al., 2007 supra). Another form of FH-associated aHUS occurs in patients who have anti-FH autoantibodies resulting in a loss of FH functional activity; most of these patients have deletions in genes encoding five FH-related proteins (Loirat and Fremeaux-Bacchi, 2011, supra).

Similar to FH, MCP inhibits complement activation by regulating C3b deposition on target cells. MCP mutations result in proteins with low C3b-binding and cofactor activity, thus allowing for dysregulated alternative pathway activation.

FI is a serine protease that cleaves C3b and C4b in the presence of cofactors, such as FH and MCP, and thereby prevents the formation of C3 and C5 convertases and inhibits both the alternative and the classical complement pathways. Most of the FI-associated aHUS mutations result in reduced FI activity for the degradation of C3b and C4b (Zheng and Stadler, 2008, supra). FB is a zymogen that carries the catalytic sites of the alternative pathway convertase C3bBb. Functional analysis showed that the aHUS associated FB mutations result in increased alternative pathway activation (Loirat and Fremeaux-Bacchi, 2011, supra). Heterozygous mutations in C3 are associated with aHUS. Most C3 mutations induce a defect of C3 to bind MCP, leading to an increased capacity of FB to bind C3b and increased formation of C3 convertase (Loirat and Fremeaux-Bacchi, 2011, supra). Thus, aHUS is a disease closely associated with mutations in the complement genes that lead to inadequate control of the alternative pathway amplification loop. Since the alternative pathway amplification loop is dependent on factor B proteolytic activity, and since LEA-1 is required for factor B activation (either by MASP-3 dependent cleavage or by factor D-mediated cleavage wherein the MASP-1 contributes to the maturation of factor D), LEA-1-blocking agents are expected to prevent uncontrolled complement activation in susceptible individuals. As a result, it is expected that LEA-1 blocking agents will effectively treat aHUS.

While the central role of a deregulated alternative pathway amplification loop in aHUS is widely accepted, the triggers initiating complement activation and the molecular pathways involved are unresolved. Not all individuals carrying the above-described mutations develop aHUS. In fact, familial studies have suggested that the penetrance of aHUS is only ~50% (Sullivan M. et al., Ann Hum Genet 74:17-26 2010). The natural history of the disease suggests that aHUS most often develops after an initiating event such as an infectious episode or an injury. Infectious agents are well known to activate the complement system. In the absence of pre-existing adaptive immunity, complement activation by infectious agents may be primarily initiated via LEA-1 or LEA-2. Thus, lectin-dependent complement activation triggered by an infection may represent the initiating trigger for subsequent pathological amplification of complement activation in aHUS-predisposed individuals, which may ultimately lead to disease progression. Accordingly, another aspect of the present invention comprises treating a patient suffering with aHUS secondary to an infection by administering an effective amount of a LEA-1- or a LEA-2-inhibitory agent.

Other forms of injury to host tissue will activate complement via LEA-2, in particular injury to the vascular endothelium. Human vascular endothelial cells subject to oxidative stress, for example, respond by expressing surface moieties that bind lectins and activate the LEA-2 pathway of complement (Collard et al., Am J. Pathol 156(5):1549-56, 2000). Vascular injury following ischemia/reperfusion also activates complement via LEA-2 *in vivo* (Moller-Kristensen et al., Scand J Immunol 61(5):426-34, 2005). Lectin pathway activation in this setting has pathological consequences for the host, and as shown in Examples 22 and 23, inhibition of LEA-2 by blocking MASP-2 prevents further host tissue injury and adverse outcomes (see also Schwaeble PNAS, 2011, supra).

Thus, other processes that precipitate aHUS are also known to activate LEA-1 or LEA-2. It is therefore likely that the LEA-1 and/or LEA-2 pathway may represent the initial complement activating mechanism that is inappropriately amplified in a deregulated fashion in individuals genetically predisposed to aHUS, thus initiating aHUS pathogenesis. By inference, agents that block activation of complement via LEA-1 and/or LEA-2 are expected to prevent disease progression or reduce exacerbations in aHUS susceptible individuals.

In further support of this concept, recent studies have identified *Streptococcus-pneumoniae* as an important etiological agent in pediatric cases of aHUS. (Lee, C.S. et al, Nephrology, 17(1):48-52 (2012); Banerjee R. et al., Pediatr Infect Dis J., 30(9):736-9 (2011)). This particular etiology appears to have an unfavorable prognosis, with significant mortality and long-term morbidity. Notably, these cases involved non-enteric infections leading to manifestations of microangiopathy, uremia and hemolysis without evidence of concurrent mutations in complement genes known to predispose to aHUS. It is important to note that *S. pneumoniae* is particularly effective at activating complement, and does so predominantly through LEA-2. Thus, in cases of non-enteric HUS associated with pneumococcal infection, manifestations of microangiopathy, uremia and hemolysis are expected to be driven predominantly by activation of LEA-2, and agents that block LEA-2, including MASP-2 antibodies, are expected to prevent progression of aHUS or reduce disease severity in these patients. Accordingly, another aspect of the present invention comprises treating a patient suffering with non-enteric aHUS that is associated with *S. pneumoniae* infection by administering an effective amount of a MASP-2 inhibitory agent.

TTP

Thrombotic thrombocytopenic purpura (TTP) is a life-threatening disorder of the blood-coagulation system caused by autoimmune or hereditary dysfunctions that activate the coagulation cascade or the complement system (George, JN, N Engl J Med; 354:1927-35, 2006). This results in numerous microscopic clots, or thromboses, in small blood vessels throughout the body, which is a characteristic feature of TMAs. Red blood cells are subjected to shear stress, which damages their membranes, leading to intravascular hemolysis. The resulting reduced blood flow and endothelial injury results in organ damage, including brain, heart, and kidneys. TTP is clinically characterized by thrombocytopenia, microangiopathic hemolytic anemia, neurological changes, renal failure and fever. In the era before plasma exchange, the fatality rate was 90% during acute episodes. Even with plasma exchange, survival at six months is about 80%.

TTP may arise from genetic or acquired inhibition of the enzyme ADAMTS-13, a metalloprotease responsible for cleaving large multimers of von Willebrand factor (vWF) into smaller units. ADAMTS-13 inhibition or deficiency ultimately results in increased coagulation (Tsai, H. J Am Soc Nephrol 14: 1072-1081, 2003). ADAMTS-13 regulates the activity of vWF; in the absence of ADAMTS-13, vWF forms large multimers that are more likely to bind platelets and predisposes patients to platelet aggregation and thrombosis in the microvasculature.

Numerous mutations in ADAMTS13 have been identified in individuals with TTP. The disease can also develop due to autoantibodies against ADAMTS-13. In addition, TTP can develop during breast, gastrointestinal tract, or prostate cancer (George JN., Oncology (Williston Park). 25:908-14, 2011), pregnancy (second trimester or postpartum), (George JN., Curr Opin Hematol 10:339-344, 2003), or is associated with diseases, such as HIV or autoimmune diseases like systemic lupus erythematosis (Hamasaki K, et al., Clin Rheumatol.22:355-8, 2003). TTP can also be caused by certain drug therapies, including heparin, quinine, immune mediated ingredient, cancer chemotherapeutic agents (bleomycin, cisplatin, cytosine arabinoside, daunomycin gemcitabine, mitomycin C, and tamoxifen), cyclosporine A, oral contraceptives, penicillin, rifampin and anti-platelet drugs including ticlopidine and clopidogrel (Azarm, T. et al., J Res Med Sci., 16: 353-357, 2011). Other factors or conditions associated with TTP are toxins such as bee venoms, sepsis, splenic sequestration, transplantation, vasculitis, vascular surgery, and infections like *Streptococcus* pneumoniae and cytomegalovirus (Moake JL., N Engl JMed., 347:589-600, 2002). TTP due to transient functional ADAMTS-13 deficiency can occur as a consequence of endothelial cell injury associated with *S. pneumoniae* infection (Pediatr Nephrol, 26:631-5, 2011).

Plasma exchange is the standard treatment for TTP (Rock GA, et al., N Engl J Med 325:393-397, 1991). Plasma exchange replaces ADAMTS-13 activity in patients with genetic defects and removes ADAMTS-13 autoantibodies in those patients with acquired autoimmune TTP (Tsai, H-M, Hematol Oncol Clin North Am., 21(4): 609-v, 2007). Additional agents such as immunosuppressive drugs are routinely added to therapy (George, JN, N Engl J Med, 354:1927-35, 2006). However, plasma exchange is not successful for about 20% of patients, relapse occurs in more than a third of patients, and plasmapheresis is costly and technically demanding. Furthermore, many patients are unable to tolerate plasma exchange. Consequently there remains a critical need for additional and better treatments for TTP.

Because TTP is a disorder of the blood coagulation cascade, treatment with antagonists of the complement system may aid in stabilizing and correcting the disease. While pathological activation of the alternative complement pathway is linked to aHUS, the role of complement activation in TTP is less clear. The functional deficiency of ADAMTS13 is important for the susceptibility to TTP, however it is not sufficient to cause acute episodes. Environmental factors and/or other genetic variations may contribute to the manifestation of TTP. For example, genes encoding proteins involved in the regulation of the coagulation cascade, vWF, platelet function, components of the endothelial vessel surface, or the complement system may be implicated in the development of acute thrombotic microangiopathy (Galbusera, M. et al., Haematologica, 94: 166-170, 2009). In particular, complement activation has been shown to play a critical role; serum from thrombotic microangiopathy associated with ADAMTS-13 deficiency has been shown to cause C3 and MAC deposition and subsequent neutrophil activation which could be abrogated by complement inactivation (Ruiz-Torres MP, et al., Thromb Haemost, 93:443-52, 2005). In addition, it has recently been shown that during acute episodes of TTP there are increased levels of C4d, C3bBbP, and C3a (M. Réti et al., J Thromb Haemost. 10(5):791-798, 2012), consistent with activation of the classical, lectin and alternative pathways. This increased amount of complement activation in acute episodes may initiate the terminal pathway activation and be responsible for further exacerbation of TTP.

The role of ADAMTS-13 and vWF in TTP clearly is responsible for activation and aggregation of platelets and their subsequent role in shear stress and deposition in microangiopathies. Activated platelets interact with and trigger both the classical and alternative pathways of complement. Platelet-mediated complement activation increases the inflammatory mediators C3a and C5a (Peerschke E. et al., Mol Immunol, 47:2170-5 (2010)). Platelets may thus serve as targets of classical complement activation in inherited or autoimmune TTP.

As described above, the lectin-dependent activation of complement, by virtue of the thrombin-like activity of MASP-1 and the LEA-2-mediated prothombin activation, is the dominant molecular pathway linking endothelial injury to the coagulation and microvascular thrombosis that occurs in HUS. Similarly, activation of LEA-1 and LEA-2 may directly drive the coagulation system in TTP. LEA-1 and LEA-2 pathway activation may be initiated in response to the initial endothelium injury caused by ADAMTS-13 deficiency in TTP. It is therefore expected that LEA-1 and LEA-2 inhibitors, including but not limited to antibodies that block MASP-2 function, MASP-1 function, MASP-3 function, or MASP-1 and MASP-3 function will mitigate the microangiopathies associated with microvascular coagulation, thrombosis, and hemolysis in patients suffering from TTP.

Patients suffering from TTP typically present in the emergency room with one or more of the following: purpura, renal failure, low platelets, anemia and/or thrombosis, including stroke. The current standard of care for TTP involves intra-catheter delivery (e.g., intravenous or other form of catheter) of replacement plasmapheresis for a period of two weeks or longer, typically three times a week, but up to daily. If the subject tests positive for the presence of an inhibitor of ADAMTS13 (*i.e.,* an endogenous antibody against ADAMTS13), then the plasmapheresis may be carried out in combination with immunosuppressive therapy (e.g., corticosteroids, rituxan, or cyclosporine). Subjects with refractory TTP (approximately 20% of TTP patients) do not respond to at least two weeks of plasmapheresis therapy.

In accordance with the foregoing, in one embodiment, in the setting of an initial diagnosis of TTP, or in a subject exhibiting one or more symptoms consistent with a diagnosis of TTP (e.g., central nervous system involvement, severe thrombocytopenia (a platelet count of less than or equal to 5000/μL if off aspirin, less than or equal to 20,000/μL if on aspirin), severe cardiac involvement, severe pulmonary involvement, gastro-intestinal infarction or gangrene), a method is provided for treating the subject with an effective amount of a LEA-2 inhibitory agent (e.g., a MASP-2 antibody) or a LEA-1 inhibitory agent (e.g., a MASP-1 or MASP-3 antibody) as a first line therapy in the absence of plasmapheresis, or in combination with plasmapheresis. As a first-line therapy, the LEA-1 and/or LEA-2 inhibitory agent may be administered to the subject systemically, such as by intra-arterial, intravenous, intramuscular, inhalational, nasal, subcutaneous or other parenteral administration. In some embodiments, the LEA-1 and/or LEA-2 inhibitory agent is administered to a subject as a first-line therapy in the absence of plasmapheresis to avoid the potential complications of plasmapheresis, such as hemorrhage, infection, and exposure to disorders and/or allergies inherent in the plasma donor, or in a subject otherwise averse to plasmapheresis, or in a setting where plasmapheresis is unavailable. In some embodiments, the LEA-1 and/or LEA-2 inhibitory agent is administered to the subject suffering from TTP in combination (including co-administration) with an immunosuppressive agent (e.g., corticosteroids, rituxan or cyclosporine) and/or in combination with concentrated ADAMTS-13.

In some embodiments, the method comprises administering a LEA-1 and/or LEA-2 inhibitory agent to a subject suffering from TTP via a catheter (e.g., intravenously) for a first time period (e.g., an acute phase lasting at least one day to a week or two weeks) followed by administering a LEA-1 and/or LEA-2 inhibitory agent to the subject subcutaneously for a second time period (e.g., a chronic phase of at least two weeks or longer). In some embodiments, the administration in the first and/or second time period occurs in the absence of plasmapheresis. In some embodiments, the method is used to maintain the subject to prevent the subject from suffering one or more symptoms associated with TTP.

In another embodiment, a method is provided for treating a subject suffering from refractory TTP (i.e., a subject that has not responded to at least two weeks of plasmaphoresis therapy), by administering an amount of a LEA-1 and/or LEA-2 inhibitor effective to reduce one or more symptoms of TTP. In one embodiment, the LEA-1 and/or LEA-2 inhibitor is administered to a subject with refractory TTP on a chronic basis, over a time period of at least two weeks or longer via subcutaneous or other parenteral administration. Administration may be repeated as determined by a physician until the condition has been resolved or is controlled.

In some embodiments, the method further comprises determining the level of at least one complement factor (e.g., C3, C5) in the subject prior to treatment, and optionally during treatment, wherein the determination of a reduced level of the at least one complement factor in comparison to a standard value or healthy control subject is indicative of the need for continued treatment with the LEA-1 and/or LEA-2 inhibitory agent.

In some embodiments, the method comprises administering, either subcutaneously or intravenously, a LEA-1 and/or LEA-2 inhibitory agent to a subject suffering from, or at risk for developing, TTP. Treatment is preferably daily, but can be as infrequent as monthly. Treatment is continued until the subject's platelet count is greater than 150,000/ml for at least two consecutive days.

In summary, LEA-1 and LEA-2 inhibitors are expected to have independent therapeutic benefit in the treatment of TMAs, including HUS, aHUS and TTP. In addition, LEA-1 and LEA-2 inhibitors used together are expected to achieve additional treatment benefit compared to either agent alone, or may provide effective treatment for a wider spectrum of patient subsets suffering from variant forms of TMA. Combined LEA-1 and LEA-2 inhibition may be accomplished by co-administration of a LEA-1 blocking agent and a LEA2 blocking agent. Optimally, LEA-1 and LEA-2 inhibitory function may be encompassed in a single molecular entity, such as a bispecific antibody composed of MASP-1/3 and a MASP-2-specific binding site, or a dual specificity antibody where each binding site can bind to and block MASP-1/3 or MASP-2.

In accordance with the foregoing, an aspect of the invention thus provides a method for inhibiting LEA-1-dependent complement activation for treating, preventing, or reducing the severity of a thrombotic microangiopathy, such as hemolytic uremic syndrome (HUS), atypical hemolytic uremic syndrome (aHUS) or thrombotic thrombocytopenic purpura (TTP) comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing a thrombotic microangiopathy. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In one embodiment, the method according to this aspect of the invention further comprises inhibiting LEA-2-dependent complement activation for treating, preventing, or reducing the severity of a thrombotic microangiopathy, such as hemolytic uremic syndrome (HUS), atypical hemolytic uremic syndrome (aHUS) or thrombotic thrombocytopenic purpura (TTP) comprising administering a therapeutically effective amount of a MASP-2 inhibitory agent and a MASP-1, MASP-3, or MASP-1/3 inhibitory agent to a subject suffering from, or at risk for developing a thrombotic microangiopathy. As detailed above, the use of a combination of pharmacologic agents that individually block LEA-1 and LEA-2, is expected to provide an improved therapeutic outcome in treating or preventing or reducing the severity of a thrombotic microangiopathy as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

IX. APPLICATION OF THE MASP-3 INHIBITORY COMPOSITIONS AND OPTIONAL MASP-2 INHIBITORY COMPOSITIONS OF THE PRESENT INVENTION MAY BE CARRIED OUT BY A SINGLE ADMINISTRATION OF THE COMPOSITION (E.G., A SINGLE COMPOSITION COMPRISING MASP-2 AND MASP-3 INHIBITORY AGENTS, OR BISPECIFIC OR DUAL INHIBITORY AGENTS, OR CO-ADMINISTRATION OF SEPARATE COMPOSITIONS), OR A LIMITED SEQUENCE OF ADMINISTRATIONS, FOR TREATING, PREVENTING OR REDUCING THE SEVERITY OF A THROMBOTIC MICROANGIOPATHY IN A SUBJECT SUFFERING FROM, OR AT RISK FOR DEVELOPING, A THROMBOTIC MICROANGIOPATHY. ALTERNATIVELY, THE COMPOSITION MAY BE ADMINISTERED AT PERIODIC INTERVALS SUCH AS DAILY, BIWEEKLY, WEEKLY, EVERY OTHER WEEK, MONTHLY OR BIMONTHLY OVER AN EXTENDED PERIOD OF TIME FOR TREATMENT OF A SUBJECT IN NEED THEREOF.THE ROLE OF MASP-2 AND MASP-3 IN ASTHMA AND THERAPEUTIC METHODS USING MASP-2 AND MASP-3 INHIBITORY AGENTS

Asthma is a common chronic inflammatory disease of the airways. Approximately 25 million people in the United States have asthma, including seven million children under the age of 18, with more than half experiencing at least one asthma attack each year, leading to more than 1.7 million emergency department visits and 450,000 hospitalizations annually (world-wide-web at gov/health/prof/lung/asthma/naci/asthma-info/index.htm., accessed on May 4, 2012). The disease is heterogeneous with multiple clinical phenotypes. The most common phenotype is allergic asthma. Other phenotypes include nonallergic asthma, aspirin-exacerbated respiratory disease, post-infectious asthma, occupational asthma, airborne irritant-induced asthma, and exercise-induced asthma. The cardinal features of allergic asthma include airway hyperresponsiveness (AHR) to a variety of specific and nonspecific stimuli, excessive airway mucus production, pulmonary eosinophilia, and elevated concentration of serum IgE. The symptoms of asthma include coughing, wheezing, chest tightness, and shortness of breath. The goal of asthma treatment is to control the disease and minimize exacerbations, daily symptoms, and allow patients to be physically active. Current treatment guidelines recommend stepwise treatments until asthma control is attained. The first treatment step is as needed rapid-acting inhaled $\beta_2$-agonist, followed by addition of controller medications such as inhaled corticosteroids, long-acting inhaled $\beta_2$-agonists, leukotriene modifier drugs, theophylline, oral glucocorticosteroids, and anti-IgE monoclonal antibody.

Although asthma is multifactorial in origin, it is generally accepted that it arises as a result of inappropriate immunological responses to common environmental antigens in genetically susceptible individuals. Asthma is associated with complement activation and the anaphylatoxins (AT) C3a and C5a have proinflammatory and immunoregulatory properties that are relevant to the development and modulation of the allergic response (Zhang, X. and Kohl, J. Expert. Rev. Clin. Immunol., 6:269-277, 2010). However, the relative involvement of the classical, alternative, and lectin pathways of complement in asthma is not well understood. The alternative pathway may be activated on the surface of allergens and the lectin pathway may be activated through recognition of allergen polysaccharide structures, both processes leading to the generation of AT. Complement may be activated by different pathways depending on the causative allergen involved. Highly allergic grass pollen of the Parietaria family for example is very effective at promoting MBL-dependent activation of C4, implicating LEA-2. Conversely, house dust mite allergen does not require MBL for complement activation (Varga et al. Mol Immunol., 39(14):839-46, 2003).

Environmental triggers of asthma may activate complement by the alternative pathway. For example, *in vitro* exposure of human serum to cigarette smoke or diesel exhaust particles resulted in activation of complement and the effect was unaffected by the presence of EDTA, suggesting activation was via the alternative rather than classical pathway (Robbins, R.A. et al, Am. J. Physiol. 260:L254-L259, 1991; Kanemitsu, H., et al., Biol. Pharm. Bull. 21:129-132, 1998). The role of complement pathways in allergic airway inflammation was evaluated in a mouse ovalbumin sensitization and challenge model. Wild-type mice developed AHR and airway inflammation in response to aeroallergen challenge. A Crry-Ig fusion protein which inhibits all pathways of complement activation, was effective in preventing AHR and lung inflammation when administered systemically or locally by inhalation in the mouse ovalbumine model of allergic lung inflammation (Taube et al., Am J Respir Crit Care Med., 168(11):1333-41, 2003).

In comparison to wild-type mice, factor B-deficient mice demonstrated less AHR and airway inflammation whereas C4-deficient mice had similar effects as wild-type mice (Taube, C., et al., Proc. Natl. Acad. Sci. USA 103:8084-8089, 2006). These results support a role for alternative pathway and not classical pathway involvement in the murine aeroallergen challenge model. Further evidence for the importance of the alternative pathway was provided in a study of factor H (FH) using the same mouse model (Takeda, K., et al., J. Immunol. 188:661-667, 2012). FH is a negative regulator of the alternative pathway and acts to prevent autologous injury of self tissues. Endogenous FH was found to be present in airways during allergen challenge and inhibition of FH with a recombinant competitive antagonist increased the extent of AHR and airway inflammation (Takeda et al., 2012, supra). Therapeutic delivery of CR2-fH, a chimeric protein that links the iC3b/C3d binding region of CR2 to the complement-regulatory region of FH which targets the complement regulatory activity of fH to sites of existing complement activation, protected the development of AHR and eosinophil infiltration into the airways after allergen challenge (Takeda et al., 2012, supra). The protective effect was demonstrated with ovalbumin as well as ragweed allergen, which is a relevant allergen in humans.

The role of lectin-dependent complement activation in asthma was evaluated in a mouse model of fungal asthma (Hogaboam et al., J. Leukocyte Biol. 75:805-814, 2004). These studies used mice genetically deficient in mannan-binding lectin-A (MBL-A), a carbohydrate binding protein that functions as the recognition component for activation of the lectin complement pathways. MBL-A(+/+) and MBL-A(-/-) *Aspergillus. fumigatus*-sensitized mice were examined at days 4 and 28 after an *i.t.* challenge with *A. fumigatus* conidia. AHR in sensitized MBL-A(-/-) mice was significantly attenuated at both times after conidia challenge compared with the sensitized MBL-A (+/+) group. Lung TH2 cytokine levels (IL-4, IL-5 and IL-13) were significantly lower in *A. fumigatus*-sensitized MBL-A(-/-) mice compared to the wild-type group at day 4 after conidia. These results indicate that MBL-A and the lectin pathway have a major role in the development and maintenance of AHR during chronic fungal asthma.

The findings detailed above suggest the involvement of lectin-dependent complement activation in the pathogenesis of asthma. Experimental data suggest that factor B activation plays a pivotal role. In light of the fundamental role for LEA-1 in the lectin-dependent activation of factor B and subsequent activation of the alternative pathway, it is expected that LEA-1 blocking agents will be beneficial for the treatment of certain forms of asthma mediated by the alternative pathway. Such a treatment may thus be particularly useful in house dust mite-induced asthma, or asthma caused by environmental triggers such as cigarette smoke or diesel exhaust. Asthmatic responses triggered by grass pollen on the other hand are likely to invoke LEA-2-dependent complement activation. Therefore, LEA-2-blocking agents are expected to be particularly useful in treating the asthmatic conditions in this subset of patients.

In view of the data detailed above, the inventors believe that LEA-1 and LEA-2 mediate pathologic complement activation in asthma. Depending on the inciting allergic agent, LEA-1 or LEA-2 may be preferentially involved. Thus, a LEA-1-blocking agent combined with a LEA-2-blocking agent may have utility in the treatment of multiple forms of asthma regardless of the underlying etiology. LEA-1 and LEA-2-blocking agents may have complementary, additive or synergistic effects in preventing, treating or reversing pulmonary inflammation and symptoms of asthma.

Combined LEA-1 and LEA-2 inhibition may be accomplished by co-administration of a LEA-1-blocking agent and a LEA2-blocking agent. Optimally, LEA-1 and LEA-2 inhibitory function may be encompassed in a single molecular entity, such as a bispecific antibody composed of MASP-1/3 and a MASP-2-specific binding site, or a dual specificity antibody where each binding site can bind to and block MASP-1/3 or MASP-2.

In accordance with the foregoing, an aspect of the invention thus provides a method for inhibiting LEA-1-dependent complement activation for treating, preventing, or reducing the severity of asthma, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing asthma. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In one embodiment, the method according to this aspect of the invention further comprises inhibiting LEA-2-dependent complement activation for treating, preventing, or reducing the severity of asthma, comprising administering a therapeutically effective amount of a MASP-2 inhibitory agent and a MASP-1, MASP-3, or MASP-1/3 inhibitory agent to a subject suffering from, or at risk for developing asthma. As detailed above, the use of a combination of pharmacologic agents that individually block LEA-1 and LEA-2, is expected to provide an improved therapeutic outcome in treating or preventing or reducing the severity of asthma as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual inhibitory agents, or co-administration of separate compositions), or a limited sequence of administrations, for treating, preventing or reducing the severity of a asthma in a subject suffering from, or at risk for developing asthma. Alternatively, the composition may be administered at periodic intervals such as daily, biweekly, weekly, every other week, monthly or bimonthly over an extended period of time for treatment of a subject in need thereof.

X. THE ROLE OF MASP-2 AND MASP-3 IN DENSE DEPOSIT DISEASE, AND THERAPEUTIC METHODS USING MASP-2 AND MASP-3 INHIBITORY AGENTS

Membranoproliferative glomerulonephritis (MPGN) is a kidney disorder characterized morphologically by mesangial cell proliferation and thickening of the glomerular capillary wall due to subendothelial extension of the mesangium. MPGN is classified as primary (also referred to as idiopathic) or secondary, with underlying diseases such as infectious diseases, systemic immune complex diseases, neoplasms, chronic liver disease, and others. Idiopathic MPGN includes three morphologic types. Type I, or classical MPGN, is characterized by subendothelial deposits of immune complexes and activation of the classical complement pathway. Type II, or dense deposit disease (DDD), is characterized by additional intra-membraneous dense deposits. Type III is characterized by additional subepithelial deposits. Idiopathic MPGN is rare, accounting for approximately 4 to 7% of primary renal causes of nephrotic syndrome (Alchi, B. and Jayne, D. Pediatr. Nephrol. 25:1409-1418, 2010). MPGN primarily affects children and young adults and may present as nephrotic syndrome, acute nephritic syndrome, asymptomatic proteinuria and hematuria, or recurrent gross hematuria. Renal dysfunction occurs in the majority of patients and the disease has a slowly progressive course, with approximately 40% of patients developing end-stage renal disease within 10 years of diagnosis (Alchi and Jayne, 2010, supra). Current treatment options include corticosteroids, immunosuppressives, antiplatelet regimens, and plasma exchange.

DDD is diagnosed by the absence of immunoglobulin and presence of C3 by immunofluorescence staining of renal biopsies, and electron microscopy shows characteristic dense osmiophilic deposits along the glomerular basement membranes. DDD is caused by dysregulation of the alternative pathway of complement (Sethi et al, Clin J Am Soc Nephrol. 6(5):1009-17, 2011), which can arise from a number of different mechanisms. The most common complement system abnormality in DDD is the presence of C3 nephritic factors which are autoantibodies to the alternative pathway C3 convertase (C3bBb) that increases its half-life and therefore activation of the pathway (Smith, R.J.H. et al., Mol. Immunol. 48:1604-1610, 2011). Other alternative pathway abnormalities include factor H autoantibody that blocks the function of factor H, gain of function C3 mutations, and genetic deficiency of factor H (Smith et al., 2011, supra). Recent case reports show that eclizumab (anti-C5 monoclonal antibody) treatment was associated with improvements in renal function in two patients with DDD (Daina, E. et al., New Engl. J. Med. 366:1161-1163, 2012; Vivarelli, M. et al., New Engl. J. Med. 366:1163-1165, 2012), suggesting a causative role for complement activation in renal outcomes.

Given the above genetic, functional and immunohistochemical and anecdotal clinical data, the central role for complement in the pathogenesis of DDD is well established. Thus, interventions that block the disease-causing mechanisms of complement activation, or the subsequent complement activation products, are expected to be therapeutically useful to treat this condition.

While the human genetic data suggest that inappropriate control or excessive activation of the alternative pathways amplification loop plays a key role, complement-initiating events have not been identified. Immunohistochemical studies in renal biopsies show evidence of MBL deposition in diseased tissue, suggesting involvement of the lectin pathways in the initiation of pathological complement activation in DDD (Lhotta et al, Nephrol Dial Transplant., 14(4):881-6, 1999). The importance of the alternative pathway has been further corroborated in experimental models. Factor H-deficient mice develop progressive proteinuria and the renal pathological lesions characteristic of the human condition (Pickering et al., Nat Genet., 31(4):424, 2002). Pickering et al. further demonstrated that ablation of factor B, which mediates LEA-1-dependent activation of the alternative pathway, fully protects factor H-deficient mice from DDD (Pickering et al., Nat Genet., 31(4):424, 2002).

Thus it is expected that agents that block LEA-1 will effectively block lectin-dependent activation of the alternative pathway, and will thus provide an effective treatment for DDD. Given that the alternative pathway amplification loop is dysregulated in DDD patients, it can further be expected that agents that block the amplification loop will be effective. Since LEA-1-targeting agents that block MASP-1 or MASP-1 and MASP-3 inhibit the maturation of factor D, such agents are predicted to effectively block the alternative pathway amplification loop.

As detailed above, pronounced MBL deposition has been found in diseased renal specimens, highlighting the probable involvement of lectin-driven activation events in DDD pathogenesis. Once an initial tissue injury to the glomerular capillaries is established, it is likely that additional MBL binding to injured glomerular endothelium and underlying mesangial structures occurs. Such tissue injuries are well known to lead to activation of LEA-2, which can thus cause further complement activation. Therefore, LEA-2-blocking agents are also expected to have utility in preventing further complement activation on injured glomerular structures, and thus forestall further disease progression towards end stage renal failure.

The data detailed above suggest that LEA-1 and LEA-2 promote separate pathologic complement activation processes in DDD. Thus, a LEA-1-blocking agent and a LEA-2 blocking agent, either alone or in combination are expected to be useful for treating DDD.

When used in combination, LEA-1- and LEA-2-blocking agents are expected to be more efficacious than either agent alone, or useful for treating different stages of the disease. LEA-1- and LEA-2-blocking agents may thus have complementary, additive or synergistic effects in preventing, treating or reversing DDD-associated renal dysfunction.

Combined LEA-1 and LEA-2 inhibition may be accomplished by co-administration of a LEA-1 blocking agent and a LEA2 blocking agent. Optimally, LEA-1 and LEA-2 blocking agents with inhibitory function may be encompassed in a single molecular entity, such as a bispecific antibody composed of MASP-1/3 and a MASP-2-specific binding site, or a dual-specificity antibody where each binding site can bind to and block MASP-1/3 or MASP-2.

In accordance with the foregoing, an aspect of the invention thus provides a method for inhibiting LEA-1-dependent complement activation for treating, preventing, or reducing the severity of dense deposit disease, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing dense deposit disease. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, preventing, or reducing the severity of dense deposit disease, comprising administering a therapeutically effective amount of a MASP-2 inhibitory agent to a subject suffering from, or at risk for developing dense deposit disease. In another aspect, a method is provided comprising inhibiting both LEA-1 and LEA-2-dependent complement activation for treating, preventing, or reducing the severity of dense deposit disease, comprising administering a therapeutically effective amount of a MASP-2 inhibitory agent and a MASP-1, MASP-3, or MASP-1/3-inhibitory agent to a subject suffering from, or at risk for developing dense deposit disease.

In some embodiments, the method comprises inhibiting both LEA-1-dependent complement activation and LEA-2-dependent complement activation. As detailed above, the use of a combination of pharmacologic agents that individually block LEA-1 and LEA-2, is expected to provide an improved therapeutic outcome in treating, preventing or reducing the severity of dense deposit disease as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The LEA-1 and/or LEA-2 inhibitory agents may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating, preventing or reducing the severity of dense deposit disease in a subject in need thereof. Alternatively, the composition may be administered at periodic intervals such as daily, biweekly, weekly, every other week, monthly or bimonthly over an extended period of time for treatment of a subject in need thereof.

## XI. THE ROLE OF MASP-2 AND MASP-3 IN PAUCI-IMMUNE NECROTIZING CRESCENTIC GLOMERULONEPHRITIS, AND THERAPEUTIC METHODS USING MASP-2 AND MASP-3 INHIBITORY AGENTS

Pauci-immune necrotizing crescentic glomerulonephritis (NCGN) is a form of rapidly progressive glomerulonephritis in which glomerular capillary walls show signs of inflammation yet have a paucity of detectable immunocomplex deposition or antibodies against the glomerular basement membrane. The condition is associated with a rapid decline in renal function. Most patients with NCGN are found to have antineutrophil cytoplasmic autoantibodies (ANCA) and thus belong to a group of diseases termed ANCA-associated vasculitis. Vasculitis is a disorder of blood vessels characterized by inflammation and fibrinoid necrosis of the vessel wall. Systemic vasculitides are classified based on vessel size: large, medium, and small. Several forms of small-vessel vasculitis are associated with the presence of ANCA, namely Wegener granulomatosis, microscopic polyangiitis, Churg-Strauss syndrome, and renal-limited vasculitis (NCGN). They can also be a manifestation of underlying conditions such as systemic lupus erythematosus. The target antigens for ANCA include proteinase-3 (PR3) and myeloperoxidase (MPO). Pauci-immune NCGN is rare, with a reported incidence of approximately 4 per million in Wessex, United Kingdom (Hedger, N. et al., Nephrol. Dial. Transplant. 15:1593-1599, 2000). In the Wessex series of 128 patients with pauci-immune NCGN, 73% were ANCA-positive and initial dialysis was required by 59% of patients and 36% needed long-term dialysis. Treatments for pauci-immune NCGN include corticosteroids and immunsuppressive agents such as cyclophosphamide and azathioprine. Additional treatment options for ANCA-associated vasculitides include rituximab and plasma exchange (Chen, M. and Kallenberg, C.G.M. Nat. Rev. Rheumatol. 6:653-664, 2010).

Although NCGN is characterized by a paucity of complement deposition, the alternative pathway of complement has been implicated in its pathogenesis. A renal biopsy evaluation of 7 patients with MPO-ANCA-associated pauci-immune NCGN detected the presence of membrane attack complex, C3d, factor B, and factor P (which were not detected in biopsies from normal controls or patients with minimal change disease), whereas C4d and mannose binding lectin were not detected, suggesting selective activation of the alternative pathway (Xing, G.Q. et al. J. Clin. Immunol. 29:282-291, 2009). Experimental NCGN can be induced by transfer of anti-MPO IgG into wild-type mice or anti-MPO splenocytes into immune-deficient mice (Xiao, H. et al. J. Clin. Invest. 110:955-963, 2002). In this mouse model of NCGN, the role of specific complement activation pathways was investigated using knockout mice. After injection of anti-MPO IgG, $C4^{-/-}$ mice developed renal disease comparable to wild-type mice whereas $C5^{-/-}$ and factor $B^{-/-}$ mice did not develop renal disease, indicating that the alternative pathway was involved in this model and the classical and lectin pathways were not (Xiao, H. et al. Am. J. Pathol. 170:52-64, 2007). Moreover, incubation of MPO-ANCA or PR3-ANCA IgG from patients with TNF-$\alpha$-primed human neutrophils caused release of factors that resulted in complement activation in normal human serum as detected by generation of C3a; this effect was not observed with IgG from healthy subjects, suggesting the potential pathogenic role of ANCA in neutrophil and complement activation (Xiao et al., 2007, supra).

Based on the role outlined above for the alternative pathway in this condition, it is expected that blocking the activation of the alternative pathway will have utility in the treatment of ANCA positive NCGN. Given the requirement for fB activation for pathogenesis, it is expected that inhibitors of LEA-1 will be particularly useful in treating this condition, and in preventing the further decline in renal function in these patients.

Yet another subset of patients develops progressive renal vasulitis with crescent formation accompanied by a rapid decline in renal function in the absence of ANCA. This form of the condition is termed ANCA-negative NCGN and constitutes about one third of all patients with pauci immune NCGN (Chen et al, JASN 18(2): 599-605, 2007). These patients tend to be younger, and renal outcomes tend to be particularly severe. (Chen et al., Nat Rev Nephrol., 5(6):313-8, 2009). A discriminating pathological feature of these patients is the deposition of MBL and C4d in renal lesions (Xing et al., J Clin Immunol. 30(1):144-56, 2010). MBL and C4d staining intensity in renal biopsies correlated negatively with renal function (Xing et al., 2010, supra). These findings suggest an important role for lectin-dependent complement activation in pathogenesis. The fact that C4d, but not factor B is commonly found in diseased tissue specimens indicates LEA-2 involvement.

Based on the role of lectin-dependent complement activation in ANCA negative NCGN described above, it is expected that blocking the activation of the LEA-2 pathway will have utility in the treatment of ANCA negative NCGN.

The data detailed above suggest that LEA-1 and LEA-2 mediate pathologic complement activation in ANCA-positive and ANCA-negative NCGN, respectively. Thus, a LEA-1-blocking agent combined with a LEA-2-blocking agent is expected to have utility in the treatment of all forms of pauci-immune NCGN, regardless of the underlying etiology. LEA-1- and LEA-2-blocking agents may thus have complementary, additive or synergistic effects in preventing, treating or reversing NCGN-associated renal dysfunction.

LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1 blocking agent and a LEA2 blocking agent. Optimally, LEA-1 and LEA-2 inhibitory function may be encompassed in a single molecular entity, such as a bispecific antibody composed of MASP-1/3 and a MASP-2-specific binding site, or a dual-specificity antibody where each binding site can bind to and block MASP-1/3 or MASP-2.

In accordance with the foregoing, an aspect of the invention thus provides a method for inhibiting LEA-1-dependent complement activation for treating, preventing, or reducing the severity of pauci-immune necrotizing crescentic glomerulonephritis, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing pauci-immune necrotizing crescentic glomerulonephritis. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, preventing, or reducing the severity of pauci-immune necrotizing crescentic glomerulonephritis, comprising administering a therapeutically effective amount of a MASP-2 inhibitory agent to a subject suffering from, or at risk for developing pauci-immune necrotizing crescentic glomerulonephritis. In another aspect, a method is provided comprising inhibiting both LEA-1 and LEA-2-dependent complement activation for treating, preventing, or reducing the severity of pauci-immune necrotizing crescentic glomerulonephritis, comprising administering a therapeutically effective amount of a MASP-2 inhibitory agent and a MASP-1, MASP-3, or MASP-1/3 inhibitory agent to a subject in need thereof.

In some embodiments, the method comprises inhibiting both LEA-1-dependent complement activation and LEA-2-dependent complement activation. As detailed above, the use of a combination of pharmacologic agents that individually block LEA-1 and LEA-2, is expected to provide an improved therapeutic outcome in treating or preventing or reducing the severity of pauci-immune necrotizing crescentic glomerulonephritis as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating, preventing or reducing the severity of pauci-immune necrotizing crescentic glomerulonephritis. Alternatively, the composition may be administered at periodic intervals such as daily, biweekly, weekly, every other week, monthly or bimonthly over an extended period of time for treatment of a subject in need thereof.

XII. THE ROLE OF MASP-2 AND MASP-3 IN TRAUMATIC BRAIN INJURY, AND THERAPEUTIC METHODS USING MASP-2 AND MASP-3 INHIBITORY AGENTS

Traumatic brain injury (TBI) is a major global health problem that leads to at least 10 million deaths or hospitalizations annually (Langlois, J.A. et al., J. Head Trauma Rehabil. 21:375-378, 2006). In 2003 there were an estimated 1.6 million TBIs in the United States, including 1.2 million emergency department visits, 290,000 hospitalizations, and 51,000 deaths (Rutland-Brown, W. et al., J. Head Trauma Rehabil. 21:544-548, 2006). The majority of TBIs in the United States are caused by falls and motor vehicle traffic. TBI can result in long-term or lifelong physical, cognitive, behavioral, and emotional consequences. Over 5 million Americans are living with long-term or lifelong disability associated with a TBI (Langlois et al., 2006, supra).

TBI may involve penetration of the brain substance ("penetrating" injuries) or injuries that do not penetrate the brain ("closed" injuries). The injury profiles and associated neurobehavioral sequelae can be quite different between penetrating and closed TBI. Although each injury is unique, certain brain regions are particularly vulnerable to trauma-induced damage, including the frontal cortex and subfrontal white matter, the basal ganglia and diencephalon, the rostral brain stem, and the temporal lobes including the hippocampi (McAllister, T.W. Dialogues Clin. Neurosci. 13:287-300, 2011). TBI can lead to changes in several neurotransmitter systems, including release of glutamate and other excitatory amino acids during the acute phase and chronic alterations in the catecholaminergic and cholinergic systems, which may be associated with neurobehavioral disability (McAllister, 2011, supra). Survivors of significant TBI often suffer from cognitive defects, personality changes, and increased psychiatric disorders, particularly depression, anxiety, and post-traumatic stress disorder. Despite intense research, no clinically effective treatment for TBI that can reduce mortality and morbidity and improve functional outcome has yet to be found.

*Complement factors and TBI*

Numerous studies have identified a relationship of complement proteins and neurological disorders, including Alzheimer's disease, multiple sclerosis, myasthenia gravis, Guillain-Barre syndrome, cerebral lupus, and stroke (reviewed in Wagner, E., et al., Nature Rev Drug Disc. 9: 43-56, 2010). Recently a role for C1q and C3 in synapse elimination has been demonstrated, thus complement factors are likely involved in both normal CNS function and neurodegenerative disease (Stevens, B. et al., Cell 131: 1164-1178, 2007). The gene for MASP-1 and MASP-3 is extensively expressed in the brain and also in a glioma cell line, T98G (Kuraya, M. et al., Int Immunol., 15:109-17, 2003), consistent with a role of the lectin pathway in the CNS.

MASP-1 and MASP-3 are key to immediate defense against pathogens and altered self-cells, but the lectin pathway also is responsible for severe tissue damage after stroke, heart attack, and other ischemia reperfusion injuries. Similarly, MASP-1 and MASP-3 are likely mediators in the tissue damage caused by TBI. Inhibition of Factor B in the alternative pathway has been shown to attenuate TBI in two mouse models. Factor B knockout mice are protected from complement-mediated neuroinflammation and neuropathology after TBI (Leinhase I, et al., BMC Neurosci. 7:55, 2006). In addition, anti-factor B antibody attenuated cerebral tissue damage and neuronal cell death in TBI induced mice (Leinhase I, et al., J Neuroinflammation 4:13, 2007). MASP-3 directly activates Factor B (Iwaki, D. et al., J Immunol. 187:3751-8, 2011) and therefore is also a likely mediator in TBI. Similar to inhibition of Factor B, LEA-1 inhibitors, such as antibodies against MASP-3 are expected to provide a promising strategy for treating tissue damage and subsequent sequelae in TBI.

Thus, LEA-1 and LEA-2 inhibitors may have independent therapeutic benefit in TBI. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA2-blocking agent. Optimally, LEA-1 and LEA-2 inhibitory function may be encompassed in a single molecular entity, such as a bispecific antibody composed of MASP-1/3 and a MASP-2-specific binding site, or a dual-specificity antibody where each binding site can bind to and block MASP-1/3 or MASP-2.

In accordance with the foregoing, an aspect of the invention thus provides a method for inhibiting LEA-1-dependent complement activation for treating, or reducing the severity of traumatic brain injury, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from a traumatic brain injury. The MASP-1, MASP-3, or MASP-1/3 inhibitory composition may be administered to the subject systemically, such as by intra-arterial, intravenous, intramuscular, inhalational, nasal, intracranial, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, or reducing the severity of traumatic brain injury, comprising administering a therapeutically effective amount of a MASP-2 inhibitory agent to a subject suffering from a traumatic brain injury. In another aspect, a method is provided comprising inhibiting both LEA-1 and LEA-2-dependent complement activation for treating, or reducing the severity of traumatic brain injury, comprising administering a therapeutically effective amount of a MASP-2 inhibitory agent and a MASP-1, MASP-3, or MASP-1/3 inhibitory agent to a subject suffering from a traumatic brain injury.

In some embodiments, the method comprises inhibiting both LEA-1-dependent complement activation and LEA-2-dependent complement activation. As detailed above, the use of a combination of pharmacologic agents that individually block LEA-1 and LEA-2 is expected to provide an improved therapeutic outcome in treating or reducing the severity of traumatic brain injury as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject systemically, such as by intra-arterial, intravenous, intramuscular, inhalational, nasal, subcutaneous, intracranial, or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating or reducing the severity of traumatic brain injury. Alternatively, the composition may be administered at periodic intervals such as daily, biweekly, weekly, every other week, monthly or bimonthly over an extended period of time for treatment of a subject in need thereof.

XIII. THE ROLE OF MASP-2 AND MASP-3 IN ASPIRATION PNEUMONIA, AND THERAPEUTIC METHODS USING MASP-2 AND MASP-3 INHIBITORY AGENTS

Aspiration is defined as the inhalation of either oropharyngeal or gastric contents into the lower airways. Aspiration may result in complications of aspiration (chemical) pneumonitis, primary bacterial aspiration pneumonia, or secondary bacterial infection of chemical pneumonitis. Risk factors for aspiration include decreased levels of consciousness (e.g., head trauma, alcohol or drug-induced alterations in sensorium, stroke), various gastrointestinal and esophageal abnormalities, and neuromuscular diseases. It is estimated that 5-15% of the 4.5 million cases of community-acquired pneumonia are due to aspiration pneumonia (Marik, P.E. New Engl. J. Med. 344:665-671, 2001). Treatment of chemical pneumonitis is mainly supportive and the use of empiric antibiotics is controversial. Treatment of bacterial aspiration pneumonia is with appropriate antibiotics, which is based on whether the aspiration occurred in the community or in the hospital as the likely causative organisms differ between these settings. Measures should be taken to prevent aspiration in high-risk patients, for example elderly patients in nursing homes who have impaired gag reflexes. Measures that have been shown to be effective prophylaxis include elevation of the head of the bed while feeding, dental prophylaxis, and good oral hygiene. Prophylactic antibiotics have not been shown to be effective and are discouraged as they may lead to the emergence of resistant organisms.

Modulation of complement components has been proposed for numerous clinical indications, including infectious disease---sepsis, viral, bacterial, and fungal infectionsand pulmonary conditions---respiratory distress syndrome, chronic obstructive pulmonary disease, and cystic fibrosis (reviewed in Wagner, E., et al., Nature Rev Drug Disc. 9: 43-56, 2010). Support for this proposal is provided by numerous clinical and genetic studies. For example, there is a significantly decreased frequency of patients with low MBL levels with clinical tuberculosis (Soborg et al., Journal of Infectious Diseases 188:777-82, 2003), suggesting that low levels of MBL are associated with protection from disease.

In a murine model of acid aspiration injury, Weiser MR et al., J. Appl. Physiol. 83(4): 1090-1095, 1997, demonstrated that C3-knockout mice were protected from serious injury; whereas C4-knockout mice were not protected, indicating that complement activation is mediated by the alternative pathway. Consequently, blocking the alternative pathway with LEA-1 inhibitors is expected to provide a therapeutic benefit in aspiration pneumonia.

Thus, LEA-1 and LEA-2 inhibitors may have independent therapeutic benefit in aspiration pneumonia. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bi-specific antibody composed of MASP-1/3 and a MASP-2-specific binding site, or a dual-specificity antibody where each binding site binds to and blocks MASP-1/3 or MASP-2

An aspect of the invention thus provides a method for inhibiting LEA-1-dependent complement activation to treat aspiration pneumonia by administering a composition comprising a therapeutically effective amount of a MASP-1 inhibitory agent, a MASP-3 inhibitory agent, or a combination of a MASP-1/3 inhibitory agent, in a pharmaceutical carrier to a subject suffering from such a condition or other complement-mediated pneumonia. The MASP-1, MASP-3, or MASP-1/3 inhibitory composition may be administered locally to the lung, as by an inhaler. Alternately, the MASP-1, MASP-3, or MASP-1/3 inhibitory agent may be administered to the subject systemically, such as by intra-arterial, intravenous, intramuscular, inhalational, nasal, subcutaneous or other parenteral administration, or potentially by oral administration. Administration may be repeated as determined by a physician until the condition has been resolved or is controlled.

In accordance with the foregoing, an aspect of the invention thus provides a method for inhibiting LEA-1-dependent complement activation for treating, preventing or reducing the severity of aspiration pneumonia, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing aspiration pneumonia. The MASP-1, MASP-3, or MASP-1/3 inhibitory composition may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, preventing or reducing the severity of aspiration pneumonia, comprising administering a therapeutically effective amount of a MASP-2 inhibitory agent to a subject suffering from, or at risk for developing aspiration pneumonia. In another aspect, a method is provided comprising inhibiting both LEA-1 and LEA-2-dependent complement activation for treating, or reducing the severity of aspiration pneumonia, comprising administering a therapeutically effective amount of a MASP-2 inhibitory agent and a MASP-1, MASP-3, or MASP-1/3 inhibitory agent to a subject suffering from aspiration pneumonia.In some embodiments, the method comprises inhibiting both LEA-1-dependent complement activation and LEA-2-dependent complement activation. As detailed above, the use of a combination of pharmacologic agents that individually block LEA-1 and LEA-2, is expected to provide an improved therapeutic outcome in treating or reducing the severity of aspiration pneumonia as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered to the subject systemically, such as by intra-arterial, intravenous, intramuscular, inhalational, nasal, subcutaneous, or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual-inhibitory agents, or co-administration of separate compositions), or a limited sequence of administrations, for treating, preventing or reducing the severity of aspiration pneumonia in a subject in need thereof. Alternatively, the composition may be administered at periodic intervals such as daily, biweekly, weekly, every other week, monthly or bimonthly over an extended period of time for treatment of a subject in need thereof.

XIV. THE ROLE OF MASP-2 AND MASP-3 IN ENDOPHTHALMITIS, AND THERAPEUTIC METHODS USING MASP-2 AND MASP-3 INHIBITORY AGENTS

Endophthalmitis is an inflammatory condition of the intraocular cavities and is usually caused by infection. Endophthalmitis may be endogenous, resulting from hematogenous spread of organisms from a distant source of infection (e.g., endocarditis), or exogenous, from direct inoculation of an organism from the outside as a complication of ocular surgery, foreign bodies, and/or blunt or penetrating ocular trauma. Exogeneous endophthalmitis is much more common than endogenous and most cases of exogeneous endophthalmitis occur following ocular surgery. In the United States, cataract surgery is the leading cause of endophthalmitis and occurs in 0.1-0.3% of this procedure, with an apparent increase in the incidence over the last decade (Taban, M. et al., Arch. Ophthalmol. 123:613-620, 2005). Post-surgical endophthalmitis may present either acutely, within 2 weeks of surgery, or delayed, months after surgery. Acute endophthalmitis typically presents with pain, redness, lid swelling, and decreased visual acuity. Delayed-onset endophthalmitis is less common than the acute form and patients may report only mild pain and photosensitivity. Treatment of endophthalmitis depends on the underlying cause and may include systemic and/or intravitreal antibiotics. Endophthalmitis may result in decreased or loss of vision.

As previously described for AMD, multiple complement pathway genes have been associated with ophthalmologic disorders, and these specifically include genes of the lectin pathway. For example, MBL2 has been identified with subtypes of AMD (Dinu V, et al., Genet Epidemiol 31: 224-37, 2007). The LEA-1 and LEA-2 pathways are likely to be involved in ocular inflammatory conditions such as endophthalmitis (Chow SP et al., Clin Experiment Ophthalmol. 39:871-7, 2011). Chow et al. examined MBL levels of patients with endophthalmitis and demonstrated that both MBL levels and functional lectin pathway activity are significantly elevated in inflamed human eyes but virtually undetectable in non-inflamed control eyes. This suggests a role for MBL and the lectin pathway in sight-threatening ocular inflammatory conditions, particularly endophthalmitis. Furthermore, in a murine model of corneal fungal keratitis, the MBL-A gene was one of five upregulated inflammatory pathway genes (Wang Y., et al., Mol Vis 13: 1226-33, 2007).

Thus, LEA-1 and LEA-2 inhibitors are expected to have independent therapeutic benefit in treating endophthalmitis. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bi-specific antibody composed of MASP-1/3 and a MASP-2-specific binding site, or a dual-specificity antibody where each binding site binds to and blocks MASP-1/3 or MASP-2

In accordance with the foregoing, an aspect of the invention thus provides a method for inhibiting LEA-1-dependent complement activation for treating, preventing, or reducing the severity of endophthalmitis, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing endophthalmitis. The MASP-1, MASP-3, or MASP-1/3 inhibitory composition may be administered locally to the eye, such as by irrigation or application of the composition in the form of a topical gel, salve or drops, or by intravitreal administration. Alternately, the MASP-1, MASP-3, or MASP-1/3 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, preventing, or reducing the severity of endophthalmitis, comprising administering a therapeutically effective amount of a MASP-2 inhibitory agent to a subject suffering from, or at risk for developing endophthalmitis. In another aspect, a method is provided comprising inhibiting both LEA-1 and LEA-2-dependent complement activation for treating, or reducing the severity of endophthalmitis, comprising administering a therapeutically effective amount of a MASP-2 inhibitory agent and a MASP-1, MASP-3, or MASP-1/3 inhibitory agent to a subject suffering from endophthalmitis.

In some embodiments, the method comprises inhibiting both LEA-1-dependent complement activation and LEA-2-dependent complement activation. As detailed above, the use of a combination of pharmacologic agents that individually block LEA-1 and LEA-2 is expected to provide an improved therapeutic outcome in treating or preventing or reducing the severity of endophthalmitis, as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered locally to the eye, such as by irrigation or application of the composition in the form of a topical gel, salve or drops, or by intravitreal injection. Alternately, the MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating, preventing or reducing the severity of endophthalmitis in a subject in need thereof. Alternatively, the composition may be administered at periodic intervals such as daily, biweekly, weekly, every other week, monthly or bimonthly over an extended period of time for treatment of a subject in need thereof.

XV. THE ROLE OF MASP-2 AND MASP-3 IN NEUROMYELITIS OPTICA, AND THERAPEUTIC METHODS USING MASP-2 AND MASP-3 INHIBITORY AGENTS

Neuromyelitis optica (NMO) is an autoimmune disease that targets the optic nerves and spinal cord. This results in inflammation of the optic nerve, known as optic neuritis, and the spinal cord, known as myelitis. Spinal cord lesions in NMO may lead to weakness or paralysis in the legs or arms, blindness, bladder and bowel dysfunction, and sensory dysfunction.

NMO shares several similarities to multiple sclerosis (MS), since both are due to immune attack of CNS targets and both result in demyelination (Papadopoulos and Verkman, Lancet Neurol., 11(6):535-44, 2013). However, the molecular targets, treatments, and lesions for NMO are distinct from those of MS. While MS is largely mediated by T cells, NMO patients typically have antibodies that target the water channel protein aquaporin 4 (AQP4), a protein found in astrocytes that surround the blood-brain barrier. Interferon beta is the most commonly used therapy for MS, but it is generally acknowledged to be harmful in NMO. The inflammatory lesions of NMO are found in the spinal cord and optic nerve and may progress to the brain, including white and gray matter. The demyelination that occurs in NMO lesions is mediated by complement (Papadopoulos and Verkman, Lancet Neurol., 11(6):535-44, 2013).

Complement-dependent cytotoxicity appears to be the major mechanism causing development of NMO. Over 90% of NMO patients have IgG antibodies against AQP4 (Jarius and Wildemann, Jarius S, Wildemann B., Nat Rev Neurol. 2010 Jul;6(7):383-92). These antibodies initiate formation of a lesion at the blood brain barrier. The initial antigen-antibody complex---AQP4/AQP4-IgG---on the surface of astrocytes activates the classical pathway of complement. This results in formation of the membrane attack complex on the astrocyte surface, leading to granulocyte infiltration, demyelination, and ultimately necrosis of astrocytes, oligodendrocytes and neurons (Misu et al., Acta Neuropathol 125(6):815-27, 2013). These cellular events are reflected in tissue destruction and formation of cystic, necrotic lesions.

The classical pathway of complement clearly is critical for NMO pathogenesis. NMO lesions show a vasculocentric deposition of immunoglobulin and activated complement components (Jarius et al., Nat Clin Pract Neurol. 4(4):202-14, 2008). In addition, complement proteins such as C5a have been isolated from cerebrospinal fluid of NMO patients (Kuroda et al., J Neuroimmunol.,254(1-2):178-82, 2013). Furthermore, serum IgG obtained from NMO patients can cause complement-dependent cytotoxicity in a mouse NMO model (Saadoun et al., Brain, 133(Pt 2):349-61, 2010). A monoclonal antibody against C1q prevents the complement mediated destruction of astrocytes and lesions in a mouse model of NMO (Phuan et al., Acta Neuropathol, 125(6):829-40, 2013).

The alternative pathway of complement serves to amplify overall complement activity. Harboe and colleagues (2004) demonstrated that selective blockade of the alternative pathway inhibited more than 80% of membrane attack complex formation induced by the classical pathway (Harboe et al., Clin Exp Immunol 138(3):439-46, 2004). Tüzün and colleagues (2013) examined both classical and alternative pathway products in NMO patients (Tüzün E, et al., J Neuroimmunol. 233(1-2): 211-5, 2011). C4d, the breakdown product of C4, was measured to evaluate classical pathway activity and was increased in NMO patient sera compared to controls (an elevation of 2.14 fold). In addition, an increase of Factor Bb, the breakdown product of the alternative pathway Factor B, was observed in NMO patients compared to MS patients or normal control individuals (an elevation of 1.33 fold). This suggests that alternative pathway function is also increased in NMO. This activation would be expected to increase overall complement activation, and in fact sC5b-9, the final product of the complement cascade, was significantly increased (a 4.14 fold elevation).

Specific inhibitors of MASP-3 are expected to provide benefit in treating patients suffering from NMO. As demonstrated in Examples 17 and 18, serum lacking MASP-3 is unable to activate Factor B, an essential component of C5 convertase, or Factor D, the central activator of the alternative pathway. Therefore, blocking MASP-3 activity with an inhibitory agent such as an antibody or small molecule would also be expected to inhibit activation of Factor B and Factor D. Inhibition of these two factors will arrest the amplification of the alternative pathway, resulting in diminished overall complement activity. MASP-3 inhibition should thus significantly improve therapeutic outcomes in NMO.

Thus, LEA-1 and/or LEA-2 inhibitors are expected to have independent therapeutic benefit in treating NMO. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bi-specific antibody composed of MASP-1/3 and a MASP-2-specific binding site, or a dual-specificity antibody where each binding site binds to and blocks MASP-1/3 or MASP-2

In accordance with the foregoing, an aspect of the invention thus provides a method for inhibiting LEA-1-dependent complement activation for treating, preventing, or reducing the severity of NMO, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing NMO. The MASP-1, MASP-3, or MASP-1/3 inhibitory composition may be administered locally to the eye, such as by irrigation or application of the composition in the form of a topical gel, salve or drops, or by intravitreal administration. Alternately, the MASP-1, MASP-3, or MASP-1/3 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

In another aspect, a method is provided for inhibiting LEA-2-dependent complement activation for treating, preventing, or reducing the severity of NMO, comprising administering a therapeutically effective amount of a MASP-2 inhibitory agent to a subject suffering from, or at risk for developing NMO. In another aspect, a method is provided comprising inhibiting both LEA-1 and LEA-2-dependent complement activation for treating, or reducing the severity of NMO, comprising administering a therapeutically effective amount of a MASP-2 inhibitory agent and a MASP-1, MASP-3, or MASP-1/3 inhibitory agent to a subject suffering from NMO.

In some embodiments, the method comprises inhibiting both LEA-1-dependent complement activation and LEA-2-dependent complement activation. As detailed above, the use of a combination of pharmacologic agents that individually block LEA-1 and LEA-2 is expected to provide an improved therapeutic outcome in treating or preventing or reducing the severity of NMO, as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered locally to the eye, such as by irrigation or application of the composition in the form of a topical gel, salve or drops, or by intravitreal injection. Alternately, the MASP-2 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating, preventing or reducing the severity of NMO in a subject in need thereof. Alternatively, the composition may be administered at periodic intervals such as daily, biweekly, weekly, every other week, monthly or bimonthly over an extended period of time for treatment of a subject in need thereof.


XVI. THE ROLE OF MASP-2 AND MASP-3 IN BEHCET'S DISEASE, AND THERAPEUTIC METHODS USING MASP-2 AND MASP-3 INHIBITORY AGENTS


Behçet's disease, or Behçet's syndrome, is a rare, immune-mediated small-vessel systemic vasculitis that often presents with mucous membrane ulceration and ocular problems. Behçet's disease (BD) was named in 1937 after the Turkish dermatologist Hulusi Behçet, who first described the triple-symptom complex of recurrent oral ulcers, genital ulcers, and uveitis. BD is a systemic, relapsing inflammatory disorder of unknown cause. The inflammatory perivasculitis of BD may involve the gastrointestinal tract, pulmonary, musculoskeletal, cardiovascular, and neurological systems. BD can be fatal due to ruptured vascular aneurysms or severe neurological complications. Optic neuropathy and atrophy may result from vasculitis and occlusion of the vessels supplying the optic nerve. See Al-Araji A, et al., Lancet Neurol., 8(2): 192-204, 2009.

The highest incidence of BD is in the Middle East and Far East regions, but it is rare in Europe and North America. BD is often initially controlled with corticosteroids and immunosuppressants, but many cases are refractory with serious morbidity and mortality. Biologic agents, including interferon-alpha, IVIG, anti-TNF, anti-IL-6, and anti-CD20, have shown benefit in some cases, but there is no consensus on best treatment.

While BD is clearly an inflammatory disorder, its pathobiology is not clear. There are genetic associations with HLA antigens, and genome wide association studies have implicated numerous cytokine genes (Kirino et al., Nat Genet, 45(2):202-7, 2013). The hyperactivity of the immune system appears to be regulated by the complement system. Increased levels of C3 have been observed in BD patient sera (Bardak and Aridogan, Ocul Immunol Inflamm 12(1):53-8, 2004), and elevated C3 and C4 in the cerebrospinal fluid correlates with disease (Jongen et al., Arch Neurol, 49(10):1075-8, 1992).

Tüzün and colleagues (2013) examined both classical and alternative pathway products in sera of BD patients (Tüzün E, et al., J Neuroimmunol, 233(1-2):211-5, 2011). 4d, the breakdown product of C4, is generated upstream of the alternative pathway and was measured to evaluate initial classical pathway activity. C4d was increased in BD patient sera compared to controls (an elevation of 2.18 fold). Factor Bb is the breakdown product of Factor B, and was measured to determine activity of the alternative pathway. BD patients had an increase of factor Bb compared to normal control individuals (an elevation of 2.19 fold) consistent with an increase in BD alternative pathway function. Because the alternative pathway of complement serves to amplify overall complement activity, this activation would be expected to increase overall complement activation. Harboe and colleagues (2004) demonstrated that selective blockade of the alternative pathway inhibited more than 80% of membrane attack complex formation induced by the classical pathway (Harboe M, et al., Clin Exp Immunol, 138(3):439-46, 2004). In fact sC5b-9, the final product of the complement cascade, was significantly increased in BD patients (a 5.46 fold elevation). Specific inhibitors of MASP-3 should provide benefit in BD. Blocking MASP-3 should inhibit activation of Factor B and Factor D. This will stop the amplification of the alternative pathway, resulting in a diminished response of overall complement activity. MASP-3 inhibition should thus significantly improve therapeutic outcomes in BD.Thus, LEA-1 and/or LEA-2 inhibitors are expected to have independent therapeutic benefit in treating BD. In addition, LEA-1 and LEA-2 inhibitors used together may achieve additional treatment benefit compared to either agent alone, or may provide effective treatment for a wider spectrum of patient subsets. Combined LEA-1 and LEA-2 inhibition may be accomplished by co-administration of a LEA-1-blocking agent and a LEA-2-blocking agent. Optimally, LEA-1 and LEA-2 inhibitory function may be encompassed in a single molecular entity, such as a bispecific antibody composed of MASP-1/3 and a MASP-2-specific binding site, or a dual-specificity antibody where each binding site binds to and blocks MASP-1/3 or MASP-2.In accordance with the foregoing, an aspect of the invention thus provides a method for inhibiting LEA-1-dependent complement activation for treating, preventing, or reducing the severity of BD, comprising administering a composition comprising a therapeutically effective amount of a LEA-1 inhibitory agent comprising a MASP-1 inhibitory agent, a MASP-3 inhibitory agent, or a combination of a MASP-1/3 inhibitory agent, in a pharmaceutical carrier to a subject suffering from, or at risk for developing BD. The MASP-1, MASP-3, or MASP-1/3 inhibitory composition may be administered locally to the eye, such as by irrigation or application of the composition in the form of a topical gel, salve or drops, or by intravitreal administration. Alternately, the MASP-1, MASP-3, or MASP-1/3 inhibitory agent may be administered to the subject systemically, such as by intra-arterial, intravenous, intramuscular, inhalational, nasal, subcutaneous or other parenteral administration, or potentially by oral administration for non-peptidergic agents. Administration may be repeated as determined by a physician until the condition has been resolved or is controlled.In another aspect, a method is provided for inhibiting LEA-2-dependent complement activation for treating, preventing, or reducing the severity of BD, comprising administering a therapeutically effective amount of a MASP-2 inhibitory agent to a subject suffering from, or at risk for developing BD. In another aspect, a method is provided comprising inhibiting both LEA-1 and LEA-2-dependent complement activation for treating, or reducing the severity of BD, comprising administering a therapeutically effective amount of a MASP-2 inhibitory agent and a MASP-1, MASP-3, or MASP-1/3 inhibitory agent to a subject suffering from BD.

In some embodiments, the method comprises inhibiting both LEA-1-dependent complement activation and LEA-2-dependent complement activation. As detailed above, the use of a combination of pharmacologic agents that individually block LEA-1 and LEA-2 is expected to provide an improved therapeutic outcome in treating or preventing or reducing the severity of BD, as compared to the inhibition of LEA-1 alone. This outcome can be achieved for example, by co-administration of an antibody that has LEA-1-blocking activity together with an antibody that has LEA-2-blocking activity. In some embodiments, LEA-1- and LEA-2-blocking activities are combined into a single molecular entity, and that such entity with combined LEA-1- and LEA-2-blocking activity. Such an entity may comprise or consist of a bispecific antibody where one antigen-combining site specifically recognizes MASP-1 and blocks LEA-1 and the second antigen-combining site specifically recognizes MASP-2 and blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 while the second antigen-combining site specifically recognized MASP-2 and blocks LEA-2.

The MASP-2 inhibitory agent may be administered locally to the eye, such as by irrigation or application of the composition in the form of a topical gel, salve or drops, or by intravitreal injection. Alternately, the MASP-2 inhibitory agent may be administered to the subject systemically, such as by intra-arterial, intravenous, intramuscular, inhalational, nasal, subcutaneous or other parenteral administration, or potentially by oral administration for non-peptidergic agents.

Administration may be repeated as determined by a physician until the condition has been resolved or is controlled.

Application of the MASP-3 inhibitory compositions and/or the MASP-2 inhibitory compositions of the present invention may be carried out by a single administration of the composition (e.g., a single composition comprising MASP-2 and/or MASP-3 inhibitory agents, or bispecific or dual inhibitory agents, or co-administration of separate compositions), or a limited sequence of administrations, for treating, preventing or reducing the severity of BD in a subject in need thereof. Alternatively, the composition may be administered at periodic intervals such as daily, biweekly, weekly, every other week, monthly or bimonthly over an extended period of time for treatment of a subject in need thereof.

## XVII. MASP INHIBITORY AGENTS

With the recognition that the lectin pathway of complement is composed of two major complement activation arms, LEA-1 and LEA-2, and that there also is a lectin-independent complement activation arm, comes the realization that it would be highly desirable to specifically inhibit one or more of these effector arms that cause a pathology associated with at least one of paroxysmal nocturnal hemoglobinuria (PNH), ), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) and thrombotic thrombocytopenic purpura (TTP)), asthma, dense deposit disease, pauci-immune necrotizing crescentic glomerulonephtiris, traumatic brain injury, aspiration pneumonia, endophthalmitis, neuromyelitis optica or Behcet's disease without completely shutting down the immune defense capabilities of complement (i.e., leaving the classical pathway intact). This would leave the C1q-dependent complement activation system intact to handle immune complex processing and to aid in host defense against infection.

### i. Compositions for inhibiting LEA-1-mediated complement activation

As described herein, the inventors have unexpectedly discovered that activation of LEA-1, leading to lysis, is MASP-3-dependent. As further described herein, under physiological conditions, MASP-3-dependent LEA-1 activation also contributes to opsonization, thereby providing an additive effect with LEA-2-mediated complement activation. As demonstrated in Example 7, in the presence of $Ca^{++}$, factor D is not required, as MASP-3 can drive activation of LEA-1 in factor $D^{-/-}$ sera. MASP-3, MASP-1, and HTRA-1 are able to convert pro-factor D to active factor D. Likewise, MASP-3 activation appears, in many instances, to be dependent on MASP-1, since MASP-3 (in contrast to MASP-1 and MASP-2) is not an auto-activating enzyme and is incapable of converting into its active form without the help of MASP-1 (Zundel, S. et al., J.Immunol. 172: 4342-4350 (2004); Megyeri et al., J. Biol. Chem. 288:8922-8934 (2013). As MASP-3 does not autoactivate and, in many instances, requires the activity of MASP-1 to be converted into its enzymatically active form, the MASP-3-mediated activation of the alternative pathway C3 convertase C3Bb can either be inhibited by targeting the MASP-3 zymogen or already-activated MASP-3, or by targeting MASP-1-mediated activation of MASP-3, or both, since, in many instances, in the absence of MASP-1 functional activity, MASP-3 remains in its zymogen form and is not capable of driving LEA-1 through direct formation of the alternative pathway C3 convertase (C3bBb).

Therefore, in one aspect of the invention, the preferred protein component to target in the development of therapeutic agents to specifically inhibit LEA-1 is an inhibitor of MASP-3 (including inhibitors of MASP-1-mediated MASP-3 activation (e.g., a MASP-1 inhibitor that inhibits MASP-3 activation)).

In accordance with the foregoing, in one aspect, the invention provides methods of inhibiting the adverse effects of LEA-1 (i.e., hemolysis and opsonization) in a subject suffering from, or at risk for developing , a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) and thrombotic thrombocytopenic purpura (TTP), asthma, dense deposit disease, pauci-immune necrotizing crescentic glomerulonephritis, traumatic brain injury, aspiration pneumonia, endophthalmitis, neuromyelitis optica and Behcet's disease, comprising administering to the subject a pharmaceutical composition comprising an amount of a MASP-3 inhibitory agent effective to inhibit MASP-3-dependent complement activation and a pharmaceutically acceptable carrier.

MASP-3 inhibitory agents are administered in an amount effective to inhibit MASP-3-dependent complement activation in a living subject suffering from, or at risk for developing, paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica or Behcet's disease. In the practice of this aspect of the invention, representative MASP-3 inhibitory agents include: molecules that inhibit the biological activity of MASP-3, including molecules that inhibit at least one or more of the following: lectin MASP-3-dependent activation of factor B, lectin MASP-3-dependent activation of pro-factor D, MASP-3-dependent, lectin-independent activation of factor B, and MASP-3-dependent, lectin-independent activation of pro-factor D (such as small-molecule inhibitors, MASP-3 antibodies and fragments thereof, or blocking peptides which interact with MASP-3 or interfere with a protein-protein interaction), and molecules that decrease the expression of MASP-3 (such as MASP-3 antisense nucleic acid molecules, MASP-3 specific RNAi molecules and MASP-3 ribozymes). A MASP-3 inhibitory agent may effectively block MASP-3 protein-to-protein interactions, interfere with MASP-3 dimerization or assembly, block Ca$^{++}$ binding, interfere with the MASP-3 serine protease active site, or reduce MASP-3 protein expression, thereby preventing MASP-3 from activating LEA-1-mediated, or lectin-independent, complement activation. The MASP-3 inhibitory agents can be used alone as a primary therapy or in combination with other therapeutics as an adjuvant therapy to enhance the therapeutic benefits of other medical treatments, as further described herein.

In one embodiment, the MASP-3 inhibitory agent specifically binds to a portion of MASP-3 (SEQ ID NO:8) with a binding affinity of at least 10 times greater than to other components in the complement system. In another embodiment, a MASP-3 inhibitory agent specifically binds to a portion of MASP-3 (SEQ ID NO:8) with a binding affinity of at least 100 times greater than to other components in the complement system. In one embodiment, the MASP-3 inhibitory agent specifically binds to the serine protease domain of MASP-3 (aa 450-711 of SEQ ID NO:8) and inhibits MASP-3-dependent complement activation, with the proviso that the inhibitory agent does not bind to the serine protease domain of MASP-1 (SEQ ID NO:10), and it does not bind to the serine protease domain of MASP-2 (SEQ ID NO:5). In one embodiment, the MASP-3 inhibitory agent is a MASP-3 monoclonal antibody, or fragment thereof, that specifically binds to MASP-3.

In another embodiment, the MASP-3 inhibitory agent specifically binds to a portion of MASP-1 (SEQ ID NO:10) with a binding affinity of at least 10 times greater than to other components in the complement system, and inhibits MASP-1-mediated activation of MASP-3. In another embodiment, the MASP-3 inhibitory agent specifically binds to a portion of MASP-1 (SEQ ID NO:10) with a binding affinity of at least 100 times greater than to other components (i.e., polypeptides, or fragments thereof) in the complement system, and inhibits MASP-1-mediated activation of MASP-3. In some embodiments, the MASP-3 inhibitory agent specifically binds to the serine protease domain of MASP-1 (aa 449-694 of SEQ ID NO:10) and inhibits MASP-1-mediated activation of MASP-3, with the proviso that the inhibitory agent does not bind to the serine protease domain of MASP-2 (SEQ ID NO:5), and it does not bind to the serine protease domain of MASP-3 (SEQ ID NO:8). In one embodiment, the MASP-3 inhibitory agent is a MASP-1 monoclonal antibody, or fragment thereof, that specifically binds to MASP-1 and inhibits MASP-1-mediated activation of MASP-3. In some embodiments, the MASP-3 inhibitory agent that binds to MASP-1 inhibits MASP-1-mediated activation of MASP-3 and further inhibits MASP-1-mediated maturation of factor D.

In another embodiment, the MASP-3 inhibitory agent binds to a portion of MASP-3 (SEQ ID ON:8) and also binds to a portion of MASP-1 (SEQ ID NO:10), with the proviso that the inhibitory agent does not bind to MASP-2 (SEQ ID NO:5), or MAp19 (SEQ ID NO:3). In one embodiment, the MASP-3 inhibitory agent binds to a portion of MASP-3 (SEQ ID ON:8) and also binds to a portion of MASP-1 (SEQ ID NO:10), with the proviso that the inhibitory agent does not bind to MASP-2 (SEQ ID NO:5) or MAp19 (SEQ ID NO:3). In one embodiment, the MASP-3 inhibitory agent binds to a portion of MASP-3 (SEQ ID ON:8) and also binds to a portion of MASP-1 (SEQ ID NO:10), with the proviso that the inhibitory agent does not bind to MASP-2 (SEQ ID NO:5), MAp19 (SEQ ID NO:3), or MAp44 (SEQ ID NO:11), thereby providing allowing for a lower effective dose for inhibiting MASP-3-dependent complement activation due to the lack of binding to MAp44, which is present at a high concentration in human serum.

In one embodiment, the MASP-3 inhibitory agent is a MASP-1/MASP-3 dual inhibitory agent that binds to an epitope within the amino acid region that is conserved between MASP-1 and MASP-3, such as the CUBI-CCP2 domain (aa 25-432 of SEQ ID NO:10), as illustrated in **FIGURES 3-5**. In one embodiment, the MASP-3 inhibitory agent is a MASP-1/MASP-3 dual inhibitory agent that binds to an epitope within the amino acid region that is conserved between MASP-1 and MASP-3, with the proviso that the inhibitory agent does not bind to MAp44, such as the CCP domain (aa 367-432 of SEQ ID NO:10). In another embodiment, the MASP-3 inhibitory agent is a bispecific inhibitory agent, such as a bispecific monoclonal antibody, that specifically binds to an epitope on the MASP-3 protein (SEQ ID NO:8) and an epitope on the MASP-1 protein (SEQ ID NO:10). In some embodiments, the MASP-3 inhibitory agent is a bispecific monoclonal antibody that binds to the serine protease domain of MASP-1 (aa 449-694 of SEQ ID NO:10) and also binds to a domain in the serine protease of MASP-3 (aa 450-711 of SEQ ID NO:8).

The binding affinity of the MASP-3 inhibitory agents can be determined using a suitable binding assay.

The inhibition of MASP-3-dependent complement activation is characterized by at least one of the following changes in a component of the complement system that occurs as a result of administration of a MASP-3 inhibitory agent in accordance with the methods of the invention: the inhibition of LEA-1-mediated complement activation (inhibition of hemolysis and/or opsonization); inhibition of lectin-independent conversion of factor B; inhibition of lectin-independent conversion of factor D, inhibition of MASP-3 serine protease substrate-specific cleavage, the reduction of hemolysis (measured, for example as described in Example 5) or the reduction of C3 cleavage and C3b deposition (measured, for example, as described in Example 4 and Example 11).

In some embodiments, the MASP-3 inhibitory agents selectively inhibit MASP-3-dependent complement activation (i.e., LEA-1-mediated complement activation and/or lectin-independent conversion of factor B and/or lectin-independent conversion of factor D), leaving the C1q-dependent complement activation system functionally intact.

In some embodiments, the MASP-3 inhibitory agents are antibodies, or fragments thereof, including MASP-3 antibodies and MASP-3 binding fragments thereof, MASP-1 antibodies and fragments thereof, natural and synthetic peptides, or small-molecules. In some embodiments, the MASP-3 inhibitory agents are small-molecule protease inhibitors that are selective for MASP-1, or selective for MASP-3, or selective for MASP-1 and MASP-3.

ii. Compositions for inhibiting activation of LEA-2

As described herein, LEA-2-mediated complement activation is MASP-2-dependent, leading to opsonization and/or lysis. Therefore, the preferred protein component to target in the development of therapeutic agents to specifically inhibit the LEA-2 lectin-dependent complement system is MASP-2. Several proteins have been shown to bind to, or interact with MASP-2 through protein-to-protein interactions. For example, MASP-2 is known to bind to, and form calcium-dependent complexes with, the lectin proteins MBL, H-ficolin and L-ficolin and collectin-11. Ma Y., et al., J Innate Immun. Epub Dec 4 (2012). Each MASP-2/lectin complex has been shown to activate complement through the MASP-2-dependent cleavage of proteins C4 and C2 (Ikeda, K., et al., J. Biol. Chem. 262:7451-7454, (1987); Matsushita, M., et al., J. Exp. Med. 176:1497-2284, (2000); Matsushita, M., et al., J. Immunol. 168:3502-3506, (2002)). Studies have shown that the CUB1-EGF domains of MASP-2 are essential for the association of MASP-2 with MBL (Thielens, N.M., et al., J. Immunol. 166:5068, (2001)). It has also been shown that the CUB1EGFCUBII domains mediate dimerization of MASP-2, which is required for formation of an active MBL complex (Wallis, R., et al., J. Biol. Chem. 275:30962-30969, 2000). Therefore, MASP-2 inhibitory agents can be identified that bind to or interfere with MASP-2 target regions known to be important for MASP-2-dependent complement activation.

In accordance with the foregoing, in one aspect, the invention provides methods of inhibiting the adverse effects of LEA-2-mediated complement activation in a subject suffering from, or at risk for developing a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, comprising administering to the subject a pharmaceutical composition comprising an amount of a MASP-2 inhibitory agent effective to inhibit MASP-2-dependent complement activation and a pharmaceutically acceptable carrier.

In some embodiments, the invention provides methods of inhibiting the adverse effects of LEA-2-mediated complement activation in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica and Behcet's disease, comprising administering to the subject a pharmaceutical composition comprising an amount of a MASP-2 inhibitory agent effective to inhibit MASP-2 dependent complement activation and a pharmaceutically acceptable carrier. MASP-2 inhibitory agents are administered in an amount effective to inhibit MASP-2-dependent LEA-2 in a living subject suffering from, or at risk for developing PNH, age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica or Behcet's disease. In the practice of this aspect of the invention, representative MASP-2 inhibitory agents include: molecules that inhibit the biological activity of MASP-2 (such as small-molecule inhibitors, MASP-2 antibodies or blocking peptides that interact with MASP-2 or interfere with a protein-protein interaction), and molecules that decrease the expression of MASP-2 (such as MASP-2 antisense nucleic acid molecules, MASP-2 specific RNAi molecules and MASP-2 ribozymes), thereby preventing MASP-2 from activating LEA-2.

A MASP-2 inhibitory agent may effectively block MASP-2 protein-to-protein interactions, interfere with MASP-2 dimerization or assembly, block $Ca^{++}$ binding, interfere with the MASP-2 serine protease active site, or may reduce MASP-2 protein expression, thereby preventing MASP-2 from activating LEA-2. The MASP-2 inhibitory agents can be used alone as a primary therapy or in combination with other therapeutics as an adjuvant therapy to enhance the therapeutic benefits of other medical treatments, as further described herein.

In one embodiment, the MASP-2 inhibitory agent specifically binds to a portion of MASP-2 (SEQ ID NO:5) with a binding affinity of at least 10 times greater than to other antigens in the complement system. In another embodiment, the MASP-2 inhibitory agent specifically binds to a portion of MASP-2 (SEQ ID NO:5) with a binding affinity of at least 100 times greater than to other antigens in the complement system. In one embodiment, the MASP-2 inhibitory agent specifically binds to at least one of (i) the CCP1-CCP2 domain (aa 300-431 of SEQ ID NO:5) or the serine protease domain of MASP-2 (aa 445-682 of SEQ ID NO:5) and inhibits MASP-2-dependent complement activation, with the proviso that the inhibitory agent does not bind to the serine protease domain of MASP-1 (SEQ ID NO:10), and it does not bind to the serine protease domain of MASP-3 (SEQ ID NO:8). In one embodiment, the MASP-2 inhibitory agent is a MASP-2 monoclonal antibody, or fragment thereof that specifically binds to MASP-2.

The binding affinity of the MASP-2 inhibitory agent can be determined using a suitable binding assay.

The inhibition of MASP-2-dependent complement activation is characterized by at least one of the following changes in a component of the complement system that occurs as a result of administration of a MASP-2 inhibitory agent in accordance with the methods of the invention: the inhibition of the generation or production of MASP-2-dependent complement-activation-system products C4b, C3a, C5a and/or C5b-9 (MAC) (measured, for example, as described in Example 2 of US Patent No. 7,919,094), the reduction of C4 cleavage and C4b deposition (measured, for example as described in Example 8 or Example 9), or the reduction of C3 cleavage and C3b deposition (measured, for example, as described in Example 11).

In some embodiments, the MASP-2 inhibitory agents selectively inhibit MASP-2 complement activation (i.e., LEA-2), leaving the C1q-dependent complement activation system functionally intact.

In some embodiments, the MASP-2 inhibitory agents are antibodies, or fragments thereof, including MASP-2 antibodies and MASP-2 binding fragments thereof, natural and synthetic peptides, or small-molecules. In some embodiments, the MASP-2 inhibitory agents are small-molecule protease inhibitors that are selective for MASP-2.

iii. Compositions for inhibiting LEA-1-mediated complement activation and LEA-2-mediated complement activation

In another aspect, the invention provides methods for inhibiting the adverse effects of LEA-1 and inhibiting the adverse effects of LEA-2 in a subject suffering from, or at risk for developing a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, comprising administering to the subject a composition comprising an amount of at least one of a MASP-1 inhibitory agent and/or a MASP-3 inhibitory agent effective to inhibit MASP-3-dependent complement activation.

In one embodiment, the composition comprises a MASP-1 inhibitory agent. In one embodiment, the MASP-1 inhibitory agent inhibits MASP-3-mediated complement activation and also inhibits MASP-2-mediated complement activation.

In one embodiment, the composition comprises a MASP-3 inhibitory agent. In one embodiment, the MASP-3 inhibitory agent inhibits at least one of: lectin MASP-3-dependent activation of factor B; lectin MASP-3-dependent activation of factor D; MASP-3-dependent, lectin-independent activation of factor B; and/or MASP-3-dependent, lectin-independent, activation of factor D.

In one embodiment, the composition comprises a MASP-1 inhibitory agent and a MASP-3 inhibitory agent.

In some embodiments, the method further comprises administering to the subject a composition comprising a MASP-2 inhibitory agent.

In another embodiment, this aspect of the invention comprises administering to a subject suffering from PNH a pharmaceutical composition comprising an amount of a MASP-2 inhibitory agent effective to inhibit MASP-2-dependent complement activation and an amount of a MASP-3 inhibitory agent effective to inhibit MASP-3-dependent complement activation and a pharmaceutically acceptable carrier.

In some embodiments, the composition comprises a single agent that inhibits both LEA-1 and LEA-2 (i.e., a dual MASP-2/MASP-3 inhibitory agent, a dual MASP-1/MASP-2 inhibitory agent, a bispecific MASP-2/MASP-3 inhibitory agent, a bispecific MASP-1/MASP-2 inhibitory agent, or a pan-MASP-1/2/3 inhibitory agent or a trispecific MASP-1/2/3 inhibitory agent). In some embodiments, the composition comprises a combination of LEA-1 and LEA-2 inhibitory agents, for example, a combination of dual inhibitory agents plus a single inhibitory agent, a combination of bispecific inhibitory agents plus a single inhibitory agent, or a combination of any of the MASP-1, MASP-2 and/or MASP-3 inhibitory agents as described herein that in combination inhibit both LEA-1 and LEA-2, as further described herein.

In one embodiment, the invention provides a pharmaceutical composition for inhibiting both LEA-1 and LEA-2, comprising at least one MASP-3 inhibitory agent and at least one MASP-2 inhibitory agent and a pharmaceutically acceptable carrier. In one embodiment, the pharmaceutical composition comprises a combination of a first molecule that is a MASP-3 inhibitory agent and a second molecule that is a MASP-2 inhibitory agent. In another embodiment, the pharmaceutical composition comprises a single molecular entity that includes activity as a MASP-3 inhibitory agent and activity as a MASP-2 inhibitory agent (i.e., an inhibitory agent that inhibits both MASP-2-mediated LEA-2 activation and MASP-3-mediated LEA-1 activation). In one embodiment, the inhibitory agent is a MASP-2/MASP-3 dual inhibitory agent that binds to an epitope within an amino acid region that is conserved between MASP-2 (SEQ ID NO:5) and MASP-3 (SEQ ID NO:8), such as the serine protease domain, for example the N-terminal region of the beta chain (e.g., the first 150 aa of the N-terminal region of the beta chain of SEQ ID NO5 and SEQ ID NO:8:), as shown in **FIGURES 4**, **6** and **7C.** In one embodiment, the inhibitory agent is a bispecific inhibitory agent, such as a bispecific monoclonal antibody, that specifically binds to an epitope on the MASP-2 protein (SEQ ID NO:5) and an epitope on the MASP-3 protein (SEQ ID NO:8). In some embodiments, the inhibitory agent is a bispecific monoclonal antibody that binds to at least one of the CCP1-CCP2 domain of MASP-2 (aa 300-431 of SEQ ID NO:5) or the serine protease domain of MASP-2 (aa 445-682 of SEQ ID NO:5) and also binds to an epitope in the serine protease of MASP-3 (aa 450-711 of SEQ ID NO:8).

In another embodiment, the invention provides a composition for inhibiting both LEA-1 and LEA-2, comprising an inhibitory agent that inhibits both MASP-2-mediated LEA-2 activation and MASP-1-mediated activation of MASP-3, thereby inhibiting MASP-3-mediated LEA-1 activation (and optionally also inhibiting the MASP-1-mediated maturation of factor D). In one embodiment, the inhibitory agent is a MASP-1/MASP-2 dual inhibitory agent that binds to an epitope within an amino acid region that is conserved between MASP-1 (SEQ ID NO:10) and MASP-2 (SEQ ID NO:5), such as the serine protease domain, as shown in **FIGURES 4**, **6** and **7A**. In one embodiment, the inhibitory agent is a bispecific inhibitory agent, such as a bispecific monoclonal antibody, that specifically binds to an epitope on the MASP-1 protein (SEQ ID NO:10) and an epitope on the MASP-2 protein (SEQ ID NO:5). In some embodiments, the inhibitory agent is a bispecific monoclonal antibody that binds to the serine protease domain of MASP-1 (aa 449-694 of SEQ ID NO:10) and also binds to at least one of the CCP1-CCP2 domain of MASP-2 (aa 300-431 of SEQ ID NO:5) or the serine protease domain of MASP-2 (aa 445-682 of SEQ ID NO:5).

In another embodiment, the invention provides a composition for inhibiting both LEA-1 and LEA-2, comprising an inhibitory agent that inhibits MASP-2-mediated LEA-2 activation, MASP-3-mediated LEA-1 activation by directly binding to MASP-3 and also inhibits MASP-1-mediated activation of MASP-3, thereby inhibiting MASP-3-mediated LEA-1 activation (and optionally also inhibiting the MASP-1-mediated maturation of factor D). In one embodiment, the inhibitory agent is a pan-MASP inhibitor that binds to an amino acid region that is conserved between MASP-1 (SEQ ID NO:10), MASP-2 (SEQ ID NO:5) and MASP-3 (SEQ ID NO:8), for example a conserved region in the CUBI-EGF-CUB2 domain, as shown in **FIGURES 4** and **5**. As illustrated in **FIGURES 4** and **5**, there are numerous patches of identity shared between MASP-1, MASP-2 and MASP-3 in the CUBI-EGF-CUBII domains, thereby allowing for the generation of pan-specific MASP antibodies. In some embodiments, the pan-specific MASP antibody can bind to an epitope within the CUB2 domain of MASP-1 (aa 185-296 of SEQ ID NO:10), MASP-2 (aa 184-295 of SEQ ID NO:5) and MASP-3 (aa 185-296 of SEQ ID NO:8). It is noted that a pan-specific MASP inhibitor that binds to CUBI-EGF of MASP-1, MASP-2 and MASP-3 would also bind to MAp19 and MAp44, therefore the effective therapeutic dosage of such an inhibitor would be adjusted to a higher level to compensate for this binding. It is further noted that a pan-specific MASP inhibitor that binds to the CUBII domain of MASP-1, MASP-2 and MASP-3 would also bind to MAp44, therefore the effective therapeutic dosage of such an inhibitor would be adjusted to a higher level to compensate for this binding.

In one embodiment, the inhibitory agent is a trispecific MASP-1/2/3 inhibitor that binds to an epitope on the MASP-1 protein (SEQ ID NO:10), an epitope on the MASP-2 protein (SEQ ID NO:5) and an epitope on the MASP-3 protein (SEQ ID NO:8). In some embodiments, the inhibitory agent is a trispecific monoclonal antibody that binds to the serine protease domain of MASP-1 (aa 449-694 of SEQ ID NO:10), binds to at least one of the CCP1-CCP2 domain of MASP-2 (aa 300-431 of SEQ ID NO:5) or the serine protease domain of MASP-2 (aa 445-682 of SEQ ID NO:5) and also binds to an epitope in the serine protease of MASP-3 (aa 450-711 of SEQ ID NO:8).

Exemplary inhibitory agents for inhibiting LEA-1, LEA-2 or LEA-1 and LEA-2 are described below in **TABLE 2**.

**TABLE 2: MASP Inhibitory Agents**

| Type of MASP inhibitor | Inhibitor Binding domain(s) | Cross-reactivity* | Assay for inhibitory activity | Therapeutic Utility |
|---|---|---|---|---|
| **MASP-3 specific** | MASP-3 serine protease domain (aa 450-711 of SEQ ID NO:8) | Binds to MASP-3; not to MASP-1; MASP-2; MAp44 or MAp19 | Inhibition of MASP-3 serine protease substrate-specific cleavage; LEA-1 inhibition, assay for inhibition of factor D activation; inhibition of hemolysis of non-human RBCs by human serum | Inhibit LEA-1-mediated complement activation (inhibit lysis and opsonization) |
| **MASP-2 specific** | MASP-2 CCP1-CCP2 domain (aa 300-431 of SEQ ID NO:5); or MASP-2 serine protease domain (aa 445-682 of SEQ ID NO:5) | Binds to MASP-2; not to MASP-1; MASP-3; MAP19 or MAp44 | Inhibition of MASP-2-specific protease substrate-specific cleavage, LEA-2 inhibition | Inhibit LEA-2-mediated complement activation (inhibit opsonization and/or lysis) |

(continued)

| Type of MASP inhibitor | Inhibitor Binding domain(s) | Cross-reactivity* | Assay for inhibitory activity | Therapeutic Utility |
|---|---|---|---|---|
| **MASP-1 specific** | MASP-1 serine protease domain (aa449-694 of SEQ ID NO: 10) | Binds to MASP-1; not to MASP-2, MASP-3, MAp44 or MAp19 | Inhibition of MASP-1-specific protease substrate-specific cleavage; LEA-1 and LEA2 inhibition, Assay for inhibition of factor D activation; assay for restoration of AP-1 activity in factor D depleted serum supplemented with pro-factor D | Inhibit LEA-1 and LEA-2mediated complement activation (inhibit lysis and/or opsonization) |
| **MASP-2/MASP-3 dual inhibitor (one antibody binds to conserved region)** | Region of serine protease domain conserved between MASP-2 and MASP-3, especially the N-terminal region of beta chain (first 150aa) | Binds MASP-2 and MASP-3; not MASP-1, MAp44, or MAp19. | Assay for MASP-2- and MASP-3 protease substrate-specific cleavage, inhibition of LEA-1 and LEA-2 | Inhibit LEA-1 and LEA-2-mediated complement activation (inhibit lysis and/or opsonization) |
| **MASP-1/3 dual inhibitor that excludes MAp44** | MASP-1/3 CCP2 domain (aa 367-432 of SEQ ID NO: 10) | Binds MASP-1 and MASP-3; not MAp44, MASP-2, or MAp19 | Assay for MASP-3 and MASP-1 protease substrate-specific cleavage and inhibition of factor D activation, LEA-1 and LEA-2 inhibition | Inhibit LEA-1- and LEA-2-mediated complement activation (inhibit lysis and/or opsonization) |
| **MASP-1/3 dual inhibitor that includes MAp44** | MASP-1/3 CUBI-CCP1 domain (aa25-363 of SEQ ID NO: 10) | Binds MASP-1, MASP-3, and MAp44; not MASP-2 or MAp19 | Assay for MASP-3 and MASP-1 protease substrate-specific cleavage and inhibition of factor D activation, LEA-1 and LEA-2 inhibition | Inhibit LEA-1- and LEA-2-mediated complement activation (inhibit lysis and/or opsonization) |
| **MASP-1/2 dual inhibitor** | Region of serine protease domain conserved between MASP-1 and MASP-2 | Binds MASP-1 and MASP-2; not MASP-3, MAp19 or MAp44 | Assay for inhibition of MASP-1 and MASP-2 serine protease substrate-specific cleavage; LEA-1 and LEA-2 inhibition | Inhibit LEA-1- and LEA-2-mediated complement activation (inhibit lysis and/or opsonization) |
| **MASP-1/2/3 pan inhibitor** | Conserved region of CUB1-EGF-CUB2, especially CUB2 domain (common interaction site) | In addition to MASP-1/2/3 would bind to MAp44, and possibly Map 19 | Assay for MASP-1-, MASP-2- and MASP-3-specific protease substrate-specific cleavage and inhibition of factor D activation; inhibition of LEA-1 and LEA-2 | Inhibit LEA-1- and LEA-2-mediated complement activation (inhibit lysis and/or opsonization) |

(continued)

| Type of MASP inhibitor | Inhibitor Binding domain(s) | Cross-reactivity* | Assay for inhibitory activity | Therapeutic Utility |
|---|---|---|---|---|
| MASP-2/MASP-3 bispecific inhibitor | MASP-2-specific binding to CCP1-CCP2 (aa 300-431 of SEQ ID NO:5); or MASP-2 serine protease domain (aa 445-682 of SEQ ID NO:5) and MASP-3-specific binding to serine protease domain (aa 450-711 of SEQ ID NO:8) | Binds MASP-2 and MASP-3; not MASP-1, MAp44 or MAp19 | Assay for MASP-2- and MASP-3-specific protease substrate-specific cleavage, inhibition of LEA-1 and LEA-2 | Inhibit LEA-1- and LEA-2-mediated complement activation (inhibit lysis and/or opsonization) |
| MASP-1/MASP-2 bispecific inhibitor | MASP-1 serine protease domain (aa449-694 of SEQ ID NO:10), and MASP-2-specific binding to CCP1-CCP2 (aa 300-431 of SEQ ID NO:5); or MASP-2 serine protease domain (aa 445-682 of SEQ ID NO:5) | Binds to MASP-1 and to MASP-2; not MASP-3, MAp19 or MAp44 | Assay for inhibition of MASP-1- and MASP-2-specific serine protease substrate-specific cleavage; LEA-1 and LEA-2 inhibition | Inhibit LEA-1- and LEA-2-mediated complement activation (inhibit lysis and/or opsonization) |
| MASP-1/MASP-3 bispecific | MASP-1 serine protease domain (aa449-694 of SEQ ID NO:10) and MASP-3-specific binding to serine protease domain (aa 450-711 of SEQ ID NO:8) | Binds to MASP-1 and MASP-3; not to MASP-2, MAp44 or MAp19 | Assay for MASP-1 and MASP-3-protease sub strate-specificcleavage and inhibition of factor D activation, LEA-1 and LEA-2 inhibition | Inhibit LEA-1- and LEA-2-mediated complement activation (inhibit lysis and/or opsonization) |
| MASP-1/MASP-2/MASP-3 trispecific | MASP-1 serine protease domain, MASP-2 serine protease domain or CCP-CCP2 domain and MASP-3 serine protease domain | Binds to MASP-1 and MASP-2 and MASP-3; not MAp19 or MAp44 | Assay for MASP-1-, MASP-2- and MASP-3-protease substrate-specific cleavage and inhibition of factor D activation, inhibition of LEA-1 and LEA-2 | Inhibit LEA-1- and LEA-2-mediated complement activation (inhibit lysis and/or opsonization) |

*With regard to cross-reactivity column as set forth in **TABLE 2**, the designated MASP inhibitor binds to the inhibitor binding domain with a binding affinity of at least 10 times greater (e.g., at least 20 times, at least 50 times or at least 100 times greater) than to the other complement components (*i.e.*, polypeptides or fragments thereof) listed as "not" binding.

In some embodiments, the composition comprises a combination of LEA-1 and LEA-2 inhibitory agents, for example, a combination of single inhibitory agents as described above and shown in **TABLE 2.** For example, in one embodiment, the composition comprises a combination of a MASP-1 antibody and a MASP-2 antibody. In one embodiment, the composition comprises a combination of a MASP-1 antibody and a MASP-3 antibody. In one embodiment, the composition comprises a combination of a MASP-2 antibody and a MASP-3 antibody. In one embodiment, the composition comprises a combination of a MASP-1, and MASP-2 and a MASP-3 antibody. In some embodiments, the methods of the invention comprise administration of a single composition comprising a combination of inhibitory agents. In other embodiments, the methods of the invention comprise co-administering separate compositions.

In some embodiments, the compositions comprise a combination of a dual inhibitory agent plus a single inhibitory agent (i.e., a MASP-2/3 dual inhibitor plus a MASP-1 inhibitor; a MASP-1/3 dual inhibitor plus a MASP-2 inhibitor; or a MASP-1/2 dual inhibitor plus a MASP-3 inhibitor). In other embodiments, the methods of the invention comprise co-administering separate compositions comprising a dual inhibitor and a single inhibitor.

In some embodiments, the compositions comprise a combination of a bispecific inhibitory agent plus a single inhibitory agent (i.e., a MASP-2/3 bispecific inhibitor plus a MASP-1 inhibitor; a MASP-1/3 bispecific inhibitor plus a MASP-2 inhibitor; or a MASP-1/2 bispecific inhibitor plus a MASP-3 inhibitor). In other embodiments, the methods of the invention comprise co-administering separate compositions comprising a bispecific inhibitor and a single inhibitor.

In accordance with various embodiments of the invention, it is noted that MASP-3 inhibitory agents and/or MASP-2 inhibitory agents and/or MASP-1 inhibitory agents would be used to clear the target protein from the plasma as compared to a C5 antibody which must localize to the site of action.

**MASP ANTIBODIES**

In some embodiments of this aspect of the invention, the MASP inhibitory agent comprises a MASP antibody (e.g., a MASP-1, MASP-2 or MASP-3 antibody) that inhibits at least one of the LEA-1 and/or LEA-2 complement activation pathways. The MASP antibodies useful in this aspect of the invention include polyclonal, monoclonal or recombinant antibodies derived from any antibody producing mammal and may be multispecific (i.e., bispecific or trispecific), chimeric, humanized, fully human, anti-idiotype, and antibody fragments. Antibody fragments include Fab, Fab', F(ab)$_2$, F(ab')$_2$, Fv fragments, scFv fragments and single-chain antibodies as further described herein.

MASP antibodies can be screened for the ability to inhibit the LEA-1 or LEA-2-dependent complement activation system using the assays described herein. Several MASP-1, MASP-2 and MASP-3 antibodies have been described in the literature and some have been newly generated, some of which are listed below in **TABLE 3**. These exemplary MASP antibodies can be screened for the ability to inhibit the LEA-1- and/or LEA-2-dependent complement activation system using the assays described herein. For example, as described in Examples 11-13 herein, anti-rat MASP-2 Fab2 antibodies have been identified that block MASP-2-dependent complement activation. As further described in Example 14, fully human MASP-2 scFv antibodies have been identified that block MASP-2-dependent complement activation. As further described in Example 15, MASP-3 antibodies have been generated. Once a MASP antibody is identified that functions as an inhibitor of LEA-1 or LEA-2, it can be used in a pharmaceutical composition as descrbed herein, and it can also be used to generate bispecific and trispecific inhibitory agents, as set forth in **TABLE 2** and further described herein (see e.g., Example 8).

**TABLE 3**: MASP-1, MASP-2 and MASP-3 SPECIFIC ANTIBODIES

| TARGET | ANTIGEN | ANTIBODY TYPE | REFERENCE |
|---|---|---|---|
| MASP-2 | Recombinant MASP-2 | Rat Polyclonal | Peterson, S.V., et al., Mol. Immunol. 37:803-811, 2000 |
| MASP-2 | Recombinant human CCP1/2-SP fragment (MoAb 8B5) | Rat MoAb (subclass IgG1) | Moller-Kristensen, M., et al., J. of Immunol. Methods 282:159-167, 2003 |
| MASP-2 | Recombinant human MAp19 (MoAb 6G12) (cross-reacts with MASP-2) | Rat MoAb (subclass IgG1) | Moller-Kristensen, M., et al., J. of Immunol. Methods 282:159-167, 2003 |
| MASP-2 | hMASP-2 | Mouse MoAb (S/P) Mouse MoAb (N-term) | Peterson, S.V., et al., Mol. Immunol. 35:409, April 1998 |
| MASP-2 | hMASP-2 (CCP1-CCP2-SP domain | rat MoAb: Nimoab101, produced by hybridoma cell line 03050904 (ECACC) | WO 2004/106384 |
| MASP-2 | hMASP-2 (full-length his-tagged) | murine MoAbs: NimoAb 104, produced by hybridoma cell line M0545YM035 (DSMZ) NimoAb 108, produced by hybridoma cell line M0545YM029 (DSMZ) NimoAb 109 produced by hybridoma cell line M0545YM046 (DSMZ) NimoAb 110 produced by hybridoma cell line M0545YM048 (DSMZ) | WO 2004/106384 |

(continued)

| TARGET | ANTIGEN | ANTIBODY TYPE | REFERENCE |
|---|---|---|---|
| MASP-2 | Rat MASP-2 (full-length) | MASP-2 Fab2 antibody fragments | Examples 11-12 |
| MASP-2 | hMASP-2 (full-length) | Fully human scFv clones | Example 14 |
| MASP-1 | hMASP-1 (full-length) | Mouse MoAbs: MoaAbs1E2 and 2B11 produced by hybridoma line 1E2 and 2B11 (do not cross-react with MASP-2). Both abs recognize the heavy chain common to both MASP-1 and MASP-3 | Terai I. et al., Clin Exp Immunol 110: 317-323 (1997); MoAb1E2: Commercially available from Hycult Biotech Cat#HM2092 MoAb2B 11: commercially available from Hycult Biotech: Cat#HM2093 |
| MASP-1 | hMASP-1 (full-length) | Mouse MoAb 4C2 | Endo M. et al., Nephrol Dial Transplant 13:1984-1990 (1998) |
| MASP-1 | hMASP-1 (full-length) | MASP-1 chicken abs | Example 15 |
| MASP-3 | hMASP-3 (full-length) | Mouse MoAbs: MoAb-7D8;MoAb-7B7; MoAb-8B3; and MoAb-5H3 MoAb-7D8 and mAb-5H3 are MASP-3-specific, others cross-react with MASP-1 | Skjoedt et al., Immunobiology 215 (11):921-31 (2010) |
| MASP-3 | hMASP-3 (full-length) | Rat MoAb 38:12-3, Does not recognize MASP-1 | Moller-Kristensen et al., Int Immunol 19:141 (2007); Commercially available from Hycult Biotech: Cat #HM2216 |
| MASP-3 | hMASP-3 (full-length) | MASP-3 chicken abs | Example 15 |

i. MASP antibodies with reduced effector function

In some embodiments of this aspect of the invention, the MASP antibodies described herein have reduced effector function in order to reduce inflammation that may arise from the activation of the classical complement pathway. The ability of IgG molecules to trigger the classical complement pathway has been shown to reside within the Fc portion of the molecule (Duncan, A.R., et al., Nature 332:738-740 (1988)). IgG molecules in which the Fc portion of the molecule has been removed by enzymatic cleavage are devoid of this effector function (see Harlow, Antibodies: A Laboratory Manual, Cold Spring Harbor Laboratory, New York, 1988). Accordingly, antibodies with reduced effector function can be generated as the result of lacking the Fc portion of the molecule by having a genetically engineered Fc sequence that minimizes effector function, or being of either the human $IgG_2$ or $IgG_4$ isotype.

Antibodies with reduced effector function can be produced by standard molecular biological manipulation of the Fc portion of the IgG heavy chains as described in Jolliffe et al., Int'l Rev. Immunol. 10:241-250, (1993), and Rodrigues et al., J. Immunol. 151:6954-6961, (1998). Antibodies with reduced effector function also include human IgG2 and IgG4 isotypes that have a reduced ability to activate complement and/or interact with Fc receptors (Ravetch, J.V., et al., Annu. Rev. Immunol. 9:457-492, (1991); Isaacs, J.D., et al., J. Immunol. 148:3062-3071, 1992; van de Winkel, J.G., et al., Immunol. Today 14:215-221, (1993)). Humanized or fully human antibodies specific to human MASP-1, MASP-2 or MASP-3 (including dual, pan, bispecific or trispecific antibodies) comprised of IgG2 or IgG4 isotypes can be produced by one of several methods known to one of ordinary skilled in the art, as described in Vaughan, T.J., et al., Nature Biotechnical 16:535-539, (1998).

ii. Production of MASP antibodies

MASP-1, MASP-2 or MASP-3 antibodies can be produced using MASP-1, MASP-2 or MASP-3 polypeptides (e.g., full-length MASP-1, MASP-1 or MASP-3) or using antigenic MASP-1, 2 or 3 epitope-bearing peptides (e.g., a portion of the MASP-2 polypeptide). Immunogenic peptides may be as small as five amino acid residues. For example, the MASP-2 polypeptide including the entire amino acid sequence of SEQ ID NO:5 may be used to induce MASP-2 antibodies useful in the method of the invention. Particular MASP domains known to be involved in protein-protein interactions, such as the CUBI, and CUBI-EGF domains, as well as the region encompassing the serine-protease active site, for example, as set forth in **TABLE 2**, may be expressed as recombinant polypeptides using methods well known in the art and used as antigens. In addition, peptides comprising a portion of at least 6 amino acids of the MASP-1 polypeptide (SEQ ID NO:10), or of the MASP-2 polypeptide (SEQ ID NO:5) or of the MASP-3 polypeptide (SEQ ID NO:8) are also useful to induce MASP-1, MASP-2 or MASP-3 antibodies, respectively. The MASP peptides and polypeptides used to raise antibodies may be isolated as natural polypeptides, or recombinant or synthetic peptides and catalytically inactive recombinant polypeptides. Antigens useful for producing MASP antibodies also include fusion polypeptides, such as fusions of a MASP polypeptide or a portion thereof with an immunoglobulin polypeptide or with maltose-binding protein. The polypeptide immunogen may be a full-length molecule or a portion thereof. If the polypeptide portion is hapten-like, such portion may be advantageously joined or linked to a macromolecular carrier (such as keyhole limpet hemocyanin (KLH), bovine serum albumin (BSA) or tetanus toxoid) for immunization.

### iii. Polyclonal antibodies

Polyclonal antibodies against MASP-1, MASP-2 or MASP-3 can be prepared by immunizing an animal with MASP-1, MASP-2 or MASP-3 polypeptide or an immunogenic portion thereof using methods well known to those of ordinary skill in the art. See, for example, Green et al., "Production of Polyclonal Antisera," in Immunochemical Protocols (Manson, ed.). The immunogenicity of a MASP polypeptide can be increased through the use of an adjuvant, including mineral gels, such as aluminum hydroxide or Freund's adjuvant (complete or incomplete), surface active substances such as lysolecithin, pluronic polyols, polyanions, oil emulsions, KLH and dinitrophenol. Polyclonal antibodies are typically raised in animals such as horses, cows, dogs, chicken, rats, mice, rabbits, guinea pigs, goats, or sheep. Alternatively, a MASP antibody useful in the present invention may also be derived from a subhuman primate. General techniques for raising diagnostically and therapeutically useful antibodies in baboons may be found, for example, in Goldenberg et al., International Patent Publication No. WO 91/11465, and in Losman, M.J., et al., Int. J. Cancer 46:310, (1990). Sera containing immunologically active antibodies are then produced from the blood of such immunized animals using standard procedures well known in the art.

### iv. Monoclonal antibodies

In some embodiments, the LEA-2 inhibitory agent is a MASP-2 monoclonal antibody and/or the LEA-1 inhibitory agent is a MASP-3 monoclonal antibody or a MASP-1 monoclonal antibody. As described above, in some embodiments, MASP-1, MASP-2 and MASP-3 monoclonal antibodies are highly specific, being directed against a single MASP-1, MASP-2 or MASP-3 epitope. As used herein, the modifier "monoclonal" indicates the character of the antibody as being obtained from a substantially homogenous population of antibodies, and is not to be construed as requiring production of the antibody by any particular method. Monoclonal antibodies can be obtained using any technique that provides for the production of antibody molecules by continuous cell lines in culture, such as the hybridoma method described by Kohler, G., et al., Nature 256:495, (1975), or they may be made by recombinant DNA methods (see, e.g., U.S. Patent No. 4,816,567 to Cabilly). Monoclonal antibodies may also be isolated from phage antibody libraries using the techniques described in Clackson, T., et al., Nature 352:624-628, (1991), and Marks, J.D., et al., J. Mol. Biol. 222:581-597, (1991). Such antibodies can be of any immunoglobulin class including IgG, IgM, IgE, IgA, IgD and any subclass thereof.

For example, monoclonal antibodies can be obtained by injecting a suitable mammal (e.g., a BALB/c mouse) with a composition comprising a MASP-1 polypeptide, a MASP-2 polypeptide or a MASP-3 polypeptide, or portion thereof. After a predetermined period of time, splenocytes are removed from the mouse and suspended in a cell culture medium. The splenocytes are then fused with an immortal cell line to form a hybridoma. The formed hybridomas are grown in cell culture and screened for their ability to produce a monoclonal antibody against MASP-1, MASP-2 or MASP-3. (See also Current Protocols in Immunology, Vol. 1., John Wiley & Sons, pages 2.5.1-2.6.7, 1991.)

Human monoclonal antibodies may be obtained through the use of transgenic mice that have been engineered to produce specific human antibodies in response to antigenic challenge. In this technique, elements of the human immunoglobulin heavy and light chain locus are introduced into strains of mice derived from embryonic stem cell lines that contain targeted disruptions of the endogenous immunoglobulin heavy chain and light chain loci. The transgenic mice can synthesize human antibodies specific for human antigens, such as the MASP-2 antigens described herein, and the mice can be used to produce human MASP-2 antibody-secreting hybridomas by fusing B-cells from such animals to suitable myeloma cell lines using conventional Kohler-Milstein technology. Methods for obtaining human antibodies from transgenic mice are described, for example, by Green, L.L., et al., Nature Genet. 7:13, 1994; Lonberg, N., et al., Nature 368:856, 1994; and Taylor, L.D., et al., Int. Immun. 6:579, 1994.

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, The Humana Press, Inc., Vol. 10, pages 79-104, 1992).

Once produced, polyclonal, monoclonal or phage-derived antibodies are first tested for specific MASP-1, MASP-2 or MASP-3 binding or, where desired, dual MASP-1/3, MASP-2/3 or MASP-1/2 binding. Methods for determining whether an antibody binds to a protein antigen and/or the affinity for an antibody to a protein antigen are known in the art. For example, the binding of an antibody to a protein antigen can be detected and/or quantified using a variety of techniques such as, but not limited to, Western blot, dot blot, plasmon surface resonance method (e.g., BIAcore system; Pharmacia Biosensor AB, Uppsala, Sweden and Piscataway, NJ), or enzyme-linked immunosorbent assays (ELISA). See, e.g., Harlow and Lane (1988) "Antibodies: A Laboratory Manual" Cold Spring Harbor Laboratory Press, Cold Spring Harbor, N. Y.; Benny K. C. Lo (2004) "Antibody Engineering: Methods and Protocols," Humana Press (ISBN: 1588290921); Borrebaek (1992) "Antibody Engineering, A Practical Guide," W.H. Freeman and Co., NY; Borrebaek (1995) "Antibody Engineering," 2nd Edition, Oxford University Press, NY, Oxford; Johne et al. (1993), Immunol. Meth. 160:191-198; Jonsson et al. (1993) Ann. Biol. Clin. 51: 19-26; and Jonsson et al. (1991) Biotechniques 11:620-627. See also, U.S. Patent No. 6,355,245.

The affinity of MASP monoclonal antibodies can be readily determined by one of ordinary skill in the art (see, e.g., Scatchard, A., NY Acad. Sci. 51:660-672, 1949). In one embodiment, the MASP-1, MASP-2 or MASP-3 monoclonal antibodies useful for the methods of the invention bind to MASP-1, MASP-2, or MASP-3 with a binding affinity of <100 nM, preferably <10 nM and most preferably <2 nM.

Once antibodies are identified that specifically bind to MASP-1, MASP-2 or MASP-3, the MASP-1, MASP-2 or MASP-3 antibodies are tested for the ability to function as a LEA-1 inhibitory agent or a LEA-2 inhibitory agent in one of several functional assays, for example as described in **TABLE 2**. For example, antibodies identified that specifically bind to MASP-2 are tested for the ability to function as a LEA-2 inhibitory agent in one of several assays, such as, for example, as described in **TABLE 2** (e.g., a lectin-specific C4 cleavage assay (such as the assay described in Example 8 or Example 9), or a C3b deposition assay (such as the assay described in Example 4 or Example 11)). As a further example, antibodies identified that specifically bind to MASP-1 or MASP-3 are tested for the ability to function as a LEA-1 inhibitory agent in one of several assays, such as, for example, as described in **TABLE 2** (e.g., the reduction of hemolysis, measured, for example as described in Example 5, or the reduction of C3 cleavage and C3b deposition, measured, for example, as described in Example 4 and Example 11).

V. Chimeric/humanized antibodies

Monoclonal antibodies useful in the method of the invention include chimeric antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies (U.S. Patent No. 4,816,567, to Cabilly; and Morrison, S.L., et al., Proc. Nat'l Acad. Sci. USA 81:6851-6855, (1984)).

One form of a chimeric antibody useful in the invention is a humanized monoclonal MASP-1, MASP-2 or MASP-3 antibody. Humanized forms of non-human (e.g., murine) antibodies are chimeric antibodies, which contain minimal sequence derived from non-human immunoglobulin. Humanized monoclonal antibodies are produced by transferring the non-human (e.g., mouse) complementarity determining regions (CDR), from the heavy and light variable chains of the mouse immunoglobulin into a human variable domain. Typically, residues of human antibodies are then substituted in the framework regions of the non-human counterparts. Furthermore, humanized antibodies may comprise residues that are not found in the recipient antibody or in the donor antibody. These modifications are made to further refine antibody performance. In general, the humanized antibody will comprise substantially all of at least one, and typically two, variable domains in which all or substantially all of the hypervariable loops correspond to those of a non-human immunoglobulin and all or substantially all of the Fv framework regions are those of a human immunoglobulin sequence. The humanized antibody optionally also will comprise at least a portion of an immunoglobulin constant region (Fc), typically that of a human immunoglobulin. For further details, see Jones, P.T., et al., Nature 321:522-525, (1986); Reichmann, L., et al., Nature 332:323-329, (1988); and Presta, Curr. Op. Struct. Biol. 2:593-596, (1992).

The humanized antibodies useful in the invention include human monoclonal antibodies including at least a MASP-1, MASP-2, or MASP-3 binding CDR3 region. In addition, the Fc portions may be replaced so as to produce IgA or IgM as well as human IgG antibodies. Such humanized antibodies will have particular clinical utility because they will specifically recognize human MASP-1, MASP-2 or MASP-3 but will not evoke an immune response in humans against the antibody itself. Consequently, they are better suited for *in vivo* administration in humans, especially when repeated or long-term administration is necessary.

Techniques for producing humanized monoclonal antibodies are also described, for example, by Jones, P.T.,

et al., Nature 321:522, (1986); Carter, P., et al., Proc. Nat'l. Acad. Sci. USA 89:4285, (1992); Sandhu, J.S., Crit. Rev. Biotech. 12:437, (1992); Singer, I.I., et al., J. Immun. 150:2844, (1993); Sudhir (ed.), Antibody Engineering Protocols, Humana Press, Inc., (1995); Kelley, "Engineering Therapeutic Antibodies," in Protein Engineering: Principles and Practice, Cleland et al. (eds.), John Wiley & Sons, Inc., pages 399-434, (1996); and by U.S. Patent No. 5,693,762, to Queen, 1997. In addition, there are commercial entities that will synthesize humanized antibodies from specific murine antibody regions, such as Protein Design Labs (Mountain View, CA).

### vi. Recombinant antibodies

MASP-1, MASP-2 or MASP-3 antibodies can also be made using recombinant methods. For example, human antibodies can be made using human immunoglobulin expression libraries (available for example, from Stratagene, Corp., La Jolla, CA) to produce fragments of human antibodies ($V_H$, $V_L$, Fv, Factor D, Fab or $F(ab')_2$). These fragments are then used to construct whole human antibodies using techniques similar to those for producing chimeric antibodies.

### vii. Anti-idiotype antibodies

Once MASP-1, MASP-2 or MASP-3 antibodies are identified with the desired inhibitory activity, these antibodies can be used to generate anti-idiotype antibodies that resemble a portion of MASP-1, MASP-2 or MASP-3 using techniques that are well known in the art. See, e.g., Greenspan, N.S., et al., FASEB J. 7:437, (1993). For example, antibodies that bind to MASP-2 and competitively inhibit a MASP-2 protein interaction required for complement activation can be used to generate anti-idiotypes that resemble the MBL binding site on MASP-2 protein and therefore bind and neutralize a binding ligand of MASP-2 such as, for example, MBL.

### viii. Immunoglobulin fragments

The MASP-2 and MASP-3 inhibitory agents useful in the method of the invention encompass not only intact immunoglobulin molecules but also the well known fragments including Fab, Fab', $F(ab)_2$, $F(ab')_2$ and Fv fragments, scFv fragments, diabodies, linear antibodies, single-chain antibody molecules and multispecific (e.g., bispecific and trispecific) antibodies formed from antibody fragments.

It is well known in the art that only a small portion of an antibody molecule, the paratope, is involved in the binding of the antibody to its epitope (see, e.g., Clark, W.R., The Experimental Foundations of Modern Immunology, Wiley & Sons, Inc., NY, 1986). The pFc' and Fc regions of the antibody are effectors of the classical complement pathway but are not involved in antigen binding. An antibody from which the pFc' region has been enzymatically cleaved, or which has been produced without the pFc' region, is designated an $F(ab')_2$ fragment and retains both of the antigen binding sites of an intact antibody. An isolated $F(ab')_2$ fragment is referred to as a bivalent monoclonal fragment because of its two antigen binding sites. Similarly, an antibody from which the Fc region has been enzymatically cleaved, or which has been produced without the Fc region, is designated a Fab fragment, and retains one of the antigen binding sites of an intact antibody molecule.

Antibody fragments can be obtained by proteolytic hydrolysis, such as by pepsin or papain digestion of whole antibodies by conventional methods. For example, antibody fragments can be produced by enzymatic cleavage of antibodies with pepsin to provide a 5S fragment denoted $F(ab')_2$. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, U.S. Patent No. 4,331,647 to Goldenberg; Nisonoff, A., et al., Arch. Biochem. Biophys. 89:230, (1960); Porter, R.R., Biochem. J. 73:119, (1959); Edelman, et al., in Methods in Enzymology 1:422, Academic Press, (1967); and by Coligan at pages 2.8.1-2.8.10 and 2.10.-2.10.4.

In some embodiments, the use of antibody fragments lacking the Fc region are preferred to avoid activation of the classical complement pathway which is initiated upon binding Fc to the Fcγ receptor. There are several methods by which one can produce a monoclonal antibody that avoids Fcγ receptor interactions. For example, the Fc region of a monoclonal antibody can be removed chemically using partial digestion by proteolytic enzymes (such as ficin digestion), thereby generating, for example, antigen-binding antibody fragments such as Fab or $F(ab)_2$ fragments (Mariani, M., et al., Mol. Immunol. 28:69-71, (1991)). Alternatively, the human γ4 IgGisotype, which does not bind Fcγ receptors, can be used during construction of a humanized antibody as described herein. Antibodies, single chain antibodies and antigen-binding domains that lack the Fc domain can also be engineered using recombinant techniques described herein.

ix. Single-chain antibody fragments

Alternatively, one can create single peptide chain binding molecules specific for MASP-1, MASP-2 or MASP-3 in which the heavy and light chain Fv regions are connected. The Fv fragments may be connected by a peptide linker to form a single-chain antigen binding protein (scFv). These single-chain antigen binding proteins are prepared by constructing a structural gene comprising DNA sequences encoding the $V_H$ and $V_L$ domains which are connected by an oligonucleotide. The structural gene is inserted into an expression vector, which is subsequently introduced into a host cell, such as *E. coli.* The recombinant host cells synthesize a single polypeptide chain with a linker peptide bridging the two V domains. Methods for producing scFvs are described for example, by Whitlow, et al., "Methods: A Companion to Methods in Enzymology" 2:97, (1991); Bird, et al., Science 242:423, (1988); U.S. Patent No. 4,946,778, to Ladner; Pack, P., et al., Bio/Technology 11:1271, (1993).

As an illustrative example, a MASP-3-specific scFv can be obtained by exposing lymphocytes to MASP-3 polypeptide *in vitro* and selecting antibody display libraries in phage or similar vectors (for example, through the use of immobilized or labeled MASP-3 protein or peptide). Genes encoding polypeptides having potential MASP-3 polypeptide binding domains can be obtained by screening random peptide libraries displayed on phage or on bacteria such as *E. coli.* These random peptide display libraries can be used to screen for peptides which interact with MASP-3. Techniques for creating and screening such random peptide display libraries are well known in the art (U.S. Patent No. 5,223,409, to Lardner; U.S. Patent No. 4,946,778, to Ladner; U.S. Patent No. 5,403,484, to Lardner; U.S. Patent No. 5,571,698, to Lardner; and Kay et al., Phage Display of Peptides and Proteins Academic Press, Inc., 1996) and random peptide display libraries and kits for screening such libraries are available commercially, for instance from CLONTECH Laboratories, Inc. (Palo Alto, Calif.), Invitrogen Inc. (San Diego, Calif.), New England Biolabs, Inc. (Beverly, Mass.), and Pharmacia LKB Biotechnology Inc. (Piscataway, N.J.).

Another form of a MASP-3 antibody fragment useful in this aspect of the invention is a peptide coding for a single complementarity-determining region (CDR) that binds to an epitope on a MASP-3 antigen and inhibits MASP-3-dependent complement activation (i.e., LEA-1). Another form of a MASP-1 antibody fragment useful in this aspect of the invention is a peptide coding for a single complementarity-determining region (CDR) that binds to an epitope on a MASP-1 antigen and inhibits MASP-3-dependent complement activation (i.e., LEA-1). Another form of a MASP-2 antibody fragment useful in this aspect of the invention is a peptide coding for a single complementarity-determining region (CDR) that binds to an epitope on a MASP-2 antigen and inhibits MASP-2-dependent complement activation (i.e., LEA-2).

CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106, (1991); Courtenay-Luck, "Genetic Manipulation of Monoclonal Antibodies," in Monoclonal Antibodies: Production, Engineering and Clinical Application, Ritter et al. (eds.), page 166, Cambridge University Press, (1995); and Ward et al., "Genetic Manipulation and Expression of Antibodies," in Monoclonal Antibodies: Principles and Applications, Birch et al. (eds.), page 137, Wiley-Liss, Inc., 1995).

The MASP antibodies described herein are administered to a subject in need thereof to inhibit LEA-1, LEA-2 or a combination of LEA-1 and LEA-2 complement activation. In some embodiments, the MASP inhibitory agent is a high-affinity human or humanized monoclonal MASP-1, MASP-2 or MASP-3 antibody with reduced effector function.

x. Bispecific antibodies

The MASP-2 and MASP-3 inhibitory agents useful in the method of the invention encompass multispecific (i.e., bispecific and trispecific) antibodies. Bispecific antibodies are monoclonal, preferably human or humanized, antibodies that have binding specificities for at least two different antigens. As described above and shown in **TABLE 2**, in one embodiment, the method comprises the use of a bispecific antibody comprising a binding specificity for MASP-2 (e.g., binding to at least one of CCP1-CCP2 or serine protease domain of MASP-2) and a binding specificity for MASP-3 (e.g., binding to the serine protease domain of MASP-3). In another embodiment, the method comprises the use of a bispecific antibody comprising a binding specificity for MASP-1 (e.g., binding to the serine protease domain of MASP-1) and a binding specificity for MASP-2 (e.g., binding to at least one of CCP1-CCP2 or serine protease domain of MASP-2). In another embodiment, the method comprises the use of a bispecific antibody comprising a binding specificity for MASP-1 (e.g., binding to the serine protease domain of MASP-1) and a binding specificity for MASP-3 (e.g., binding to the serine protease domain of MASP-3). In another embodiment, the method comprises the use of a trispecific antibody comprising a binding specificity for MASP-1 (e.g., binding to the serine protease domain of MASP-1), a binding specificity for MASP-2 (e.g., binding to at least one of CCP1-CCP2 or serine protease domain of MASP-2) and a binding specificity for MASP-3 (e.g., binding to the serine protease domain of MASP-3).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature 305:537-539 (1983)). Antibody variable domains with the desired binding specificities (antibody-antigen combining sites) can be fused to immunoglobulin constant domain sequences. The fusion preferably is with an immunoglobulin heavy-chain constant domain, including at least part of the hinge, $C_H2$, and $C_H3$ regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, e.g., Suresh et al., Methods in Enzymology 121:210 (1986); WO96/27011; Brennan et al., Science 229:81 (1985); Shalaby et al., J. Exp. Med. 175:217-225 (1992); Kostelny et al., J. Immunol. 148(5):1547-1553 (1992); Hollinger et al. Proc. Natl. Acad. Sci USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross -linking methods. Suitable crosslinking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. (See, e.g., Kostelny et al. J.Immunol. 148(5):1547-1553 (1992)). The "diabody" technology described by Hollinger et al. Proc. Natl. Acad. Sci USA 90:6444-6448 (1993), has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Bispecific diabodies, as opposed to bispecific whole antibodies, may also be particularly useful because they can be readily constructed and expressed in E. coli. Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

Another strategy for making bispecific antibody fragments by the use of single-chain Fv (scFv) dimers has also been reported. (See, e.g., Gruber et al. J. Immunol., 152:5368 (1994)). Alternatively, the antibodies can be "linear antibodies" as described in, e.g., Zapata et al., Protein Eng. 8(10):1057-1062 (1995). Briefly described, these antibodies comprise a pair of tandem Factor D segments ($V_H$-$C_H$I-$V_H$-$C_H$I) which form a pair of antigen binding regions. Linear antibodies can be bispecific or monospecific. The methods of the invention also embrace the use of variant forms of bispecific antibodies such as the tetravalent dual variable domain immunoglobulin (DVD-Ig) molecules described in Wu et al., Nat Biotechnol 25:1290-1297 (2007). The DVD-Ig molecules are designed such that two different light chain variable domains (VL) from two different parent antibodies are linked in tandem directly or via a short linker by recombinant DNA techniques, followed by the light chain constant domain. Methods for generating DVD-Ig molecules from two parent antibodies are further described in, e.g., WO08/024188 and WO07/024715, the disclosures of each of which are incorporated herein by reference in their entirety.

## NON-PEPTIDE INHIBITORS

In some embodiments, the MASP-3 or MASP-2 inhibitory agent is a MASP-3 or a MASP-2 or a MASP-1 inhibitory peptide or a non-peptide inhibitor of MASP-3, or of MASP-2 or of MASP-1. Non-peptide MASP inhibitory agents may be administered to the subject systemically, such as by intra-arterial, intravenous, intramuscular, subcutaneous or other parenteral administration, or by oral administration. The MASP inhibitory agent may be administered periodically over an extended period of time for treatment or control of a chronic condition, or may be by single or repeated administration in the period before, during or following acute trauma or injury.

## XVIII. PHARMACEUTICAL COMPOSITIONS AND DELIVERY METHODS

## DOSING

In another aspect, the invention provides compositions for inhibiting the adverse effects of MASP-3-dependent complement activation in a subject suffering from a hemolytic disease, such as PNH, or a disease or disorder selected from the group consisting of age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, comprising administering to the subject a composition comprising an amount of a MASP-3 inhibitory agent effective to inhibit MASP-3-dependent complement activation and a pharmaceutically acceptable carrier. In some embodiments, the method further comprises administering a composition comprising a MASP-2 inhibitory agent. The MASP-3 and MASP-2 inhibitory agents can be administered to a subject in need thereof, at therapeutically effective doses to treat or ameliorate conditions associated with MASP-3-dependent complement activation (LEA-1), and optionally also MASP-2-dependent complement activation (LEA-2). A therapeutically effective dose refers to the amount of the MASP-3 inhibitory agent, or a combination of a MASP-3 inhibitory agent and a MASP-2 inhibitory agent sufficient to result in amelioration of symptoms of the condition.

Toxicity and therapeutic efficacy of MASP-3 and MASP-2 inhibitory agents can be determined by standard pharmaceutical procedures employing experimental animal models. Using such animal models, the NOAEL (no observed adverse effect level) and the MED (the minimally effective dose) can be determined using standard methods. The dose ratio between NOAEL and MED effects is the therapeutic ratio, which is expressed as the ratio NOAEL/MED. MASP-3 inhibitory agents and MASP-2 inhibitory agents that exhibit large therapeutic ratios or indices are most preferred. The data obtained from the cell culture assays and animal studies can be used in formulating a range of dosages for use in humans. The dosage of the MASP-3 inhibitory agent and MASP-2 inhibitory agent preferably lies within a range of circulating concentrations that include the MED with little or no toxicity. The dosage may vary within this range depending upon the dosage form employed and the route of administration utilized.

For any compound formulation, the therapeutically effective dose can be estimated using animal models. For example, a dose may be formulated in an animal model to achieve a circulating plasma concentration range that includes the MED. Quantitative levels of the MASP-3 inhibitory agent or MASP-2 inhibitory agent in plasma may also be measured, for example, by high performance liquid chromatography.

In addition to toxicity studies, effective dosage may also be estimated based on the amount of target MASP protein present in a living subject and the binding affinity of the MASP-3 or MASP-2 inhibitory agent.

It has been reported that MASP-1 levels in normal human subjects is present in serum in levels in the range of from 1.48 to 12.83 $\mu$g/mL (Terai I. et al, Clin Exp Immunol 110:317-323 (1997); Theil et al., Clin. Exp. Immunol. 169:38 (2012)). The mean serum MASP-3 concentrations in normal human subjects has been reported to be in the range of about 2.0 to 12.9 $\mu$g/mL (Skjoedt M et al., Immunobiology 215(11):921-31 (2010); Degn et al., J. Immunol Methods, 361-37 (2010); Csuka et al., Mol. Immunol. 54:271 (2013). It has been shown that MASP-2 levels in normal human subjects is present in serum in low levels in the range of 500 ng/mL, and MASP-2 levels in a particular subject can be determined using a quantitative assay for MASP-2 described in Moller-Kristensen M., et al., J. Immunol. Methods 282:159-167 (2003) and Csuka et al., Mol. Immunol. 54:271 (2013).

Generally, the dosage of administered compositions comprising MASP-3 inhibitory agents or MASP-2 inhibitory agents varies depending on such factors as the subject's age, weight, height, sex, general medical condition, and previous medical history. As an illustration, MASP-3 inhibitory agents or MASP-2 inhibitory agents (such as MASP-3 antibodies, MASP-1 antibodies or MASP-2 antibodies), can be administered in dosage ranges from about 0.010 to 100.0 mg/kg, preferably 0.010 to 10 mg/kg, preferably 0.010 to 1.0 mg/kg, more preferably 0.010 to 0.1 mg/kg of the subject body weight. In some embodiments, MASP-2 inhibitory agents (such as MASP-2 antibodies) are administered in dosage ranges from about preferably 0.010 to 10 mg/kg, preferably 0.010 to 1.0 mg/kg, more preferably 0.010 to 0.1 mg/kg of the subject body weight. In some embodiments, MASP-1 inhibitory agents (such as MASP-1 antibodies) or MASP-3 inhibitory agents (such as MASP-3 antibodies) are administered in dosage ranges from about 0.010 to 100.0 mg/kg, preferably 0.010 to 10 mg/kg, preferably 0.010 to 1.0 mg/kg, more preferably 0.010 to 0.1 mg/kg of the subject body weight.

Therapeutic efficacy of MASP-3 inhibitory compositions, optionally in combination with MASP-2 inhibitory compositions, or of MASP-1 inhibitory compositions, optionally in combination with MASP-2 inhibitory compositions, and methods of the present invention in a given subject, and appropriate dosages, can be determined in accordance with complement assays well known to those of skill in the art. Complement generates numerous specific products. During the last decade, sensitive and specific assays have been developed and are available commercially for most of these activation products, including the small activation fragments C3a, C4a, and C5a and the large activation fragments iC3b, C4d, Bb, and sC5b-9. Most of these assays utilize monoclonal antibodies that react with new antigens (neoantigens) exposed on the fragment, but not on the native proteins from which they are formed, making these assays very simple and specific. Most rely on ELISA technology, although radioimmunoassay is still sometimes used for C3a and C5a. These latter assays measure both the unprocessed fragments and their 'desArg' fragments, which are the major forms found in the circulation. Unprocessed fragments and C5a$_{desArg}$ are rapidly cleared by binding to cell surface receptors and are hence present in very low concentrations, whereas C3a$_{desArg}$ does not bind to cells and accumulates in plasma. Measurement of C3a provides a sensitive, pathway-independent indicator of complement activation. Alternative pathway activation can be assessed by measuring the Bb fragment and/or measurement of factor D activation. Detection of the fluid-phase product of membrane attack pathway activation, sC5b-9, provides evidence that complement is being activated to completion. Because both the lectin and classical pathways generate the same activation products, C4a and C4d, measurement of these two fragments does not provide any information about which of these two pathways has generated the activation products.

The inhibition of MASP-3-dependent complement activation is characterized by at least one of the following changes in a component of the complement system that occurs as a result of administration of a MASP-3 inhibitory agent in accordance with the methods of the invention: the inhibition of LEA-1-mediated complement activation (inhibition of hemolysis and opsonization); inhibition of MASP-3 serine protease substrate-specific cleavage, the reduction of hemolysis (measured, for example as described in Example 5) or the reduction of C3 cleavage and C3b deposition (measured, for example, as described in Example 4 or Example 11).

The inhibition of MASP-2-dependent complement activation is characterized by at least one of the following changes in a component of the complement system that occurs as a result of administration of a MASP-2 inhibitory agent in accordance with the methods of the invention: the inhibition of the generation or production of MASP-2-dependent complement activation system products C4b, C3a, C5a and/or C5b-9 (MAC) (measured, for example, as described in measured, for example, as described in Example 2 of US Patent No. 7,919,094), the reduction of C4 cleavage and C4b deposition (measured, for example as described in Example 8 or Example 9), or the reduction of C3 cleavage and C3b deposition (measured, for example, as described in Example 11).

## i. Pharmaceutical carriers and delivery vehicles

In general, the MASP-3 inhibitory agent compositions and the MASP-2 inhibitory agent compositions of the present invention, or compositions comprising a combination of MASP-2 and MASP-3 inhibitory agents, may be combined with any other selected therapeutic agents, are suitably contained in a pharmaceutically acceptable carrier. The carrier is non-toxic, biocompatible and is selected so as not to detrimentally affect the biological activity of the MASP-3 inhibitory agent or the MASP-2 inhibitory agent (and any other therapeutic agents combined therewith). Exemplary pharmaceutically acceptable carriers for peptides are described in U.S. Patent No. 5,211,657 to Yamada. The MASP antibodies useful in the invention, as described herein, may be formulated into preparations in solid, semi-solid, gel, liquid or gaseous forms such as tablets, capsules, powders, granules, ointments, solutions, depositories, inhalants and injections allowing for oral, parenteral or surgical administration. The invention also contemplates local administration of the compositions by coating medical devices and the like.

Suitable carriers for parenteral delivery via injectable, infusion or irrigation and topical delivery include distilled water, physiological phosphate-buffered saline, normal or lactated Ringer's solutions, dextrose solution, Hank's solution, or propanediol. In addition, sterile, fixed oils may be employed as a solvent or suspending medium. For this purpose any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay or regulate) the delivery of the agent(s) or to enhance the delivery, uptake, stability or pharmacokinetics of the therapeutic agent(s). Such a delivery vehicle may include, by way of non-limiting example, microparticles, microspheres, nanospheres or nanoparticles composed of proteins, liposomes, carbohydrates, synthetic organic compounds, inorganic compounds, polymeric or copolymeric hydrogels and polymeric micelles. Suitable hydrogel and micelle delivery systems include the PEO:PHB:PEO copolymers and copolymer/cyclodextrin complexes disclosed in WO 2004/009664 A2 and the PEO and PEO/cyclodextrin complexes disclosed in U.S. Patent Application Publication No. 2002/0019369 A1. Such hydrogels may be injected locally at the site of intended action, or subcutaneously or intramuscularly to form a sustained release depot.

Compositions of the present invention may be formulated for delivery subcutaneously, intra-muscularly, intravenously, intra-arterially or as an inhalant.

For intra-articular delivery, the MASP-3 inhibitory agent or the MASP-2 inhibitory agent may be carried in above-described liquid or gel carriers that are injectable, above-described sustained-release delivery vehicles that are injectable, or a hyaluronic acid or hyaluronic acid derivative.

For oral administration of non-peptidergic agents, the MASP-3 inhibitory agent or MASP-2 inhibitory agent may be carried in an inert filler or diluent such as sucrose, cornstarch, or cellulose.

For topical administration, the MASP-3 inhibitory agent or MASP-2 inhibitory agent may be carried in ointment, lotion, cream, gel, drop, suppository, spray, liquid or powder, or in gel or microcapsular delivery systems via a transdermal patch.

Various nasal and pulmonary delivery systems, including aerosols, metered-dose inhalers, dry powder inhalers, and nebulizers, are being developed and may suitably be adapted for delivery of the present invention in an aerosol, inhalant, or nebulized delivery vehicle, respectively.

For intrathecal (IT) or intracerebroventricular (ICV) delivery, appropriately sterile delivery systems (e.g., liquids; gels, suspensions, etc.) can be used to administer the present invention.

The compositions of the present invention may also include biocompatible excipients, such as dispersing or wetting agents, suspending agents, diluents, buffers, penetration enhancers, emulsifiers, binders, thickeners, flavoring agents (for oral administration).

ii. Pharmaceutical carriers for antibodies and peptides

More specifically with respect to MASP antibodies, as described herein, exemplary formulations can be parenterally administered as injectable dosages of a solution or suspension of the compound in a physiologically acceptable diluent with a pharmaceutical carrier that can be a sterile liquid such as water, oils, saline, glycerol or ethanol. Additionally, auxiliary substances such as wetting or emulsifying agents, surfactants, pH buffering substances and the like can be present in compositions comprising MASP antibodies. Additional components of pharmaceutical compositions include petroleum (such as of animal, vegetable or synthetic origin), for example, soybean oil and mineral oil. In general, glycols such as propylene glycol or polyethylene glycol are preferred liquid carriers for injectable solutions.

The MASP antibodies can also be administered in the form of a depot injection or implant preparation that can be formulated in such a manner as to permit a sustained or pulsatile release of the active agents.

## XVIX. MODES OF ADMINISTRATION

The pharmaceutical compositions comprising the MASP-3 inhibitory agents or MASP-2 inhibitory agents may be administered in a number of ways depending on whether a local or systemic mode of administration is most appropriate for the condition being treated. Further, the compositions of the present invention can be delivered by coating or incorporating the compositions on or into an implantable medical device.

i. Systemic delivery

As used herein, the terms "systemic delivery" and "systemic administration" are intended to include but are not limited to oral and parenteral routes including intramuscular (IM), subcutaneous, intravenous (IV), intraarterial, inhalational, sublingual, buccal, topical, transdermal, nasal, rectal, vaginal and other routes of administration that effectively result in dispersement of the delivered agent to a single or multiple sites of intended therapeutic action. Preferred routes of systemic delivery for the present compositions include intravenous, intramuscular, subcutaneous, intraarterial and inhalational. It will be appreciated that the exact systemic administration route for selected agents utilized in particular compositions of the present invention will be determined in part to account for the agent's susceptibility to metabolic transformation pathways associated with a given route of administration. For example, peptidergic agents may be most suitably administered by routes other than oral.

The MASP inhibitory antibodies, as described herein, can be delivered into a subject in need thereof by any suitable means. Methods of delivery of MASP antibodies and polypeptides include administration by oral, pulmonary, parenteral (e.g., intramuscular, intraperitoneal, intravenous (IV) or subcutaneous injection), inhalation (such as via a fine powder formulation), transdermal, nasal, vaginal, rectal, or sublingual routes of administration, and can be formulated in dosage forms appropriate for each route of administration.

By way of representative example, MASP inhibitory antibodies and peptides can be introduced into a living body by application to a bodily membrane capable of absorbing the polypeptides, for example the nasal, gastrointestinal and rectal membranes. The polypeptides are typically applied to the absorptive membrane in conjunction with a permeation enhancer. (See, e.g., Lee, V.H.L., Crit. Rev. Ther. Drug Carrier Sys. 5:69, (1988); Lee, V.H.L., J. Controlled Release 13:213, (1990); Lee, V.H.L., Ed., Peptide and Protein Drug Delivery, Marcel Dekker, New York (1991); DeBoer, A.G., et al., J. Controlled Release 13:241, (1990). For example, STDHF is a synthetic derivative of fusidic acid, a steroidal surfactant that is similar in structure to the bile salts, and has been used as a permeation enhancer for nasal delivery. (Lee, W.A., Biopharm. 22, Nov./Dec. 1990.)

The MASP inhibitory antibodies as described herein may be introduced in association with another molecule, such as a lipid, to protect the polypeptides from enzymatic degradation. For example, the covalent attachment of polymers, especially polyethylene glycol (PEG), has been used to protect certain proteins from enzymatic hydrolysis in the body and thus prolong half-life (Fuertges, F., et al., J. Controlled Release 11:139, (1990)). Many polymer systems have been reported for protein delivery (Bae, Y.H., et al., J. Controlled Release 9:271, (1989); Hori, R., et al., Pharm. Res. 6:813, (1989); Yamakawa, I., et al., J. Pharm. Sci. 79:505, (1990); Yoshihiro, I., et al., J. Controlled Release 10:195, (1989); Asano, M., et al., J. Controlled Release 9:111, (1989); Rosenblatt, J., et al., J. Controlled Release 9:195, (1989); Makino, K., J. Controlled Release 12:235, (1990); Takakura, Y., et al., J. Pharm. Sci. 78:117, (1989); Takakura, Y., et al., J. Pharm. Sci. 78:219, (1989)).

Recently, liposomes have been developed with improved serum stability and circulation half-times (see, e.g., U.S. Patent No. 5,741,516, to Webb). Furthermore, various methods of liposome and liposome-like preparations as potential drug carriers have been reviewed (see, e.g., U.S. Patent No. 5,567,434, to Szoka; U.S. Patent No. 5,552,157, to Yagi; U.S. Patent No. 5,565,213, to Nakamori; U.S. Patent No. 5,738,868, to Shinkarenko; and U.S. Patent No. 5,795,587, to Gao).

For transdermal applications, the MASP inhibitory antibodies, as described herein, may be combined with other suitable ingredients, such as carriers and/or adjuvants. There are no limitations on the nature of such other ingredients, except that they must be pharmaceutically acceptable for their intended administration, and cannot degrade the activity of the active ingredients of the composition. Examples of suitable vehicles include ointments, creams, gels, or suspensions, with or without purified collagen. The MASP inhibitory antibodies may also be impregnated into transdermal patches, plasters, and bandages, preferably in liquid or semi-liquid form.

The compositions of the present invention may be systemically administered on a periodic basis at intervals determined to maintain a desired level of therapeutic effect. For example, compositions may be administered, such as by subcutaneous injection, every two to four weeks or at less frequent intervals. The dosage regimen will be determined by the physician considering various factors that may influence the action of the combination of agents. These factors will include the extent of progress of the condition being treated, the patient's age, sex and weight, and other clinical factors. The dosage for each individual agent will vary as a function of the MASP-3 inhibitory agent or the MASP-2 inhibitory agent that is included in the composition, as well as the presence and nature of any drug delivery vehicle (e.g., a sustained release delivery vehicle). In addition, the dosage quantity may be adjusted to account for variation in the frequency of administration and the pharmacokinetic behavior of the delivered agent(s).

ii. Local delivery

As used herein, the term "local" encompasses application of a drug in or around a site of intended localized action, and may include for example topical delivery to the skin or other affected tissues, ophthalmic delivery, intrathecal (IT), intracerebroventricular (ICV), intra-articular, intracavity, intracranial or intravesicular administration, placement or irrigation. Local administration may be preferred to enable administration of a lower dose, to avoid systemic side effects, and for more accurate control of the timing of delivery and concentration of the active agents at the site of local delivery. Local administration provides a known concentration at the target site, regardless of interpatient variability in metabolism, blood flow, etc. Improved dosage control is also provided by the direct mode of delivery.

Local delivery of a MASP-3 inhibitory agent or a MASP-2 inhibitory agent may be achieved in the context of surgical methods for treating a disease or condition, such as for example during procedures such as arterial bypass surgery, atherectomy, laser procedures, ultrasonic procedures, balloon angioplasty and stent placement. For example, a MASP-3 inhibitory agent or a MASP-2 inhibitory agent can be administered to a subject in conjunction with a balloon angioplasty procedure. A balloon angioplasty procedure involves inserting a catheter having a deflated balloon into an artery. The deflated balloon is positioned in proximity to the atherosclerotic plaque and is inflated such that the plaque is compressed against the vascular wall. As a result, the balloon surface is in contact with the layer of vascular endothelial cells on the surface of the blood vessel. The MASP-3 inhibitory agent or MASP-2 inhibitory agent may be attached to the balloon angioplasty catheter in a manner that permits release of the agent at the site of the atherosclerotic plaque. The agent may be attached to the balloon catheter in accordance with standard procedures known in the art. For example, the agent may be stored in a compartment of the balloon catheter until the balloon is inflated, at which point it is released into the local environment. Alternatively, the agent may be impregnated on the balloon surface, such that it contacts the cells of the arterial wall as the balloon is inflated. The agent may also be delivered in a perforated balloon catheter such as those disclosed in Flugelman, M.Y., et al., Circulation 85:1110-1117, (1992). See also published PCT Application WO 95/23161 for an exemplary procedure for attaching a therapeutic protein to a balloon angioplasty catheter. Likewise, the MASP-3 inhibitory agent or MASP-2 inhibitory agent may be included in a gel or polymeric coating applied to a stent, or may be incorporated into the material of the stent, such that the stent elutes the MASP-3 inhibitory agent or MASP-2 inhibitory agent after vascular placement.

MASP-3 inhibitory agents or MASP-2 inhibitory agents used in the treatment of arthritides and other musculoskeletal disorders may be locally delivered by intra-articular injection. Such compositions may suitably include a sustained release delivery vehicle. As a further example of instances in which local delivery may be desired, MASP-2 inhibitory compositions used in the treatment of urogenital conditions may be suitably instilled intravesically or within another urogenital structure.

## XX. TREATMENT REGIMENS

In prophylactic applications, the pharmaceutical compositions are administered to a subject susceptible to, or otherwise at risk of, a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) and thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, in an amount sufficient to eliminate or reduce the risk of developing symptoms of the condition. In therapeutic applications, the pharmaceutical compositions are administered to a subject suspected of, or already suffering from, a disease or disorder selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, in a therapeutically effective amount sufficient to relieve, or at least partially reduce, the symptoms of the condition.

In one embodiment, the pharmaceutical composition is administered to a subject suffering from, or at risk for developing PNH In accordance with this the subject's red blood cells are opsonized by fragments of C3 in the absence of the composition, and administration of the composition to the subject increases the survival of red blood cells in the subject. In one embodiment, the subject exhibits one or more symptoms in the absence of the composition selected from the group consisting of (i) below normal levels of hemoglobin, (ii) below normal levels of platelets; (iii) above normal levels of reticulocytes, and (iv) above normal levels of bilirubin, and administration of the composition to the subject improves at least one or more of the symptoms, resulting in (i) increased, normal, or nearly normal levels of hemoglobin (ii) increased, normal or nearly normal levels of platelets, (iii) decreased, normal or nearly normal levels of reticulocytes, and/or (iv) decreased, normal or nearly normal levels of bilirubin.

In both prophylactic and therapeutic regimens for the treatment, prevention or reduction in severity of a disease or condition selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease, compositions comprising MASP-3 inhibitory agents and optionally MASP-2 inhibitory agents may be administered in several dosages until a sufficient therapeutic outcome has been achieved in the subject. In one embodiment of the invention, the MASP-3 and/or MASP-2 inhibitory agent comprises a MASP-1 antibody, a MASP-2 antibody or a MASP-3 antibody, which suitably may be administered to an adult patient (e.g., an average adult weight of 70 kg) in a dosage of from 0.1 mg to 10,000 mg, more suitably from 1.0 mg to 5,000 mg, more suitably 10.0 mg to 2,000 mg, more suitably 10.0 mg to 1,000 mg and still more suitably from 50.0 mg to 500 mg, or 10 to 200 mg. For pediatric patients, dosage can be adjusted in proportion to the patient's weight.

Application of the MASP-3 inhibitory compositions and optional MASP-2 inhibitory compositions of the present invention may be carried out by a single administration of the composition (e.g., a single composition comprising MASP-2 and MASP-3 inhibitory agents, or bispecific or dual inhibitory agents, or co-administration of separate compositions), or a limited sequence of administrations, for treatment of a disease or disorder selected form the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease. Alternatively, the composition may be administered at periodic intervals such as daily, biweekly, weekly, every other week, monthly or bimonthly over an extended period of time for as determined by a physician for optimal therapeutic effect.

In some embodiments, a first composition comprising at least one MASP-3 inhibitory agent and a second composition comprising at least one MASP-2 inhibitory agent are administered to a subject suffering from, or at risk for developing a disease or condition selected from the group consisting of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica and Behcet's disease. In one embodiment, the first composition comprising at least one MASP-3 inhibitory agent and a second composition comprising at least one MASP-2 inhibitory agent are administered simultaneously (i.e., within a time separation of no more than about 15 minutes or less, such as no more than any of 10, 5 or 1 minute). In one embodiment, the first composition comprising at least one MASP-3 inhibitory agent and a second composition comprising at least one MASP-2 inhibitory agent are administered sequentially (i.e., the first composition is administered either prior to or after the administration of the second composition, wherein the time separation of administration is more than 15 minutes). In some embodiments, the first composition comprising at least one MASP-3 inhibitory agent and a second composition comprising at least one MASP-2 inhibitory agent are administered concurrently (i.e., the administration period of the first composition overlaps with the administration of the second composition). For example, in some embodiments, the first composition and/or the second composition are administered for a period of at least one, two, three or four weeks or longer. In one embodiment, at least one MASP-3 inhibitory agent and at least one MASP-2 inhibitory agent are combined in a unit dosage form. In one embodiment, a first composition comprising at least one MASP-3 inhibitory agent and a second composition comprising at least one MASP-2 inhibitory agent are packaged together in a kit for use in treatment of paroxysmal nocturnal hemoglobinuria (PNH), age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica or Behcet's disease.

In some embodiments, the subject suffering from PNH, age-related macular degeneration (AMD), ischemia-reperfusion injury, arthritis, disseminated intravascular coagulation, thrombotic microangiopathy (including hemolytic uremic syndrome (HUS), atypical hemolytic uremic syndrome (aHUS) or thrombotic thrombocytopenic purpura (TTP)), asthma, dense deposit disease, pauci-immune necrotizing crescentic glomerulonephritis, traumatic brain injury, aspiration pneumonia, endophthalmitis, neuromyelitis optica or Behcet's disease, has previously undergone, or is currently undergoing treatment with a terminal complement inhibitor that inhibits cleavage of complement protein C5. In some embodiments, the method comprises administering to the subject a composition of the invention comprising a MASP-3 and optionally a MASP-2 inhibitor and further administering to the subject a terminal complement inhibitor that inhibits cleavage of complement protein C5. In some embodiments, the terminal complement inhibitor is a humanized anti-C5 antibody or antigen-binding fragment thereof. In some embodiments, the terminal complement inhibitor is eculizumab.

## XXI. EXAMPLES

The following examples merely illustrate the best mode now contemplated for practicing the invention, but should not be construed to limit the invention. All literature citations herein are expressly incorporated by reference.

## EXAMPLE 1

This Example demonstrates that MASP-2 deficient mice are protected from *Neisseria meningitidis* induced mortality after infection with either *N. meningitidis* serogroup A or *N. meningitidis* serogroup B.

## Methods:

MASP-2 knockout mice (MASP-2 KO mice) were generated as described in Example 1 of US 7,919,094, hereby incorporated herein by reference. 10-week-old MASP-2 KO mice (n=10) and wild-type (WT) C57/BL6 mice (n=10) were inoculated by intraperitoneal (i.p.) injection with a dosage of $2.6 \times 10^7$ CFU of *N. meningitidis* serogroup A Z2491 in a volume of 100 $\mu$l. The infective dose was administered to mice in conjunction with iron dextran at a final concentration of 400 mg/kg. Survival of the mice after infection was monitored over a 72-hour time period.

In a separate experiment, 10-week-old MASP-2 KO mice (n=10) and WT C57/BL6 mice (n=10) were inoculated by i.p. injection with a dosage of $6 \times 10^6$ CFU of *N. meningitidis* serogroup B strain MC58 in a volume of 100 $\mu$l. The infective dose was administered to mice in conjunction with iron dextran at a final dose of 400 mg/kg. Survival of the mice after infection was monitored over a 72-hour time period. An illness score was also determined for the WT and MASP-2 KO mice during the 72-hour time period after infection, based on the illness scoring parameters described below in **TABLE 4**, which is based on the scheme of Fransen et al. (2010) with slight modifications.

**TABLE 4: Illness Scoring associated with clinical signs in infected mice**

| Signs | Score |
|---|---|
| Normal | 0 |
| Slightly ruffled fur | 1 |
| Ruffled fur, slow and sticky eyes | 2 |
| Ruffled fur, lethargic and eyes shut | 3 |
| Very sick and no movement after stimulation | 4 |
| Dead | 5 |

Blood samples were taken from the mice at hourly intervals after infection and analyzed to determine the serum level (log cfu/mL) of *N. meningitidis* in order to verify infection and determine the rate of clearance of the bacteria from the serum.

**Results:**

**FIGURE 8** is a Kaplan-Meyer plot graphically illustrating the percent survival of MASP-2 KO and WT mice after administration of an infective dose of $2.6 \times 10^7$ cfu of *N. meningitidis* serogroup A Z2491. As shown in **FIGURE 8**, 100% of the MASP-2 KO mice survived throughout the 72-hour period after infection. In contrast, only 80% of the WT mice ($p$=0.012) were still alive 24 hours after infection, and only 50% of the WT mice were still alive at 72 hours after infection. These results demonstrate that MASP-2-deficient mice are protected from *N. meningitidis* serogroup A Z2491-induced mortality.

**FIGURE** 9 is a Kaplan-Meyer plot graphically illustrating the percent survival of MASP-2 KO and WT mice after administration of an infective dose of $6 \times 10^6$ cfu of *N. meningitidis* serogroup B strain MC58. As shown in **FIGURE 9**, 90% of the MASP-2 KO mice survived throughout the 72-hour period after infection. In contrast, only 20% of the WT mice ($p$=0.0022) were still alive 24 hours after infection. These results demonstrate that MASP-2-deficient mice are protected from *N. meningitidis* serogroup B strain MC58-induced mortality.

**FIGURE 10** graphically illustrates the log cfu/mL of *N. meningitidis* serogroup B strain MC58 recovered at different time points in blood samples taken from the MASP-2 KO and WT mice after i.p. infection with $6 \times 10^6$ cfu of *N. meningitidis* serogroup B strain MC58 (n=3 at different time points for both groups of mice). The results are expressed as Means±SEM. As shown in **FIGURE 10**, in WT mice the level of *N. meningitidis* in the blood reached a peak of about 6.0 log cfu/mL at 24 hours after infection and dropped to about 4.0 log cfu/mL by 36 hours after infection. In contrast, in the MASP-2 KO mice, the level of *N. meningitidis* reached a peak of about 4.0 log cfu/mL at 12 hours after infection and dropped to about 1.0 log cfu/mL by 36 hours after infection (the symbol "*" indicates p<0.05; the symbol "**" indicates p=0.0043). These results demonstrate that although the MASP-2 KO mice were infected with the same dose of *N. meningitidis* serogroup B strain MC58 as the WT mice, the MASP-2 KO mice have enhanced clearance of bacteraemia as compared to WT.

**FIGURE 11** graphically illustrates the average illness score of MASP-2 KO and WT mice at 3, 6, 12 and 24 hours after infection with $6 \times 10^6$ cfu of *N. meningitidis* serogroup B strain MC58. As shown in **FIGURE 11**, the MASP-2-deficient mice showed high resistance to the infection, with much lower illness scores at 6 hours (symbol "*" indicates p=0.0411), 12 hours (symbol "**" indicates p=0.0049) and 24 hours (symbol "***" indicates p=0.0049) after infection, as compared to WT mice. The results in **FIGURE 11** are expressed as means±SEM.

In summary, the results in this Example demonstrate that MASP-2-deficient mice are protected from *N. meningitides*-induced mortality after infection with either *N. meningitidis* serogroup A or *N. meningitidis* serogroup B.


## EXAMPLE 2

This Example demonstrates that the administration of MASP-2 antibody after infection with *N. meningitidis* increases the survival of mice infected with *N. meningitidis.*

**Background/Rationale:**

As described in Example 24 of US Patent 7,919,094, incorporated herein by reference, rat MASP-2 protein was utilized to pan a Fab phage display library, from which Fab2 #11 was identified as a functionally active antibody. Full-length antibodies of the rat IgG2c and mouse IgG2a isotypes were generated from Fab2 #11. The full-length MASP-2 antibody of the mouse IgG2a isotype was characterized for pharmacodynamic parameters (as described in Example 38 of US Patent 7,919,094).

In this Example, the mouse MASP-2 full-length antibody derived from Fab2 #11 was analyzed in the mouse model of *N. meningitidis* infection.

**Methods:**

The mouse IgG2a full-length MASP-2 antibody isotype derived from Fab2 #11, generated as described above, was tested in the mouse model of *N. meningitidis* infection as follows.

***1. Administration of mouse-MASP-2 monoclonal antibodies (MoAb) after infection***

9-week-old C57/BL6 Charles River mice were treated with inhibitory mouse MASP-2 antibody (1.0 mg/kg) (n=12) or control isotype antibody (n=10) at 3 hours after i.p. injection with a high dose ($4 \times 10^6$ cfu) of *N. meningitidis* serogroup B strain MC58.

**Results:**

**FIGURE 12** is a Kaplan-Meyer plot graphically illustrating the percent survival of mice after administration of an infective dose of $4\times10^6$ cfu of *N. meningitidis* serogroup B strain MC58, followed by administration 3 hours post-infection of either inhibitory MASP-2 antibody (1.0 mg/kg) or control isotype antibody. As shown in **FIGURE 12**, 90% of the mice treated with MASP-2 antibody survived throughout the 72-hour period after infection. In contrast, only 50% of the mice treated with isotype control antibody survived throughout the 72-hour period after infection. The symbol "∗" indicates p=0.0301, as determined by comparison of the two survival curves.

These results demonstrate that administration of a MASP-2 antibody is effective to treat and improve survival in subjects infected with N. *meningitidis.*

As demonstrated herein, the use of MASP-2 antibody in the treatment of a subject infected with *N. meningitidis* is effective when administered within 3 hours post-infection, and is expected to be effective within 24 hours to 48 hours after infection. Meningococcal disease (either meningococcemia or meningitis) is a medical emergency, and therapy will typically be initiated immediately if meningococcal disease is suspected (i.e., before *N. meningitidis* is positively identified as the etiological agent).

In view of the results in the MASP-2 KO mouse demonstrated in EXAMPLE 1, it is believed that administration of MASP-2 antibody prior to infection with *N. meningitidis* would also be effective to prevent or ameliorate the severity of infection.

**EXAMPLE 3**

This Example demonstrates the complement-dependent killing of *N. meningitidis* in human sera is MASP-3-dependent.

**Rationale:**

Patients with decreased serum levels of functional MBL display increased susceptibility to recurrent bacterial and fungal infections (Kilpatrick et al., Biochim Biophys Acta 1572:401-413 (2002)). It is known that *N. meningitidis* is recognized by MBL, and it has been shown that MBL-deficient sera do not lyse *N. meningitidis.*

In view of the results described in Examples 1 and 2, a series of experiments were carried out to determine the efficacy of administration of MASP-2 antibody to treat *N. meningitidis* infection in complement-deficient and control human sera. Experiments were carried out in a high concentration of serum (20%) in order to preserve the complement pathway.

**Methods:**

***1. Serum bactericidal activity in various complement-deficient human sera and in human sera treated with human MASP-2 antibody***

The following complement-deficient human sera and control human sera were used in this experiment:

**TABLE 5: Human serum samples tested (as shown in FIGURE 13)**

| Sample | Serum type |
|--------|-----------|
| A | Normal human sera (NHS) + human MASP-2 Ab |
| B | NHS + isotype control Ab |
| C | MBL -/- human serum |
| D | NHS |
| E | Heat-Inactivated (HI) NHS |

A recombinant antibody against human MASP-2 was isolated from a combinatorial Antibody Library (Knappik, A., et al., J. Mol. Biol. 296:57-86 (2000)), using recombinant human MASP-2A as an antigen (Chen, C.B. and Wallis, J. Biol. Chem. 276:25894-25902 (2001)). An anti-human scFv fragment that potently inhibited lectin pathway-mediated activation of C4 and C3 in human plasma (IC50~20 nM) was identified and converted to a full-length human IgG4 antibody.

*N. meningitidis* serogroup B-MC58 was incubated with the different sera show in **TABLE 5**, each at a serum concentration of 20%, with or without the addition of inhibitory human MASP-2 antibody (3 μg in 100 μl total volume) at 37°C with shaking. Samples were taken at the following time points: 0-, 30-, 60- and 90-minute intervals, plated out and then viable counts were determined. Heat-inactivated human serum was used as a negative control.

**Results:**

**FIGURE 13** graphically illustrates the log cfu/mL of viable counts of *N. meningitidis* serogroup B-MC58 recovered at different time points in the human sera samples shown in **TABLE 5. TABLE 6** provides the Student's t-test results for **FIGURE 13.**

**TABLE 6**: Student's t-test Results for **FIGURE 13** (time point 60 minutes)

|  | **Mean Diff. (Log)** | **Significant? P<0.05?** | **P value summary** |
|---|---|---|---|
| AvsB | -0.3678 | Yes | ***(0.0002) |
| A vs C | -1.1053 | Yes | ***(p<0.0001) |
| AvsD | -0.2111 | Yes | **(0.0012) |
| CvsD | 1.9 | Yes | ***(p<0.0001) |

As shown in **FIGURE 13** and **TABLE 6**, complement-dependent killing of *N. meningitidis* in human 20% serum was significantly enhanced by the addition of the human MASP-2 inhibitory antibody.

*2. Serum bactericidal activity in various complement-deficient human sera*

The following complement-deficient human sera and control human sera were used in this experiment:

**TABLE 7:** Human serum samples tested (as shown in **FIGURE 14**)

| **Sample** | **Serum Type** |
|---|---|
| A | Normal human serum (NHS) |
| B | Heat-inactivated NHS |
| C | MBL -/- |
| D | MASP-3 -/- (MASP-1 +) |

Note: The MASP-3 -/- (MASP-1 +) serum in sample D was taken from a subject with 3MC syndrome, which is a unifying term for the overlapping Carnevale, Mingarelli, Malpuech and Michels syndromes. As further described in Example 4, the mutations in exon 12 of the MASP-1/3 gene render the serine protease domain of MASP-3, but not MASP-1 dysfunctional. As described in Example 19, pro-factor D is preferentially present in 3MC serum, whereas activated factor D is preferentially present in normal human serum.

*N. meningitidis* serogroup B-MC58 was incubated with different complement-deficient human sera, each at a serum concentration of 20%, at 37°C with shaking. Samples were taken at the following time points: 0-, 15-, 30-, 45-, 60-, 90- and 120-minute intervals, plated out and then viable counts were determined. Heat-inactivated human serum was used as a negative control.

**Results:**

**FIGURE 14** graphically illustrates the log cfu/mL of viable counts of *N. meningitidis* serogroup B-MC58 recovered at different time points in the human sera samples shown in **TABLE 7.** As shown in **FIGURE 14**, the WT (NHS) serum has the highest level of bactericidal activity for *N. meningitidis.* In contrast, the MBL -/- and MASP-3 -/- (which is MASP-1-sufficient) human sera do not have any bactericidal activity. These results indicate that complement-dependent killing of *N. meningitidis* in human 20% (v/v) serum is MASP-3- and MBL-dependent. **TABLE** 8 provides the Student's t-test results for **FIGURE 14.**

TABLE 8: Student's t-test Results for **FIGURE 14**

| Comparison | Time Point (min) | Mean Diff. (Log) | Significant? P<0.05? | P value Summary |
|---|---|---|---|---|
| AvsB | 60 | -0.8325 | Yes | ***(p<0.0001) |
| AvsB | 90 | -1.600 | Yes | ***(p<0.0001) |
| A vs C | 60 | -1.1489 | Yes | ***(p<0.0001) |
| A vs C | 90 | -1.822 | Yes | ***(p<0.0001) |
| AvsD | 60 | -1.323 | Yes | ***(0.0005) |
| AvsD | 90 | -2.185 | Yes | ***(p<0.0001) |

In summary, the results shown in **FIGURE 14** and **TABLE 8** demonstrate that complement-dependent killing of *N. meningitidis* in 20% human serum is MASP-3- and MBL-dependent.

**3. Complement-dependent killing of *N. meningitidis* in 20% (v/v) mouse sera deficient of MASP-2, MASP-1/3 or MBL A/C.**

The following complement-deficient mouse sera and control mouse sera were used in this experiment:

TABLE 9: Mouse serum samples tested (as shown in **FIGURE 15**)

| Sample | Serum Type |
|---|---|
| A | WT |
| B | MASP-2 -/- |
| C | MASP-1/3 -/- |
| D | MBL A/C -/- |
| E | WT heat-inactivated (HIS) |

*N. meningitidis* serogroup B-MC58 was incubated with different complement-deficient mouse sera, each at a serum concentration of 20%, at 37°C with shaking. Samples were taken at the following time points: 0-, 15-, 30-, 60-, 90- and 120-minute intervals, plated out and then viable counts were determined. Heat-inactivated human serum was used as a negative control.

**Results:**

**FIGURE 15** graphically illustrates the log cfu/mL of viable counts of *N. meningitidis* serogroup B-MC58 recovered at different time points in the mouse serum samples shown in **TABLE 9.** As shown in **FIGURE 15**, the MASP-2 -/- mouse sera have a higher level of bactericidal activity for *N. meningitidis* than WT mouse sera. In contrast, the MASP-1/3 -/- mouse sera do not have any bactericidal activity. The symbol "**" indicates p=0.0058, the symbol "***" indicates p=0.001. **TABLE 10** provides the Student's t-test results for **FIGURE 15.**

TABLE 10: Student's t-test Results for **FIGURE 15**

| Comparison | Time point | Mean Diff. (LOG) | Significant? (p<0.05)? | P value summary |
|---|---|---|---|---|
| A vs. B | 60 min. | 0.39 | yes | ** (0.0058) |
| A vs. B | 90 min. | 0.6741 | yes | *** (0.001) |

In summary, the results in this Example demonstrate that MASP-2 -/- serum has a higher level of bactericidal activity for *N. meningitidis* than WT serum and that complement-dependent killing of *N. meningitidis* in 20% serum is MASP-3- and MBL-dependent.

**EXAMPLE 4**

This Example describes a series of experiments that were carried out to determine the mechanism of the MASP-3-dependent resistance to *N. meningitidis* infection observed in MASP-2 KO mice, as described in Examples 1-3.

**Rationale:**

In order to determine the mechanism of MASP-3-dependent resistance to *N. meningitidis* infection observed in MASP-2 KO mice (described in Examples 1-3 above), a series of experiments were carried out as follows.

**1. MASP-1/3-deficient mice are not deficient of lectin pathway functional activity (also referred to as "LEA-2")**

**Methods:**

In order to determine whether MASP-1/3-deficient mice are deficient of lectin pathway functional activity (also referred to as LEA-2), an assay was carried out to measure the kinetics of C3 convertase activity in plasma from various complement-deficient mouse strains tested under lectin activation pathway-specific assay conditions (1% plasma), as described in Schwaeble W. et al., PNAS vol 108(18):7523-7528 (2011), hereby incorporated herein by reference.

Plasma was tested from WT, C4-/-, MASP-1/3-/-; Factor B-/-, and MASP-2-/- mice as follows.

To measure C3 activation, microtiter plates were coated with mannan (1 $\mu$g/well), zymosan (1 $\mu$g/well) in coating buffer (15 mM $Na_2CO_3$, 35 mM $NaHCO_3$), or immune complexes, generated *in situ* by coating with 1% human serum albumin (HSA) in coating buffer then adding sheep anti-HAS serum (2 $\mu$g/mL) in TBS (10mM Tris, 140 mM NaCl, pH 7.4) with 0.05% Tween 20 and 5 mM $Ca^{++}$. Plates were blocked with 0.1% HSA in TBS and washed three times with TBS/Tween20/ $Ca^{++}$. Plasma samples were diluted in 4 mM barbital, 145 mM NaCl, 2 mM $CaCl_2$, 1 mM $MgCl_2$, pH 7.4, added to the plates and incubated for 1.5 h at 37°C. After washing, bound C3b was detected using rabbit anti-human C3c (Dako), followed by alkaline phosphatase-conjugated goat anti-rabbit IgG and p-nitrophenyl phosphate.

**Results:**

The kinetics of C3 activation (as measured by C3b deposition on mannan-coated plates with 1% serum) under lectin pathway-specific conditions is shown in **FIGURE 16.** No C3 cleavage was seen in MASP-2-/- plasma. Factor B-/- (Factor B -/-) plasma cleaved C3 at half the rate of WT plasma, likely due to the loss of the amplification loop. A significant delay in the lectin pathway-dependent conversion of C3 to C3b was seen in C4-/- ($T_{1/2}$=33min) as well as in MASP-1/3-/- deficient plasma ($T_{1/2}$=49 min). This delay of C3 activation in MASP-1/3 -/- plasma has been shown to be MASP-1- rather than MASP-3-dependent. (See Takahashi M. et al., J Immunol 180:6132-6138 (2008)). These results demonstrate that MASP-1/3-deficient mice are not deficient of lectin pathway functional activity (also referred to as "LEA-2").

**2. Effect of hereditary MASP-3 deficiency on alternative pathway activation.**

**Rationale:**

The effect of hereditary MASP-3 deficiency on alternative pathway activation was determined by testing serum of a MASP-3-deficient patient with 3MC syndrome caused by a frame-shift mutation in the exon encoding the serine protease of MASP-3. The 3MC syndrome is a unifying term for the overlapping Carneavale, Mingarelli, Malpuech and Michels syndromes. These rare autosomal recessive disorders exhibit a spectrum of developmental features, including characteristic facial dysmorphism, cleft lip and/or palate, craniosynostosis, learning disability and genital, limb and vesicorenal abnormalities. Rooryck et al., Nature Genetics 43:197-203 (2011) studied 11 families with 3MC syndrome and identified two mutated genes, COLEC11 and MASP-1. The mutations in the MASP-1 gene render the exon encoding the serine protease domain of MASP-3, but not the exons encoding the serine protease of MASP-1, dysfunctional. Therefore, 3MC patients with mutations in the exon encoding the serine protease of MASP-3 are deficient of MASP-3 but sufficient in MASP-1.

**Methods:**

MASP-3-deficient serum was obtained from a 3MC patient, the mother and father of the 3MC patient (both heterozygous for the allele bearing a mutation that renders the exon encoding the MASP-3 serine protease domain dysfunctional), as well as from a C4-deficient patient (deficient in both human C4 genes) and an MBL-deficient subject. An alternative pathway assay was carried out under traditional AP-specific conditions (BBS/ $Mg^{++}$/EGTA, without $Ca^{++}$,

wherein BBS = barbital buffered saline containing sucrose), as described in Bitter-Suermann et al., Eur. J. Immunol 11:291-295 (1981)), on zymosan-coated microtiter plates at serum concentrations ranging from 0.5 to 25% and C3b deposition was measured over time.

**Results:**

**FIGURE 17** graphically illustrates the level of alternative pathway-driven C3b deposition on zymosan-coated microtiter plates as a function of serum concentration in serum samples obtained from MASP-3-deficient, C4-deficient and MBL-deficient subjects. As shown in **FIGURE 17**, MASP-3-deficient patient serum has residual alternative pathway (AP) activity at high serum concentrations (25%, 12.5%, 6.25% serum concentrations), but a significantly higher $AP_{50}$ (i.e.. 9.8% of serum needed to achieve 50% of maximum C3 deposition).

**FIGURE 18** graphically illustrates the level of alternative pathway-driven C3b deposition on zymosan-coated microtiter plates under "traditional" alternative pathway-specific (AP-specific) conditions (i.e., BBS/EGTA/Mg$^{++}$ without Ca$^{++}$) as a function of time in 10% human serum samples obtained from MASP-3-deficient, C4-deficient and MBL-deficient human subjects.

**TABLE 11** below summarizes the $AP_{50}$ results shown in **FIGURE 17** and the half-times for C3b deposition shown in **FIGURE 18**.

**TABLE 11: Summary of Results shown in FIGURES 17 and 18**

| Serum type | APso (%) | $T_{1/2}$ (min) |
|---|---|---|
| MASP-3-deficient (3MC patient) | 9.8 | 37.4 |
| Mother of 3MC patient (heterozygous) | 4.3 | 17.2 |
| Father of 3MC patient (heterozygous) | 4.3 | 20.9 |
| C4-deficient | 4.0 | 11.6 |
| MBL-deficient | 4.8 | 11.0 |
| Note: In BBS/ Mg$^{++}$/EGTA buffer, the lectin pathway-mediated effects are deficient due to absence of Ca$^{++}$ in this buffer. | | |

In summary, under the conditions of these assays, the alternative pathway is significantly compromised in the 3MC patient.

### 3. Measurement of C3b deposition on mannan, zymosan and S. pneumonia D39 in mouse sera deficient of MASP-2 or MASP-1/3.

**Methods:**

C3b deposition was measured on mannan, zymosan and *S. pneumonia* D39-coated microtiter plates using mouse serum concentrations ranging from 0% to 20% obtained from MASP-2-/-, MASP-1/3-/- and WT mice. The C3b deposition assays were carried out under either "traditional" alternative pathway-specific conditions (i.e. BBS/EGTA/Mg$^{++}$ without Ca$^{++}$), or under physiological conditions allowing both the lectin pathway and the alternative pathway to function (i.e., BBS/Mg$^{++}$/Ca$^{++}$).

**Results:**

**FIGURE 19A** graphically illustrates the level of C3b deposition on mannan-coated microtiter plates as a function of serum concentration in serum samples obtained from WT, MASP-2-deficient, and MASP-1/3-deficient mice under traditional alternative pathway-specific conditions (i.e., BBS/EGTA/Mg$^{++}$ without Ca$^{++}$), or under physiological conditions allowing both the lectin pathway and the alternative pathway to function (BBS/Mg$^{++}$/Ca$^{++}$). **FIGURE 19B** graphically illustrates the level of C3b deposition on zymosan-coated microtiter plates as a function of serum concentration in serum samples from WT, MASP-2-deficient, and MASP-1/3-deficient mice under traditional AP-specific conditions (i.e., BBS/EGTA/Mg$^{++}$ without Ca$^{++}$), or under physiological conditions allowing both the lectin pathway and the alternative pathway to function (BBS/Mg$^{++}$/Ca$^{++}$). **FIGURE 19C** graphically illustrates the level of C3b deposition on *S. pneumoniae* D39-coated microtiter plates as a function of serum concentration in serum samples from WT, MASP-2-deficient, and MASP-1/3-deficient mice under traditional AP-specific conditions (i.e., BBS/EGTA/Mg$^{++}$ without Ca$^{++}$), or under physiological conditions allowing both the lectin pathway and the alternative pathway to function (BBS/Mg$^{++}$/Ca$^{++}$).

**FIGURE 20A** graphically illustrates the results of a C3b deposition assay in highly diluted sera carried out on mannan-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), using serum concentrations ranging from 0 % up to 1.25%. **FIGURE 20B** graphically illustrates the results of a C3b deposition assay carried out on zymosan-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/EGTA/Mg$^{++}$/Ca$^{++}$), using serum concentrations ranging from 0 % up to 1.25%. **FIGURE 20C** graphically illustrates the results of a C3b deposition assay carried out on *S. pneumoniae* D39-coated microtiter plates under traditional AP-specific conditions (*i.e.* BBS/EGTA/Mg$^{++}$ without Ca$^{++}$) or under physiological conditions allowing both the lectin pathway and the alternative pathway to function (BBS/EGTA/Mg$^{++}$/Ca$^{++}$), using serum concentrations ranging from 0 % up to 1.25%.

As shown in **FIGURES 20A-C**, C3b deposition assays were also carried out under traditional alternative pathway-specific conditions (*i.e.* BBS/EGTA/Mg$^{++}$ without Ca$^{++}$) or under physiological conditions allowing both the lectin pathway and the alternative pathway to function (BBS/Mg$^{++}$/Ca$^{++}$), using higher dilutions ranging from 0 % up to 1.25% serum on mannan-coated plates (**FIGURE 20A**); zymosan-coated plates (**FIGURE 20B**) and *S. pneumoniae* D39-coated plates (**FIGURE 20C**). The alternative pathway tails off under higher serum dilutions, so the activity observed in the MASP-1/3-deficient serum in the presence of Ca$^{++}$ is MASP-2-mediated LP activity, and the activity in MASP-2-deficient serum in the presence of Ca$^{++}$ is MASP-1/3-mediated residual activation of the AP.

## Discussion:

The results described in this Example demonstrate that a MASP-2 inhibitor (or MASP-2 KO) provides significant protection from *N. meningitidis* infection by promoting MASP-3-driven alternative pathway activation. The results of the mouse serum bacteriolysis assays and the human serum bacteriolysis assays further show, by monitoring the serum bactericidal activity against *N. meningitidis,* that bactericidal activity against *N. meningitidis* is absent in MBL-deficient (mouse MBL A and MBL C double-deficient and human MBL-deficient sera).

**FIGURE 1** illustrates the new understanding of the lectin pathway and alternative pathway based on the results provided herein. **FIGURE 1** delineates the role of LEA-2 in both opsonization and lysis. While MASP-2 is the initiator of "downstream" C3b deposition (and resultant opsonization) in multiple lectin-dependent settings physiologically (**FIGURE 20A**, **20B**, **20C**), it also plays a role in lysis of serum-sensitive bacteria. As illustrated in **FIGURE 1**, the proposed molecular mechanism responsible for the increased bactericidal activity of MASP-2-deficient or MASP-2-depleted serum/plasma for serum-sensitive pathogens such as *N. meningitidis* is that, for the lysis of bacteria, lectin pathway recognition complexes associated with MASP-1 and MASP-3 have to bind in close proximity to each other on the bacterial surface, thereby allowing MASP-1 to cleave MASP-3. In contrast to MASP-1 and MASP-2, MASP-3 is not an auto-activating enzyme, but, in many instances, requires activation/cleavage by MASP-1 to be converted into its enzymatically active form.

As further shown in **FIGURE 1**, activated MASP-3 can then cleave C3b-bound factor B on the pathogen surface to initiate the alternative pathway activation cascade by formation of the enzymatically active alternative pathway C3 and C5 convertase C3bBb and C3bBb(C3b)n, respectively. MASP-2-bearing lectin-pathway activation complexes have no part in the activation of MASP-3 and, in the absence or after depletion of MASP-2, all-lectin pathway activation complexes will either be loaded with MASP-1 or MASP-3. Therefore, in the absence of MASP-2, the likelihood is markedly increased that on the microbial surface MASP-1 and MASP-3-bearing lectin-pathway activation complexes will come to sit in close proximity to each other, leading to more MASP-3 being activated and thereby leading to a higher rate of MASP-3-mediated cleavage of C3b-bound factor B to form the alternative pathway C3 and C5 convertases C3bBb and C3bBb(C3b)n on the microbial surface. This leads to the activation of the terminal activation cascades C5b-C9 that forms the Membrane Attack Complex, composed of surface-bound C5b associated with C6, C5bC6 associated with C7, C5bC6C7 associated with C8, and C5bC6C7C8, leading to the polymerization of C9 that inserts into the bacterial surface structure and forms a pore in the bacterial wall, which will lead to osmolytic killing of the complement-targeted bacterium.

The core of this novel concept is that the data provided herein clearly show that the lectin-pathway activation complexes drive the following two distinct activation routes, as illustrated in **FIGURE 1**:

## EXAMPLE 5

This Example demonstrates the inhibitory effect of MASP-2 deficiency and/or MASP-3 deficiency on lysis of red blood cells from blood samples obtained from a mouse model of paroxysmal nocturnal hemoglobinuria (PNH).

**Background/Rationale:**

Paroxysmal nocturnal hemoglobinuria (PNH), also referred to as Marchiafava-Micheli syndrome, is an acquired, potentially life-threatening disease of the blood, characterized by complement-induced intravascular hemolytic anemia. The hallmark of PNH is the chronic complement-mediated intravascular hemolysis that is a consequence of unregulated activation of the alternative pathway of complement due to the absence of the complement regulators CD55 and CD59 on PNH erythrocytes, with subsequent hemoglobinuria and anemia. Lindorfer, M.A., et al., Blood 115(11) (2010), Risitano, A.M, Mini-Reviews in Medicinal Chemistry, 11:528-535 (2011). Anemia in PNH is due to destruction of red blood cells in the bloodstream. Symptoms of PNH include red urine, due to appearance of hemoglobin in the urine, back pain, fatigue, shortness of breath and thrombosis. PNH may develop on its own, referred to as "primary PNH" or in the context of other bone marrow disorders such as aplastic anemia, referred to as "secondary PNH". Treatment for PNH includes blood transfusion for anemia, anticoagulation for thrombosis and the use of the monoclonal antibody eculizumab (Soliris®), which protects blood cells against immune destruction by inhibiting the complement system (Hillmen P. et al., N. Engl. J. Med. 350(6):552-9 (2004)). Eculizumab (Soliris®) is a humanized monoclonal antibody that targets the complement component C5, blocking its cleavage by C5 convertases, thereby preventing the production of C5a and the assembly of MAC. Treatment of PNH patients with eculizumab has resulted in a reduction of intravascular hemolysis, as measured by lactate dehydrogenase (LDH), leading to hemoglobin stabilization and transfusion independence in about half of the patients (Hillmen P, et al., Mini-Reviews in Medicinal Chemistry, vol 11(6) (2011)). While nearly all patients undergoing therapy with eculizumab achieve normal or almost normal LDH levels (due to control of intravascular hemolysis), only about one third of the patients reach a hemoglobin value about 11gr/dL, and the remaining patients on eculizumab continue to exhibit moderate to severe (*i.e.,* transfusion-dependent) anemia, in about equal proportions (Risitano A.M. et al., Blood 113:4094-100 (2009)). As described in Risitano et al., Mini-Reviews in Medicinal Chemistry 11:528-535 (2011), it was demonstrated that PNH patients on eculizumab contained C3 fragments bound to a substantial portion of their PNH erythrocytes (while untreated patients did not), leading to the conclusion that membrane-bound C3 fragments work as opsonins on PNH erythrocytes, resulting in their entrapment in the reticuloendotheli cells through specific C3 receptors and subsequent extravascular hemolysis. Therefore, therapeutic strategies in addition to the use of eculizumab are needed for those patients developing C3 fragment-mediated extravascular hemolysis because they continue to require red cell transfusions.

This Example describes methods to assess the effect of MASP-2- and MASP-3-deficient serum on lysis of red blood cells from blood samples obtained from a mouse model of PNH and demonstrates the efficacy of MASP-2 inhibition and/or MASP-3 inhibition to treat subjects suffering from PNH, and also supports the use of inhibitors of MASP-2 and/or inhibitors of MASP-3 (including dual or bispecific MASP-2/MASP-3 inhibitors) to ameliorate the effects of C3 fragment-mediated extravascular hemolysis in PNH subjects undergoing therapy with a C5 inhibitor such as eculizumab.

**Methods:**

**PNH animal model:**

Blood samples were obtained from gene-targeted mice with deficiencies of Crry and C3 (Crry/C3-/-) and CD55/CD59-deficient mice. These mice are missing the respective surface complement regulators on their erythrocytes and these erythrocytes are, therefore, susceptible to spontaneous complement autolysis as are PNH human blood cells.

In order to sensitize these erythrocytes even more, these cells were used with and without coating by mannan and then tested for hemolysis in WT C56/BL6 plasma, MBL null plasma, MASP-2 -/- plasma, MASP-1/3 -/- plasma, human NHS, human MBL -/plasma, and NHS treated with human MASP-2 antibody.

***1. Hemolysis assay of Crry/C3 and CD55/CD59 double-deficient murine erythrocytes in MASP-2-deficient/depleted sera and controls***

Day 1. Preparation of murine RBC (± mannan coating).

Materials included: fresh mouse blood, BBS/Mg$^{++}$/ Ca$^{++}$ (4.4 mM barbituric acid, 1.8 mM sodium barbitone, 145 mM NaCl, pH 7.4, 5mM Mg$^{++}$, 5mM Ca$^{++}$), chromium chloride, CrCl$_3$·6H$_2$0 (0.5mg/mL in BBS/Mg$^{++}$/ Ca$^{++}$) and mannan, 100 μg/mL in BBS/Mg$^{++}$/Ca$^{++}$.

Whole blood (2 mL) was spun down for 1-2 min at 2000xg in a refrigerated centrifuge at 4°C. The plasma and buffy coat were aspirated off. The sample was then washed 3x by re-suspending RBC pellet in 2 mL ice-cold BBS/gelatin/Mg$^{++}$/Ca$^{++}$ and repeating centrifugation step. After the third wash, the pellet was re-suspended in 4 mL BBS/Mg$^{++}$/Ca$^{++}$. A 2 mL aliquot of the RBC was set aside as an uncoated control. To the remaining 2 mL, 2 mL CrCl3 and 2 mL mannan were added and the sample was incubated with gentle mixing at RT for 5min. The reaction was terminated by adding 7.5 mL BBS/gelatin/Mg$^{++}$/Ca$^{++}$. The sample was spun down as above, re-suspended in 2 mL BBS/gelatin/Mg$^{++}$/Ca$^{++}$ and washed a further two times as above, then stored at 4°C.

**Day 2.** Hemolysis assay

Materials included BBS/gelatin/Mg$^{++}$/Ca$^{++}$ (as above), test sera, 96-well roundbottomed and flat-bottomed plates and a spectrophotometer that reads 96-well plates at 410-414 nm.

The concentration of the RBC was first determined and the cells were adjusted to 10$^9$/mL, and stored at this concentration. Before use, the cells were diluted in assay buffer to 10$^8$/mL, and then 100 ul per well was used. Hemolysis was measured at 410-414 nm (allowing for greater sensitivity than 541nm). Dilutions of test sera were prepared in ice-cold BBS/gelatin/Mg$^{++}$/Ca$^{++}$. 100 $\mu$l of each serum dilution was pipetted into roundbottomed plate. 100 $\mu$l of appropriately diluted RBC preparation was added (i.e., 10$^8$/mL), incubated at 37°C for about 1 hour, and observed for lysis. (The plates may be photographed at this point.) The plate was then spun down at maximum speed for 5 minutes. 100 $\mu$l of the fluid phase was aspirated, transferred to flat-bottom plates, and the OD was recorded at 410-414 nm. The RBC pellets were retained (these can be subsequently lysed with water to obtain an inverse result).

## Experiment #1

Fresh blood was obtained from CD55/CD59 double-deficient mice and blood of Crry/C3 double-deficient mice and erythrocytes were prepared as described in detail in the above protocol. The cells were split and half of the cells were coated with mannan and the other half were left untreated, adjusting the final concentration to 10$^8$/mL, of which 100 $\mu$l was used in the hemolysis assay, which was carried out as described above.

## Results of Experiment #1: The lectin pathway is involved in erythrocyte lysis in the PNH animal model

In an initial experiment, it was determined that non-coated WT mouse erythrocytes were not lysed in any mouse serum. It was further determined that mannan-coated Crry-/- mouse erythrocytes were slowly lysed (more than 3 hours at 37 degrees) in WT mouse serum, but they were not lysed in MBL null serum. (Data not shown).

It was determined that mannan-coated Crry-/- mouse erythrocytes were rapidly lysed in human serum but not in heat-inactivated NHS. Importantly, mannan-coated Crry-/- mouse erythrocytes were lysed in NHS diluted down to 1/640 (i.e., 1/40, 1/80, 1/160, 1/320 and 1/640 dilutions all lysed). (Data not shown). In this dilution, the alternative pathway does not work (AP functional activity is significantly reduced below 8% serum concentration).

## Conclusions from Experiment #1

Mannan-coated Crry-/- mouse erythrocytes are very well lysed in highly diluted human serum with MBL but not in that without MBL. The efficient lysis in every serum concentration tested implies that the alternative pathway is not involved or needed for this lysis. The inability of MBL-deficient mouse serum and human serum to lyse the mannan-coated Crry-/- mouse erythrocytes indicates that the classical pathway also has nothing to do with the lysis observed. As lectin pathway recognition molecules are required (i.e., MBL), this lysis is mediated by the lectin pathway.

## Experiment #2

Fresh blood was obtained from the Crry/C3 and CD55/CD59 double-deficient mice and mannan-coated Crry-/- mouse erythrocytes were analyzed in the haemolysis assay as described above in the presence of the following human serum: MASP-3 -/-; MBL null; WT; NHS pretreated with human MASP-2 antibody; and heat-inactivated NHS as a control.

## *Results of Experiment #2: MASP-2 inhibitors and MASP-3 deficiency prevents erythrocyte lysis in PNH animal model*

With the mannan-coated Crry-/- mouse erythrocytes, NHS was incubated in the dilutions diluted down to 1/640 (i.e., 1/40, 1/80, 1/160, 1/320 and 1/640), human MBL-/- serum, human MASP-3-deficient serum (from 3MC patient), and NHS pretreated with MASP-2 mAb, and heat-inactivated NHS as a control.

The ELISA microtiter plate was spun down and the non-lysed erythrocytes were collected on the bottom of the round-well plate. The supernatant of each well was collected and the amount of hemoglobin released from the lysed erythrocytes was measured by reading the OD415 nm in an ELISA reader.

It was observed that MASP-3-/- serum did not lyse mannan-coated mouse erythrocytes at all. In the control heat-inactivated NHS (negative control), as expected, no lysis was observed. MBL-/- human serum lysed mannan-coated mouse erythrocytes at 1/8 and 1/16 dilutions. MASP-2-antibody-pretreated NHS lysed mannan-coated mouse erythrocytes at 1/8 and 1/16 dilutions while WT human serum lysed mannan-coated mouse erythrocytes down to dilutions of 1/32.

**FIGURE 21** graphically illustrates hemolysis (as measured by hemoglobin release of lysed mouse erythrocytes (Crry/C3-/-) into the supernatant measured by photometry) of mannan-coated murine erythrocytes by human serum over a range of serum dilutions in serum from MASP-3-/-, heat-inactivated (HI) NHS, MBL-/-, NHS pretreated with MASP-2 antibody, and NHS control.

**FIGURE 22** graphically illustrates hemolysis (as measured by hemoglobin release of lysed mouse erythrocytes (Crry/C3-/-) into the supernatant measured by photometry) of mannan-coated murine erythrocytes by human serum over a range of serum concentration in serum from MASP-3-/-, heat-inactivated (HI) NHS, MBL-/-, NHS pretreated with MASP-2 antibody, and NHS control.

From the results shown in **FIGURE 21** and **22**, it is demonstrated that inhibiting MASP-3 will prevent any complement-mediated lysis of sensitized erythrocytes with deficient protection from autologous complement activation. MASP-2 inhibition with MASP-2 antibody significantly shifted the $CH_{50}$ and was protective to some extent, but MASP-3 inhibition was more effective.

### Experiment #3

Non-coated Crry-/- mouse erythrocytes obtained from fresh blood from the Crry/C3 and CD55/CD59 double-deficient mice were analyzed in the hemolysis assay as described above in the presence of the following sera: MASP-3-/-; MBL-/-; WT; NHS pretreated with human MASP-2 antibody, and heat-inactivated NHS as a control.

### Results:

**FIGURE 23** graphically illustrates hemolysis (as measured by hemoglobin release of lysed WT mouse erythrocytes into the supernatant measured by photometry) of non-coated murine erythrocytes over a range of serum concentrations in human sera from a 3MC (MASP-3-/-) patient, heat inactivated (HI) NHS, MBL-/-, NHS pretreated with MASP-2 antibody, and NHS control. As shown in **FIGURE 23** and summarized in **TABLE 12**, it is demonstrated that inhibiting MASP-3 inhibits complement-mediated lysis of non-sensitized WT mouse erythrocytes.

**FIGURE 24** graphically illustrates hemolysis (as measured by hemoglobin release of lysed mouse erythrocytes (CD55/59 -/-) into the supernatant measured by photometry) of non-coated murine erythrocytes by human serum over a range of serum concentrations in human sera from heat-inactivated (HI) NHS, MBL-/-, NHS pretreated with MASP-2 antibody, and NHS control. As shown in **FIGURE 24** and summarized in **TABLE 12**, it is demonstrated that inhibiting MASP-2 was protective to a limited extent.

**TABLE 12**: $CH_{50}$ values expressed as serum concentrations

| Serum | WT | CD55/59 -/- |
|---|---|---|
| 3MC patient | No lysis | No lysis |
| Heat-inactivated NHS | No lysis | No lysis |
| MBL AO/XX donor (MBL deficient) | 7.2% | 2.1% |
| NHS + MASP-2 antibody | 5.4% | 1.5% |
| NHS | 3.1% | 0.73% |
| Note: "$CH_{50}$" is the point at which complement-mediated hemolysis reachs 50%. | | |

In summary, the results in this Example demonstrate that inhibiting MASP-3 prevents any complement lysis of sensitized and non-sensitized erythrocytes with deficient protection from autologous complement activation. MASP-2 inhibition also is protective to some extent. Therefore, MASP-2 and MASP-3 inhibitors alone or in combination (*i.e.*, co-administered, administered sequentially) or MASP-2/MASP-3 bispecific or dual inhibitors may be used to treat subjects suffering from PNH, and may also be used to ameliorate (*i.e.*, inhibit, prevent or reduce the severity of) extravascular hemolysis in PNH patients undergoing treatment with a C5 inhibitor such as eculizumab (Soliris®).

### EXAMPLE 6

This Example describes a hemolysis assay testing mannan-coated rabbit erythrocytes for lysis in the presence of WT or MASP-1/3-/- mouse sera.

**Methods:**

***1. Hemolysis assay of rabbit RBC (mannan coated) in mouse MASP-1/3-deficient sera and WT control sera***

**Day 1. Preparation of rabbit RBC.**

Materials included: fresh rabbit blood, BBS/ $Mg^{++}$/$Ca^{++}$ (4.4 mM barbituric acid, 1.8 mM sodium barbitone, 145 mM NaCl, pH 7.4, 5 mM $Mg^{++}$, 5 mM $Ca^{++}$), BBS/ $Mg^{++}$/$Ca^{++}$with 0.1% gelatin, chromium chloride contained in buffer; i.e., $CrCl_3$.6 $H_2O$ (0.5 mg /mL in BBS/ $Mg^{++}$/$Ca^{++}$) and mannan, 100 $\mu$g/mL in BBS/ $Mg^{++}$/$Ca^{++}$.

1. Rabbit whole blood (2 mL) was split into two 1.5 mL eppendorf tubes and centrifuged for 3 minutes at 8000 rpm (approximately 5.9 rcf) in a refrigerated eppendorf centrifuge at 4°C. The RBC pellet was washed three times after re-suspending in ice-cold BBS/$Mg^{++}$/$Ca^{++}$. After the third wash, the pellet was re-suspended in 4 mL BBS/$Mg^{++}$/$Ca^{++}$. Two mL of this aliquot were added to a 15-mL falcon tube to be used as the uncoated control. The remaining 2 mL of the RBCs aliquot were diluted in 2 mL of $CrCl_3$ buffer, 2 mL of the mannan solution were added and the suspension was incubated at room temperature for 5 minutes with gentle mixing. The reaction was terminated by adding 7.5 mL of BBS/0.1% gelatin/$Mg^{++}$/$Ca^{++}$ to the mixture. The erythrocytes were pelleted and the RBCs were washed twice with BBS/0.1% gelatin/$Mg^{++}$/$Ca^{++}$ as described above. The RBCs suspension was stored in BBS/0.1% gelatin/ $Mg^{++}$/$Ca^{++}$ at 4°C.

2. 100 $\mu$l of suspended RBCs were diluted with 1.4 mL water and spun down at 8000 rpm (approximately 5.9 rcf) for 3 minutes and the OD of the supernatant was adjusted to 0.7 at 541nm (an OD of 0.7 at 541nm corresponds to approximately $10^9$ erythrocyte s/mL).

3. The re-suspended RBCs were diluted with BBS/0.1% gelatin/$Mg^{++}$/$Ca^{++}$ to a concentration of $10^8$ /mL.

4. Dilutions of the test sera were prepared in ice-cold BBS/gelatin/ $Mg^{++}$/$Ca^{++}$ and 100 $\mu$l of each serum dilution were pipetted into the corresponding well of round-bottom plate. 100 $\mu$l of appropriately diluted RBC (108/mL) were added to each well. As a control for complete lysis, purified water (100 $\mu$L) was mixed with the diluted RBC (100 $\mu$L) to cause 100% lysis, while BBS/0.1% gelatin/ $Mg^{++}$/$Ca^{++}$ without serum (100 $\mu$L) was used as a negative control. The plate was then incubated for 1 hour at 37°C.

5. The round-bottom plate was centrifuged at 3250 rpm for 5 minutes. The supernatant from each well (100 $\mu$L) was transferred into the corresponding wells of a flat-bottom plate and OD was read in an ELISA reader at 415-490nm. Results are reported as the ratio of the OD at 415 nm to that at 49 0nm.

**Results**:

**FIGURE 25** graphically illustrates hemolysis (as measured by hemoglobin release of lysed rabbit erythrocytes into the supernatant measured by photometry) of mannan-coated rabbit erythrocytes by mouse serum over a range of serum concentrations in serum from MASP-1/3-/- and WT control. As shown in **FIGURE 25**, it is demonstrated that inhibiting MASP-3 prevents complement-mediated lysis of mannan-coated WT rabbit erythrocytes. These results further support the use of MASP-3 inhibitors for the treatment of one or more aspects of PNH as described in Example 5.

**EXAMPLE 7**

This Example demonstrates that the alternative pathway is activated in factor D-deficient serum in the presence of $Ca^{++}$.

***Experiment #1: C3b deposition assay under alternative pathway specific conditions***

**Methods:**

A C3b deposition assay on a zymosan-coated microtiter plate was carried out under alternative pathway-specific conditions (BBS/EGTA/$Mg^{++}$, no $Ca^{++}$) using increasing dilutions of the following mouse sera: factor D-/-; MASP- -/-; and WT.

**Results:**

**FIGURE 26** graphically illustrates the level of C3b deposition (OD 405 nm) as a function of serum concentration in serum samples from factor D-/-, MASP-2 -/-; and WT mice sera in a C3 deposition assay carried out under alternative pathway specific conditions. As shown in **FIGURE 26**, under these conditions, factor D -/- mouse serum does not activate C3 at all and the alternative pathway is not working. MASP-2-/- serum shows alternative pathway activation at a similar rate as WT serum. These results confirm that, in the absence of Ca$^{++}$, factor D is required for C3b deposition. This is consistent with the evidence that MASP-3 cannot be converted into its enzymatically active form under these conditions because the interactions of MASP-1, the MASP-3 activating enzyme, and MASP-3 with their respective carbohydrate recognition components is Ca$^{++}$-dependent.

***Experiment #2: C3b deposition assay under physiological conditions***

**Methods:**

A C3b deposition assay was carried under physiological conditions (BBS/Ca$^{++}$/Mg$^{++}$) (allowing both the LP and AP to function) using increasing dilutions of the following mouse sera: factor D -/-; MASP-2 -/-; and WT.

**Results:**

**FIGURE 27** graphically illustrates the level of C3b deposition (OD 405 nm) as a function of serum concentration using samples of sera from factor D-/-; MASP-2-/-; and WT mice in a C3b deposition assay carried out under physiological conditions (in the presence of Ca$^{++}$). As shown in **FIGURE 27**, factor D-/- mouse serum activates C3 via both the lectin and the alternative pathway with no difference as compared to WT serum through the serum dilutions indicated. MASP--/- serum shows the turnover of C3 in lower serum dilutions by the alternative pathway only (i.e., MASP-3-driven alternative pathway activation). These results indicate that in the presence of Ca$^{++}$, factor D is not required given that MASP-3 can drive alternative pathway activity.

***Experiment #3: C3b Deposition Assay Using Mouse Sera deficient in factor B or factor D in the presence or absence of MASP-2 mAb***

**Methods:**

A C3b deposition assay was carried out under physiological conditions (BBS/Ca$^{++}$/Mg$^{++}$) on mannan coated microtiter plates as follows:

1. Micro-titer ELISA plates were coated overnight at 4°C with mannan (1 μg/mL) in coating buffer (15 mM Na$_2$CO$_3$, 35 mM NaHCo$_3$, 0.02% sodium azide, pH 9.6).
2. The next day, residual protein binding sites were blocked for 2 hours at room temperature with 250 μl/well with 0.1% HSA in BBS (4 mM barbital, 145 mM NaCl, 2 mM CaCl$_2$, 1 mM MgCl$_2$, pH 7.4).
3. Plates were washed three times with wash buffer (TBS with 0.05% Tween 20 and 5 mM CaCl$_2$).
4. 1:10 diluted serum samples in BBS were added to the wells at the specified time points. Wells receiving only buffer were used as negative controls. The plate was incubated at 37°C for up to 40 minutes.
5. The plates were then washed 3 times with the wash buffer.
6. Then 100 μl of rabbit anti-human C3c (Dako) diluted 1:5000 in washing buffer was added to the wells and plates were incubated for 90 minutes at 37°C.
7. After being washed for three times with washing buffer, 100 μl of alkaline phosphatase-conjugated anti-rabbit diluted 1:5000 in washing buffer was added to the wells followed by incubation for 90 minutes at room temperature.
8. After washing, alkaline phosphatase was detected by adding 100 μl of substrate solution.
9. After incubation for 15 minutes, the optical density was measured at OD 405 nm.

**Results:**

**FIGURE 28** graphically illustrates the level of C3b deposition (OD 405 nm) as a function of serum incubation time (minutes) in mouse serum samples obtained from factor D-/- or factor B-/- mice in the presence or absence of MASP-2 mAb in a C3b deposition assay carried out under physiological conditions (in the presence of Ca$^{++}$). As shown in **FIGURE 28**, there is no difference in the amount of C3b deposition in WT and factor D/- serum, providing strong support for the conclusion that MASP-3 can initiate alternative pathway activation, even in the absence of factor D. The observed signal is thought to be due to both lectin pathway and alternative pathway activation. As further shown in **FIGURE 28**, factor D-/- plus MASP-2 mAb shows MASP-3-mediated alternative pathway activation only. The factor B-/- plus MASP-2 mAb was background only (data not shown). Heat-inactivated serum was used as the background control value, which was identical to factor D-/- and factor B-/- with MASP-2 (data not shown).

In summary, the results in this Example demonstrate that factor D is only essential under non-physiological conditions (i.e. when testing for alternative pathway activation in BBS/EGTA/ Mg$^{++}$ in the absence of Ca$^{++}$). In contrast, when testing for alternative pathway activation under physiological conditions (in the presence of Ca$^{++}$), which allows the alternative pathway to be activated via MASP-3, factor D-deficient serum is not at all deficient in alternative pathway activity as compared to the WT control. Therefore, under physiological conditions, factor D is redundant, in that the initiation of alternative pathway activation is driven by MASP-3. These results support the conclusion that the lectin pathway directs AP activation through a MASP-3-dependent activation event.

## EXAMPLE 8

This example describes exemplary methods for producing murine monoclonal antibodies against human MASP-1, MASP-2 or MASP-3 polypeptides, and for generating dual, bispecific or pan-specific MASP antibodies.

### 1. Methods for generating MASP antibodies

Male A/J mice (Harlan, Houston, Tex.), 8 to 12 weeks of age, are injected subcutaneously with 100 $\mu$g human full-length polypeptides: rMASP-1 (SEQ ID NO:10), rMASP-2 (SEQ ID NO:5) or rMASP-3 (SEQ ID NO:8), or antigen fragments thereof, for example as set forth in **TABLE 2**, in complete Freund's adjuvant (Difco Laboratories, Detroit, Mich.) in 200 $\mu$l of phosphate buffered saline (PBS) pH 7.4. Two weeks later, the mice are injected subcutaneously with 50 $\mu$g of the same human polypeptide in incomplete Freund's adjuvant. At the sixth week, the mice are injected with 50 $\mu$g of the same human polypeptide in PBS and are fused 4 days later.

For each fusion, single-cell suspensions are prepared from the spleen of an immunized mouse and used for fusion with Sp2/0 myeloma cells. $5 \times 10^8$ of the Sp2/0 and $5 \times 10^8$ spleen cells are fused in a medium containing 50% polyethylene glycol (M.W. 1450) (Kodak, Rochester, N.Y.) and 5% dimethylsulfoxide (Sigma Chemical Co., St. Louis, Mo.). The cells are then adjusted to a concentration of $1.5 \times 10^5$ spleen cells per 200 $\mu$l of the suspension in Iscove medium (Gibco, Grand Island, N.Y.), supplemented with 10% fetal bovine serum, 100 units/mL of penicillin, 100 $\mu$g/mL of streptomycin, 0.1 mM hypoxanthine, 0.4 $\mu$M aminopterin and 16 $\mu$M thymidine. Two hundred microliters of the cell suspension are added to each well contained in roughly twenty 96-well microculture plates. After about ten days, culture supernatants are withdrawn for screening for reactivity with the target purified antigen (MASP-1, MASP-2 or MASP-3, or the antigen fragment from **TABLE 2**) in an ELISA assay.

**ELISA Assay** (described with reference to MASP-2): Wells of Immulon 2 (Dynatech Laboratories, Chantilly, Va.) microtest plates are coated by adding 50 $\mu$l of purified hMASP-2 at 50 ng/mL overnight at room temperature. The low concentration of MASP-2 used for coating enables the selection of high-affinity antibodies. After the coating solution is removed by flicking the plate, 200 $\mu$l of BLOTTO (non-fat dry milk) in PBS is added to each well for one hour to block the non-specific sites. An hour later, the wells are then washed with a buffer PBST (PBS containing 0.05% Tween 20). The culture supernatants from each fusion well (50 uL) are mixed with 50 $\mu$l of BLOTTO and then added to individual MASP-2-coated wells of the microtest plates. After one hour of incubation, the wells are washed with PBST and antibody binding to MASP-2 is detected by adding horseradish peroxidase (HRP)-conjugated goat anti-mouse IgG (Fc specific) (Jackson ImmunoResearch Laboratories, West Grove, Pa.). The HRP-conjugated anti-mouse IgG is diluted appropriately in BLOTTO to provide an appropriate signal to noise ratio, and added to each sample-containing well. After washing, the bound HRP-conjugated antibody is detected with the peroxidase substrate solution. Peroxidase substrate solution containing 0.1% 3,3,5,5 tetramethyl benzidine (Sigma, St. Louis, Mo.) and 0.0003% hydrogen peroxide (Sigma) is added to the wells for color development for 30 minutes. The reaction is terminated by addition of 50 $\mu$l of 2M H$_2$SO$_4$ per well and the optical density at 450 nm of the reaction mixture is measured with a BioTek ELISA Reader (BioTek Instruments, Winooski, Vt.).

**Binding Assay** (described with reference to MASP-2):

Culture supernatants that test positive in the MASP-2 ELISA assay described above can be tested in a binding assay to determine the binding affinity that the MASP-2 inhibitory agents have for MASP-2. A similar assay can also be used to determine if the inhibitory agents bind to other antigens in the complement system.

Polystyrene microtiter plate wells (96-well medium binding plates, Corning Costar, Cambridge, MA) are coated with MASP-2 (20 ng/100 $\mu$l/well, Advanced Research Technology, San Diego, CA) in phosphate-buffered saline (PBS) pH 7.4 overnight at 4°C. After aspirating the MASP-2 solution, wells are blocked with PBS containing 1% bovine serum albumin (BSA; Sigma Chemical) for 2 hours at room temperature. Wells without MASP-2 coating serve as the background controls. Aliquots of hybridoma supernatants or purified MASP-2 MoAbs, at varying concentrations in BSA PBS blocking solution, are added to the wells. Following a two-hour incubation at room temperature, the wells are extensively rinsed with PBS. MASP-2-bound MASP-2 MoAb is detected by the addition of peroxidase-conjugated goat anti-mouse IgG (Sigma Chemical) in blocking solution, which is allowed to incubate for 1 hour at room temperature. The plate is rinsed again thoroughly with PBS, and 100 µl of 3,3',5,5'-tetramethyl benzidine (TMB) substrate (Kirkegaard and Perry Laboratories, Gaithersburg, MD) is added. The reaction of TMB is quenched by the addition of 100 µl of 1M phosphoric acid, and the plate is read at 450 nm in a microplate reader (SPECTRA MAX 250, Molecular Devices, Sunnyvale, CA).

The culture supernatants from the positive wells are then tested for the ability to inhibit complement activation in a functional assay such as the C4 cleavage assay as described herein (Example 9). The cells in positive wells are then cloned by limiting dilution. The MoAbs are tested again for reactivity with hMASP-2 in an ELISA assay as described above. The selected hybridomas are grown in spinner flasks and the spent culture supernatant collected for antibody purification by protein A affinity chromatography.

MASP-2 antibodies may be assayed for LEA-2 inhibitory activity in a C4 cleavage assay, for example as described in Example 9.

While the ELISA and Binding Assay above are described with reference to MASP-2, it will be understood by those of skill in the art that the same ELISA and binding assays may be carried out using MASP-1 or MASP-3 polypeptides and antigen fragments thereof (e.g., as described in **TABLE 2**). MASP-3 antibodies may be assayed for inhibition of MASP-3 serine protease cleavage of a MASP-3 substrate and for LEA-1 inhibitory activity in a C3b deposition assay, for example as described in Example 4, in a hemolysis assay as described in Example 5. MASP-1 antibodies may be assayed for inhibition of MASP-1 serine protease cleavage of a MASP-1 substrate, for inhibition of MASP-3 activation, and for LEA-1 inhibitory activity in a C3b deposition assay, for example as described in Example 4, and in a hemolysis assay as described in Example 5.

### 2. Methods for Generating Dual-MASP antibodies

MASP-2/3 dual inhibitory antibodies: As shown in **FIGURES 4**, **6** and **7C**, there are regions conserved between MASP-2 and MASP-3 in the serine protease domain, encoded by the beta chain of SEQ ID NO:5 and SEQ ID NO:8. Therefore, a dual MASP-2/3 antibody can be generated using an antigen comprising or consisting of the serine protease domain of MASP-2 (or MASP-3), such as the beta chain of SEQ ID NO:5 (or SEQ ID NO:8) to generate a monoclonal antibody as described above, or alternatively, these antigen(s) may be used to screen a phage library for clones that specifically bind to these antigen(s), followed by screening for dual binding to MASP-3 (or MASP-1). The dual MASP-2/3 antibodies are then screened for inhibitory activity in a functional assay, for example as described in **TABLE 2.**

MASP-1/3 dual inhibitory antibodies: As shown in **FIGURES 3-5**, MASP-1 and MASP-3 share an identical conserved region in the CUBI-CCP2 domain (aa 25-432 of SEQ ID NO:10), which is also shared by MAp44. As shown in **FIGURE 3**, MAp44 does not contain a CCP2 domain. Therefore, a dual MASP-1/3 antibody inclusive of MAp44 is generated using an antigen comprising or consisting of the CUBI-CCP-2 domain of MASP-1 (or MASP-3) to generate a monoclonal antibody as described above, or alternatively, this antigen is used to screen a phage library for clones that specifically bind to this antigen, followed by screening for dual binding to MASP-3 (or MASP-1). A dual MASP-1/3 antibody exclusive of MAp44 is generated in a similar manner, using an antigen comprising or consisting of the CCP2 domain of MASP-1 (or MASP-3). The dual MASP-1/3 antibodies are then screened for inhibitory activity in a functional assay, for example as described in **TABLE 2.**

MASP-1/2 dual inhibitory antibodies: As shown in **FIGURES 4**, **6** and **7A**, the serine protease domain of MASP-1 and MASP-2 contains regions that are conserved. Therefore, a dual MASP-1/2 antibody is generated using an antigen comprising or consisting of the serine protease domain of MASP-1 (or MASP-2) to generate a monoclonal antibody as described above, or alternatively, this antigen is used to screen a phage library for clones that specifically bind to this antigen, followed by screening for dual binding to MASP-2 (or MASP-1). The dual MASP-1/2 antibodies are then screened for inhibitory activity in a functional assay, for example as described in **TABLE 2.**

### 3. Methods for Generating Pan-specific MASP antibodies:

Alpha Chain: Numerous patches of identity between MASP-2 and MASP-1/3 suggest that it may be possible to generate monoclonal antibodies that bind MASP-1/3 and MASP-2. In particular, most of the identity lies within the CUB1-EGF-CUB2 domains, as shown in **FIGURE 5**. The various domains illustrated in **FIGURE 5** were identified according to Yongqing, et al., Biochemica et Biophysica Acta 1824:253-262 (2012); Teillet et al., J. Biol. Chem. 283:25715-25724 (2008); and Marchler-Bauer et al., Nucleic Acids Res. 39:D225-229 (2011).

Beta Chain: Numerous patches of identity between MASP-2 and MASP-1/3, as shown in **FIGURE 6**, would allow for the generation of a pan-specific MASP-1/2/3 inhibitor.

### Methods:

Pan-specific MASP inhibitory antibodies (i.e., antibodies that inhibit MASP-1, 2 and 3 activity) are generated as follows:

1. Screen a library against MASP-1/3 and MASP-2 Alpha-chain CUB1-EGF-CUB2 domains and select clones that cross-react to both MASP-1/3 and MASP-2.

2. Screen the clones for the ability to inhibit functional activity, for example as described in **TABLE 2.**

3. Use the DTLacO affinity/functionality maturation technology (Yabuki et al., PLoS ONE, 7(4):e36032 (2012)) to optimize both binding to all three proteins and inhibitory function.

4. As described in **TABLE 2**, pan-MASP inhibitors can be used to inhibit LEA-1- and LEA-2-mediated complement activation.

### *4. Methods for generating bispecific MASP-2/3 antibodies*

Bispecific MASP-2/3 inhibitory antibodies are generated as follows:

1. Exemplary MASP-2 specific inhibitory antibodies that bind to the CCP1 domain and inhibit MASP-2-dependent complement activation have been identified, as described in Examples 11-14.

2. A MASP-3 specific inhibitory antibody is generated by screening a library against the MASP-3 polypeptide and identifying MASP-3 antibodies, as described in Example 15, followed by assaying the antibodies for LEA-1 inhibitory activity in a functional assay, for example as described in **TABLE 2.** Exemplary MASP-3 antibodies are described in Example 15.

3. The antigen binding region specific for MASP-2 and MASP-3 are cloned into a framework to generate a bi-specific antibody. Numerous bispecific antibody formats have been described, including immunoglobulin G-like formats as well as various fusion protein and single chain variable fragment configurations (Holmes, Nature Reviews, Drug Discovery 10:798-800 (2011), Müller and Kontermann, Biodrugs 24:89-98 (2010)). In one example, bispecific antibodies can be generated by fusing two hybridomas expressing antibodies against two distinct antigens, resulting in various heavy and light chain pairings, a percentage of which comprise a heavy and light chain specific for one antigen paired with a heavy and light chain specific for the other antigen (Milstein and Cuello, Nature 305:537-539 (1983)). A similar bispecific antibody may be generated recombinantly, by co-expressing two immunoglobulin heavy-chain/light-chain pairs, where the two heavy chains have different specificities Antibody variable domains with the desired binding specificities (antibody-antigen combining sites) (e.g. MASP-2 antibodies as described in Examples 11-14, MASP-3 antibodies as described in Example 15) can be fused to immunoglobulin constant domain sequences. The fusion preferably is with an immunoglobulin heavy-chain constant domain, including at least part of the hinge, $C_H2$, and $C_H3$ regions. DNAs encoding the immunoglobulin heavy-chain fusions and, if desired, the immunoglobulin light chain, are inserted into separate expression vectors, and are co-transfected into a suitable host organism.

In addition to paired immunoglobulin heavy and light chains, linkage of single chain variable fragments specific for two different targets is exemplified in Kipriyanov et al., J. Mol. Biol. 293:41 (1999)). In this example, a single polynucleotide expression construct is designed to encode two pairs of heavy and light chain variable regions separated by linker peptides, with each pair imparting specificity for a distinct protein target. Upon expression, the polypeptide assembles in a configuration in which the heavy and light chain pair specific for one target forms one antigen-binding surface of the protein, and the other pair forms a separate antigen-binding surface, creating a molecule termed a single chain diabody. Depending on the length of the linker between the central heavy and light chain variable region pair, the polypeptide can also be forced to dimerize, resulting in the formation of a tandem diabody.

For example, DNA encoding the following immunoglobulin polypeptides may be inserted into one or more vectors and expressed in a suitable host organism to generate the following illustrative, non-limiting examples of a bi-specific antibodies.

### MASP-2/3 bispecific antibodies

In one embodiment, a bispecific antibody is provided that binds human MASP-2 and human MASP-3 and comprises:

(I) a MASP-2 specific binding region comprising at least one or more of the following:a) a heavy chain variable region comprising: i) a heavy chain CDR1 comprising the amino acid sequence from 31-35 of SEQ ID NO:21; and ii) a heavy chain CDR2 comprising the amino acid sequence from 50-65 of SEQ ID NO:21; and iii) a heavy chain CDR3 comprising the amino acid sequence from 95-102 of SEQ ID NO:21; and/or at least one or more of the following:b) a light chain variable region comprising: i) a light chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light chain CDR3 comprising the amino acid sequence from 89-97 of either SEQ ID NO:25 or SEQ ID NO:27; and

(II) a MASP-3 specific binding region, optionally a MASP-3 specific binding region comprising at least one of a) a heavy chain variable region comprising: i) a heavy chain CDR1 comprising the amino acid sequence from 31-35 of SEQ ID NO:25 or SEQ ID NO:26; and ii) a heavy chain CDR2 comprising the amino acid sequence from 50-65 of SEQ ID NO:25 or SEQ ID NO:26; and iii) a heavy chain CDR3 comprising the amino acid sequence from 95-102 of SEQ ID NO:25 or SEQ ID NO:26; and

b) a light chain variable region comprising: i) a light chain CDR1 comprising the amino acid sequence from 24-34 of either SEQ ID NO:28 or SEQ ID NO:29; and ii) a light chain CDR2 comprising the amino acid sequence from 50-56 of either SEQ ID NO:28 or SEQ ID NO:29; and iii) a light chain CDR3 comprising the amino acid sequence from 89-97 of either SEQ ID NO:28 or SEQ ID NO:29.

**MASP-1/2 bispecific antibodies**

In one embodiment, a bispecific antibody is provided that binds human MASP-1 and human MASP-2 and comprises:

(I) a MASP-2 specific binding region comprising at least one or more of the following:a) a heavy-chain variable region comprising: i) a heavy-chain CDR1 comprising the amino acid sequence from 31-35 of SEQ ID NO:21; and ii) a heavy-chain CDR2 comprising the amino acid sequence from 50-65 of SEQ ID NO:21; and iii) a heavy-chain CDR3 comprising the amino acid sequence from 95-102 of SEQ ID NO:21; and/or at least one or more of the following:b) a light-chain variable region comprising: i) a light-chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light-chain CDR3 comprising the amino acid sequence from 89-97 of either SEQ ID NO:25 or SEQ ID NO:27; and

(II) a MASP-1 specific binding region.

4. Testing for functional inhibitory activity against MASP-2 and/or MASP-3 is carried out, for example as described in **TABLE 2**, and as further described herein.

**EXAMPLE 9**

This example describes an *in vitro* C4 cleavage assay used as a functional screen to identify MASP-2 inhibitory agents capable of blocking MASP-2-dependent complement activation via L-ficolin/P35, H-ficolin, M-ficolin or mannan.

**C4 Cleavage Assay:** A C4 cleavage assay has been described by Petersen, S.V., et al., J. Immunol. Methods 257:107, 2001, which measures lectin pathway activation resulting from lipoteichoic acid (LTA) on *S. aureus,* which binds to L-ficolin.

**Reagents:** Formalin-fixed *S. aureus* (DSM20233) is prepared as follows: bacteria is grown overnight at 37°C in tryptic soy blood medium, washed three times with PBS, then fixed for 1 hour at room temperature in PBS/0.5% formalin, and washed a further three times with PBS, before being resuspended in coating buffer (15 mM $Na_2CO_3$, 35 mM $NaHCO_3$, pH 9.6).

**Assay:** The wells of a Nunc MaxiSorb microtiter plate (Nalgene Nunc International, Rochester, NY) are coated with: 100 $\mu$l of formalin-fixed *S. aureus* DSM20233 ($OD_{550}$ = 0.5) in coating buffer with 1 $\mu$g of L-ficolin in coating buffer. After overnight incubation, wells are blocked with 0.1% human serum albumin (HSA) in TBS (10 mM Tris-HCl, 140 mM NaCl, pH 7.4), then are washed with TBS containing 0.05% Tween 20 and 5 mM $CaCl_2$ (wash buffer). Human serum samples are diluted in 20 mM Tris-HCl, 1 M NaCl, 10 mM $CaCl_2$, 0.05% Triton X-100, 0.1% HSA, pH 7.4, which prevents activation of endogenous C4 and dissociates the C1 complex (composed of C1q, C1r and C1s). MASP-2 inhibitory agents, including MASP-2 MoAbs, are added to the serum samples in varying concentrations. The diluted samples are added to the plate and incubated overnight at 4°C. After 24 hours, the plates are washed thoroughly with wash buffer, then 0.1 $\mu$g of purified human C4 (obtained as described in Dodds, A.W., Methods Enzymol. 223:46, 1993) in 100 $\mu$l of 4 mM barbital, 145 mM NaCl, 2 mM $CaCl_2$, 1 mM $MgCl_2$, pH 7.4 is added to each well. After 1.5 hours at 37°C, the plates are washed again and C4b deposition is detected using alkaline phosphatase-conjugated chicken anti-human C4c (obtained from Immunsystem, Uppsala, Sweden) and measured using the colorimetric substrate p-nitrophenyl phosphate.

**C4 Assay on mannan:** The assay described above is adapted to measure lectin pathway activation via MBL by coating the plate with LSP and mannan prior to adding serum mixed with various MASP-2 inhibitory agents.

**C4 assay on H-ficolin (Hakata Ag):** The assay described above is adapted to measure lectin pathway activation via H-ficolin by coating the plate with LPS and H-ficolin prior to adding serum mixed with various MASP-2 inhibitory agents.

**EXAMPLE 10**

The following assay is used to test whether a MASP inhibitory agent blocks the classical pathway by analyzing the effect of a MASP inhibitory agent under conditions in which the classical pathway is initiated by immune complexes.

Methods: To test the effect of a MASP inhibitory agent on conditions of complement activation where the classical pathway is initiated by immune complexes, triplicate 50 μl samples containing 90% NHS are incubated at 37°C in the presence of 10 μg/mL immune complex or PBS, and parallel triplicate samples (+/- immune complexes) are also included containing 200 nM anti-properdin monoclonal antibody during the 37°C incubation. After a two-hour incubation at 37°C, 13 mM EDTA is added to all samples to stop further complement activation and the samples are immediately cooled to 5°C. The samples are then stored at -70°C prior to being assayed for complement activation products (C3a and sC5b-9) using ELISA kits (Quidel, Catalog Nos. A015 and A009) following the manufacturer's instructions.

**EXAMPLE 11**

This example describes the identification of high-affinity MASP-2 Fab2 antibody fragments that block MASP-2 activity.

Background and rationale: MASP-2 is a complex protein with many separate functional domains, including: binding site(s) for MBL and ficolins, a serine protease catalytic site, a binding site for proteolytic substrate C2, a binding site for proteolytic substrate C4, a MASP-2 cleavage site for autoactivation of MASP-2 zymogen, and two $Ca^{++}$ binding sites. Fab2 antibody fragments were identified that bind with high affinity to MASP-2, and the identified Fab2 fragments were tested in a functional assay to determine if they were able to block MASP-2 functional activity.

To block MASP-2 functional activity, an antibody or Fab2 antibody fragment must bind and interfere with a structural epitope on MASP-2 that is required for MASP-2 functional activity. Therefore, many or all of the high-affinity binding MASP-2 Fab2s may not inhibit MASP-2 functional activity unless they bind to structural epitopes on MASP-2 that are directly involved in MASP-2 functional activity.

A functional assay that measures inhibition of lectin pathway C3 convertase formation was used to evaluate the "blocking activity" of MASP-2 Fab2s. It is known that the primary physiological role of MASP-2 in the lectin pathway is to generate the next functional component of the lectin-mediated complement pathway, namely the lectin pathway C3 convertase. The lectin pathway C3 convertase is a critical enzymatic complex (C4bC2a) that proteolytically cleaves C3 into C3a and C3b. MASP-2 is not a structural component of the lectin pathway C3 convertase (C4bC2a); however, MASP-2 functional activity is required in order to generate the two protein components (C4b, C2a) that comprise the lectin pathway C3 convertase. Furthermore, all of the separate functional activities of MASP-2 listed above appear to be required in order for MASP-2 to generate the lectin pathway C3 convertase. For these reasons, a preferred assay to use in evaluating the "blocking activity" of MASP-2 Fab2s is believed to be a functional assay that measures inhibition of lectin pathway C3 convertase formation.

Generation of High Affinity Fab2s: A phage display library of human variable light and heavy chain antibody sequences and automated antibody selection technology for identifying Fab2s that react with selected ligands of interest was used to create high-affinity Fab2s to rat MASP-2 protein (SEQ ID NO: 13). A known amount of rat MASP-2 (~1 mg, >85% pure) protein was utilized for antibody screening. Three rounds of amplification were utilized for selection of the antibodies with the best affinity. Approximately 250 different hits expressing antibody fragments were picked for ELISA screening. High affinity hits were subsequently sequenced to determine uniqueness of the different antibodies.

Fifty unique MASP-2 antibodies were purified and 250 μg of each purified Fab2 antibody was used for characterization of MASP-2 binding affinity and complement pathway functional testing, as described in more detail below.

**Assays used to Evaluate the Inhibitory (blocking) Activity of MASP-2 Fab2s**

***1. Assay to Measure Inhibition of Formation of Lectin Pathway C3 Convertase:***

Background: The lectin pathway C3 convertase is the enzymatic complex (C4bC2a) that proteolytically cleaves C3 into the two potent proinflammatory fragments, anaphylatoxin C3a and opsonic C3b. Formation of C3 convertase appears to be a key step in the lectin pathway in terms of mediating inflammation. MASP-2 is not a structural component of the lectin pathway C3 convertase (C4bC2a); therefore MASP-2 antibodies (or Fab2) will not directly inhibit activity of preexisting C3 convertase. However, MASP-2 serine protease activity is required in order to generate the two protein components (C4b, C2a) that comprise the lectin pathway C3 convertase. Therefore, MASP-2 Fab2, which inhibits MASP-2 functional activity (i.e., blocking MASP-2 Fab2) will inhibit *de novo* formation of lectin pathway C3 convertase. C3 contains an unusual and highly reactive thioester group as part of its structure. Upon cleavage of C3 by C3 convertase in this assay, the thioester group on C3b can form a covalent bond with hydroxyl or amino groups on macromolecules immobilized on the bottom of the plastic wells via ester or amide linkages, thus facilitating detection of C3b in the ELISA assay.

Yeast mannan is a known activator of the lectin pathway. In the following method to measure formation of C3 convertase, plastic wells coated with mannan were incubated for 30 minutes at 37°C with diluted rat serum to activate the lectin pathway. The wells were then washed and assayed for C3b immobilized onto the wells using standard ELISA methods. The amount of C3b generated in this assay is a direct reflection of the *de novo* formation of lectin pathway C3 convertase. MASP-2 Fab2s at selected concentrations were tested in this assay for their ability to inhibit C3 convertase formation and consequent C3b generation.

**Methods:**

96-well Costar Medium Binding plates were incubated overnight at 5°C with mannan diluted in 50 mM carbonate buffer, pH 9.5 at 1 $\mu$g/50 $\mu$l/well. After overnight incubation, each well was washed three times with 200 $\mu$l PBS. The wells were then blocked with 100 $\mu$l/well of 1% bovine serum albumin in PBS and incubated for one hour at room temperature with gentle mixing. Each well was then washed three times with 200 $\mu$l of PBS. The MASP-2 Fab2 samples were diluted to selected concentrations in Ca$^{++}$ and Mg$^{++}$ containing GVB buffer (4.0 mM barbital, 141 mM NaCl, 1.0 mM MgCl$_2$, 2.0 mM CaCl$_2$, 0.1% gelatin, pH 7.4) at 5°C. A 0.5% rat serum was added to the above samples at 5°C and 100 $\mu$l was transferred to each well. Plates were covered and incubated for 30 minutes in a 37°C waterbath to allow complement activation. The reaction was stopped by transferring the plates from the 37°C waterbath to a container containing an ice-water mix. Each well was washed five times with 200 $\mu$l with PBS-Tween 20 (0.05% Tween 20 in PBS), then washed two times with 200 $\mu$l PBS. A 100 $\mu$l/well of 1:10,000 dilution of the primary antibody (rabbit anti-human C3c, DAKO A0062) was added in PBS containing 2.0 mg/mL bovine serum albumin and incubated 1 hour at room temperature with gentle mixing. Each well was washed 5 times with 200 $\mu$l PBS. 100 $\mu$l/well of 1:10,000 dilution of the secondary antibody (peroxidase-conjugated goat anti-rabbit IgG, American Qualex A102PU) was added in PBS containing 2.0 mg/mL bovine serum albumin and incubated for one hour at room temperature on a shaker with gentle mixing. Each well was washed five times with 200 $\mu$l with PBS. 100 $\mu$l/well of the peroxidase substrate TMB (Kirkegaard & Perry Laboratories) was added and incubated at room temperature for 10 minutes. The peroxidase reaction was stopped by adding 100 Tl/well of 1.0 M H$_3$PO$_4$ and the OD$_{450}$ was measured.

### 2. *Assay to Measure Inhibition of MASP-2-dependent C4 Cleavage*

Background: The serine protease activity of MASP-2 is highly specific and only two protein substrates for MASP-2 have been identified; C2 and C4. Cleavage of C4 generates C4a and C4b. MASP-2 Fab2 may bind to structural epitopes on MASP-2 that are directly involved in C4 cleavage (e.g., MASP-2 binding site for C4; MASP-2 serine protease catalytic site) and thereby inhibit the C4 cleavage functional activity of MASP-2.

Yeast mannan is a known activator of the lectin pathway. In the following method to measure the C4 cleavage activity of MASP-2, plastic wells coated with mannan were incubated for 30 minutes at 37°C with diluted rat serum to activate the lectin pathway. Since the primary antibody used in this ELISA assay only recognizes human C4, the diluted rat serum was also supplemented with human C4 (1.0 $\mu$g/mL). The wells were then washed and assayed for human C4b immobilized onto the wells using standard ELISA methods. The amount of C4b generated in this assay is a measure of MASP-2-dependent C4 cleavage activity. MASP-2 Fab2 at selected concentrations was tested in this assay for ability to inhibit C4 cleavage.

**Methods:** 96-well Costar Medium Binding plates were incubated overnight at 5°C with mannan diluted in 50 mM carbonate buffer, pH 9.5 at 1.0 Tg/50 $\mu$l/well. Each well was washed 3 times with 200 $\mu$l PBS. The wells were then blocked with 100 $\mu$l/well of 1% bovine serum albumin in PBS and incubated for one hour at room temperature with gentle mixing. Each well was washed 3 times with 200 $\mu$l of PBS. MASP-2 Fab2 samples were diluted to selected concentrations in Ca$^{++}$ and Mg$^{++}$ containing GVB buffer (4.0 mM barbital, 141 mM NaCl, 1.0 mM MgCl$_2$, 2.0 mM CaCl$_2$, 0.1% gelatin, pH 7.4) at 5°C. 1.0 $\mu$g/mL human C4 (Quidel) was also included in these samples. 0.5% rat serum was added to the above samples at 5°C and 100 $\mu$l was transferred to each well. The plates were covered and incubated for 30 minutes in a 37°C waterbath to allow complement activation. The reaction was stopped by transferring the plates from the 37°C waterbath to a container containing an ice-water mix. Each well was washed 5 times with 200 $\mu$l with PBS-Tween 20 (0.05% Tween 20 in PBS), then each well was washed with 2 times with 200 $\mu$l PBS. 100 $\mu$l/well of 1:700 dilution of biotin-conjugated chicken anti-human C4c (Immunsystem AB, Uppsala, Sweden) was added in PBS containing 2.0 mg/mL bovine serum albumin (BSA) and incubated one hour at room temperature with gentle mixing. Each well was washed 5 times with 200 $\mu$l PBS. 100 $\mu$l/well of 0.1 $\mu$g/mL of peroxidase-conjugated streptavidin (Pierce Chemical #21126) was added in PBS containing 2.0 mg/mL BSA and incubated for one hour at room temperature on a shaker with gentle mixing. Each well was washed 5 × 200 $\mu$l with PBS. 100 $\mu$l/well of the peroxidase substrate TMB (Kirkegaard & Perry Laboratories) was added and incubated at room temperature for 16 min. The peroxidase reaction was stopped by adding 100 $\mu$l/well of 1.0 M H$_3$PO$_4$ and the OD$_{450}$ was measured.

**3. *Binding Assay of anti-rat MASP-2 Fab2 to 'Native' rat MASP-2***

Background: MASP-2 is usually present in plasma as a MASP-2 dimer complex that also includes specific lectin molecules (mannose-binding protein (MBL) and ficolins). Therefore, if one is interested in studying the binding of MASP-2 Fab2 to the physiologically relevant form of MASP-2, it is important to develop a binding assay in which the interaction between the Fab2 and 'native' MASP-2 in plasma, rather than purified recombinant MASP-2, is used. In this binding assay, the 'native' MASP-2-MBL complex from 10% rat serum was first immobilized onto mannan-coated wells. The binding affinity of various MASP-2 Fab2s to the immobilized 'native' MASP-2 was then studied using a standard ELISA methodology.

**Methods:** 96-well Costar High Binding plates were incubated overnight at 5°C with mannan diluted in 50 mM carbonate buffer, pH 9.5 at 1 $\mu$g/50 $\mu$l/well. Each well was washed 3 times with 200 $\mu$l PBS. The wells were blocked with 100 $\mu$l/well of 0.5% nonfat dry milk in PBST (PBS with 0.05% Tween 20) and incubated for one hour at room temperature with gentle mixing. Each well was washed 3 times with 200 $\mu$l of TBS/Tween/Ca$^{++}$ Wash Buffer (Tris-buffered saline, 0.05% Tween 20, containing 5.0 mM CaCl$_2$, pH 7.4. 10% rat serum in High Salt Binding Buffer (20 mM Tris, 1.0 M NaCl, 10 mM CaCl$_2$, 0.05% Triton-XlOO, 0.1% (w/v) bovine serum albumin, pH 7.4) was prepared on ice. 100 $\mu$l/well was added and incubated overnight at 5°C. Wells were washed 3 times with 200 $\mu$l of TBS/Tween/Ca$^{++}$ Wash Buffer. Wells were then washed 2 times with 200 $\mu$l PBS. 100 $\mu$l/well of selected concentration of MASP-2 Fab2 diluted in Ca$^{++}$ and Mg$^{++}$ containing GVB Buffer (4.0 mM barbital, 141 mM NaCl, 1.0 mM MgCl$_2$, 2.0 mM CaCl$_2$, 0.1% gelatin, pH 7.4) was added and incubated for one hour at room temperature with gentle mixing. Each well was washed 5 times with 200 $\mu$l PBS. 100 $\mu$l/well of HRP-conjugated goat anti-Fab2 (Biogenesis Cat No 0500-0099) diluted 1:5000 in 2.0 mg/mL bovine serum albumin in PBS was added and incubated for one hour at room temperature with gentle mixing. Each well was washed 5 times with 200 $\mu$l PBS. 100 $\mu$l/well of the peroxidase substrate TMB (Kirkegaard & Perry Laboratories) was added and incubated at room temperature for 70 minutes. The peroxidase reaction was stopped by adding 100 $\mu$l/well of 1.0 M H$_3$PO$_4$ and OD$_{450}$ was measured.

**RESULTS:**

Approximately 250 different Fab2s that reacted with high affinity to the rat MASP-2 protein were picked for ELISA screening. These high-affinity Fab2s were sequenced to determine the uniqueness of the different antibodies, and 50 unique MASP-2 antibodies were purified for further analysis. 250 ug of each purified Fab2 antibody was used for characterization of MASP-2 binding affinity and complement pathway functional testing. The results of this analysis are shown below in **TABLE 13.**

**TABLE 13**: MASP-2 FAB2 THAT BLOCK LECTIN PATHWAY COMPLEMENT ACTIVATION

| Fab2 antibody # | C3 Convertase (IC$_{50}$ (nM) | K$_d$ (nM) | C4 Cleavage IC$_{50}$ (nM) |
|---|---|---|---|
| 88 | 0.32 | 4.1 | ND |
| 41 | 0.35 | 0.30 | 0.81 |
| 11 | 0.46 | 0.86 | <2 nM |
| 86 | 0.53 | 1.4 | ND |
| 81 | 0.54 | 2.0 | ND |
| 66 | 0.92 | 4.5 | ND |
| 57 | 0.95 | 3.6 | <2 nM |
| 40 | 1.1 | 7.2 | 0.68 |
| 58 | 1.3 | 2.6 | ND |
| 60 | 1.6 | 3.1 | ND |
| 52 | 1.6 | 5.8 | <2 nM |
| 63 | 2.0 | 6.6 | ND |
| 49 | 2.8 | 8.5 | <2 nM |
| 89 | 3.0 | 2.5 | ND |
| 71 | 3.0 | 10.5 | ND |

(continued)

| Fab2 antibody # | C3 Convertase (IC$_{50}$ (nM) | K$_d$ (nM) | C4 Cleavage IC$_{50}$ (nM) |
|---|---|---|---|
| 87 | 6.0 | 2.5 | ND |
| 67 | 10.0 | 7.7 | ND |

As shown above in **TABLE 13**, of the 50 MASP-2 Fab2s tested, 17 were identified as MASP-2-blocking Fab2s that potently inhibit C3 convertase formation with IC$_{50}$ equal to or less than 10 nM Fab2s (a 34% positive hit rate). Eight of the 17 Fab2s identified have IC$_{50}$s in the subnanomolar range. Furthermore, all seventeen of the MASP-2 blocking Fab2s shown in **TABLE 13** gave essentially complete inhibition of C3 convertase formation in the lectin pathway C3 convertase assay. This is an important consideration, since it is theoretically possible that a "blocking" Fab2 may only fractionally inhibit MASP-2 function even when each MASP-2 molecule is bound by the Fab2.

Although mannan is a known activator of the lectin pathway, it is theoretically possible that the presence of anti-mannan antibodies in the rat serum might also activate the classical pathway and generate C3b via the classical pathway C3 convertase. However, each of the seventeen blocking MASP-2 Fab2s listed in this example potently inhibits C3b generation (>95 %), thus demonstrating the specificity of this assay for lectin pathway C3 convertase.

Binding assays were also performed with all seventeen of the blocking Fab2s in order to calculate an apparent K$_d$ for each. The results of the binding assays of anti-rat MASP-2 Fab2s to native rat MASP-2 for six of the blocking Fab2s are also shown in **TABLE 13.** Similar binding assays were also carried out for the other Fab2s, the results of which are shown in **TABLE 13.** In general, the apparent K$_d$s obtained for binding of each of the six Fab2s to 'native' MASP-2 corresponds reasonably well with the IC$_{50}$ for the Fab2 in the C3 convertase functional assay. There is evidence that MASP-2 undergoes a conformational change from an 'inactive' to an 'active' form upon activation of its protease activity (Feinberg et al., EMBO J 22:2348-59 (2003); Gal et al., J. Biol. Chem. 280:33435-44 (2005)). In the normal rat plasma used in the C3 convertase formation assay, MASP-2 is present primarily in the 'inactive' zymogen conformation. In contrast, in the binding assay, MASP-2 is present as part of a complex with MBL bound to immobilized mannan; therefore, the MASP-2 would be in the 'active' conformation (Petersen et al., J. Immunol Methods 257:107-16, 2001). Consequently, one would not necessarily expect an exact correspondence between the IC$_{50}$ and K$_d$ for each of the seventeen blocking Fab2 tested in these two functional assays because, in each assay, the Fab2 would be binding a different conformational form of MASP-2. Nevertheless, with the exception of Fab2 #88, there appears to be a reasonably close correspondence between the IC$_{50}$ and apparent K$_d$ for each of the other sixteen Fab2 tested in the two assays (see **TABLE 13**).

Several of the blocking Fab2s were evaluated for inhibition of MASP-2-mediated cleavage of C4. As shown in **TABLE 13**, all of the Fab2s tested were found to inhibit C4 cleavage with IC$_{50}$s similar to those obtained in the C3 convertase assay.

Although mannan is a known activator of the lectin pathway, it is theoretically possible that the presence of anti-mannan antibodies in the rat serum might also activate the classical pathway and thereby generate C4b by C1s-mediated cleavage of C4. However, several MASP-2 Fab2s have been identified that potently inhibit C4b generation (>95 %), thus demonstrating the specificity of this assay for MASP-2-mediated C4 cleavage. C4, like C3, contains an unusual and highly reactive thioester group as part of its structure. Upon cleavage of C4 by MASP-2 in this assay, the thioester group on C4b can form a covalent bond with hydroxyl or amino groups on macromolecules immobilized on the bottom of the plastic wells *via* ester or amide linkages, thus facilitating detection of C4b in the ELISA assay.

These studies clearly demonstrate the creation of high-affinity FAB2s to rat MASP-2 protein that functionally block both C4 and C3 convertase activity, thereby preventing lectin pathway activation.

## EXAMPLE 12

This Example describes the epitope mapping for several of the blocking anti-rat MASP-2 Fab2 antibodies that were generated as described in Example 11.

**Methods:**

The following proteins, all with N-terminal 6X His tags were expressed in CHO cells using the pED4 vector:

rat MASP-2A, a full-length MASP-2 protein, inactivated by altering the serine at the active center to alanine (S613A); rat MASP-2K, a full-length MASP-2 protein altered to reduce autoactivation (R424K); CUBI-II, an N-terminal fragment of rat MASP-2 that contains the CUBI, EGF-like and CUBII domains only; and

CUBI/EGF-like, an N-terminal fragment of rat MASP-2 that contains the CUBI and EGF-like domains only.

These proteins were purified from culture supernatants by nickel-affinity chromatography, as previously described (Chen et al., J. Biol. Chem. 276:25894-02 (2001)).

A C-terminal polypeptide (CCPII-SP), containing CCPII and the serine protease domain of rat MASP-2, was expressed in *E. coli* as a thioredoxin fusion protein using pTrxFus (Invitrogen). Protein was purified from cell lysates using Thiobond affinity resin. The thioredoxin fusion partner was expressed from empty pTrxFus as a negative control.

All recombinant proteins were dialyzed into TBS buffer and their concentrations determined by measuring the OD at 280 nm.

*Dot Blot Analysis:*

Serial dilutions of the five recombinant MASP-2 polypeptides described above (and the thioredoxin polypeptide as a negative control for CCPII-serine protease polypeptide) were spotted onto a nitrocellulose membrane. The amount of protein spotted ranged from 100 ng to 6.4 pg, in five-fold steps. In later experiments, the amount of protein spotted ranged from 50 ng down to 16 pg, again in five-fold steps. Membranes were blocked with 5% skimmed milk powder in TBS (blocking buffer) then incubated with 1.0 $\mu$g/mL MASP-2 Fab2s in blocking buffer (containing 5.0 mM Ca$^{++}$). Bound Fab2s were detected using HRP-conjugated anti-human Fab (AbD/Serotec; diluted 1/10,000) and an ECL detection kit (Amersham). One membrane was incubated with polyclonal rabbit-anti human MASP-2 Ab (described in Stover et al., J Immunol 163:6848-59 (1999)) as a positive control. In this case, bound Ab was detected using HRP-conjugated goat anti-rabbit IgG (Dako; diluted 1/2,000).

*MASP-2 Binding Assay:*

ELISA plates were coated with 1.0 $\mu$g/well of recombinant MASP-2A or CUBI-II polypeptide in carbonate buffer (pH 9.0) overnight at 4°C. Wells were blocked with 1% BSA in TBS, then serial dilutions of the MASP-2 Fab2s were added in TBS containing 5.0 mM Ca$^{++}$. The plates were incubated for one hour at RT. After washing three times with TBS/tween/Ca$^{++}$, HRP-conjugated anti-human Fab (AbD/Serotec) diluted 1/10,000 in TBS/Ca$^{++}$ was added and the plates incubated for a further one hour at room temperature. Bound antibody was detected using a TMB peroxidase substrate kit (Biorad).

Results:

Results of the dot blot analysis demonstrating the reactivity of the Fab2s with various MASP-2 polypeptides are provided below in **TABLE 14.** The numerical values provided in **TABLE 14** indicate the amount of spotted protein required to give approximately half-maximal signal strength. As shown, all of the polypeptides (with the exception of the thioredoxin fusion partner alone) were recognized by the positive control Ab (polyclonal anti-human MASP-2 sera, raised in rabbits).

**TABLE 14**: REACTIVITY WITH VARIOUS RECOMBINANT RAT MASP-2 POLYPEPTIDES ON DOT BLOTS

| Fab2 Antibody # | MASP-2A | CUBI-II | CUBI/EGF-like | CCPII-SP | Thioredoxin |
|---|---|---|---|---|---|
| 40 | 0.16 ng | NR | NR | 0.8 ng | NR |
| 41 | 0.16 ng | NR | NR | 0.8 ng | NR |
| 11 | 0.16 ng | NR | NR | 0.8 ng | NR |
| 49 | 0.16 ng | NR | NR | >20 ng | NR |
| 52 | 0.16 ng | NR | NR | 0.8 ng | NR |
| 57 | 0.032 ng | NR | NR | NR | NR |
| 58 | 0.4 ng | NR | NR | 2.0 ng | NR |
| 60 | 0.4 ng | 0.4 ng | NR | NR | NR |
| 63 | 0.4 ng | NR | NR | 2.0 ng | NR |
| 66 | 0.4 ng | NR | NR | 2.0 ng | NR |
| 67 | 0.4 ng | NR | NR | 2.0 ng | NR |

(continued)

| Fab2 Antibody # | MASP-2A | CUBI-II | CUBI/EGF-like | CCPII-SP | Thioredoxin |
|---|---|---|---|---|---|
| 71 | 0.4 ng | NR | NR | 2.0 ng | NR |
| 81 | 0.4 ng | NR | NR | 2.0 ng | NR |
| 86 | 0.4 ng | NR | NR | 10 ng | NR |
| 87 | 0.4 ng | NR | NR | 2.0 ng | NR |
| Positive Control | <0.032 ng | 0.16 ng | 0.16 ng | <0.032 ng | NR |
| NR = No reaction. The positive control antibody is polyclonal anti-human MASP-2 sera, raised in rabbits. | | | | | |

All of the Fab2s reacted with MASP-2A as well as MASP-2K (data not shown). The majority of the Fab2s recognized the CCPII-SP polypeptide but not the N-terminal fragments. The two exceptions are Fab2 #60 and Fab2 #57. Fab2 #60 recognizes MASP-2A and the CUBI-II fragment, but not the CUBI/EGF-like polypeptide or the CCPII-SP polypeptide, suggesting it binds to an epitope in CUBII, or spanning the CUBII and the EGF-like domain. Fab2 # 57 recognizes MASP-2A but not any of the MASP-2 fragments tested, perhaps indicating that this Fab2 recognizes an epitope in CCP1. Fab2 #40 and #49 bound only to complete MASP-2A. In the ELISA binding assay, Fab2 #60 also bound to the CUBI-II polypeptide, albeit with a slightly lower apparent affinity (data not shown).

These finding demonstrate the identification of unique blocking Fab2s to multiple regions of the MASP-2 protein.

## EXAMPLE 13

This Example describes the pharmacodynamic analysis of representative high-affinity MASP-2 Fab2 antibodies that were identified as described in Example 11.

### Background/Rationale:

As described in **Example 11**, in order to identify high-affinity antibodies that block the rat lectin pathway, rat MASP-2 protein was utilized to pan a phage display library. This library was designed to provide for high immunological diversity and was constructed using entirely human immunoglobin gene sequences. As shown in **Example 11**, approximately 250 individual phage clones were identified that bound with high affinity to the rat MASP-2 protein by ELISA screening. Sequencing of these clones identified 50 unique MASP-2 antibody-encoding phage. Fab2 protein was expressed from these clones, purified and analyzed for MASP-2 binding affinity and lectin complement pathway functional inhibition.

As shown in **TABLE 13** of **Example 11**, 17 MASP-2 Fab2s with functional blocking activity were identified as a result of this analysis (a 34% hit rate for blocking antibodies). Functional inhibition of the lectin complement pathway by Fab2s was apparent at the level of C4 deposition, which is a direct measure of C4 cleavage by MASP-2. Importantly, inhibition was equally evident when C3 convertase activity was assessed, demonstrating functional blockade of the lectin complement pathway. The 17 MASP-2 blocking Fab2s identified as described in **Example 11** potently inhibit C3 convertase formation with $IC_{50}$ values equal to or less than 10 nM. Eight of the 17 Fab2s identified have $IC_{50}$ values in the sub-nanomolar range. Furthermore, all 17 of the MASP-2 blocking Fab2s gave essentially complete inhibition of the C3 convertase formation in the lectin pathway C3 convertase assay, as summarized in **TABLE 13** of Example 11. Moreover, each of the 17 blocking MASP-2 Fab2s shown in **TABLE 13** potently inhibit C3b generation (>95%), thus demonstrating the specificity of this assay for lectin pathway C3 convertase.

Rat IgG2c and mouse IgG2a full-length antibody isotype variants were derived from Fab2 #11. This Example describes the *in vivo* characterization of these isotypes for pharmacodynamic parameters.

### Methods:

As described in **Example 11**, rat MASP-2 protein was utilized to pan a Fab phage display library, from which Fab2 #11 was identified. Rat IgG2c and mouse IgG2a full-length antibody isotype variants were derived from Fab2 #11. Both rat IgG2c and mouse IgG2a full-length antibody isotypes were characterized *in vivo* for pharmacodynamic parameters as follows.

*In vivo study in mice:*

A pharmacodynamic study was carried out in mice to investigate the effect of MASP-2 antibody dosing on the plasma lectin pathway activity *in vivo.* In this study, C4 deposition was measured *ex vivo* in a lectin pathway assay at various time points following subcutaneous (sc) and intraperitoneal (ip) administration of 0.3 mg/kg or 1.0 mg/kg of the mouse MASP-2 MoAb (mouse IgG2a full-length antibody isotype derived from Fab2#11).

**FIGURE 29A** graphically illustrates lectin pathway specific C4b deposition on a zymosan-coated microtiter plate, measured *ex vivo* in undiluted serum samples taken from mice (n=3 mice/group) at various time points after subcutaneous dosing of either 0.3 mg/kg or 1.0 mg/kg of the mouse MASP-2 MoAb. Serum samples from mice collected prior to antibody dosing served as negative controls (100% activity), while serum supplemented *in vitro* with 100 nM of the same blocking MASP-2 antibody was used as a positive control (0% activity).

The results shown in **FIGURE 29A** demonstrate a rapid and complete inhibition of C4b deposition following subcutaneous administration of 1.0 mg/kg dose of mouse MASP-2 MoAb. A partial inhibition of C4b deposition was seen following subcutaneous administration of a dose of 0.3 mg/kg of mouse MASP-2 MoAb.

The time course of lectin pathway recovery was followed for three weeks following a single ip administration of mouse MASP-2 MoAb at 0.6 mg/kg in mice. As shown in **FIGURE 29B**, a precipitous drop in lectin pathway activity occurred after antibody dosing followed by complete lectin pathway inhibition that lasted for about 7 days after i.p. administration. Slow restoration of lectin pathway activity was observed over the second and third weeks, with complete lectin pathway restoration in the mice by 17 days following MASP-2 MoAb administration.

These results demonstrate that the mouse MASP-2 Moab derived from Fab2 #11 inhibits the lectin pathway of mice in a dose-responsive manner when delivered systemically.

## EXAMPLE 14

This example describes the identification, using phage display, of fully human scFv antibodies that bind to MASP-2 and inhibit lectin-mediated complement activation (LEA-2) while leaving the classical (C1q-dependent) pathway component of the immune system intact.

### Overview:

Fully human, high-affinity MASP-2 antibodies were identified by screening a phage display library. The variable light and heavy chain fragments of the antibodies were isolated in both a scFv format and in a full-length IgG format. The human MASP-2 antibodies are useful for inhibiting cellular injury associated with lectin pathway-mediated alternative complement pathway activation while leaving the classical (C1q-dependent) pathway component of the immune system intact. In some embodiments, the subject MASP-2 inhibitory antibodies have the following characteristics: (a) high affinity for human MASP-2 (e.g., a $K_D$ of 10 nM or less), and (b) inhibit MASP-2-dependent complement activity in 90% human serum with an $IC_{50}$ of 30 nM or less.

### Methods:

*Expression of full-length catalytically inactive MASP-2:*

The full-length cDNA sequence of human MASP-2 (SEQ ID NO: 4), encoding the human MASP-2 polypeptide with leader sequence (SEQ ID NO:5) was subcloned into the mammalian expression vector pCI-Neo (Promega), which drives eukaryotic expression under the control of the CMV enhancer/promoter region (described in Kaufman R.J. et al., Nucleic Acids Research 19:4485-90, 1991; Kaufman, Methods in Enzymology, 185:537-66 (1991)).

In order to generate catalytically inactive human MASP-2A protein, site-directed mutagenesis was carried out as described in US2007/0172483, hereby incorporated herein by reference. The PCR products were purified after agarose gel electrophoresis and band preparation and single adenosine overlaps were generated using a standard tailing procedure. The adenosine-tailed MASP-2A was then cloned into the pGEM-T easy vector and transformed into *E. coli.* The human MASP-2A was further subcloned into either of the mammalian expression vectors pED or pCI-Neo.

The MASP-2A expression construct described above was transfected into DXB1 cells using the standard calcium phosphate transfection procedure (Maniatis et al., 1989). MASP-2A was produced in serum-free medium to ensure that preparations were not contaminated with other serum proteins. Media was harvested from confluent cells every second day (four times in total). The level of recombinant MASP-2A averaged approximately 1.5 mg/liter of culture medium. The MASP-2A (Ser-Ala mutant described above) was purified by affinity chromatography on MBP-A-agarose columns

*MASP-2A ELISA on ScFv Candidate Clones identified by panning/scFv conversion and filter screening*

A phage display library of human immunoglobulin light- and heavy-chain variable region sequences was subjected to antigen panning followed by automated antibody screening and selection to identify high-affinity scFv antibodies to human MASP-2 protein. Three rounds of panning the scFv phage library against HIS-tagged or biotin-tagged MASP-2A were carried out. The third round of panning was eluted first with MBL and then with TEA (alkaline). To monitor the specific enrichment of phages displaying scFv fragments against the target MASP-2A, a polyclonal phage ELISA against immobilized MASP-2A was carried out. The scFv genes from panning round 3 were cloned into a pHOG expression vector and run in a small-scale filter screening to look for specific clones against MASP-2A.

Bacterial colonies containing plasmids encoding scFv fragments from the third round of panning were picked, gridded onto nitrocellulose membranes and grown overnight on non-inducing medium to produce master plates. A total of 18,000 colonies were picked and analyzed from the third panning round, half from the competitive elution and half from the subsequent TEA elution. Panning of the scFv phagemid library against MASP-2A followed by scFv conversion and a filter screen yielded 137 positive clones. 108/137 clones were positive in an ELISA assay for MASP-2 binding (data not shown), of which 45 clones were further analyzed for the ability to block MASP-2 activity in normal human serum.

*Assay to Measure Inhibition of Formation of Lectin Pathway C3 Convertase*

A functional assay that measures inhibition of lectin pathway C3 convertase formation was used to evaluate the "blocking activity" of the MASP-2 scFv candidate clones. MASP-2 serine protease activity is required in order to generate the two protein components (C4b, C2a) that comprise the lectin pathway C3 convertase. Therefore, a MASP-2 scFv that inhibits MASP-2 functional activity (i.e., a blocking MASP-2 scFv), will inhibit *de novo* formation of lectin pathway C3 convertase. C3 contains an unusual and highly reactive thioester group as part of its structure. Upon cleavage of C3 by C3 convertase in this assay, the thioester group on C3b can form a covalent bond with hydroxyl or amino groups on macromolecules immobilized on the bottom of the plastic wells via ester or amide linkages, thus facilitating detection of C3b in the ELISA assay.

Yeast mannan is a known activator of the lectin pathway. In the following method to measure formation of C3 convertase, plastic wells coated with mannan were incubated with diluted human serum to activate the lectin pathway. The wells were then washed and assayed for C3b immobilized onto the wells using standard ELISA methods. The amount of C3b generated in this assay is a direct reflection of the *de novo* formation of lectin pathway C3 convertase. MASP-2 scFv clones at selected concentrations were tested in this assay for their ability to inhibit C3 convertase formation and consequent C3b generation.

## Methods:

The 45 candidate clones identified as described above were expressed, purified and diluted to the same stock concentration, which was again diluted in $Ca^{++}$ and $Mg^{++}$ containing GVB buffer (4.0 mM barbital, 141 mM NaCl, 1.0 mM $MgCl_2$, 2.0 mM $CaCl_2$, 0.1% gelatin, pH 7.4) to assure that all clones had the same amount of buffer. The scFv clones were each tested in triplicate at the concentration of 2 $\mu$g/mL. The positive control was OMS100 Fab2 and was tested at 0.4 $\mu$g/mL. C3c formation was monitored in the presence and absence of the scFv/IgG clones.

Mannan was diluted to a concentration of 20 $\mu$g/mL (1 $\mu$g/well) in 50mM carbonate buffer (15mM $Na_2CO_3$ + 35mM $NaHCO_3$ + 1.5 mM $NaN_3$), pH 9.5 and coated on an ELISA plate overnight at 4°C. The next day, the mannan-coated plates were washed 3 times with 200 $\mu$l PBS. 100 $\mu$l of 1% HSA blocking solution was then added to the wells and incubated for 1 hour at room temperature. The plates were washed 3 times with 200 $\mu$l PBS, and stored on ice with 200 $\mu$l PBS until addition of the samples.

Normal human serum was diluted to 0.5% in CaMgGVB buffer, and scFv clones or the OMS100 Fab2 positive control were added in triplicates at 0.01 $\mu$g/mL; 1 $\mu$g/mL (only OMS100 control) and 10 $\mu$g/mL to this buffer and preincubated 45 minutes on ice before addition to the blocked ELISA plate. The reaction was initiated by incubation for one hour at 37°C and was stopped by transferring the plates to an ice bath. C3b deposition was detected with a Rabbit $\alpha$-Mouse C3c antibody followed by Goat $\alpha$-Rabbit HRP. The negative control was buffer without antibody (no antibody = maximum C3b deposition), and the positive control was buffer with EDTA (no C3b deposition). The background was determined by carrying out the same assay except that the wells were mannan-free. The background signal against plates without mannan was subtracted from the signals in the mannan-containing wells. A cut-off criterion was set at half of the activity of an irrelevant scFv clone (VZV) and buffer alone.

**Results:** Based on the cut-off criterion, a total of 13 clones were found to block the activity of MASP-2. All 13 clones producing > 50% pathway suppression were selected and sequenced, yielding 10 unique clones. All ten clones were found to have the same light chain subclass, $\lambda$3, but three different heavy chain subclasses: VH2, VH3 and VH6. In the functional assay, five out of the ten candidate scFv clones gave $IC_{50}$ nM values less than the 25 nM target criteria using 0.5% human serum.

To identify antibodies with improved potency, the three mother scFv clones, identified as described above, were subjected to light-chain shuffling. This process involved the generation of a combinatorial library consisting of the VH of each of the mother clones paired up with a library of naive, human lambda light chains (VL) derived from six healthy donors. This library was then screened for scFv clones with improved binding affinity and/or functionality.

**TABLE 15**: Comparison of functional potency in $IC_{50}$ (nM) of the lead daughter clones and their respective mother clones (all in scFv format)

| scFv clone | 1% human serum C3 assay ($IC_{50}$ nM) | 90% human serum C3 assay ($IC_{50}$ nM) | 90% human serum C4 assay ($IC_{50}$ nM) |
|---|---|---|---|
| 17D20mc | 38 | nd | nd |
| 17D20m_d3521N11 | 26 | >1000 | 140 |
| 17N16mc | 68 | nd | nd |
| 17N16m_d17N9 | 48 | 15 | 230 |

Presented below are the heavy-chain variable region (VH) sequences for the mother clones and daughter clones shown above in **TABLE 15**, and listed below in

**TABLES 16A-F.**

The Kabat CDRs (31-35 (HI), 50-65 (H2) and 95-102 (H3)) are bolded; and the Chothia CDRs (26-32 (HI), 52-56 (H2) and 95-101 (H3)) are underlined.

17D20 35VH-21N11VL heavy chain variable region (VH) (SEQ ID NO:15, encoded by SEQ ID NO:14)

QVTLKESGPVLVKPTETLTLTCTVS<u>GFSL</u>**S<u>RGKMG</u>**VSWIRQPPGKALEW**LAH<u>IFSS</u>DEKSYRTSL**KSRLTISKDTSKNQVVLTMTNMDPVDTAT**YYCARIR<u>R</u>**GGIDYWGQGTLVTVSS

d17N9 heavy chain variable region (VH) (SEQ ID NO:16)

QVQLQQSGPGLVKPSQTLSLTCAIS<u>GDSVS</u>**S<u>TSAA</u>**WNWIRQSPSRGLEW**LG<u>RTY</u>YRS**KWYNDYAVSVKSRITINPDTSKNQFSLQLNSVTPEDT**AVYYCARD**PFGVPFDIWGQGTMVTVSS

**Heavy Chain Variable Region**

**TABLE 16A**: Heavy chain (aa 1-20)

| Heavy chain | | | | | | | | | | | | | | | | | | | | |
|---|---|---|---|---|---|---|---|---|---|---|---|---|---|---|---|---|---|---|---|---|
| **aa** | **1** | **2** | **3** | **4** | **5** | **6** | **7** | **8** | **9** | **10** | **11** | **12** | **13** | **14** | **15** | **16** | **17** | **18** | **19** | **20** |
| d3521N11 (SEQ: 15) | Q | V | T | L | K | E | S | G | P | V | L | V | K | P | T | E | T | L | T | L |
| d17N9 (SEQ:16) | Q | V | Q | L | Q | Q | S | G | P | G | L | V | K | P | S | Q | T | L | S | L |

**TABLE 16B**: Heavy chain (aa 21-40)

| Heavy chain | | | | | | | | | | | | CDR-H1 | | | | | | | | |
|---|---|---|---|---|---|---|---|---|---|---|---|---|---|---|---|---|---|---|---|---|
| aa | 21 | 22 | 23 | 24 | 25 | 26 | 27 | 28 | 29 | 30 | 31 | 32 | 33 | 34 | 35 | 36 | 37 | 38 | 39 | 40 |
| d3521N11 (SEQ:15) | T | C | T | V | S | G | F | S | L | S | R | G | K | M | G | V | S | W | I | R |
| d17N9 (SEQ:16) | T | C | A | I | S | G | D | S | V | S | S | T | S | A | A | W | N | W | I | R |

105

**TABLE 16C**: Heavy chain (aa 41-60)

| Heavy chain | | | | | | | | | | CDR-H2 | | | | | | | | | | |
|---|---|---|---|---|---|---|---|---|---|---|---|---|---|---|---|---|---|---|---|---|
| aa | 41 | 42 | 43 | 44 | 45 | 46 | 47 | 48 | 49 | 50 | 51 | 52 | 53 | 54 | 55 | 56 | 57 | 58 | 59 | 60 |
| d3521N11 (SEQ:15) | Q | P | P | G | K | A | L | E | W | L | A | H | I | F | S | S | D | E | K | S |
| d17N9 (SEQ:16) | Q | S | P | S | R | G | L | E | W | L | G | R | T | Y | Y | R | S | K | W | Y |

**TABLE 16D**: Heavy chain (aa 61-80)

| Heavy chain | CDR-H2 (cont'd) | | | | | | | | | | | | | | | | | | | |
|---|---|---|---|---|---|---|---|---|---|---|---|---|---|---|---|---|---|---|---|---|
| aa | 61 | 62 | 63 | 64 | 65 | 66 | 67 | 68 | 69 | 70 | 71 | 72 | 73 | 74 | 75 | 76 | 77 | 78 | 79 | 80 |
| d3521N11 (SEQ:15) | Y | R | T | S | L | K | S | R | L | T | I | S | K | D | T | S | K | N | Q | V |
| d17N9 (SEQ:16) | N | D | Y | A | V | S | V | K | S | R | I | T | I | N | P | D | T | S | K | N |

**TABLE 16E**: Heavy chain (aa 81-100)

| Heavy chain | | | | | | | | | | | | | | CDR-H3 | | | | | |
|---|---|---|---|---|---|---|---|---|---|---|---|---|---|---|---|---|---|---|---|
| **aa** | **81** | **82** | **83** | **84** | **85** | **86** | **87** | **88** | **89** | **90** | **91** | **92** | **93** | **94** | **95** | **96** | **97** | **98** | **99** | **100** |
| d3521N11 (SEQ:15) | V | L | T | M | T | N | M | D | P | V | D | T | A | T | <u>Y</u> | <u>Y</u> | <u>C</u> | <u>A</u> | <u>R</u> | <u>I</u> |
| d17N9 (SEQ:16) | Q | F | S | L | Q | L | N | S | V | T | P | E | D | T | <u>A</u> | <u>V</u> | <u>Y</u> | <u>Y</u> | <u>C</u> | <u>A</u> |

**TABLE 16F**: heavy chain (aa 101-118)

| Heavy chain | CDR-H3 (cont'd) | | | | | | | | | | | | | | | | | | | |
|---|---|---|---|---|---|---|---|---|---|---|---|---|---|---|---|---|---|---|---|---|
| aa | 101 | 102 | 103 | 104 | 105 | 106 | 107 | 108 | 109 | 110 | 111 | 112 | 113 | 114 | 115 | 116 | 117 | 118 | 119 | 120 |
| d3521N11 (SEQ: 15) | **R** | **R** | G | G | I | D | Y | w | G | Q | G | T | L | V | T | V | S | S | | |
| d17N9 (SEQ:16) | **R** | **D** | P | F | G | V | P | F | D | I | w | G | Q | G | T | M | V | T | V | S |

Presented below are the light-chain variable region (VL) sequences for the mother clones and daughter clones listed below in **TABLES 17A-F.**

The Kabat CDRs (24-34 (L1); 50-56 (L2); and 89-97 (L3) are bolded; and the Chothia CDRs (24-34 (L1); 50-56 (L2) and 89-97 (L3) are underlined. These regions are the same whether numbered by the Kabat or Chothia system.

17D20m_d3521N11 light chain variable region (VL) (SEQ ID NO:17)

QPVLTQPPSLSVSPGQTASITCS**GEKLGDKYAYW**YQQKPGQSPVLVMYQ**DKQRPSG**IPERFSGSNSGNTATLTISGTQAMDEADYYCQ**AWDSSTAVF**GGGTKLTVL

17N16m_d17N9 light chain variable region (VL) (SEQ ID NO:19, encoded by SEQ ID NO:18)

SYELIQPPSVSVAPGQTATITCA**GDNLGKKRVHW**YQQRPGQAPVLVIYD**DSDRPSG**IPDRFSASNSGNTATLTITRGEAGDEADYYCQ**VWDIATDHV**VFGGGTKLTVLAAAGSEQKLISE

**TABLE 17A**: Light chain (aa 1-20)

| Light chain | | | | | | | | | | | | | | | | | | | |
|---|---|---|---|---|---|---|---|---|---|---|---|---|---|---|---|---|---|---|---|
| **aa** | **1** | **2** | **3** | **4** | **5** | **6** | **7** | **8** | **9** | **10** | **11** | **12** | **13** | **14** | **15** | **16** | **17** | **18** | **19** | **20** |
| d3521N11 (SEQ: 17) | Q | P | v | L | T | Q | P | P | S | **L** | S | V | S | P | G | Q | T | A | S | I |
| d17N9 (SEQ:19) | S | Y | **E** | L | **I** | Q | P | P | S | V | S | V | A | P | G | Q | T | A | **T** | I |

**TABLE 17B**: Light chain (aa 21-40)

| Light chain | | | | CDR-L1 | | | | | | | | | | | | | | | |
|---|---|---|---|---|---|---|---|---|---|---|---|---|---|---|---|---|---|---|---|
| aa | 21 | 22 | 23 | 24 | 25 | 26 | 27 | 28 | 29 | 30 | 31 | 32 | 33 | 34 | 35 | 36 | 37 | 38 | 39 | 40 |
| d3521N11 (SEQ:17) | T | C | S | G | E | K | L | G | D | K | Y | A | Y | W | Y | Q | Q | K | P | G |
| d17N9 (SEQ:19) | T | C | A | G | D | N | L | G | K | K | R | V | H | W | Y | Q | Q | R | P | G |

**TABLE 17C**: Light chain (aa 41-60)

| Light chain | | | | | | | | | CDR-L2 | | | | | | | | | | |
|---|---|---|---|---|---|---|---|---|---|---|---|---|---|---|---|---|---|---|---|
| aa | 41 | 42 | 43 | 44 | 45 | 46 | 47 | 48 | 49 | 50 | 51 | 52 | 53 | 54 | 55 | 56 | 57 | 58 | 59 | 60 |
| d3521N11 (SEQ:17) | Q | S | P | V | L | V | M | Y | Q | D | K | Q | R | P | S | G | I | P | E | R |
| d17N9 (SEQ:19) | Q | A | P | V | L | V | I | Y | D | D | S | D | R | P | S | G | I | P | D | R |

**TABLE 17D**: Light chain (aa 61-80)

| Light chain | CDR-L2 (cont'd) | | | | | | | | | | | | | | | | | | | |
|---|---|---|---|---|---|---|---|---|---|---|---|---|---|---|---|---|---|---|---|
| aa | 61 | 62 | 63 | 64 | 65 | 66 | 67 | 68 | 69 | 70 | 71 | 72 | 73 | 74 | 75 | 76 | 77 | 78 | 79 | 80 |
| d3521N11 (SEQ:17) | F | S | G | S | N | S | G | N | T | A | T | L | T | I | S | G | T | Q | A | M |
| d17N9 (SEQ:19) | F | S | **A** | S | N | S | G | N | T | A | T | L | T | **I** | **T** | R | **G** | E | A | G |

**TABLE 17E**: Light chain (aa 81-100)

| Light chain | | | | | | | | | CDR-L3 | | | | | | | | | | |
|---|---|---|---|---|---|---|---|---|---|---|---|---|---|---|---|---|---|---|---|
| aa | 81 | 82 | 83 | 84 | 85 | 86 | 87 | 88 | 89 | 90 | 91 | 92 | 93 | 94 | 95 | 96 | 97 | 98 | 99 | 100 |
| d3521N1 1 (SEQ:17) | D | **X** | A | D | Y | Y | C | Q | A | W | D | S | S | T | A | V | F | G | **G** | G |
| d17N9 (SEQ:19) | D | E | A | D | Y | Y | C | Q | V | W | D | **I** | **A** | T | D | H | V | V | F | G |

TABLE 17F: Light chain (aa 101-120)

| Light chain | CDR-L3 (cont'd) | | | | | | | | | | | | | | | | | | | |
|---|---|---|---|---|---|---|---|---|---|---|---|---|---|---|---|---|---|---|---|---|
| aa | 10 1 | 10 2 | 10 3 | 10 4 | 10 5 | 10 6 | 10 7 | 10 8 | 10 9 | 11 0 | 11 1 | 11 2 | 11 3 | 11 4 | 11 5 | 11 6 | 11 7 | 11 8 | 11 9 | 12 0 |
| d3521N1 1 (SEQ: 17) | T | K | **L** | T | V | L | A | A | A | G | W | E | Q | K | L | I | S | E | E | D |
| d17N9 (SEQ:19) | G | G | T | K | L | T | V | L | A | A | A | G | S | E | Q | K | L | I | S | E |

The MASP-2 antibodies OMS100 and MoAb_d3521N11VL, which have both been demonstrated to bind to human MASP-2 with high affinity and have the ability to block functional complement activity, were analyzed with regard to epitope binding by dot blot analysis. The results show that d3521N11 and OMS100 antibodies are highly specific for MASP-2 and do not bind to MASP-1/3. Neither antibody bound to MAp19 nor to MASP-2 fragments that did not contain the CCP1 domain of MASP-2, leading to the conclusion that the binding sites encompass CCP1.

Accordingly, in one embodiment, a MASP-2 inhibitory agent for use in the compositions and methods of the claimed invention comprises a human antibody that binds a polypeptide consisting of human MASP-2 (SEQ ID NO:3), wherein the antibody comprises:

I) a) a heavy chain variable region comprising: i) a heavy chain CDR1 comprising the amino acid sequence from 31-35 of SEQ ID NO:21; and ii) a heavy chain CDR2 comprising the amino acid sequence from 50-65 of SEQ ID NO:21; and iii) a heavy chain CDR3 comprising the amino acid sequence from 95-102 of SEQ ID NO:21; and

b) a light chain variable region comprising: i) a light chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light chain CDR3 comprising the amino acid sequence from 89-97 of either SEQ ID NO:25 or SEQ ID NO:27; or II) a variant thereof that is otherwise identical to said variable domains, except for up to a combined total of 6 amino acid substitutions within said CDR regions of said heavy-chain variable region and up to a combined total of 6 amino acid substitutions within said CDR regions of said light-chain variable region, wherein the antibody or variant thereof inhibits MASP-2-dependent complement activation.

## EXAMPLE 15

This Example describes the generation of MASP-1 and MASP-3 monoclonal antibodies using an *in vitro* system comprising a modified DT40 cell line, DTLacO.

### Background/Rationale:

Antibodies against human MASP-1 and MASP-3 were generated using an *in vitro* system comprising a modified DT40 cell line, DTLacO, that permits reversible induction of diversification of a particular polypeptide, as further described in WO2009029315 and US2010093033. DT40 is a chicken B cell line that is known to constitutively mutate its heavy and light chain immunoglobulin (Ig) genes in culture. Like other B cells, this constitutive mutagenesis targets mutations to the V region of Ig genes, and thus, the CDRs of the expressed antibody molecules. Constitutive mutagenesis in DT40 cells takes place by gene conversion using as donor sequences an array of non-functional V gene segments (pseudo-V genes; $\psi$V) situated upstream of each functional V region. Deletion of the $\psi$V region was previously shown to cause a switch in the mechanism of diversification from gene conversion to somatic hypermutation, the mechanism commonly observed in human B cells. The DT40 chicken B cell lymphoma line has been shown to be a promising starting point for antibody evolution *ex vivo* (Cumbers, S.J. et al. Nat Biotechnol 20, 1129-1134 (2002); Seo, H. et al. Nat Biotechnol 23, 731-735 (2005)). DT40 cells proliferate robustly in culture, with an 8-10 hour doubling time (compared to 20-24 hr for human B cell lines), and they support very efficient homologous gene targeting (Buerstedde, J.M. et al. Embo J 9, 921-927 (1990)). DT40 cells command enormous potential V region sequence diversity given that they can access two distinct physiological pathways for diversification, gene conversion and somatic hypermutation, which create templated and nontemplated mutations, respectively (Maizels, N. Annu Rev Genet 39, 23-46 (2005)). Diversified heavy and light chain immunoglobulins (Igs) are expressed in the form of a cell-surface displayed IgM. Surface IgM has a bivalent form, structurally similar to an IgG molecule. Cells that display IgM with specificity for a particular antigen can be isolated by binding either immobilized soluble or membrane displayed versions of the antigen. However, utility of DT40 cells for antibody evolution has been limited in practice because - as in other transformed B cell lines - diversification occurs at less than 1% the physiological rate.

In the system used in this example, as described in WO2009029315 and US2010093033, the DT40 cells were engineered to accelerate the rate of Ig gene diversification without sacrificing the capacity for further genetic modification or the potential for both gene conversion and somatic hypermutation to contribute to mutagenesis. Two key modifications to DT40 were made to increase the rate of diversification and, consequently, the complexity of binding specificities in our library of cells. First, Ig gene diversification was put under the control of the potent *E. coli* lactose operator/repressor regulatory network. Multimers consisting of approximately 100 polymerized repeats of the potent *E. coli* lactose operator (PolyLacO) were inserted upstream of the rearranged and expressed Ig$\lambda$ and IgH genes by homologous gene targeting. Regulatory factors fused to lactose repressor protein (LacI) can then be tethered to the LacO regulatory elements to regulate diversification, taking advantage of the high affinity ($k_D$=10$^{-14}$ M) of lactose repressor for operator DNA. DT40 PolyLacO-$\lambda_R$ cells, in which PolyLacO was integrated only at Ig$\lambda$, exhibited a 5-fold increase in Ig gene diversification rate relative to the parental DT40 cells prior to any engineering (Cummings, W.J. et al. PLoS Biol 5, e246 (2007)). Diversification was further elevated in cells engineered to carry PolyLacO targeted to both the Ig$\lambda$ and the IgH genes ("DTLacO"). DTLacO cells were demonstrated to have diversification rates 2.5- to 9.2-fold elevated relative to the 2.8% characteristic of the parental DT40 PolyLacO-$\lambda_R$ LacI-HP1 line. Thus, targeting PolyLacO elements to both the heavy and light chain genes accelerated diversification 21.7-fold relative to the DT40 parental cell line. Tethering regulatory factors to the Ig loci not only alters the frequency of mutagenesis, but also can change the pathway of mutagenesis creating a larger collection of unique sequence changes (Cummings et al. 2007; Cummings et al. 2008). Second, a diverse collection of sequence starting points for the tethered factor-accelerated Ig gene diversification was generated. These diverse sequence starting points were added to DTLacO by targeting rearranged Ig heavy-chain variable regions, isolated from a two month old chick, to the heavy chain locus. The addition of these heavy chain variable regions created a repertoire of $10^7$ new starting points for antibody diversification. Building these new starting points into the DTLacO cell line permits the identification of clones that bind a particular target, and then rapid affinity maturation by the tethered factors. Following affinity maturation, a full-length, recombinant chimeric IgG is made by cloning the matured, rearranged heavy- and light-chain variable sequences (VH and V$\lambda$; consisting of chicken framework regions and the complementarity determining regions or CDRs) into expression vectors containing human IgG1 and lambda constant regions. These recombinant mAbs are suitable for *in vitro* and *in vivo* applications, and they serve as the starting point for humanization.

## Methods:

### Selection for MASP-1 and MASP-3 antigen binding.

Initial selections were performed by binding DTLacO populations diversified by gene targeting to beads complexed with human MASP-1 (SEQ ID NO:10) and MASP-3 antigen (SEQ ID NO:8); and subsequent selections by FACS, using fluorescence-labeled soluble antigen (Cumbers, S.J. et al. Nat Biotechnol 20, 1129-1134 (2002); Seo, H. et al. Nat 23, 731-735 (2005). Because of the conserved amino acid sequence in the alpha chain that is shared between MASP-1 and MASP-3 (shown in **FIGURE 5**), and the distinct beta chain sequences (shown in **FIGURE 6**), separate, parallel screens for binders to MASP-1 and MASP-3 were carried out to identify MASP-1 specific mAbs, MASP-3 specific mAbs and also mAbs capable of binding to both MASP-1 and MASP-3 (dual-specific). Two forms of antigen were used to select and screen for binders. First, recombinant MASP-1 or MASP-3, either full-length or a fragment, fused to an Fc domain were bound to Dynal magnetic Protein G beads or used in FACS-based selections using a PECy5-labeled anti-human IgG(Fc) secondary antibody. Alternatively, recombinant versions of MASP-1 or MASP-3 proteins were directly labeled with Dylight flours and used for selections and screening.

### Binding and affinity.

Recombinant antibodies were generated by cloning PCR-amplified V regions into a vector that supported expression of human IgG1 in 293F cells (Yabuki et al., PLoS ONE, 7(4):e36032 (2012)). Saturation binding kinetics were determined by staining DTLacO cells expressing antibody binding MASP-1 or MASP-3 with various concentrations of fluorescent-labeled soluble antigen. Functional assays for MASP-3 specific activityincluding MASP-3-dependent C3b deposition and MASP-3-dependent factor D cleavage were carried out as described in Examples 17 and 18, respectively. A functional assay for MASP-1-specific activity, namely the inhibition of MASP-1-dependent C3b deposition was carried out as described below.

## Results:

Numerous MASP-1 and MASP-3 binding antibodies were generated using the methods described above. Binding, as demonstrated by FACS analysis, is described for the representative clones M3J5 and M3M1, which were isolated in screens for MASP-3 binders.

**FIGURE 30A** is a FACS histogram of MASP-3 antigen/antibody binding for DTLacO clone M3J5. **FIGURE 30B** is a FACS histogram of MASP-3 antigen/antibody binding for DTLacO clone M3M1. In **FIGURES 30A** and **30B** the gray filled curves are IgG1-stained negative control, and thick black curves are MASP-3-staining.

**FIGURE 31** graphically illustrates a saturation binding curve of clone M3J5 (Clone 5) for the MASP-3 antigen. As shown in **FIGURE 31**, the apparent binding affinity of the M3J5 antibody for MASP-3 is about 31 nM.

Sequence analysis of identified clones was performed using standard methods. All clones were compared to the common (DT40) VH and VL sequences and to each other. Sequences for the two aforementioned clones, M3J5 and M3M1 are provided in an alignment with two additional representative clones, D14 and 1E10, which were identified in screens for CCP1-CCP2-SP fragments of MASP-1 and MASP-3, respectively. D14 and 1E10 bind regions common to both MASP-1 and MASP-3.

**FIGURE 32A** is an amino acid sequence alignment of the VH regions of M3J5, M3M1, D14 and 1E10 to the chicken DT40 VH sequence.

**FIGURE 32B** is an amino acid sequence alignment of the VL regions of M3J5, M3M1, D14 and 1E10 to the chicken DT40 VL sequence.

The VH and VL amino acid sequence of each clone is provided below.

**Heavy Chain Variable Region (VH) sequences**

**FIGURE 32A** shows an amino acid alignment of the heavy-Chain Variable Region (VH) sequences for the parent DTLacO (SEQ ID NO: 24), the MASP-3-binding clones M3J5 (SEQ ID NO: 25), and M3M1 (SEQ ID NO: 26), and the MASP-1/MASP-3 dual binding clones D14 (SEQ ID NO:30), and 1E10.

The Kabat CDRs in the VH sequences below are located at the following amino acid positions::H1:aa 31-35; H2:aa 50-62; and H3:aa 95-102.

The Chothia CDRs in the VH sequences below are located at the following amino acid positions: H1:aa 26-32; H2: aa 52-56; and H3: aa 95-101.

Parent DTLacO VH (SEQ ID NO:24)

AVTLDESGGGLQTPGGALSLVCKASGFTFSSNAMGWVRQAPGKGLEWVAGIDD
DGSGTRYAPAVKGRATISRDNGQSTLRLQLNNLRAEDTGTYYCTKCAYSSGCDY
EGGYIDAWGHGTEVIVSS

Clone M3J5 VH: (SEQ ID NO:25)

AVTLDESGGGLQTPGG**G**LSLVCKASGFTFSSYAMGWMRQAPGKGLEYVAGIRS
DGSFTLYATAVKGRATISRDNGQSTVRLQLNNLRAEDTATYFCTRSGNVGDIDA
WGHGTEVIVSS

Clone M3M1 VH: (SEQ ID NO:26)

AVTLDESGGGLQTPGGGLSLVCKASGFDFSSYQMNWIRQAPGKGLEFVAAINRF
GNSTGHGAAVKGRVTISRDDGQSTVRLQLSNLRAEDTATYYCAKGVYGYCGSY
SCCGVDTIDAWGHGTEVIVSS

Clone D14 VH: (SEQ ID NO:30)

AVTLDESGGGLQTPGGALSLVCKASGFTFSSYAMHWVRQAPGKGLEWVAGIYK
SGAGTNYAPAVKGRATISRDNGQSTVRLQLNNLRAEDTGTYYCAKTTGSGCSSG
YRAEYIDAWGHGTEVIVSS

Clone 1E10 VH: (SEQ ID NO:32)

AVTLDESGGGLQTPGGALSLVCKASGFTFSSYDMVWVRQAPGKGLEFVAGISRN
DGRYTEYGSAVKGRATISRDNGQSTVRLQLNNLRAEDTATYYCARDAGGSAYW
FDAGQIDAWGHGTEVIVSS

**Light Chain Variable Region (VL) sequences**

**FIGURE 32B** shows an amino acid alignment of the light-Chain Variable Region (VL) sequences for the parent DTLacO (SEQ ID NO:27) and the MASP-3-binding clones M3J5 (SEQ ID NO:28), and M3M1 (SEQ ID NO:29), and the MASP-1/MASP-3 dual binding clones D14 (SEQ ID NO:31) and 1E10 (SEQ ID NO: 33).

Parent DTLacO VL (SEQ ID NO:27):

ALTQPASVSANLGGTVKITCSGGGSYAGSYYYGWYQQKSPGSAPVTVIYDNDKR
PSDIPSRFSGSLSGSTNTLTITGVRADDEAVYFCGSADNSGAAFGAGTTLTVL

Clone M3J5 VL (SEQ ID NO:28):

ALTQPASVSANPGETVKITCSGGYSGYAGSYYYGWYQQKAPGSAPVTLIYYNNK
RPSDIPSRFSGSLSGSTNTLTITGVRADDEAVYFCGSADNSGAAFGAGTTLTVL

Clone M3M1 VL (SEQ ID NO:29):

ALTQPASVSANPGETVKITCSGGGSYAGSYYYGWYQQKAPGSAPVTLIYYNNKR
PSDIPSRFSGSLSGSTNTLTITGVRADDEAVYFCGSADNSGAAFGAGTTLTVL

Clone D14 VL: (SEQ ID NO:31)

ALTQPASVSANPGETVKITCSGGGSYAGSYYYGWYQQKAPGSAPVTLIYYNNKR
PSDIPSRFSGSLSGSTNTLTITGVRADDEAVYFCGSADNSGAAFGAGTTLTVL

Clone 1E10 VL: (SEQ ID NO:33)

ALTQPASVSANPGETVKITCSGGGSYAGSYYYGWYQQKAPGSAPVTLIYYNNKR
PSDIPSRFSGSLSGSTNTLTITGVRADDEAVYFCGSADNSGAAFGAGTTLTVL

### *LEA-2 (MASP-2-dependent) Functional Assay*

MASP-1 contributes to LEA-2 via its ability to activate MASP-2 (see **FIGURE 1**). The Wieslab® Complement System Screen MBL assay (Euro Diagnostica, Malmö, Sweden) measures C5b-C9 deposition under conditions that isolate LEA-2-dependent activation (i.e., traditional lectin pathway activity). The assay was carried out according to the manufacturer's instructions with representative clone 1E10 tested as a final concentration of 400 nM.

**FIGURE 33** is a bar graph showing the inhibitory activity of the mAb 1E10 in comparison to the positive serum provided with the assay kit, as well as an isotype control antibody. As shown in **FIGURE 33**, mAb 1E10 demonstrates partial inhibition of LEA-2-dependent activation (via inhibition of MASP-1-dependent activation of MASP-2), whereas the isotype control antibody does not. Stronger inhibition should be achieved by continued affinity maturation of this antibody for MASP-1 binding using the tethered factors in the DTLacO system.

LEA-1 (MASP-3-dependent) Function Assays for representative mAbs are described below in Examples 17 and 18.

### Summary of Results:

The above results showed that the DTLacO platform permitted rapid *ex vivo* discovery of MASP-1 and MASP-3 monoclonal antibodies with inhibitory properties on LEA-1 (as shown below in Examples 17 and 18) and on LEA-2 (as shown in this Example).

**EXAMPLE 16**

This Example describes the generation of polypeptide inhibitors of MASP-1 and MASP-2.

**Rationale:**

The generation of specific inhibitors of MASP-1 and MASP-2, termed SGMI-1 and SGMI-2, respectively, is described in Heja et al., J Biol Chem 287:20290 (2012) and Heja et al., PNAS 109:10498 (2012), each of which is hereby incorporated herein by reference. SGMI-1 and SGMI-2 are each 36 amino acid peptides which were selected from a phage library of variants of the *Schistocerca gregaria* protease inhibitor 2 in which six of the eight positions of the protease binding loop were fully randomized. Subsequent *in vitro* evolution yielded mono-specific inhibitors with single digit nM $K_I$ values (Heja et al., J. Biol. Chem. 287:20290, 2012). Structural studies revealed that the optimized protease binding loop forms the primary binding site that defines the specificity of the two inhibitors. The amino acid sequences of the extended secondary and internal binding regions are common to the two inhibitors and contribute to the contact interface (Heja et al., 2012. J. Biol. Chem. 287:20290). Mechanistically, both SGMI-1 and SGMI-2 block the lectin pathway of complement activation without affecting the classical or alternative pathways (Heja et al., 2012. Proc. Natl. Acad. Sci. 109:10498).

The amino acid sequences of the SGMI-1 and SGMI-2 inhibitors are set forth below: SGMI-1-full-length: LEVTCEPGTTFKDKCNTCRCGSDGKSAFCTRKLCYQ (SEQ ID NO:34) SGMI-2-full-length: LEVTCEPGTTFKDKCNTCRCGSDGKSAVCTKLWCNQ (SEQ ID NO:35)

SGMI-1 and SGMI-2 are highly specific inhibitors of MASP-1 and MASP-2, respectively. However, as peptides they have limited potential for use in biological studies. To address these limitations, we engrafted these bioactive peptide amino acid sequences onto the amino terminus of human IgG1 Fc region to create an Fc-fusion protein,

**Methods:**

To express the SGMI-IgG1 Fc fusion proteins, polynucleotides encoding the SGMI-1 (SEQ ID NO:34) and SGMI-2 (SEQ ID NO:35) peptides were synthesized (DNA 2.0) and inserted into the expression vector pFUSE-hIgG1-Fc2 (InvivoGen) between nucleotide sequences encoding the IL-2 signal sequence and the human IgG1 Fc region (SEQ ID NO:36). A flexible polypeptide linker (e.g., SEQ ID NO:37 or SEQ ID NO:38) was included between the SGMI peptide and the IgG1 Fc region.

Flexible Polypeptide Linker Sequences:

GTGGGSGSSSRS (SEQ ID NO:37)
GTGGGSGSSS (SEQ ID NO:38)

The resulting constructs are described as follows:
A polynucleotide encoding the polypeptide fusion comprising the human IL-2 signal sequence, SGMI-1, linker and human IgG1-Fc (pFUSE-SGMI-1Fc), is set forth as SEQ ID NO:39, which encodes the mature polypeptide fusion comprising SGMI-1 (underlined), linker region (italicized) and human IgG1-Fc (together referred to as "SGMI-1Fc"), which is set forth as SEQ ID NO:40.

## SEQ ID NO:40

LEVTCEPGTTFKDKCNTCRCGSDGKSAFCTRKLCYQ*GTGGGSGSSSRS*DK THTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEALHNHYT QKSLSLSPGK

A polynucleotide encoding the polypeptide fusion comprising the human IL-2 signal sequence, SGMI-2, linker and human IgG1-Fc (pFUSE-SGMI-2Fc), is set forth as SEQ ID NO:41, which encodes the mature polypeptide fusion comprising SGMI-2 (underlined), linker region (italicized) and human IgG1-Fc (together referred to as "SGMI-2Fc"), which is set forth as SEQ ID NO:42:

## SEQ ID NO:42

LEVTCEPGTTFKDKCNTCRCGSDGKSAVCTKLWCNQ*GTGGGSGSSSRS*DK THTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEALHNHYT QKSLSLSPGK

### Production of Recombinant proteins:

Freestyle 293-F or Expi293F cells (Invitrogen) were transiently transfected according to the supplier's protocol with one of the two expression plasmids (pFUSE-SGMI-1Fc (SEQ ID NO:39) and pFUSE-SGMI-2Fc (SEQ ID NO:41). After four days of incubation at 37°C, the culture media were harvested. The Fc-fusion proteins were purified by Protein A affinity chromatography.

### Assays measuring activation of the lectin pathway.

The SGMI-1Fc and SGMI-2Fc fusion proteins were tested for the ability to inhibit deposition of C3b from 1% serum on a mannan-coated 96-well plate, which is a measure of lectin pathway activity. SGMI-1Fc and SGMI-2Fc were pre-incubated with 1% normal human serum for one hour on ice before addition to wells coated with mannan (2 μg/well). C3b deposition was measured by ELISA as described in Schwaeble et al. PNAS 108:7523, 2011.

**FIGURE 34** graphically illustrates the level of C3b deposition for 1% normal human serum plus isotype control, SGMI-1Fc or SGMI-2Fc over a concentration range of 0.15 to 1000 nM. As shown in **FIGURE 34**, both SGMI-1Fc and SGMI-2Fc inhibited C3b deposition from normal serum in mannan-coated ELISA wells, with $IC_{50}$ values of approximately 27nM and 300nM, respectively.

These results demonstrate that the MASP-1 and MASP-2 inhibitory functions of the SGMI peptides are retained in the SGMI-1Fc and SGMI-2Fc fusion proteins.

## EXAMPLE 17

Analysis of the complement pathway in 3MC serum with *S. aureus*

### Background/Rationale:

It was determined that MASP-3 is not activated through exposure to non-immobilized fluid-phase mannan, zymosan A or N-acetyl cysteine either in the presence or absence of normal human serum. However it was determined that recombinant and native MASP-3 are activated on the surface of heat-inactivated *S. aureus* in the presence and absence of normal human serum (NHS) or heat-inactivated human serum (HIS) (data not shown). It was also determined that C3b deposition occurs on the surface of *S. aureus* in the presence of normal human serum, and that the deposition can be monitored using a flow cytometer. Therefore, the alternative pathway (AP) response to *S. aureus* was measured as described in this Example as a means of assessing the contribution of MASP-3 to LEA-1.

### Methods:

Recombinant MASP-3: polynucleotide sequences encoding full length recombinant human MASP-3, a truncated serine protease (SP) active version of MASP-3 (CCP1-CCP2-SP), and a SP-inactivated form of MASP-3 (S679A) were cloned into the pTriEx7 mammalian expression vector (Invivogen). The resulting expression constructs encode the full length MASP-3 or the CCP1-CCP2-SP fragment with an amino-terminal Streptag and a carboxy-terminal $His_6$ tag. The expression constructs were transfected into Freestyle 293-F or Expi293F cells (Invitrogen) according to the protocols provided by the manufacturer. After three to four days of culture in 5% $CO_2$ at 37°C, recombinant proteins were purified utilizing Streptactin affinity chromatography.

Recombinant MASP-1: the full length or truncated CCP1-CCP2-SP forms of recombinant MASP-1 with or without the stabilizing R504Q (Dobo et al., J. Immunol 183:1207, 2009) or SP inactivating (S646A) mutations and bearing an amino-terminal Steptag and a carboxy-terminal His6 tag were generated as described for recombinant MASP-3 above.

### 1. C3b deposition and factor B cleavage on S. aureus in 3MC (human) serum

An initial experiment was carried out to demonstrate that the flow cytometry assay is able to detect the presence or absence of AP-driven C3b deposition (AP-C3b) as follows. Five percent of the following sera: normal human serum, factor B (Factor B)- depleted human serum, factor D-depleted human serum and properdin-depleted human serum (obtained from Complement Technology, Tyler, Texas, USA) were mixed with test antibody in either $Mg^{++}$/EGTA buffer or EDTA at 4°C overnight. Heat-killed *S. aureus* ($10^8$/reaction) was added to each mixture to a total volume of 100 $\mu$L and rotated at 37°C for 40 minutes. Bacteria were washed in washing buffer, the bacterial pellet was re-suspended in washing buffer and a 80 $\mu$L aliquot of each sample was analyzed for C3b deposition on the bacterial surface, which was detected with anti-human C3c (Dako, UK) using flow cytometry.

The results of the flow cytometry detection of C3b are shown in **FIGURE 35A.** As shown in **FIGURE 35A**, panel 1, normal human serum in the presence of EDTA, which is known to inactivate the AP, no C3b deposition was observed (negative control). In normal human serum treated with $Mg^{++}$/EGTA, only lectin-independent complement pathways can function. In panel 2, $Mg^{++}$/EGTA buffer is used, therefore the AP is active, and AP-driven C3b deposition is observed (positive control). As shown in panel 3, 4 and 5, in factor B-depleted, factor D-depleted and properdin-depleted serum, respectively, no alternative pathway driven C3b deposition is observed, as expected. These results demonstrate that the assay is capable of detecting AP-dependent C3b deposition.

A C3b deposition on *S. aureus* assay was carried out as described above to assess the ability of recombinant MASP-3 to reconstitute the AP (LEA-1) in human 3MC serum, which is deficient in MASP-3 (Rooryck C, et al., Nat Genet. 43(3):197-203 (2011)). The following combinations of reagents were tested.

1. 5% normal human serum +EDTA
2. 5% normal human serum +Mg/EGTA
3. 5% human 3MC (MASP-3$^{-/-}$) serum + $Mg^{++}$/EGTA
4. 5% human 3MC (MASP-3$^{-/-}$) serum + $Mg^{++}$/EGTA plus active full-length rMASP-3
5. 5% human 3MC (MASP-3$^{-/-}$) serum + $Mg^{++}$/EGTA plus truncated active rMASP-3 (CCP1/CCP2/SP)
6. 5% human 3MC (MASP-3$^{-/-}$) serum + $Mg^{++}$/EGTA plus inactive rMASP-3 (S679A)
7. 5% human 3MC (MASP-3$^{-/-}$) serum + $Mg^{++}$/EGTA plus active full length rMASP-1

The various mixtures of 5% serum and recombinant proteins (5 $\mu$g of each) as shown above were incubated in the specified buffer conditions (either $Mg^{++}$/EGTA buffer or EDTA) at 4°C overnight. After the incubation overnight, $10^8$ heat-killed *S. aureus* were added to each mixture in a total volume of 100 $\mu$L and rotated at 37°C for 40 minutes. Bacteria were washed and re-suspended in washing buffer and an 80 $\mu$l aliquot of each sample was analyzed for C3b deposition by FACS. The remaining 20 $\mu$L aliquot of each sample was used to measure factor B cleavage by Western blot using anti-factor B antibody as described below.

The results of the flow cytometery detection of C3b are shown in **FIGURE 35B.** Panel numbers correspond to the numbers designated for each of the reagent combination outlined above. The negative control (panel 1) and positive control (panel 2) show the absence and presence of C3b deposition, as expected. Panel 3 shows that AP-driven C3b deposition is absent in 3MC serum. Panels 4 and 5 show that active full length rMASP-3 (panel 4) and active rMASP-3 (CCP1-CCP2-SP) (panel 5) both restore AP-driven C3b deposition in 3MC serum. Panel 6 shows that inactive rMASP-3 (S679A) does not restore AP-driven C3b deposition in 3MC serum. Panel 7 shows that rMASP-1 does not restore AP-driven C3b deposition in 3MC serum.

Taken together, these results demonstrate that MASP-3 is required for AP-driven C3b deposition on *S. aureus* in human serum.

### 2. MASP-3-dependent Activation of Factor B

In order to analyze MASP-3-dependent activation of Factor B, the various mixtures of 5% serum (either normal human serum or 3MC patient serum) and recombinant proteins as shown above were assayed as described above. From each reaction mixture, 20 $\mu$L were removed and added to protein sample loading buffer. The samples were heated at 70°C for 10 minutes and loaded onto an SDS-PAGE gel. Western blot analysis was performed using a Factor B polyclonal antibody (R&D Systems). Activation of Factor B was apparent by the formation of two lower molecular weight cleavage products (Bb and Ba) derived from the higher molecular weight pro-Factor B protein.

**FIGURE 36** shows the results of a Western blot analysis to determine factor B cleavage in response to *S. aureus* in 3MC serum in the presence or absence of rMASP-3. As shown in lane 1, the normal human serum in the presence of EDTA (negative control) demonstrates very little Factor B cleavage relative to normal human serum in the presence of $Mg^{++}$/EGTA, shown in lane 2 (positive control). As shown in lane 3, 3MC serum demonstrates very little Factor B cleavage in the presence of $Mg^{++}$/EGTA. However, as shown in lane 4, Factor B cleavage is restored by the addition and pre-incubation of full-length, recombinant MASP-3 protein (5 $\mu$g) to the 3MC serum.

### 3. Assay to determine the effect of rMASP-3 on pro-factor D in factor B/C3(H2O) Cleavage

The following assay was carried out to determine the minimal requirement for MASP-3-dependent activation/cleavage of factor B.

$C3(H_2O)$ (200ng), purified plasm factor B (20 $\mu$g), recombinant pro-factor D (200 ng) and recombinant human MASP-3 (200 ng) were mixed together in various combinations (as shown in **FIGURE 37**), in a total volume of 100 $\mu$L in BBS/$Ca^{++}$/ $Mg^{++}$ and incubated at 30°C for 30 minutes. The reaction was stopped by adding 25 uL of SDS loading dye containing 5% 2-mercaptoethanol. After boiling at 95°C for 10 minutes under shaking (300 rpm), the mixture was spun down at 1400 rpm for 5 minutes and 20 uL of the supernatant was loaded and separated on a 10% SDS gel. The gel was stained with Coomassie brilliant blue.

### Results:

**FIGURE 37** shows a Comassie-stained SDS-PAGE gel in which factor B cleavage is analyzed. As shown in lane 1, factor B cleavage is most optimal in the presence of C3, MASP-3 and pro-factor D. As shown in lane 2, C3 is absolutely required; however, as shown in lanes 4 and 5, either MASP-3 or pro-factor D are able to mediate factor B cleavage, as long as C3 is present.

### 4. Analysis of the ability of MASP-3 mAbs to inhibit MASP-3-dependent AP-driven C3b deposition

As described in this Example it was demonstrated that MASP-3 is required for AP-driven C3b deposition on *S. aureus* in human serum. Therefore, the following assay was carried out to determine if a representative MASP-3 mAb identified as described in Example 15, could inhibit activity of MASP-3. Active, recombinant MASP-3 (CCP1-CCP2-SP) fragment protein (250 ng) was pre-incubated with an isotype control mAb, mAb1A5 (control obtained from the DTLacO platform that does not bind MASP-3 or MASP-1), or mAbD14 (binds MASP-3) at three different concentrations (0.5, 2 and 4 $\mu$M) for 1 hour on ice. The enzyme-mAb mixture was exposed to 5% 3MC serum (MASP-3 deficient) and $5\times10^7$ heat-killed *S. aureus* in a final reaction volume of 50 $\mu$L. The reactions were incubated at 37°C for 30 minutes, and then stained for the detection of C3b deposition. The stained bacterial cells were analyzed by a flow cytometer.

**FIGURE 38** graphically illustrates the mean fluorescent intensities (MFI) of C3b staining obtained from the three antibodies plotted as a function of mAb concentration in 3MC serum with the presence of rMASP-3. As shown in **FIGURE 38**, mAbD14 demonstrates inhibition of C3b deposition in a concentration-dependent manner. In contrast, neither of the control mAbs inhibited C3b deposition. These results demonstrate that mAbD14 is able to inhibit MASP-3-dependent C3b deposition. Improved inhibitory activity for mAbD14 is expected following continued affinity maturation of this antibody for MASP-3 binding using the tethered factors in the DTLacO system.

### Summary of Results:

In summary, the results in this Example demonstrate a clear defect of the AP in serum deficient for MASP-3. Thus, MASP-3 has been demonstrated to make a critical contribution to the AP, using factor B activation and C3b deposition as functional endpoints. Furthermore, addition of functional, recombinant MASP-3, including the catalytically-active C-terminal portion of MASP-3 corrects the defect in factor B activation and C3b deposition in the serum from the 3MC patient. Conversely, as further demonstrated in this Example, addition of a MASP-3 antibody (e.g.,mAbD14) in 3MC serum with rMASP-3 inhibits AP-driven C3b deposition. A direct role of MASP-3 in Factor B activation, and therefore the AP, is demonstrated by the observation that recombinant MASP-3, along with C3, is sufficient to activate recombinant factor B.

**EXAMPLE 18**

This Example demonstrates that MASP-1 and MASP-3 activate factor D.

**Methods:**

Recombinant MASP-1 and MASP-3 were tested for their ability to cleave two different recombinant versions of pro-factor D. The first version (pro-factor D-His) lacks an N-terminal tag, but has a C-terminal His tag. Thus, this version of pro-factor D contains the 5 amino acid pro-peptide that is removed by cleavage during activation. The second version (ST-pro-factor D-His) has a Strep-TagII sequence on the N-terminus, thus increasing the cleaved N-terminal fragment to 15 amino acids. ST-pro-factor D also contains a $His_6$ tag at the C-terminus. The increased length of the propeptide of ST-pro-factor D-His improves the resolution between the cleaved and uncleaved forms by SDS-PAGE compared to the resolution possible with the pro-factor D-HIS form.

Recombinant MASP-1 or MASP-3 proteins (2 $\mu$g) was added to either pro-factor D-His or ST-pro-factor D-His substrates (100 ng) and incubated for 1 hour at 37°C. The reactions were electrophoresed on a 12% Bis-Tris gel to resolve pro-factor D and the active factor D cleavage product. The resolved proteins were transferred to a PVDF membrane and analyzed by Western blot by detection with a biotinylated factor D antibody (R&D Systems).

**Results:**

**FIGURE 39** shows the Western blot analysis of pro-factor D substrate cleavage.

TABLE 18: Lane Description for Western Blot shown in FIGURE 39

| Experimental conditions | Lane 1 | Lane 2 | Lane 3 | Lane 4 | Lane 5 |
|---|---|---|---|---|---|
| Pro-Factor D | + | + | + | + | + |
| rMASP-3 (full-length) | - | + | | | |
| rMASP-3a (S679A) | - | - | + | - | - |
| rMASP-1a (S646A) | - | - | - | + | - |
| rMASP-1 (CCP-1-CCP2-SP) | - | - | - | - | + |

As shown in **FIGURE 39**, only full length MASP-3 (lane 2) and the MASP-1 CCP1-CCP2-SP) fragment (lane 5) cleaved ST-pro-factor D-$His_6$. The catalytically-inactive full length MASP-3 (S679A; lane 3) and MASP-1 (S646A; lane 3) failed to cleave either substrate. Identical results were obtained with the pro-factor D-$His_6$ polypeptide (not shown). The comparison of a molar excess of MASP-1 (CCP1-CCP2-SP) relative to MASP-3 suggests that MASP-3 is a more effective catalyst of pro-factor D cleavage than is MASP-1, as least under the condtions described herein.

**Conclusions:** Both MASP-1 and MASP-3 are capable of cleaving and activating factor D. This activity directly connects LEA-1 with the activation of the AP. More specifically, activation of factor D by MASP-1 or MASP-3 will lead to factor B activation, C3b deposition, and likely opsonization and/or lysis.

***1. Assay for Inhibition of MASP-3-dependent Cleavage of pro factor D with MASP-3 antibodies***

An assay was carried out to determine the inhibitory effect of representative MASP-3 and MASP-1 mAbs, identified as described in Example 15, on MASP-3-dependent factor D cleavage as follows. Active, recombinant MASP-3 protein (80 ng) was pre-incubated with 1 $\mu$g of representative mAbs D14, M3M1 and a control antibody (which binds specifically to MASP-1, but not to MASP-3) at room temperature for 15 minutes. Pro-factor D with an N-terminal Strep-tag (ST-pro-factor D-His, 70 ng) was added and the mixture was incubated at 37°C for 75 minutes. The reactions were then electrophoresed, blotted and stained with anti-factor D as described above.

**FIGURE 40** is a Western blot showing the partial inhibitory activity of the mAbs D14 and M3M1 in comparison to a control reaction containing only MASP-3 and ST-pro-factor D-His (no mAb; lane 1), as well as a control reaction containing a mAb obtained from the DTLacO library that binds MASP-1, but not MASP-3 (lane 4). As shown in **FIGURE 40**, in the absence of an inhibitory antibody, MASP-3 cleaves approximately 50% of pro-factor D into factor D (lane 1). The control MASP-1 specific antibody (lane 4) does not change the ratio of pro-factor D to factor D. In contrast, as shown in lanes 2 and 3, both mAb D14 and mAb M3M1 inhibit MASP-3-dependent cleavage of pro-factor D to factor D, resulting in a reduction in factor D generated.

**Conclusions:** These results demonstrate that MASP-3 mAbs D14 and M3M1 are able to inhibit MASP-3-dependent factor D cleavage. Improved inhibitory activity for mAbD14 and mAb M3M1 is expected following continued affinity maturation of these antibodies for MASP-3 binding using the tethered factors in the DTLacO system.

EXAMPLE 19

This Example demonstrates that MASP-3 deficiency prevents complement-mediated lysis of mannan-coated WT rabbit erythrocytes.

**Background/Rationale:**

As described in Examples 5 and 6 herein, the effect of MASP-2- and MASP-3-deficient serum on lysis of red blood cells from blood samples obtained from a mouse model of PNH demonstrated the efficacy of MASP-2 inhibition and/or MASP-3 inhibition to treat subjects suffering from PNH, and also supported the use of inhibitors of MASP-2 and/or inhibitors of MASP-3 (including dual or bi-specific MASP-2/MASP-3 inhibitors) to ameliorate the effects of C3 fragment-mediated extravascular hemolysis in PNH subjects undergoing therapy with a C5 inhibitor such as eculizumab.
As described in this Example, C3b deposition experiments and hemolysis experiments were carried out in MASP-3 deficient serum from additional 3MC patients, confirming the results obtained in Examples 5 and 6. In addition, experiments were carried out which demonstrated that addition of rMASP-3 to 3MC serum was able to reconstitute C3b deposition and hemolytic activity.

**Methods:**

MASP-3-deficient serum was obtained from three different 3MC patients as follows:

3MC Patient 1: contains an allele bearing a mutation that renders the exon encoding the MASP-3 serine protease domain dysfunctional, supplied along with the mother and father of the 3MC patient (both heterozygous for the allele bearing a mutation that renders the exon encoding the MASP-3 serine protease domain dysfunctional),
3MC Patient 2: Has C1489T (H497Y) mutation in exon 12 of MASP-1, the exon that encodes the serine protease domain of MASP-3, resulting in nonfunctional MASP-3, but functional MASP-1 proteins.
3MC Patient 3: Has a confirmed defect in the MASP-1 gene, resulting in nonfunctional MASP-3 and nonfunctional MASP-1 proteins.

**Experiment #1**: C3b Deposition Assay

An AP assay was carried out under traditional AP-specific conditions (BBS/ $Mg^{++}$/EGTA, without $Ca^{++}$, wherein BBS= barbital buffered saline containing sucrose), as described in Bitter-Suermann et al., Eur. J. Immunol 11:291-295 (1981)), on zymosan-coated microtiter plates at serum concentrations ranging from 0.5 to 25% and C3b deposition was measured over time.

**Results:**

**FIGURE 41** graphically illustrates the level of AP-driven C3b deposition on zymosan-coated microtiter plates as a function of serum concentration in serum samples obtained from MASP-3-deficient (3MC), C4-deficient and MBL-deficient subjects. As shown in **FIGURE 41**, and summarized below in **TABLE 18**, MASP-3-deficient patient sera from Patient 2 and Patient 3 have residual AP activity at high concentrations (25%, 12.5%, 6.25% serum concentrations), but a significantly higher $AP_{50}$ (i.e., 8.2% and 12.3% of serum needed to achieve 50% of maximum C3 deposition).
**FIGURE 42A** graphically illustrates the level of AP-driven C3b deposition on zymosan-coated microtiter plates under "traditional" AP-specific conditions (i.e., BBS/EGTA/$Mg^{++}$ without $Ca^{++}$) as a function of time in 10% human serum samples obtained from MASP-3 deficient, C4-deficient and MBL-deficient human subjects.
**TABLE 19** below summarizes the $AP_{50}$ results shown in **FIGURE 41** and the half-times for C3b deposition shown in **FIGURE 42A**.

**TABLE 19**: Summary of Results shown in **FIGURES 41** and **42A**

| Serum type | $AP_{50}$ (%) | $T_{1/2}$ (min) |
|---|---|---|
| Normal | 4.5 | 26.3 |

(continued)

| Serum type | AP$_{50}$ (%) | T$_{1/2}$ (min) |
|---|---|---|
| MBL-deficient (MBL-/-) | 5.7 | 27.5 |
| C4-deficient (C4-/-) | 5.1 | 28.6 |
| 3MC (Patient 3) | 8.2 | 58.2 |
| 3MC (Patient 2) | 12.3 | 72.4 |
| Note: In BBS/Mg$^{++}$/EGTA buffer, the lectin pathway-mediated effects are deficient due to absence of Ca$^{++}$ in this buffer. | | |

Experiment #2: Analysis of pro-factor D cleavage in 3MC patient sera by Western Blot

Methods: Serum was obtained from 3MC patient #2 (MASP-3 (-/-), MASP-1 (+/+)) and from 3MC patient #3 (MASP-3 (-/-), MASP-1 (-/-)). The patient sera, along with sera from normal donors (W), were separated by SDS-polyacrylamide gel and the resolved proteins were blotted to a polyvinylidine fluoride membrane. Human pro-factor D (25,040 Da) and/or mature factor D (24,405 Da) were detected with a human factor D-specific antibody.

Results: The results of the Western blot are shown in FIGURE 42B. As shown in FIGURE 42B, in the sera from normal donors (W), the factor D antibody detected a protein of a size consistent with mature factor D (24,405 Da). As further shown in FIGURE 42B, the factor D antibody detected a slightly larger protein in the sera from 3MC patient #2 (P2) and 3MC patient #3 (P3), consistent with the presence of pro-factor D (25,040 Da) in these 3MC patients.

Experiment #3: Wieslab Complement Assays with 3MC patient sera

Methods: Sera obtained from 3MC patient #2 (MASP-3 (-/-), MASP-1 (+/+)) and from 3MC patient #3 (MASP-3 (-/-), MASP-1 (-/-)) were also tested for classical, lectin and alternative pathway activity using the Wieslab Complement System Screen (Euro-Diagnostica, Malmö, Sweden) according to the manufacturer's instructions. Normal human serum was tested in parallel as a control.

Results: FIGURE 42C graphically illustrates the results of the Weislab classical, lectin and alternative pathway assays with plasma obtained from 3MC patient #2, 3MC patient #3, and normal human serum. As shown in FIGURE 42C, under conditions of the Wieslab assay, the classical, alternative, and MBL (lectin) pathways are all functional in the normal human serum. In serum from 3MC patient #2 (MASP-3 (-/-), MASP-1 (+/+)), the classical pathway and lectin pathway are functional, however there is no detectable alternative pathway activity. In serum from 3MC patient #3 (MASP-3 (-/-), MASP-1 (-/-)), the classical pathway is functional, however there is no detectable lectin pathway activity and no detectable alternative pathway activity.

The result in FIGURES 42B and 42C further support our understanding of the role of MASP-1 and MASP-3 in the LEA-1 and LEA-2 pathways. Specifically, the absence of the alternative pathway with a nearly fully functional lectin pathway in serum from Patient 2, who lacks only MASP-3, confirms that MASP-3 is essential for activation of the alternative pathway. Serum from Patient 3, who lacks both MASP-1 and MASP-3, has lost the ability to activate the lectin pathway as well as the alternative pathway. This result confirms the requirement of MASP-1 for a functional LEA-2 pathway, and is consistent with Examples 15 and 16, and the literature demonstrating that MASP-1 activates MASP-2. The apparent inability of both sera to activate pro-factor D is also consistent with the data described in Example 18 demonstrating that MASP-3 cleaves pro-factor D. These observations are consistent with the LEA-1 and LEA-2 pathways as diagrammed in Figure 1.

**Experiment #4:** Hemolysis assay testing mannan-coated rabbit erythrocytes for lysis in the presence of human normal or 3MC serum (in the absence of Ca$^{++}$)

**Methods:**

**Preparation of rabbit RBC in the absence of Ca$^{++}$ (i.e., by using EGTA)**

Rabbit whole blood (2 mL) was split into two 1.5 mL eppendorf tubes and centrifuged for 3 minutes at 8000 rpm (approximately 5.9 rcf) in a refrigerated eppendorf centrifuge at 4°C. The RBC pellet was washed three times after re-suspending in ice-cold BBS/ Mg$^{++}$/Ca$^{++}$ (4.4 mM barbituric acid, 1.8 mM sodium barbitone, 145 mM NaCl, pH 7.4, 5 mM Mg$^{++}$, 5 mM Ca$^{++}$). After the third wash, the pellet was re-suspended in 4 mL BBS/ Mg$^{++}$/Ca$^{++}$. The erythrocytes were pelleted and the RBCs were washed with BBS/0.1% gelatin/Mg$^{++}$/Ca$^{++}$ as described above. The RBCs suspension was stored in BBS/0.1% gelatin/ $Mg^{++}/Ca^{++}$ at 4°C. Then, 100 $\mu$L of suspended RBCs were diluted with 1.4 mL water and spun down at 8000 rpm (approximately 5.9 rcf) for 3 minutes and the OD of the supernatant was adjusted to 0.7 at 541nm (an OD of 0.7 at 541nm corresponds to approximately $10^9$ erythrocytes/ml). After that, 1 mL of the resuspended RBCs at OD 0.7 were added to 9 ml of BBS/$Mg^{++}$/EGTA in order to achieve a concentration of $10^8$ erythrocytes/ml. Dilutions of the test sera or plasma were prepared in ice-cold BBS, $Mg^{++}$, EGTA and 100 $\mu$L of each serum or plasma dilution was pipetted into the corresponding well of round-bottom plate. 100 $\mu$L of appropriately diluted RBC ($10^8$ erythrocytes/ml) were added to each well. Nano-water was used to produce the positive control (100% lysis), while a dilution with BBS/$Mg^{++}$/EGTA without serum or plasma was used as a negative control. The plate was then incubated for 1 hour at 37°C. The round bottom plate was spun down at 3750 rpm for 5 minutes. Then, 100 $\mu$L of the supernatant from each well was transferred into the corresponding wells of a flat-bottom plate and OD was read at 415-490 nm.

**Results:**

**FIGURE 43** graphically illustrates the percent hemolysis (as measured by hemoglobin release of lysed rabbit erythrocytes into the supernatant measured by photometry) of mannan-coated rabbit erythrocytes over a range of serum concentrations in serum from normal subjects and from two 3MC patients (Patient 2 and Patient 3), measured in the absence of $Ca^{++}$. As shown in **FIGURE 43**, it is demonstrated that MASP-3 deficiency reduces the percentage of complement-mediated lysis of mannan-coated erythrocytes as compared to normal human serum. The differences between the two curves from the normal human serum and the two curves from the 3MC patients is significant (p=0.013, Friedman test).

**TABLE 20** below summarizes the $AP_{50}$ results shown in **FIGURE 43.**

TABLE 20: Summary of Results shown in **FIGURE 43**

| Serum type | $AP_{50}$ (%) |
|---|---|
| Normal human serum #1 | 7.1 |
| Normal human serum #2 | 8.6 |
| 3MC Patient #2 | 11.9 |
| 3MC Patient #3 | 14.3 |

It is noted that when the serum samples shown in **TABLE 20** were pooled, the $AP_{50}$ value for normal human serum = 7.9 and the $AP_{50}$ value for 3MC serum = 12.8 (p=0.031, Wilcox matched-pairs signed rank test).

**Experiment #5:** Reconstitution of human 3MC serum by recombinant MASP-3 restores AP-driven C3b deposition on zymosan coated plates

**Methods:**

An AP assay was carried out under traditional AP-specific conditions (BBS/$Mg^{++}$/EGTA, without $Ca^{++}$, wherein BBS=barbital buffered saline containing sucrose), as described in Bitter-Suermann et al., Eur. J. Immunol 11:291-295 (1981)), on zymosan-coated microtiter plates in the following serum samples (1) 5% human serum from 3MC Patient #2 with full length active rMASP-3 added in at a range of 0 to 20 $\mu$g/ml; (2) 10% human serum from 3MC Patient #2 with full length active rMASP-3 added in at a range of 0 to 20 $\mu$g/ml; and (3) 5% human serum from 3MC Patient #2 with inactive rMASP-3A (S679A) added in at a range of 0 to 20 $\mu$g/ml.

**Results:**

**FIGURE 44** graphically illustrates the level of AP-driven C3b deposition on zymosan-coated microtiter plates as a function of the concentration of rMASP-3 protein added to serum samples obtained from human 3MC Patient #2 (MASP-3-deficient). As shown in **FIGURE 44,** active recombinant MASP-3 protein reconstitutes AP-driven C3b deposition on zymosan-coated plates in a concentration-dependent manner. As further shown in **FIGURE 44,** no C3b deposition was observed in the 3MC serum containing inactive rMASP-3 (S679A).

**Experiment #6:** Reconstitution of human 3MC serum by recombinant MASP-3 restores hemolytic activity in 3MC patient serum

**Methods:**

A hemolytic assay was carried out using rabbit RBC using the methods described above in Experiment #2 with the following test sera at a range of 0 to 12% serum: (1) normal human serum; (2) 3MC patient serum; (3) 3MC patient serum plus active full length rMASP-3 (20 $\mu$g/ml); and (4) heat-inactivated human serum.

**Results:**

**FIGURE 45** graphically illustrates the percent hemolysis (as measured by hemoglobin release of lysed rabbit erythrocytes into the supernatant measured by photometry) of mannan-coated rabbit erythrocytes over a range of serum concentrations in (1) normal human serum; (2) 3MC patient serum; (3) 3MC patient serum plus active full length rMASP-3 (20 $\mu$g/ml); and (4) heat-inactivated human serum, measured in the absence of $Ca^{++}$. As shown in **FIGURE 45,** the percent lysis of rabbit RBC is significantly increased in 3MC serum including rMASP-3 as compared to the percent lysis in 3MC serum without rMASP-3 (p=0.0006).

**FIGURE 46** graphically illustrates the percentage of rabbit erythrocyte lysis in 7% human serum from 3MC Patient 2 and from 3MC Patient 3 containing active rMASP-3 at a concentration range of 0 to 110 $\mu$g/ml in BBS/$Mg^{++}$/EGTA. As shown in **FIGURE 46,** the percentage of rabbit RBC lysis is restored with the amount of rMASP-3 in a concentration-dependent manner up to 100% activity.

**Experiment #7:** Serum of MASP-3 deficient (3MC)patient has functional MASP-2 if MBL is present

**Methods:**

A C3b deposition assay was carried out using Mannan-coated ELISA plates under to examine whether 3MC serum is deficient in LEA-2. Citrate plasma was diluted in BBS buffer in serial dilutions (starting at 1:80, 1:160, 1: 320, 1:640, 1:1280, 1:2560) and plated on Mannan-coated plates. Deposited C3b was detected using a chicken anti-human C3b assay. LEA-2 driven C3b deposition (the plasma dilutions are to high for the AP and LEA-1 to work) on Mannan-coated ELISA plates was evaluated as a function of human serum concentration in serum from a normal human subject (NHS), from two 3MC patients (Patient 2 and Patient 3), from the parents of Patient 3 and from a MBL-deficient subject.

**Results:**

**FIGURE 47** graphically illustrates the level of LEA-2-driven (i.e., MASP-2-driven) C3b deposition on Mannan-coated ELISA plates as a function of the concentration of human serum diluted in BBS buffer, for serum from a normal human subject (NHS), from two 3MC patients (Patient 2 and Patient 3), from the parents of Patient 3 and from a MBL-deficient subject. These data indicate that Patient 2 is MBL sufficient. However, Patient 3 and the mother of Patient 3 are MBL deficient, and therefore their serum does not deposit C3b on Mannan via LEA-2. Replacement of MBL in these sera restores LEA-2 mediated C3b deposition in the serum of Patient 3 (who is homozygous for the SNP leading to MASP-3 deficiency) and his mother (who is heterozygous for the mutant MASP-3 allele) (data not shown). This finding demonstrates that 3MC serum is not deficient in LEA-2, but rather appears to have functional MASP-2.

**Overall summary and Conclusions:**

These results demonstrate that MASP-3 deficiency in human serum results in loss of AP activity, as manifested in reduced C3b deposition on zymosan-coated wells and reduced rabbit erythrocyte lysis. The AP can be restored in both assays by supplementing the sera with functional, recombinant human MASP-3.

**EXAMPLE 20**

This example describes the results of MASP-2-/- in a Murine Macular Degeneration Model.

**Background/Rationale:** Age-related macular degeneration (AMD) is the leading cause of blindness after age 55 in the industrialized world. AMD occurs in two major forms: neovascular (wet) AMD and atrophic (dry) AMD. The neovascular (wet) form accounts for 90% of severe visual loss associated with AMD, even though only ~20% of individuals with AMD develop the wet form. Clinical hallmarks of AMD include multiple drusen, geographic atrophy, and choroidal neovascularization (CNV). In December, 2004, the FDA approved Macugen (pegaptanib), a new class of ophthalmic drugs to specifically target and block the effects of vascular endothelial growth factor (VEGF) for treatment of the wet (neovascular) form of AMD (Ng et al., Nat Rev. Drug Discov 5:123-32 (2006)). Although Macugen represents a promising new therapeutic option for a subgroup of AMD patients, there remains a pressing need to develop additional treatments for this complex disease. Multiple, independent lines of investigation implicate a central role for complement activation in the pathogenesis of AMD The pathogenesis of choroidal neovascularization (CNV), the most serious form of AMD, may involve activation of complement pathways.

Over twenty-five years ago, Ryan described a laser-induced injury model of CNV in animals (Ryan, S.J., Tr. Am. Opth. Soc. LXXVII: 707-745, 1979). The model was initially developed using rhesus monkeys, however, the same technology has since been used to develop similar models of CNV in a variety of research animals, including the mouse (Tobe et al., Am. J. Pathol. 153:1641-46, 1998). In this model, laser photocoagulation is used to break Bruch's membrane, an act that results in the formation of CNV-like membranes. The laser-induced model captures many of the important features of the human condition (for a recent review, see Ambati et al., Survey Ophthalmology 48:257-293, 2003). The laser-induced mouse model is now well established, and is used as an experimental basis in a large, and ever increasing, number of research projects. It is generally accepted that the laser-induced model shares enough biological similarity with CNV in humans that preclinical studies of pathogenesis and drug inhibition using this model are relevant to CNV in humans.

**Methods:**

A MASP-2-/- mouse was generated as described in Example 1 and backcrossed for 10 generations with C57B1/6. The current study compared the results when MASP-2 (-/-) and MASP-2 (+/+) male mice were evaluated in the course of laser-induced CNV, an accelerated model of neovascular AMD focusing on the volume of laser-induced CNV by scanning laser confocal microscopy as a measure of tissue injury and determination of levels of VEGF, a potent angiogenic factor implicated in CNV, in the retinal pigment epithelium (RPE)/choroids by ELISA after laser injury.

**Induction of choroidal neovascularization (CNV):** Laser photocoagulation (532 nm, 200 mW, 100 ms, 75$\mu$m; Oculight GL, Iridex, Mountain View, CA) was performed on both eyes of each animal on day zero by a single individual masked to drug group assignment. Laser spots were applied in a standardized fashion around the optic nerve, using a slit lamp delivery system and a coverslip as a contact lens. The morphologic end point of the laser injury was the appearance of a cavitation bubble, a sign thought to correlate with the disruption of Bruch's membrane. The detailed methods and endpoints that were evaluated are as follows.

**Fluorescein Angiography:** Fluorescein angiography was performed with a camera and imaging system (TRC 50 1A camera; ImageNet 2.01 system; Topcon, Paramus, NJ) at 1 week after laser photocoagulation. The photographs were captured with a 20-D lens in contact with the fundus camera lens after intraperitoneal injection of 0.1 ml of 2.5% fluorescein sodium. A retina expert not involved in the laser photocoagulation or angiography evaluated the fluorescein angiograms at a single sitting in masked fashion.

**Volume of choroidal neovascularization (CNV):** One week after laser injury, eyes were enucleated and fixed with 4% paraformaldehyde for 30 min at 4°C. Eye cups were obtained by removing anterior segments and were washed three times in PBS, followed by dehydration and rehydration through a methanol series. After blocking twice with buffer (PBS containing 1% bovine serum albumin and 0.5% Triton X-100) for 30 minutes at room temperature, eye cups were incubated overnight at 4°C with 0.5% FITC-isolectin B4 (Vector laboratories, Burlingame, CA), diluted with PBS containing 0.2% BSA and 0.1% Triton X-100. The FITC-isolectin B4 binds terminal $\beta$-D-galactose residues on the surface of endothelial cells and selectively labels the murine vasculature. After two washings with PBS containing 0.1% Triton X-100, the neurosensory retina was gently detached and severed from the optic nerve. Four relaxing radial incisions were made, and the remaining RPE -choroid-sclera complex was flat mounted in antifade medium (Immu-Mount Vectashield Mounting Medium; Vector Laboratories) and cover-sli pped.

Flat-mounts were examined with a scanning laser confocal microscope (TCS SP; Leica, Heidelberg, Germany). Vessels were visualized by exciting with blue argon wavelength (488 nm) and capturing emission between 515 and 545 nm. A 40X oil-immersion objective was used for all imaging studies. Horizontal optical sections (1 $\mu$m step) were obtained from the surface of the RPE-choroid-sclera complex. The deepest focal plane in which the surrounding choroidal vascular network connecting to the lesion could be identified was judged to be the floor of the lesion. Any vessel in the laser-targeted area and superficial to this reference plane was judged as CNV. Images of each section were digitally stored. The area of CNV-related fluorescence was measured by computerized image analysis with the microscope software (TCS SP; Leica). The summation of whole fluorescent area in each horizontal section was used as an index for the volume of CNV. Imaging was performed by an operator masked to treatment group assignment.

Because the probability of each laser lesion developing CNV is influenced by the group to which it belongs (mouse, eye, and laser spot), the mean lesion volumes were compared using a linear mixed model with a split plot repeated-measures design. The whole plot factor was the genetic group to which the animal belongs, whereas the split plot factor was the eye. Statistical significance was determined at the 0.05 level. *Post hoc* comparisons of means were constructed with a Bonferroni adjustment for multiple comparisons.

**VEGF ELISA.** At three days after injury by 12 laser spots, the RPE-choroid complex was sonicated in lysis buffer (20 mM imidazole HCl, 10 mM KCl, 1 mM $MgCL_2$, 10 mM EGTA, 1% Triton X-100, 10 mM NaF, 1 mM Na molybdate, and 1 mM EDTA with protease inhibitor) on ice for 15 min. VEGF protein levels in the supernatant were determined by an ELISA kit (R&D Systems, Minneapolis, MN) that recognizes all splice variants, at 450 to 570 nm (Emax; Molecular Devices, Sunnyvale, CA), and normalized to total protein. Duplicate measurements were performed in a masked fashion by an operator not involved in photocoagulation, imaging, or angiography. VEGF numbers were represented as the mean +/- SEM of at least three independent experiments and compared using the Mann-Whitney U test. The null hypothesis was rejected at $P<0.05$.

**RESULTS:**

**Assessment of VEGF Levels:**

**FIGURE 48A** graphically illustrates the VEGF protein levels in RPE-choroid complex isolated from C57B16 wild-type and MASP-2(-/-) mice at day zero. As shown in **FIGURE 48A,** the assessment of VEGF levels indicate a decrease in baseline levels for VEGF in the MASP-2 (-/-) mice versus the C57bl/6 wild-type control mice. **FIGURE 48B** graphically illustrates VEGF protein levels measured at day three following laser-induced injury. As shown in **FIGURE 48B** VEGF levels were significantly increased in the wild-type (+/+) mice three days following laser induced injury, consistent with published studies (Nozaki et al., Proc. Natl. Acad. Sci. USA 103:2328-33 (2006)). However, surprisingly very low levels of VEGF were seen in the MASP-2 (-/-) mice.

**Assessment of choroidal neovascularization (CNV):**

In addition to the reduction in VEGF levels following laser induced macular degeneration, CNV area was determined before and after laser injury. **FIGURE 49** graphically illustrates the CNV volume measured in C57bl/6 wild-type mice and MASP-2(-/-) mice at day seven following laser induced injury. As shown in **FIGURE 49,** the MASP-2 (-/-) mice displayed about a 30% reduction in the CNV area following laser induced damage at day seven in comparison to the wild-type control mice.

These findings indicate a reduction in VEGF and CNV as seen in the MASP (-/-) mice versus the wild-type (+/+) control and that blockade of MASP-2 with an inhibitor would have a preventive or therapeutic effect in the treatment of macular degeneration.

**EXAMPLE 21**

This Example describes analysis of the mouse MASP-2 Moab derived from Fab2 #11 for efficacy in a mouse model for age-related macular degeneration.

**Background/Rationale:**

As described in Examples 11 and 12, rat MASP-2 protein was utilized to pan a Fab phage display library, from which Fab2#1 1 was identified as a functionally active antibody. Full length antibodies of the rat IgG2c and mouse IgG2a isotypes were generated from Fab2 #11. The full-length MASP-2 antibody of the mouse IgG2a isotype was characterized for pharmacodynamic parameters as described in **Example 13.** In this Example, the mouse MASP-2 full-length antibody derived from Fab2 #11 was analyzed in the mouse model of age-related macular degeneration (AMD), described by Bora P.S. et al, J Immunol 174:491-497 (2005).

**Methods:**

The mouse IgG2a full-length MASP-2 antibody isotype derived from Fab2 #11 as described in Example 13, was tested in the mouse model of age-related macular degeneration (AMD) as described in Example 20 with the following modifications.

***Administration of mouse- MASP-2 MoAbs***

Two different doses (0.3 mg/kg and 1.0 mg/kg) of mouse MASP-2 MoAb along with an isotype control MoAb treatment were injected ip into WT (+/+) mice (n= 8 mice per group) 16 hours prior to CNV induction

*Induction of choroidal neovascularization (CNV)*

The induction of choroidal neovascularization (CNV) and measurement of the volume of CNV was carried out using laser photocoagulation as described in Example 20.

**Results:**

**FIGURE 50** graphically illustrates the CNV area measured at seven days post laser injury in mice treated with either isotype control MoAb, or mouse MASP-2 MoAb (0.3 mg/kg and 1.0 mg/kg). As shown in **FIGURE 50,** in the mice pre-treated with 1.0 mg/kg MASP-2 MoAb, a statistically significant ($p <0.01$) approximately 50% reduction in CNV was observed seven days post-laser treatment. As further shown in **FIGURE 50,** it was observed that a 0.3 mg/kg dose of MASP-2 MoAb was not efficacious in reducing CNV. It is noted that the 0.3 mg/kg dose of MASP-2 MoAb was shown to have a partial and transient inhibition of C4b deposition following subcutaneous administration, as described in **Example 13** and shown in **FIGURE 29A.**

The results described in this Example demonstrate that blockade of MASP-2 with an inhibitor, such as MASP-2 MoAb, has a preventative and/or therapeutic effect in the treatment of macular degeneration. It is noted that these results are consistent with the results observed in the study carried out in the MASP-2 (-/-) mice, described in **Example 20** in which a 30% reduction in the CNV seven days post-laser treatment was observed in MASP-2 (-/-) mice in comparison to the wild-type control mice. Moreover, the results in this Example further demonstrate that systemically delivered MASP-2 antibody provides local therapeutic benefit in the eye, thereby highlighting the potential for a systemic route of administration to treat AMD patients. In summary, these results provide evidence supporting the use of MASP-2 MoAb in the treatment of AMD.

**EXAMPLE 22**

This example describes the analysis of MASP-2(-/-) mice in a Mouse Myocardial Ischemia/Reperfusion Model.

**Background/Rationale:**

The mannose-binding lectin (MBL) is a circulating molecule that initiates complement activation in an immune complex-independent fashion, in response to a wide range of carbohydrate structures. These structures can be components of infectious agents or altered endogenous carbohydrate moieties particularly within necrotic, oncotic or apoptotic cells. These forms of cell death occur in reperfused myocardium where the activation of complement likely extends injury beyond the boundary that exists at the moment when ischemia is terminated by reperfusion. Although there is compelling evidence that complement activation aggravates myocardial reperfusion, the mechanism of such activation is not well understood and inhibition of all known pathways is likely to have intolerable adverse effects. A recent study suggests that activation may involve the MBL, rather than classical pathway or alternative amplification loop (as defined in the present invention), since infarction was reduced in MBL(A/C)-, but not C1q-, null mice (Walsh M.C. et al., Jour of Immunol. 175:541-546 (2005)). However, although encouraging, these mice still harbor circulating components, such as Ficolin A, capable of activating complement through the lectin pathway.

This study investigated MASP-2(-/-) mice versus wild type (+/+) controls to determine if the MASP-2(-/-) would be less sensitive to myocardial ischemia and reperfusion injury. MASP-2(-/-) mice were subjected to regional ischemia and infarct size was compared to their wild type littermates.

**Methods:** The following protocol was based on a procedure for inducing ischemia/reperfusion injury previously described by Marber et al., J. Clin Invest. 95:1446-1456 (1995)).

A MASP-2(-/-) mouse was generated as described in Example 1 and backcrossed for at least 10 generations with C57Bl/6. Seven MASP-2 (-/-) mice and seven wild-type (+/+) mice were anesthetized with ketamine/medetomidine (100 mg/kg and 0.2 mg/kg respectively) and placed supine on a thermostatically controlled heating pad to maintain rectal temperature at $37 \pm 0.3°C$. The mice were intubated under direct vision and ventilated with room air at a respiratory rate of 110/min and a tidal volume of 225 $\mu$l/min (Ventilator - Hugo Sachs Elektronic MiniVent Type 845, Germany).

Fur was shaved and an anterolateral skin incision made from the left axilla to the processus xiphoideus. The pectoralis major muscle was dissected, cut at its sternal margin and moved into the axillary pit. The pectoralis minor muscle was cut at its cranial margin and moved caudally. The muscle was later used as a muscle flap covering the heart during coronary artery occlusion. Muscles of the 5th intercostal space and the pleura parietalis were penetrated with tweezers at a point slightly medial to the margin of the left lung, thus avoiding damage of the lung or the heart. After penetration of the pleura the tweezers were carefully directed beyond the pleura towards the sternum without touching the heart, and pleura and intercostal muscles were dissected with a battery driven cauterizer (Harvard Apparatus, UK). Special care was exercised in avoiding any bleeding. Using the same technique, the thoracotomy was extended to the mid axillary line. After cutting the 4th rib at its sternal margin the intercostal space was widened until the whole heart was exposed from base to apex. With two small artery forceps the pericardium was opened and a pericardial cradle fashioned to move the heart slightly anterior. The left anterior descending coronary artery (LAD) was exposed and an 8-0 monofilament suture with a round needle was then passed under the LAD. The site of ligation of the LAD lies just caudal of the tip of the left atrium, about 1/4 along the line running from the atrioventricular crest to the apex of the left ventricle.

All experiments were carried out in a blinded manner, with the investigator being unaware of the genotype of each animal. After completion of instrumentation and surgical procedures, mice were allowed a 15 min equilibration period. Mice then underwent 30 min of coronary artery occlusion with 120 min of reperfusion time.

**Coronary artery occlusion and reperfusion model**

Coronary artery occlusion was achieved using the hanging weight system as previously described (Eckle et al., Am J Physiol Heart Circ Physiol 291:H2533-H2540, 2006). Both ends of the monofilament ligature were passed through a 2 mm long piece of a polythene PE-10 tube and attached to a length of 5-0 suture using cyanoacrylate glue. The suture was then directed over two horizontally mounted movable metal rods, and masses of 1 g each were attached to both ends of the suture. By elevation of the rods, the masses were suspended and the suture placed under controlled tension to occlude the LAD with a defined and constant pressure. LAD occlusion was verified by paleness of the area at risk, turning color of the LAD perfusion zone from bright red to violet, indicating cessation of blood flow. Reperfusion was achieved by lowering the rods until the masses lay on the operating pad and the tension of the ligature was relieved. Reperfusion was verified by the same three criteria used to verify occlusion. Mice were excluded from further analysis if all three criteria were not met at either start of coronary artery occlusion or within 15 min of reperfusion, respectively. During coronary artery occlusion, temperature and humidity of the heart surface were maintained by covering the heart with the pectoralis minor muscle flap and by sealing the thoracotomy with a 0.9% saline wet gauze.

**Measurement of myocardial infarct size:**

Infarct size (INF) and area at risk (AAR) were determined by planometry. After i.v. injection of 500 I.U. heparin the LAD was re-occluded and 300 $\mu$l 5% (w/vol) Evans Blue (Sigma-Aldrich, Poole, UK) was slowly injected into the jugular vein to delineate the area at risk (AAR). This strategy causes dye to enter the non-ischemic region of the left ventricle and leaves the ischemic AAR unstained. After mice had been euthanized by cervical dislocation, the heart was rapidly removed. The heart was cooled on ice and mounted in a block of 5% agarose and then cut into 8 transverse slices of 800 $\mu$m thickness. All slices were incubated at 37° C for 20 min with 3% 2,3,5-triphenyltetrazolium chloride (Sigma Aldrich, Poole, UK) dissolved in 0.1 M $Na_2HPO_4$/$NaH_2PO_4$ buffer adjusted to pH 7.4. Slices were fixed overnight in 10% formaldehyde. Slices were placed between two cover slips and sides of each slice were digitally imaged using a high-resolution optical scanner. The digital images were then analyzed using SigmaScan software (SPSS, US). The size of infarcted area (pale), left ventricle (LV) area at risk (red) and normally perfused LV zone (blue) were outlined in each section by identification of their color appearance and color borders. Areas were quantified on both sides of each slice and averaged by an investigator. Infarct size was calculated as a % of risk zone for each animal.

RESULTS: **FIGURE 51A** shows the evaluation of seven WT (+/+) mice and seven MASP-2 (-/-) mice for the determination of their infarct size after undergoing the coronary artery occlusion and reperfusion technique described above. As shown in **FIGURE 51A,** MASP-2 (-/-) mice displayed a statistically significant reduction ($p<0.05$) in the infarct size versus the WT (+/+) mice, indicating a protective myocardial effect from damage in the ischemia reperfusion injury model. **FIGURE 51B** shows the distribution of the individual animals tested, indicating a clear protective effect for the MASP-2 (-/-) mice.

**EXAMPLE 23**

This Example describes the analysis of MASP-2 (-/-) mice in a Murine Myocardial Ischemia/Reperfusion Model.

**Background/Rationale:**

To assess the contribution of MASP-2 to inflammatory reperfusion damage following an ischemic insult to the coronary artery, MASP-2 (-/-) and MASP-2 (+/+) mice were compared in the murine ischemia/reperfusion (MIRP) model as described by Marber et al., J. Clin Invest. 95:1446-1456 (1995), and in a Langendorff isolated perfused mouse heart model.

**Methods:**

The MASP-2 (-/-) mice used in this study were generated as described in **Example 1.** The ischemic insult to the left ventricle was carried out in eight WT (MASP-2 (+/+) and eleven MASP-2 (-/-) mice using the methods described in Example 22. Infarct size (INF) and area at risk (AAR) were determined by planometry as described in **Example 22.**

*Langendorff isolated-perfused mouse heart model:* The method of preparing hearts from mice for the Langendorff isolated-perfused mouse heart model was carried out as described in F.J. Sutherland et al., Pharmacol Res 41: 613 (2000). See also, A.M. Kabir et al,. Am J Physiol Heart Circ Physiol 291: H1893 (2006); Y. Nishino et al., Circ Res 103:307 (2008) and I.G. Webb et al., Cardiovasc Res (2010)).

Briefly described, WT (+/+) and MASP-2 (-/-) mice were anesthetized with pentobarbital (300 mg/kg) and heparin (150 units) intra-peritoneally. Hearts were rapidly isolated and placed in ice cold modified Krebs-Henselit buffer (KH, 118.5 mmol/l NaCl, 25.0 mmol/l NaHCO$_3$, 4.75 mmol KCl, KH$_2$PO$_4$ 1.18, MgSO$_4$ 1.19, D-glucose 11.0, and CaCl$_2$ 1.41. The excised heart was mounted onto a Langendorff apparatus with a water jacket and retrogradely perfused at a constant pressure of 80 mm Hg with KH buffer equilibrated with 95% O$_2$ and 5% CO$_2$. The temperature of the perfusate was maintained at 37°C. A fluid-filled balloon inserted into the left ventricle monitored contractile function. The balloon was gradually inflated until the end-diastolic pressure was between 1 and 7 mm Hg. Atrial pacing was performed at 580 bpm with a 0.075-mm silver wire (Advent). Coronary flow was measured by timed collection of perfusate.

*Infarction Assessment in Vitro*

After retrograde perfusion commenced, the hearts were stabilized for 30 min. For inclusion, all hearts had to fulfill the following criteria: coronary flow between 1.5 and 4.5 mL/min, heart rate >300 bpm (unpaced), left ventricular developed pressure >55 mm Hg, time from thoracotomy to aortic cannulation <3 min, and no persistent dysrhythmia during stabilization. Global ischemia and reperfusion was then conducted in the absence of serum. All hearts then underwent 30 mins of global ischemia by clamping the aortic inflow tubing, followed by 2 h of reperfusion.

Electrical pacing was stopped when contraction ceased during ischemia and restarted 30 min into reperfusion. After 2 hours of reperfusion, hearts were perfused for 1 min with 5 ml of 1% triphenyl tetrazolium chloride (TTC) in KH and then placed in an identical solution at 37°C for 10 min. The atria were then removed, and the hearts were blotted dry, weighed, and stored at -20°C for up to 1 week.

Hearts were then thawed, placed in 2.5% glutaraldehyde for 1 minute, and set in 5% agarose. The agarose heart blocks were then sectioned from apex to base in 0.7mm slices using a vibratome (Agar Scientific). After sectioning, slices were placed overnight in 10% formaldehyde at room temperature before transferring into PBS for an additional day at 4°C. Sections were then compressed between Perspex plates (0.57 mm apart) and imaged using a scanner (Epson model G850A). After magnification, planimetry was carried out using image analysis software (SigmaScan Pro 5.0, SPSS) and surface area of the whole, and TTC-negative, left ventricular myocardium was transformed to volume by multiplication with tissue thickness. Within each heart, after summation of individual slices, TTC-negative infarction volume was expressed as a percentage of, or plotted against, left ventricular volume.

**Results:**

The size of infarcted area (pale), left ventricle (LV) area at risk (red) and normally perfused LV zone (blue) were outlined in each section by identification of their color appearance and color borders. Areas were quantified on both sides of each slice and averaged by an investigator. Infarct volume was calculated as a % of risk zone (% RZ) for each animal.

**FIGURE 52A** shows the evaluation of eight WT (+/+) mice and eleven MASP-2 (-/-) mice for the determination of their infarct size after undergoing the coronary artery occlusion and reperfusion technique described above. **FIGURE 52A** graphically illustrates the mean area-at-risk (AAR, a measure of the area affected by ischemia) and infarct volumes (INF, a measure of damage to the myocardium) as a percentage of total myocardial volume. As shown in **FIGURE 52A,** while there is no difference in the AAR between the two groups, the INF volumes are significantly reduced in MASP-2 (-/-) mice as compared with their WT littermates, thus indicating a protective effect from myocardial damage in the absence of MASP-2 in this model of MIRP

**FIGURE 52B** graphically illustrates the relationship between INF plotted against the AAR as a % of left ventricle (LV) myocardial volume. As shown in **FIGURE 52B,** for any given AAR, MASP-2 (-/-) animals showed a highly significant reduction in the size of their infarction in comparison with their WT littermates.

**FIGURES 52C** and **52D** show the results of myocardial infarction in the buffer-perfused hearts of WT (+/+) and MASP-2 (-/-) mice prepared in accordance with the *Langendorff isolated-perfused mouse heart model,* in which global ischemia and reperfusion was carried out in the absence of serum. As shown in **FIGURES 52C** and **52D,** there was no difference observed in the resultant infarct volume (INF) between the hearts of the MASP-2 (-/-) and WT (+/+) mice, suggesting that the difference in infarct sizes shown in **FIGURES 52A** and **52B** are caused by plasma factors, and not by a lower susceptibility of the myocardial tissue of MASP-2 (-/-) mice to ischemic damage.

Taken together, these results demonstrate that MASP-2 deficiency significantly reduces myocardial damage upon reperfusion of an ischemic heart in the Murine Myocardial Ischemia/Reperfusion Model, and support the use of MASP-2 inhibitors to treat and prevent ischemia/reperfusion injury.

**EXAMPLE 24**

This Example describes the discovery of novel lectin pathway mediated and MASP-2 dependent C4-bypass activation of complement C3.

**Rationale:**

The principal therapeutic benefit of utilizing inhibitors of complement activation to limit myocardial ischemia/reperfusion injury (MIRI) was convincingly demonstrated in an experimental rat model of myocardial infarction two decades ago: Recombinant sCR1, a soluble truncated derivative of the cell surface complement receptor type-1 (CR1), was given intravenously and its effect assessed in a rat *in vivo* model of MIRI. Treatment with sCR1 reduced infarct volume by more than 40% (Weisman, H.F., et al., Science 249:146-151 (1990)). The therapeutic potential of this recombinant inhibitor was subsequently demonstrated in a clinical trial showing that the administration of sCR1 in patients with MI prevented contractile failure in the post-ischemic heart (Shandelya, S., et al., Circulation 87:536-546 (1993)). The primary mechanism leading to the activation of complement in ischemic tissue, however, has not been ultimately defined, mainly due to the lack of appropriate experimental models, the limited understanding of the molecular processes that lead to complement activation on oxygen-deprived cells, and the cross-talk and synergisms between the different complement activation pathways.

As a fundamental component of the immune response, the complement system provides protection against invading microorganisms through both antibody-dependent and -independent mechanisms. It orchestrates many cellular and humoral interactions within the immune response, including chemotaxis, phagocytosis, cell adhesion, and B-cell differentiation. Three different pathways initiate the complement cascade: the classical pathway, the alternative pathway, and the lectin pathway. The classical pathway recognition subcomponent C1q binds to a variety of targets - most prominently immune complexes - to initiate the step-wise activation of associated serine proteases, C1r and C1s, providing a major mechanism for pathogen and immune complex clearance following engagement by the adaptive immune system. Binding of C1q to immune complexes converts the C1r zymogen dimer into its active form to cleave and thereby activate C1s. C1s translates C1q binding into complement activation in two cleavage steps: It first converts C4 into C4a and C4b and then cleaves C4b-bound C2 to form the C3 convertase C4b2a. This complex converts the abundant plasma component C3 into C3a and C3b. Accumulation of C3b in close proximity of the C4b2a complex shifts the substrate specificity for C3 to C5 to form the C5 convertase $C4b2a(C3b)_n$. The C3 and C5 convertase complexes generated via classical pathway activation are identical to those generated through the lectin pathway activation route. In the alternative pathway, spontaneous low-level hydrolysis of component C3 results in deposition of protein fragments onto cell surfaces, triggering complement activation on foreign cells, while cell-associated regulatory proteins on host tissues avert activation, thus preventing self-damage. Like the alternative pathway, the lectin pathway may be activated in the absence of immune complexes. Activation is initiated by the binding of a multi-molecular lectin pathway activation complex to **P**athogen-**A**ssociated **M**olecular **P**atterns (PAMPs), mainly carbohydrate structures present on bacterial, fungal or viral pathogens or aberrant glycosylation patterns on apoptotic, necrotic, malignant or oxygen-deprived cells (Collard, C.D., et al., Am. J. Pathol. 156:1549-1556 (2000); Walport, M.J., N. Engl. J. Med. 344:1058-1066 (2001); Schwaeble, W., et al., Immunobiology 205:455-466 (2002); and Fujita, T., Nat. Rev. Immunol. 2:346-353 (2002)).

Mannan-binding lectin (MBL) was the first carbohydrate recognition subcomponent shown to form complexes with a group of novel serine proteases, named MBL-associated Serine Proteases (MASPs) and numbered according to the sequence of their discovery (i.e., MASP-1, MASP-2 and MASP-3). In man, lectin pathway activation complexes can be formed with four alternative carbohydrate recognition subcomponents with different carbohydrate binding specificities, i.e., MBL 2, and three different members of the ficolin family, namely L-Ficolin, H-ficolin and M-ficolin and MASPs. Two forms of MBL, MBL A and MBL C, and ficolin-A form lectin activation pathway complexes with MASPs in mouse and rat plasma. We have previously cloned and characterised MASP-2 and an additional truncated MASP-2 gene product of 19 kDa, termed MAp19 or sMAP, in human, mouse and rat (Thiel, S., et al., Nature 386:506-510 (1997);. Stover, C.M., et al., J. Immunol. 162:3481-3490 (1999); Takahashi, M., et al., Int. Immunol. 11:859-863 (1999); and Stover, C.M., et al., J. Immunol. 163:6848-6859 (1999)). MAp19/ sMAP is devoid of protease activity, but may regulate lectin pathway activation by competing for the binding of MASPs to carbohydrate recognition complexes (Iwaki, D. et al., J. Immunol. 177:8626-8632 (2006)).

There is strong evidence suggesting that of the three MASPs, only MASP-2 is required to translate binding of

134

the lectin pathway recognition complexes into complement activation (Thiel, S., et al. (1997); Vorup-Jensen, T., et al., J. Immunol. 165:2093-2100 (2000); Thiel, S., et al., J. Immunol. 165:878-887 (2000); Rossi, V., et al., J. Biol. Chem. 276:40880-40887 (2001)). This conclusion is underlined by the phenotype of a most recently described mouse strain deficient in MASP-1 and MASP-3. Apart from a delay in the onset of lectin pathway mediated complement activation *in vitro* -MASP-1/3 deficient mice retain lectin pathway functional activity. Reconstitution of MASP-1- and MASP-3-deficient serum with recombinant MASP-1 overcomes this delay in lectin pathway activation implying that MASP-1 may facilitate MASP-2 activation (Takahashi, M., et al., J. Immunol. 180:6132-6138 (2008)). A recent study has shown that MASP-1 (and probably also MASP-3) are required to convert the alternative pathway activation enzyme Factor D from its zymogen form into its enzymatically active form (Takahashi, M., et al., J. Exp. Med. 207:29-37 (2010)). The physiological importance of this process is underlined by the absence of alternative pathway functional activity in plasma of MASP-1/3 deficient mice.

The recently generated mouse strains with combined targeted deficiencies of the lectin pathway carbohydrate recognition subcomponents MBL A and MBL C may still initiate lectin pathway activation via the remaining murine lectin pathway recognition subcomponent ficolin A (Takahashi, K., et al., Microbes Infect. 4:773-784 (2002)). The absence of any residual lectin pathway functional activity in MASP-2 deficient mice delivers a conclusive model to study the role of this effector arm of innate humoral immunity in health and disease.

The availability of C4- and MASP-2-deficient mouse strains allowed us to define a novel lectin pathway-specific, but MASP-2-dependent, C4-bypass activation route of complement C3. The essential contribution of this novel lectin pathway mediated C4-bypass activation route towards post-ischemic tissue loss is underlined by the prominent protective phenotype of MASP-2 deficiency in MIRI while C4-deficient mice tested in the same model show no protection.

In this Example, we describe a novel lectin pathway-mediated and MASP-2-dependent C4-bypass activation of complement C3. The physiological relevance of this new activation route is established by the protective phenotype of MASP-2 deficiency in an experimental model of myocardial ischemia/reperfusion injury (MIRI), where C4 deficient animals were not protected.

**Methods:**

**MASP-2-deficient mice show no gross abnormalities.** MASP-2 deficient mice were generated as described in **Example 1.** Both heterozygous ($^{+/-}$) and homozygous ($^{-/-}$) MASP-2 deficient mice are healthy and fertile, and show no gross abnormalities. Their life expectancy is similar to that of their WT littermates (>18 months). Prior to studying the phenotype of these mice in experimental models of disease, our MASP-2($^{-/-}$) line was backcrossed for eleven generations onto a C57BL/6 background. The total absence of MASP-2 mRNA was confirmed by Northern blotting of poly A+ selected liver RNA preparations, while the 1.2kb mRNA encoding MAp19 or sMAP (a truncated alternative splicing product of the MASP2 gene) is abundantly expressed.

qRT-PCR analysis using primer pairs specific for either the coding sequence for the serine protease domain of MASP-2 (B chain) or the remainder of the coding sequence for the A-chain showed that no B chain encoding mRNA is detectable in MASP-2 ($^{-/-}$)mice while the abundance of the disrupted A chain mRNA transcript was significantly increased. Likewise, the abundance of MAp19/sMAP encoding mRNA is increased in MASP-2 ($^{+/-}$) and MASP-2 ($^{-/-}$) mice. Plasma MASP-2 levels, determined by ELISA for five animals of each genotype, were 300ng/ml for WT controls (range 260-330ng/ml), 360ng/ml for heterozygous mice (range 330-395ng/ml) and undetectable inMASP-2($^{-/-}$)mice. Using qRT-PCR, mRNA expression profiles were established demonstrating that MASP-2$^{-/-}$mice express mRNA for MBL A, MBL C, ficolin A, MASP-1, MASP-3, C1q, C1rA, C1sA, Factor B, Factor D, C4, and C3 at an abundance similar to that of their MASP-2 sufficient littermates (data not shown).

Plasma C3 levels of MASP-2 ($^{-/-}$) (n=8) and MASP-2 ($^{+/+}$) (n=7) littermates were measured using a commercially available mouse C3 ELISA kit (Kamiya, Biomedical, Seattle, WA). C3 levels of MASP-2 deficient mice (average 0.84 mg/ml, +/- 0.34) were similar to those of the WT controls (average 0.92, +/- 0.37).

**Results:**

**MASP-2 is essential for lectin pathway functional activity.**

As described herein and in US 7,919,094, the *in vitro* analyses of MASP-2$^{-/-}$plasma showed a total absence of lectin pathway functional activity on activating mannan- and zymosan-coated surfaces for both the activation of C4 and C3. Likewise, neither lectin pathway-dependent C4 nor C3 cleavage was detectable in MASP-2 ($^{-/-}$) plasma on surfaces coated with N-acetyl glucosamine, which binds and triggers activation via MBL A, MBL C and ficolin A (data not shown).

The analyses of sera and plasma of MASP-2 (-/-) mice clearly demonstrated that MASP-2 is essentially required to activate complement via the lectin pathway and that neither MASP-1, nor MASP-3 are able to maintain or restore lectin pathway activity in MASP-2 deficiency (data not shown).

The total deficiency of lectin pathway functional activity, however, leaves the other complement activation pathways intact: MASP-2-/-plasma can still activate complement via the classical (**FIGURE 53A**) and the alternative pathway (**FIGURE 53B**). In **FIGURE 53A** and **53B,** the symbol "*" indicates serum from WT (MASP-2 (+/+)); the symbol "•" indicates serum from WT (C1q depleted); the symbol "□" indicates serum from MASP-2 (-/-); and the symbol "Δ" indicates serum from MASP-2 (-/-) (C1q depleted).

**FIGURE 53A** graphically illustrates that MASP-2 (-/-) mice retain a functional classical pathway: C3b deposition was assayed on microtiter plates coated with immune complexes (generated in situ by coating with BSA then adding goat anti-BSA IgG). **FIGURE 53B** graphically illustrates MASP-2 deficient mice retain a functional alternative pathway: C3b deposition was assayed on Zymosan coated microtiter plates under conditions that permit only alternative pathway activation (buffer containing $Mg^{2+}$ and EGTA). Results shown in **FIGURE 53A** and **FIGURE 53B** are means of duplicates and are typical of three independent experiments. Same symbols for serum sources were used throughout. These results show that a functional alternative pathway is present in MASP-2 deficient mice, as evidenced in the results shown in FIGURE 53B under experimental conditions designed to directly trigger the alternative pathway, while inactivating both the classical pathway and lectin pathway.

**The lectin pathway of complement activation critically contributes to inflammatory tissue loss in myocardial ischemia/reperfusion injury (MIRI).**

As described in **Example 23,** in order to study the contribution of lectin pathway functional activity to MIRI, we compared MASP-2 (-/-) mice and WT littermate controls in a model of MIRI following transient ligation and reperfusion of the left anterior descending branch of the coronary artery (LAD). The results described in **Example 23** clearly demonstrate that MASP-2-deficient animals show a significant degree of protection with significantly reduced infarct sizes (p<0.01) compared to their lectin pathway sufficient littermates.

The presence or absence of complement C4 has no impact on the degree of ischemic tissue loss in MIRI. Using the same procedure described in **Example 23,** we assessed the impact of C4 deficiency on infarct sizes following experimental MIRI. As shown in **FIGURE 54A** and **FIGURE 54B,** nearly identical infarct sizes were observed in both C4-deficient mice and their WT littermates. **FIGURE 54A** graphically illustrates MIRI-induced tissue loss following LAD ligation and reperfusion in C4 (-/- ) mice (n=6) and matching WT littermate controls (n=7). Areas at risk (AAR) and infarct size (INF) were determined as described in **FIGURE 52. FIGURE 54B** graphically illustrates INF as a function of AAR, clearly demonstrating that C4 (-/-) mice are as susceptible to MIRI as their WT controls (dashed line).

These results demonstrate that C4-deficient mice are not protected from MIRI. This result was unexpected, as it is in conflict with the widely accepted view that the major C4 activation fragment, C4b, is an essential component of the classical and the lectin pathway C3 convertase C4b2a. We therefore assessed whether a residual lectin pathway specific activation of complement C3 can be detected in C4-deficient mouse and human plasma.

**The lectin pathway can activate complement C3 in absence of C4 via a novel MASP-2 dependent C4-bypass activation route.**

Encouraged by historical reports indicating the existence of a C4-bypass activation route in C4-deficient guinea pig serum (May, J.E., and M. Frank, J. Immunol. 111:1671-1677 (1973)), we analyzed whether C4-deficient mice may have residual classical or lectin pathway functional activity and monitored activation of C3 under pathway-specific assay conditions that exclude contributions of the alternative pathway.

C3b deposition was assayed on mannan-coated microtiter plates using re-calcified plasma at plasma concentrations prohibitive for alternative pathway activation (1.25% and below). While no cleavage of C3 was detectable in C4-deficient plasma tested for classical pathway activation (data not shown), a strong residual C3 cleavage activity was observed in C4-deficient mouse plasma when initiating complement activation via the lectin pathway. The lectin pathway dependence is demonstrated by competitive inhibition of C3 cleavage following preincubation of C4-deficient plasma dilutions with soluble mannan (see **FIGURE 55A**). As shown in **FIGURES 55A-D,** MASP-2 dependent activation of C3 was observed in the absence of C4. **FIGURE 55A** graphically illustrates C3b deposition by C4 (+/+) (crosses) and C4 (-/-)(open circles) mouse plasma. Pre-incubating the C4 (-/) plasma with excess (1 μg/ml) fluid-phase mannan prior to the assay completely inhibits C3 deposition (filled circles). Results are typical of 3 independent experiments. **FIGURE 55B** graphically illustrates the results of an experiment in which WT (crosses), MASP-2 deficient (open squares) and C4 (-/-) (open circles) mouse plasma (1%) was mixed with various concentrations of rat MASP-2 mAbM1 1 (abscissa) and C3b deposition assayed on mannan-coated plates. Results are means (± SD) of 4 assays (duplicates of 2 of each type of plasma).

**FIGURE 55C** graphically illustrates the results of an experiment in which normal human plasma: pooled NHS (crosses), C4 (-/-) plasma (open circles) and C4 (-/-) plasma pre-incubated with 1 μg/ml mannan (filled circles) - were incubated in mannan-coated wells and C3b deposition was measured. Results are representative of three independent experiments.

**FIGURE 55D** graphically illustrates the inhibition of C3b deposition in C4-sufficient and C4-deficient human plasma (1%) by human MASP-2 mAbH3 (Means ± SD of triplicates).

As shown in **FIGURE 55B,** no lectin pathway-dependent C3 activation was detected in MASP-2 (-/-) plasma assayed in parallel, implying that this C4-bypass activation route of C3 is MASP-2 dependent.

To further corroborate these findings, we established a series of recombinant inhibitory mAbs isolated from phage display antibody libraries by affinity screening against recombinant human and rat MASP-2A (where the serine residue of the active protease domain was replaced by an alanine residue by site-directed mutagenesis to prevent autolytic degradation of the antigen). Recombinant antibodies against MASP-2 (AbH3 and AbM11) were isolated from combinatorial antibody libraries (Knappik, A., et al., J. Mol. Biol. 296:57-86 (2000)), using recombinant human and rat MASP-2A as antigens (Chen, C.B. and Wallis, J. Biol. Chem. 276:25894-25902 (2001)). An anti-rat Fab2 fragment that potently inhibited lectin pathway-mediated activation of C4 and C3 in mouse plasma (IC50~1 nM) was converted to a full-length IgG2a antibody. Polyclonal anti-murine MASP-2A antiserum was raised in rats. These tools allowed us to confirm MASP-2 dependency of this novel lectin pathway specific C4-bypass activation route of C3, as further described below.

As shown in **FIGURE 55B,** M211, an inhibitory monoclonal antibody which selectively binds to mouse and rat MASP-2 inhibited the C4-bypass activation of C3 in C4-deficient mouse as well as C3 activation of WT mouse plasma via the lectin pathway in a concentration dependent fashion with similar $IC_{50}$ values. All assays were carried out at high plasma dilutions rendering the alternative pathway activation route dysfunctional (with the highest plasma concentration being 1.25%).

In order to investigate the presence of an analogous lectin pathway specific C4-bypass activation of C3 in humans, we analyzed the plasma of a donor with an inherited deficiency of both human C4 genes (i.e., C4A and C4B), resulting in total absence of C4 (Yang, Y., et al., J. Immunol. 173:2803-2814 (2004)). **FIGURE 55C** shows that this patient's plasma efficiently activates C3 in high plasma dilutions (rendering the alternative activation pathway dysfunctional). The lectin pathway specific mode of C3 activation on mannan-coated plates is demonstrated in murine C4-deficient plasma **(FIGURE 55A**) and human C4 deficient plasma **(FIGURE 55C**) by adding excess concentrations of fluid-phase mannan. The MASP-2 dependence of this activation mechanism of C3 in human C4-deficient plasma was assessed using AbH3, a monoclonal antibody that specifically binds to human MASP-2 and ablates MASP-2 functional activity. As shown in **FIGURE 55D,** AbH3 inhibited the deposition of C3b (and C3dg) in both C4-sufficient and C4-deficient human plasma with comparable potency.

In order to assess a possible role of other complement components in the C4-bypass activation of C3, we tested plasma of MASP-1/3 (-/-) and Bf/C2 (-/-) mice alongside MASP-2 (-/-), C4 (-/-) and C1q (-/-) plasma (as controls) under both lectin pathway specific and classical pathway specific assay conditions. The relative amount of C3 cleavage was plotted against the amount of C3 deposited when using WT plasma.

**FIGURE 56A** graphically illustrates a comparative analysis of C3 convertase activity in plasma from various complement deficient mouse strains tested either under lectin activation pathway or classical activation pathway specific assay conditions. Diluted plasma samples (1%) of WT mice (n=6), MASP-2 (-/-) mice (n=4), MASP-1/3 (-/-) mice (n=2), C4 (-/-) mice (n=8), C4/MASP-1/3 (-/-) mice (n=8), Bf/C2 (-/-) (n=2) and C1q (-/-) mice (n=2) were tested in parallel. Reconstitution of Bf/C2 (-/-) plasma with 2.5μg/ml recombinant rat C2 (Bf/C2 (-/-)+C2) restored C3b deposition. Results are means (±SD). **p<0.01 (compared to WT plasma). As shown in **FIGURE 56A,** substantial C3 deposition is seen in C4 (-/-) plasma tested under lectin pathway specific assay conditions, but not under classical pathway specific conditions. Again, no C3 deposition was seen in MASP-2 deficient plasma via the lectin pathway activation route, while the same plasma deposited C3 via the classical pathway. In MASP-1/3 (-/-) plasma, C3 deposition occurred in both lectin and classical pathway specific assay conditions. No C3 deposition was seen in plasma with a combined deficiency of C4 and MASP-1/3, either using lectin pathway or classical pathway specific conditions. No C3 deposition is detectable in Bf/C2 (-/-) plasma, either via the lectin pathway, or via the classical pathway. Reconstitution of C2/Bf-/- mouse plasma with recombinant C2, however, restored both lectin pathway and classical pathway-mediated C3 cleavage. The assay conditions were validated using C1q (-/-) plasma. **FIGURE 56B** graphically illustrates time-resolved kinetics of C3 convertase activity in plasma from various complement deficient mouse strains WT, fB (-/-), C4 (-/-), MASP-1/3(-/-), and MASP-2(-/-) plasma, tested under lectin activation pathway specific assay conditions (1% plasma, results are typical of three independent experiments). As shown in **FIGURE 56B,** while no C3 cleavage was seen in MASP-2(-/-) plasma, fB(-/-) plasma cleaved C3 with similar kinetics to the WT plasma. A significant delay in the lectin pathway-dependent conversion of C3 to C3b (and C3dg) was seen in C4 (-/-) as well as in MASP-1/3 deficient plasma. This delay of C3 activation in MASP-1/3(-/-) plasma was recently shown to be MASP-1, rather than MASP-3-dependent (Takahashi, M., et al., J. Immunol. 180:6132-6138 (2008)).

**Discussion:**

The results described in this Example strongly suggest that MASP-2 functional activity is essential for the activation of C3 via the lectin pathway both in the presence and absence of C4. Furthermore, C2 and MASP-1 are required for this novel lectin pathway specific C4-bypass activation route of C3 to work. The comparative analysis of lectin pathway functional activity in MASP-2 (-/-) as well as C4 (-/-) plasma revealed the existence of a previously unrecognized C4-independent, but MASP-2-dependent activation route of complement C3 and showed that C3 can be activated in a lectin pathway-dependent mode in total absence of C4. While the detailed molecular composition and the sequence of activation events of this novel MASP-2-dependent C3 convertase remains to be elucidated, our results imply that this C4-bypass activation route additionally requires the presence of complement C2 as well as MASP-1. The loss of lectin pathway-mediated C3 cleavage activity in plasma of mice with combined C4 and MASP-1/3 deficiency may be explained by a most recently described role of MASP-1 to enhance MASP-2-dependent complement activation through direct cleavage and activation of MASP-2 (Takahashi, M., et al., J. Immunol. 180:6132-6138 (2008)). Likewise, MASP-1 may aid MASP-2 functional activity through its ability to cleave C2 (Moller-Kristensen, et al., Int. Immunol. 19:141-149 (2007)). Both activities may explain the reduced rate by which MASP-1/3-deficient plasma cleaves C3 via the lectin activation pathway and why MASP-1 may be required to sustain C3 conversion via the C4-bypass activation route.

The inability of C2/fB (-/-) plasma to activate C3 via the lectin pathway was shown to be C2-dependent as the addition of recombinant rat C2 to C2/fB (-/-) plasma restored the ability of the reconstituted plasma to activate C3 on mannan-coated plates.

The finding that C4 deficiency specifically disrupts the classical complement activation pathway while the lectin pathway retains a physiologically critical level of C3 convertase activity via a MASP-2 dependent C4-bypass activation route calls for a reassessment of the role of the lectin pathway in various disease models, including experimental *S. pneumoniae* infection (Brown, J. S., et al., Proc. Natl. Acad. Sci. U. S. A. 99:16969-16974 (2002)); Experimental Allergic Encephalomyelitis (Boos, L.A., et al., Glia 49:158-160 (2005)); and models of C3 dependent murine liver regeneration (Clark, A., et al., Mol. Immunol. 45:3125-3132 (2008)). The latter group demonstrated that C4-deficient mice can activate C3 in an alternative pathway independent fashion as *in vivo* inhibition of the alternative pathway by an antibody-mediated depletion of factor B functional activity did not affect C3 cleavage-dependent liver regeneration in C4 (-/-) mice (Clark, A., et al. (2008)). This lectin pathway-mediated C4-bypass activation route of C3 may also explain the lack of a protective phenotype of C4 deficiency in our model of MIRI as well as in a previously described model of renal allograft rejection (Lin, T., et al., Am. J. Pathol. 168:1241-1248 (2006)). In contrast, our recent results have independently demonstrated a significant protective phenotype in MASP-2 (-/-) mice in models of renal transplantation (Farrar, C.A., et al., Mol. Immunol. 46:2832 (2009)).

In summary, the results of this Example support the view that MASP-2-dependent C4-bypass activation of C3 is a physiologically relevant mechanism that may be important under conditions where availability of C4 is limiting C3 activation.

## EXAMPLE 25

This Example demonstrates that the absence of MASP-2 functional activity results in a significant degree of protection from gastrointestinal ischemia/reperfusion injury (GIRI).

**Rationale:**

We explored the role of MASP-2 in GIRI using an established murine model (Zhang, M. et al. Proc. Natl. Acad. Sci. U. S. A. 101, 3886-3891 (2004); Zhang, M. et al. J. Exp. Med. 203, 141-152 (2006)).

**Methods:**

MASP-2-deficient mice were generated as described in Example 1. MASP-2 (-/-) mice and WT littermate controls were subjected to acute intestinal ischemia by surgically clamping of the superior mesenteric artery for 40 minutes followed by reperfusion of three hours. The surgical protocol for GIRI was performed as previously described (Zhang, M., et al., Proc. Natl. Acad. Sci. U.S.A. 101:3886-3891 (2004)). Following anesthesia, a laparotomy was performed and a surgical microclip applied to the superior mesenteric artery (SMA). After 40 minutes of ischemia, the microclip was removed and the ischemic tissue allowed to reperfuse for three hours. Sham controls underwent laparotomy without clamping the SMA. Following reperfusion, animals were sacrificed and corresponding segments of the distal jejunum harvested.

Intestinal injury was assessed by semi-quantitative pathology scoring of 200-400 villi in a defined area of jejunum, 4 cm per tissue section. Cryostat sections were stained with hematoxylin and eosin, blind-coded, and examined under light microscopy. The pathology score was assessed as described (Zhang, et al., 2004, *supra*). The first set of experiments assessed GIRI in 8-week-old female MASP-2 (-/-) and their MASP-2 (+/+) littermate controls. In the second set of experiments, six groups of 8-week-old female WT C57BL/6 mice were studied: sham-operated mice and I/R-operated mice pretreated with either saline; orisotype control antibody; or MASP-2 antibody mAbM11. The antibodies (each dosed at 1 mg/kg) or the saline were injected i.p. 18 hours before surgery.

**Results:**

**FIGURE 57A** graphically illustrates that MASP-2 (-/-) mice show a significant degree of protection from severe GIRI damage following transient (40 min) occlusion of the mesenteric artery and reperfusion (3 hrs) of ischemic gut tissue. (*p<0.05 as determined by Student's test). As shown in **FIGURE 57A,** MASP-2 (-/-) mice had a significant reduction of I/R tissue damage compared with WT littermates (pathology scores of MASP-2 (-/-) I/R group: $4 \pm 1$, n=6; pathology scores of MASP-2 (+/+) I/R group: $11 \pm 3$, n=7; P <0.05).

In order to assess whether a transient inhibition of MASP-2 functional activity can be achieved by applying selective antibody-based MASP-2 inhibitors *in vivo,* we assessed the degree and duration of lectin pathway inhibitory activity of the murine specific MASP-2 inhibitor mAbM1 1 following *i.p.* injection at a dose of 0.6 mg/kg body weight. Following the bolus injection, blood was collected by cardiac puncture at time points 0 hrs, 6 hrs, 12 hrs, 24 hrs, 48 hrs, 72 hrs, and 7 days, 10 days, 14 days and 17 days, and plasma assayed for lectin pathway-mediated C4 activation according to the methods described in Petersen, et al., J. Immunol. Methods 257:107-116 (2001), incorporated herein by reference.

**FIGURE 57B** illustrates the results obtained over the time course of *in vivo* ablation of lectin pathway functional activity achieved by an intraperitoneal single dose bolus injection of recombinant anti-murine MASP-2 antibody mAbM11 (0.6 mg/kg body weight). At the indicated time points, groups of mice (n=3) were sacrificed, and sera were prepared and assayed for LP-dependent C4 activation. The relative LP functional activity was normalized to LP activity in pooled sera from naive mice measured either in the absence (100%) or in the presence of 100nM blocking antibody(0%). Results are means ($\pm$SEM) from plasma samples of three different mice for each time point.

The results shown in **FIGURE 57B** depict the relative ablation of lectin pathway dependent C4 activation as a relative percentage of lectin pathway-mediated C4 activation prior to antibody dosing. The results show that the antibody-treatment yields a complete ablation of lectin pathway functional activity within six hours following antibody dosing. Lectin pathway functional activity is completely deficient for up to 48 hrs after dosing and does not recover significantly (less than 10% of the activity levels prior to antibody treatment) for up to seven days.

To test whether a therapeutic depletion of MASP-2 functional activity can protect animals from GIRI, WT mice (male C57BL/6J, 8-10 weeks old) were injected with mAbM1 1 (i.p., 1 mg/kg body weight), or an identical dose of an irrelevant isotype control antibody (i.p., 1 mg/kg body weight) or saline 18 hrs prior to the intestinal I/R or sham surgery.

**FIGURE 57C** graphically illustrates the protective effect of MASP-2 mAb treatment on the severity of GIRI pathology. Mice were dosed with 1 mg/kg of mAbM1 1 (n=10) or an irrelevant isotype control antibody (n=10) or injected with saline only (n=10) 24 hrs before being subjected to 40 min GI ischemia followed by three hours of reperfusion. (*p<0.05 when comparing animals treated with the MASP-2 inhibitory antibody to mice treated with either the irrelevant isotype control antibody or saline). Sham animals (n=5 per group) were treated in an identical fashion except that no clamp was applied to the mesenteric artery.

**FIGURE 57D** shows histological presentation of GIRI-mediated pathology of the small intestine in WTC57BL/6 mice pre-treated with a single-dose intraperitoneal injection of either isotonic saline, an isotype control antibody (1 mg/kg body weight), or recombinant murine MASP-2 antibody mAbM1 1 (1 mg/kg body weight) 12 hours prior to the induction of GIRI and their respective sham controls. The arrowheads indicate subepithelial spaces in the luminal part of the villi (characterized by the lack of cellular content beneath the continuous epithelial layer) as typical features of GIRI pathology. (magnification, X100).

As shown in **FIGURES 57C** and **57D,** when saline-treated mice were subjected to intestinal I/R surgery, they had significant tissue damage compared with sham-operated controls ($25\pm7$, n=10; versus $1\pm0$, n=5, P<0.01). Pretreatment with the isotype control antibody gave no protection from I/R injury compared with saline control ($17\pm2$ versus $25\pm7$, n=10/per group, p>0.05). In contrast, pretreatment with mAbM1 1 significantly reduced tissue I/R damage by more than 2-fold compared with mice treated with the isotype control antibody ($8\pm2$ versus $17\pm2$, n=10/per group, p<0.01). The ischemic intestinal injury in the GIRI group treated with MASP-2 mAb was not reduced down to the baseline levels seen in the sham control group ($8\pm2$, n=10, versus $2\pm1$, n=5, p<0.01), but a significant sparing of tissue damage was evident in both MASP-2 (-/-) and MASP-2 mAb treated animals. The MASP-2 mAb results further validate the deleterious role the lectin pathway plays in ischemia reperfusion injury.

**Discussion:**

Many recent reports aimed to clarify the mechanism(s) and pathway(s) leading to complement activation on oxygen-deprived cells. The involvement of IgM antibodies in complement-dependent GIRI has been well established (Zhang, M., et al., Proc. Natl. Acad. Sci. U.S.A. 101I3886-3891 (2004); Zhang, M., et al., J. Exp. Med. 203:141-152 (2006)). With IgM being a potent activator of the classical pathway, it was assumed that mice deficient of the classical pathway (such as Clqa (-/-) mice) would be protected from complement-dependent GIRI and MIRI (described in **Example 24**). Surprisingly, two recent studies demonstrated that C1qa (-/-) mice are not protected, either in GIRI, or MIRI, while mice deficient of the lectin pathway recognition molecules MBL A and MBL C showed a significant reduction of both GIRI and MIRI (Hart, M.L., et al., J. Immunol. 174:6373-6380 (2005); Walsh, M.C.. et al.. J. Immunol. 175:541-546 (2005)). These findings were confirmed in two subsequent GIRI studies, which identified that the critical pro-inflammatory contributions of IgM-dependent complement activation occurred in absence of classical pathway activity utilizing the lectin activation pathway through direct interactions between autoreactive IgM and MBL (Zhang, M., et al., J. Immunol. 177:4727-4734 (2006); McMullen, M.E., et al., Immunobiology 211:759-766 (2006)). In contrast, the same MBL null strain (i.e., MBL null mice retain a residual lectin pathway functional activity through ficolin A) was tested in a model of renal IRI, and showed only a moderate degree of protection from tissue injury (Moller-Kristensen, M., et al., Scand. J. Immunol. 61:426-434 (2005)).

Taken together, these studies suggest that the degree of protection in MBL null mice may vary between different experimental models of IRI, as the role of the remaining lectin pathway recognition molecule ficolin A in mediating IRI is not yet understood. In humans, we have recently shown that plasma MBL is rapidly consumed in the reperfusion phase following surgically-induced ischemia during abdominal aneurism repair surgery (Norwood, M.G., et al., Eur. J. Vasc. Endovasc. Surg. 31:239-243 (2006)). In man, the situation may even be more complex in as - in addition to MBL-three different ficolins may serve as lectin pathway recognition subcomponents.

Utilizing MASP-2 (-/-) mice in a model of MIRI, we have demonstrated that lectin pathway functional activity is an essential component of the inflammatory process leading to major loss of myocardial tissue. MASP-2 (-/-) mice may still activate complement through either the classical or the alternative pathway, but are devoid of any residual lectin pathway functional activity, while having all of the three murine lectin pathway pattern recognition molecules, MBL A, MBL C and ficolin A present in plasma. Moreover, MASP-2 functional activity was also shown to be an essential component in driving post-ischemic inflammatory pathology in a model of GIRI, monitored through scoring GIRI-mediated tissue damage in MASP-2 (-/-) and MASP-2 (+/+) animals. Our results unequivocally show that neither the classical nor the alternative pathway complement activation route is sufficient to initiate the inflammatory pathology of post-ischemic tissue injury in absence of lectin pathway functional activity. It is, nevertheless, plausible that the alternative pathway may secondarily contribute towards an augmentation of complement activation in other tissues. This would explain why the deficiency of factor B may ameliorate post-ischemic inflammatory tissue loss in a model of ischemic acute renal failure (Thurman, J.M., et al., J. Immunol. 170:1517-1523 (2003)).

Finally, with regard to the phenotype of MASP-2 deficiency and the implications for therapeutic intervention, our results demonstrate that a transient and long-sustained blockade of MASP-2 and lectin pathway functional activity can be achieved *in vivo* by systemic application of inhibitory MASP-2 specific monoclonal antibodies. The high efficacy in inhibiting MASP-2 functional activity using relatively low doses of inhibitory antibodies *in vivo* may be therapeutically viable due to the relatively low abundance of MASP-2 in plasma (ranging between 260 to 330 ng/ml in mouse plasma (see Results) and between 170 to 1196 ng/ml in human plasma (Moller-Kristensen, M., et al., J. Immunol. Methods 282:159-167 (2003)), and the strict absence of any extra-hepatic MASP-2 biosynthesis (Stover, C.M., et al, J. Immunol. 163:6848-6859 (1999); Endo, Y., et al., Int. Immunol. 14:1193-1201 (2002)). Therefore, it is believed that inhibition of MASP-2 by administration of inhibitory monoclonal antibodies against MASP-2 would be effective to treat ischemia-induced inflammatory pathologies.

### EXAMPLE 26

This Example demonstrates that the absence of MASP-2 functional activity in a MASP-2 (-/-) mouse model results in a significant degree of protection from cerebral ischaemia/reperfusion injury (stroke).

### Methods:

Three-vessel occlusion (3VO) Surgery:

Transient ischemia was introduced by the three-vessel-occlusion (3VO) stroke model as described by Yanamoto et al., Exp Neurology 182(2):261-274 (2003). Briefly described, Female C57/B16 mice at the age of 8-18 weeks of age were administered with Vetergesic (analgesic) prior to the operation to minimize post-operative pain. The animals were anesthetized with 3% to 4% isofluorane with $O_2/N_2O$ followed by a reduction of isofluorane to 0.5 to 1.5% for maintenance anesthesia. The two common carotid arteries (CCA) were exposed via a ventral midline incision of the neck, followed by clipping the left CCA with an aneurism clip. This reduces bleeding during the procedure to cauterize the ipsilateral middle cerebral artery (MCA). Following the clipping of the left CCA, the left zygomatic arch was removed to enable access to the skull and the middle cerebral artery. A 1 mm thick burr hole was opened to allow access to the MCA followed by its permanent cauterization using a bipolar coagulator (Aura, Kirwan Surgical Products). After the MCA occlusion, ischemia was induced for 30 minutes by the clipping of the right CCA. During the ischemic time the head wound was closed. After the termination of ischemia both clips were removed allowing reperfusion for 24 hours and animals were culled afterwards by cervical dislocation.

**Infarct size determination**

Following 24 hours of reperfusion, mice were killed via cervical dislocation and their brains were removed and sliced into 1 mm thick slices using a pre-cooled brain matrix. Infarct volume after ischemia was determined via the reliable method using 2, 3, 5-Triphenyltetrazolium chloride (TTC), which is a metabolic cell indicator of mitochondrial activity, as described in Bederson, J.B. et al., Stroke 17:1304-1308 (1986) and Lin T.N. et al, Stroke 24:117-121 (1993). In this assay, the red coloring (shown as dark areas in the black and white photographs) in brain sections indicates the normal, non-infarcted tissue whereas non-colored, white areas indicate the infarcted tissue (Bederson et al., 1986). Upon sectioning of the brain, slices were stained with 2% TTC in saline at room temperature for 30 minutes in the dark. Afterwards the sections were fixed in 10% formalin and stored in the dark in 4°C. Digital images were taken and were analyzed in Scion Image Software to calculate the infarct volume. The infarct volume was calculated as follows to avoid overestimation of the infarct area by edema:

$$\text{Infarct volume} = \text{Infarct area} / (\text{ipsilateral area} / \text{controlateral area}) \times 1 \text{ mm}$$

$$(\text{thickness of the slice})$$

**Results:**

**FIGURE 58** graphically illustrates the cerebral infarct volume in WT and MASP-2 (-/-) mice following 30 minutes ischemia and 24 hours reperfusion. As shown in **FIGURE 58,** the infarct volume following 3-VO is significantly decreased in MASP-2 (- /-) mice in comparison to WT (MASP-2 (+/+) mice (p=0.0001)).
**FIGURE 59A** shows a series of brain sections of a WT (MASP-2 +/+) mouse after 30 minutes ischemia and 24 hours reperfusion. Panels 1-8 of **FIGURE 59A** show the different section areas of the brain corresponding to Bregma 1-8, respectively, in relation to the exit of the acoustic nerve (Bregma 0).
**FIGURE 59B** shows a series of brain sections of a MASP-2 (-/-) mouse after 30 minutes ischemia and 24 hours reperfusion. Panels 1-8 of **FIGURE 59B** show the different sections areas of the brain corresponding to Bregma 1-8, respectively, in relation to the exit of the acoustic nerve (Bregma 0).
The infarct volumes measured for the brain sections shown in **FIGURES 59A** and **59B** are provided below in **TABLE 21.**

**TABLE 21:** Infarct Volume Measurements from brain sections of mice treated with MCA occlusion for 30 minutes followed by 24 hours reperfusion (shown in **FIGURES 59A** and **59B**)

| FIG (reference panel) | Genotype | BREGMA (in relation to the Exit of the acoustic nerve, Bregma=0) | Infarct volume |
|---|---|---|---|
| Fig 59A-1 | WT (MASP2 +/+) | 1 | 1.70 mm |
| Fig 59A-2 | WT (MASP2 +/+) | 2 | 0.74 mm |
| Fig 59A-3 | WT (MASP2 +/+) | 3 | -0.10 mm |
| Fig 59A-4 | WT (MASP2 +/+) | 4 | -0.82 mm |
| Fig 59A-5 | WT (MASP2 +/+) | 5 | -1.82 mm |
| Fig 59A-6 | WT (MASP2 +/+) | 6 | -3.08 mm |
| Fig 59A-7 | WT (MASP2 +/+) | 7 | -4.04 mm |
| Fig 59A-8 | WT (MASP2 +/+) | 8 | -4.60 mm |
| Fig 59B-1 | MASP2 (-/-) | 1 | 1.54 mm |
| Fig 59B-2 | MASP2 (-/-) | 2 | 0.98 mm |
| Fig 59B-3 | MASP2 (-/-) | 3 | -0.46 mm |
| Fig 59B-4 | MASP2 (-/-) | 4 | -1.22 mm |
| Fig 59B-5 | MASP2 (-/-) | 5 | -1.70 mm |

(continued)

| FIG (reference panel) | Genotype | BREGMA (in relation to the Exit of the acoustic nerve, Bregma=0) | Infarct volume |
|---|---|---|---|
| Fig 59B-6 | MASP2 (-/-) | 6 | -2.80 mm |
| Fig 59B-7 | MASP2 (-/-) | 7 | -4.36 mm |
| Fig 59B-8 | MASP2 (-/-) | 8 | -4.72 mm |

As shown in **FIGURES 59A, 59B** and **TABLE 21,** MASP-2-deficiency limits tissue loss following transient cerebral ischemia (MCAO for 30 minutes) followed by 24 hours of reperfusion. These results demonstrate that the absence of MASP-2 functional activity in a MASP-2 (-/-) mouse model results in a significant degree of protection from cerebral ischaemia/reperfusion injury (stroke).

**EXAMPLE 27**

This example describes the results of MASP-2(-/-) in a Murine Monoclonal Antibody Induced Rheumatoid Arthritis Model.

**Background/Rationale:** The most commonly used animal model for rheumatoid arthritis (RA) is collagen-induced arthritis (CIA) (for recent review, see Linton and Morgan, Mol. Immunol. 36:905-14, 1999). Collagen type II (CII) is one of the major constituents of the articular matrix proteins and immunization with native CII in adjuvant induces autoimmune polyarthritis by a cross-reactive autoimmune response to CII in joint cartilage. As in RA, susceptibility to CIA is linked to the expression of certain class II MHC alleles. Some strains of mice, including the C57Bl/6 strain, are resistant to classic CIA because they lack an appropriate MHC haplotype and therefore do not generate high CII antibody titers. However, it has been found that consistent arthritis can be induced in all strains of mice by the *i.v.* or *i.p.* administration into mice of a cocktail of four specific monoclonal antibodies against type II collagen (Col2 MoAb). These arthritogenic monoclonal antibodies are commercially available (Chondrex, Inc., Redmond, WA). This passive transfer model of CIA has been used successfully in a number of recent published reports using the C57Bl/6 mouse strain (Kagari et al., J. Immunol. 169:1459-66, 2002; Kato et al., J. Rheumatol. 30:247-55, 2003; Banda et al, J. Immunol. 177:1904-12, 2006). The following study compared the sensitivity of WT (+/+) and MASP-2 (-/-) mice, both sharing the C57Bl/6 genetic background, to development of arthritis using the passive transfer model of CIA.

**Methods:**

Animals: A MASP-2(-/-) mouse was generated as described in **Example 1** and backcrossed for 10 generations with C57Bl/6. Fourteen male and female C57BL/6 wild type mice that were seven to eight weeks old at the time of antibody injection and ten male and female MASP-2(-/-) and WT (+/+) C57Bl/6 mice that were seven to eight weeks old at time of antibody injection were used in this study. Twenty mice were injected with a monoclonal antibody cocktail to obtain 20 solid responders (two groups of ten). The animals (ten/group) were housed with five animals/cage, and were acclimated for five to seven days prior to initiating the study.

Mice were injected intravenously with a monoclonal antibody cocktail (Chondrex, Redmond WA) (5 mg) on day 0 and day 1. The test agent was monoclonal antibody (Col2 MoAb) + LPS from Chondrex. On day 2, mice were dosed ip with LPS. Mice were weighed on days 0, 2, 4, 6, 8, 10, 12 and prior to termination on day 14. On day 14 the mice were anesthetized with isoflurane and bled terminally for serum. After blood collection, the mice were euthanized, with removal of both fore and hind limbs with knees, which were placed into formalin for future processing.

Treatment Groups:

   Group 1 (control): 4 mice of strain C57/BL/6 WT (+/+);
   Group 2 (test): 10 mice of strain C57/BL/6 WT (+/+) (received Col2 MoAb plus LPS); and
   Group 3 (test): 10 mice of strain C57/BL/MASP-2KO/6Ai (-/-) (received Col2 MoAb mAb plus LPS)

Clinical arthritic scores were assessed daily using the following scoring system: 0 = normal; 1= 1 hind or fore paw joint affected; 2= 2 hind or fore paw joints affected; 3= 3 hind or fore paw joints affected; 4= moderate (erythema and moderate swelling, or 4 digit joints affected); 5= severe (diffuse erythema and severe swelling entire paw, unable to flex digits)

**Results:**

**FIGURE 60** shows the group data plotted for the mean daily clinical arthritis score for up to two weeks. No clinical arthritis score was seen in the control group that did not receive the CoL2 MoAb treatment. The MASP (-/-) mice had a lower clinical arthritis score from day 9 to day 14. The overall clinical arthritis score with area under the curve analysis (AUC) indicated a 21% reduction in the MASP-2 (-/-) group versus the WT (+/+) mice. However, C57Bl6 mouse background as discussed previously did not provide for a robust overall arthritis clinical score. Due to the small incidence rate and group size, while positively trending, the data provided only trends (p = 0.1) and was not statistically significant at the p < 0.05 level. Additional animals in the treatment groups would be necessary to show statistical significance. Due to the reduced incidence of arthritis, the affected paw scores were evaluated for severity. No single incidence of a clinical arthritis score of greater than 3 was seen in any of the MASP-2 (-/-) mice, which was seen in 30% of the WT (+/+) mice, further suggesting that (1) the severity of the arthritis may be related to complement pathway activation and (2) that blockade of MASP-2 may have a beneficial effect in arthritis.

## EXAMPLE 28

This Example demonstrates the use of pure dust mite allergen as a potent activator of lectin pathway mediated C3 activation as a model of asthma.

**Rationale:**

A well-characterized mouse model of house dust mite (HDM)-induced allergic asthma has been developed. See X. Zhang et al., J. of Immunol. 182:5123-5130 (2009), hereby incorporated herein by reference. As described in Zhang et al. (2009), the model involves exposing mice to intratracheal HDM once a week over the course of three weeks. The intratracheal HDM administration significantly increases airway responsiveness, total cell numbers and eosinophil numbers in BAL fluid as well as serum total IgE and allergen-specific IgE levels in WT BALB/c mice. This model can be used to assess the use of MASP-2 mabs as a therapeutic for asthma.

**Methods:**

C3 deposition assays were carried out with serum samples obtained from WT mice. To measure C3 activation, microtiter plates were coated with mannan (1$\mu$g/well), then sheep anti-HSA serum (2$\mu$g/ml) in TBS/Tween/Ca$^{2+}$. Plates were blocked with 0.1% HSA in TBS and washed as above. Plasma samples were diluted in 4mM barbital, 145mM NaCl, 2mM CaCl$_2$, 1mM MgCl$_2$, pH 7.4, added to the plates and incubated for 1.5h at 37°C. After washing, bound C3b was detected using rabbit anti-human C3c (Dako), followed by alkaline phosphatase-conjugated goat anti-rabbit IgG and pNPP.

**Results:**

**FIGURE 61** graphically illustrates the results of the C3 deposition assay in serum samples obtained from WT mice in the presence of house dust mite or zymosan. As shown in **FIGURE 61,** dust mite allergen is a potent activator of lectin pathway mediated C3 activation, activating C3 at nearly the same level as zymosan. These results indicate that dust mite allergen is capable of stimulating the lectin pathway. In view of the fact that MASP-2 antibodies have been shown to block activation of the alternative complement pathway, it is expected that MASP-2 antibodies will be effective as therapeutics in treating asthma that is due to dust mite allergen-sensitized individuals.

## EXAMPLE 29

This Example demonstrates that thrombin activation can occur following lectin pathway activation under physiological conditions, and demonstrates the extent of MASP-2 involvement. In normal rat serum, activation of the lectin pathway leads to thrombin activation (assessed as thrombin deposition) concurrent with complement activation (assessed as C4 deposition). As can be seen in **FIGURES 62A** and **62B,** thrombin activation in this system is inhibited by the MASP-2 blocking antibody, HI (Fab2 format), exhibiting an inhibition concentration-response curve (**FIGURE 62B**) that parallels that for complement activation (**FIGURE 62A**). These data suggest that activation of the lectin pathway as it occurs in trauma will lead to activation of both complement and coagulation systems in a process that is entirely dependent on MASP-2. By inference, MASP-2 blocking antibodies may prove efficacious in mitigating cases of excessive systemic coagulation, e.g., disseminated intravascular coagulation, which is one of the hallmarks leading to mortality in major trauma cases.

**EXAMPLE 30**

This Example provides results generated using a localized Schwartzman reaction model of disseminated intravascular coagulation ("DIC") in MASP-2 (-/-) and WT (+/+)mice to evaluate the role of lectin pathway in DIC.

**Background/Rationale:**

As described supra, blockade of MASP-2 inhibits lectin pathway activation and reduces the generation of both anaphylatoxins C3a and C5a. C3a anaphylatoxins can be shown to be potent platelet aggregators *in vitro,* but their involvement *in vivo* is less well defined and the release of platelet substances and plasmin in wound repair may only secondarily involve complement C3. In this Example, the role of the lectin pathway was analyzed in MASP-2 (-/-) and WT (+/+) mice in order to address whether prolonged elevation of C3 activation is necessary to generate disseminated intravascular coagulation.

**Methods:**

The MASP-2 (-/-) mice used in this study were generated as described in **Example** 1. The localized Schwartzman reaction (LSR) model was used in this experiment. The LSR is a lipopolysaccharide (LPS) -induced response with well-characterized contributions from cellular and humoral elements of the innate immune system. Dependence of the LSR on complement is well established (Polak, L., et al., Nature 223:738-739 (1969); Fong J.S. et al., J Exp Med 134:642-655 (1971)). In the LSR model, the mice were primed for 4 hours with TNF alpha (500 ng, intrascrotal), then the mice were anaesthetized and prepared for intravital microscopy of the cremaster muscle. Networks of post-capillary venules (15-60 $\mu$m diameter) with good blood flow (1-4 mm/s) were selected for observation. Animals were treated with fluorescent antibodies to selectively label neutrophils, or platelets. The network of vessels was sequentially scanned and images of all vessels were digitally recorded for later analysis. After recording the basal state of the microcirculation, mice received a single intravenous injection of LPS (100 $\mu$g), either alone or with the agents listed below. The same network of vessels was then scanned every 10 minutes for 1 hour. Specific accumulation of fluorophores was identified by subtraction of background fluorescence and enhanced by thresholding the image. The magnitude of reactions was measured from recorded images. The primary measure of LSR was aggregate data.
The studies compared the WT (+/+) mice exposed to either a known complement pathway depletory agent, cobra venom factor (CVF), or a terminal pathway inhibitor (C5aR antagonist). The results (**FIGURE 63A**) demonstrate that CVF as well as a C5aR antagonist both prevented the appearance of aggregates in the vasculature. In addition, the MASP-2 (-/-) mice (**FIGURE 63B**) also demonstrated complete inhibition of the localized Schwartzman reaction, supporting lectin pathway involvement. These results clearly demonstrate the role of MASP-2 in DIC generation and support the use of MASP-2 inhibitors for the treatment and prevention of DIC.

**EXAMPLE 31**

This Example describes activation of C3 by thrombin substrates and C3 deposition on mannan in WT (+/+), MASP-2 (-/-), F11 (-/-), F11/C4 (-/-) and C4 (-/-) mice.

**Rationale:**

As described in **Example 29,** it was determined that thrombin activation can occur following lectin pathway activation under physiological conditions, and demonstrates the extent of MASP-2 involvement. C3 plays a central role in the activation of the complement system. C3 activation is required for both classical and alternative complement activation pathways. An experiment was carried out to determine whether C3 is activated by thrombin substrates.

**Methods:**

**C3 Activation by thrombin substrates**

Activation of C3 was measured in the presence of the following activated forms of thrombin substrates; human FXIa, human FVIIa, bovine FXa, human FXa, human activated protein C, and human thrombin. C3 was incubated with the various thrombin substrates, then separated under reducing conditions on 10% SDS-polyacrylamide gels. After electrophoretic transfer using cellulose membrane, the membrane was incubated with monoclonal biotin-coupled rat anti-mouse C3, detected with a streptavidin-HRP kit and developed using ECL reagent.

**Results:**

Activation of C3 involves cleavage of the intact a-chain into the truncated a' chain and soluble C3a. **FIGURE 64** shows the results of a Western blot analysis on the activation of human C3 by thrombin substrates, wherein the uncleaved C3 alpha chain, and the activation product a' chain are shown by arrows. As shown in **FIGURE 64,** incubation of C3 with the activated forms of human clotting factor XI and factor X, as well as activated bovine clotting factor X, can cleave C3 in vitro in the absence of any complement proteases.

**C3 deposition on mannan**

C3 deposition assays were carried out on serum samples obtained from WT, MASP-2 (-/-), F11(-/-), F11(-/-)/C4(-/-) and C4(-/-). F11 is the gene encoding coagulation factor XI. To measure C3 activation, microtiter plates were coated with mannan (1 $\mu$g/well), then sheep anti-HSA serum (2 $\mu$g/ml) in TBS/Tween/Ca$^{2+}$. Plates were blocked with 0.1% HSA in TBS and washed as above. Plasma samples were diluted in 4 mM barbital, 145 mM NaCl, 2 mM CaCl$_2$, 1 mM MgCl$_2$, pH 7.4, added to the plates and incubated for 1.5 h at 37°C. After washing, bound C3b was detected using rabbit anti-human C3c (Dako), followed by alkaline phosphatase-conjugated goat anti-rabbit IgG and pNPP.

**Results:**

**FIGURE 65** shows the results of the C3 deposition assay on serum samples obtained from WT, MASP-2 (-/-), F 11 (-/-), F11(-/-)/C4 (-/-) and C4 (-/-). As shown in **FIGURE 65,** there is a functional lectin pathway even in the complete absence of C4. As further shown in **FIGURE 65,** this novel lectin pathway-dependent complement activation requires coagulation factor XI.

**Discussion:**

Prior to the results obtained in this experiment, it was believed by those in the art that the lectin pathway of complement required C4 for activity. Hence, data from C4 knockout mice (and C4 deficient humans) were interpreted with the assumption that such organisms were lectin pathway-deficient (in addition to classical pathway deficiency). The present results demonstrate that this notion is false. Thus, conclusions of past studies suggesting that the lectin pathway was not important in certain disease settings based on the phenotype of C4-deficient animals may be false. As described in **Example 24,** we have demonstrated this for myocardial infarction models where MASP-2 knockout mice are protected while C4 knockout mice are not.

The data described in this Example also show that in the physiological context of whole serum the lectin pathway can activate components of the coagulation cascade. Thus, it is demonstrated that there is cross-talk between complement and coagulation involving MASP-2.

**EXAMPLE 32**

This study investigates the effect of MASP-2-deficiency in a mouse model of LPS (lipopolysaccharide)-induced thrombosis.

Rationale:

Hemolytic uremic syndrome (HUS), which is caused by Shiga toxin-producing E. coli infection, is the leading cause of acute renal failure in children. In this Example, a Schwartzman model of LPS-induced thrombosis (microvascular coagulation) was carried out in MASP-2 (-/-) mice to determine whether MASP-2 inhibition is effective to inhibit or prevent the formation of intravascular thrombi.

Methods:

MASP-2 (-/-) (n=9) and WT (n=10) mice were analyzed in a Schwarztman model of LPS-induced thrombosis (microvascular coagulation). Mice were administered Serratia LPS and thrombus formation was monitored over time. A comparison of the incidence of microthrombi and LPS-induced microvascular coagulation was carried out.

Results:

Notably, all MASP-2 -/- mice tested (9/9) did not form intravascular thrombi after Serratia LPS administration. In contrast, microthrombi were detected in 7 of 10 of the WT mice tested in parallel (p=0.0031, Fischer's exact). As shown in **FIGURE 66,** the time to onset of microvascular occlusion following LPS infection was measured in MASP-2 (-/-) and WT mice, showing the percentage of WT mice with thrombus formation measured over 60 minutes, with thrombus formation detected as early as about 15 minutes. Up to 80% of the WT mice demonstrated thrombus formation at 60 minutes. In contrast, as shown in **FIGURE 66,** none of the MASP-2 (-/-) had thrombus formation at 60 minutes (log rank: p=0.0005).

These results demonstrate that MASP-2 inhibition is protective against the development of intravascular thrombi in an HUS model.

## EXAMPLE 33

This Example describes the effect of MASP-2 antibodies in a mouse model of HUS using intraperitoneal co-injection of purified *Shiga toxin* 2 plus LPS.

Background:

A mouse model of HUS was developed using intraperitoneal co-injection of purified Shiga toxin 2 (STX2) plus LPS. Biochemical and microarray analysis of mouse kidneys revealed the STX2 plus LPS challenge to be distinct from the effects of either agent alone. Blood and serum analysis of these mice showed neutrophilia, thrombocytopenia, red cell hemolysis, and increased serum creatinine and blood urea nitrogen. In addition, histologic analysis and electron microscopy of mouse kidneys demonstrated glomerular fibrin deposition, red cell congestion, microthrombi formation, and glomerular ultrastructural changes. It was established that this model of HUS induces all clinical symptoms of human HUS pathology in C57BL/6 mice including thrombocytopenia, hemolytic anemia, and renal failure that define the human disease. (J. Immunol 187(1): 172-80 (2011))

Methods:

C57BL/6 female mice that weighed between 18 to 20 g were purchased from Charles River Laboratories and divided in to two groups (five mice in each group). One group of mice was pretreated by intraperitoneal (i.p.) injection with the recombinant MASP-2 antibody mAbM1 1 (100 $\mu$g per mouse; corresponding to a final concentration of 5 mg/kg body weight) diluted in a total volume of 150 $\mu$l saline. The control group received saline without any antibody. Six hours after i.p injection of MASP-2 antibody mAbM1 1, all mice received a combined i.p. injection of a sublethal dose (3 $\mu$g/animal; corresponding to 150 $\mu$g/kg body weight) of LPS of *Serratia marcescens* (L6136; Sigma-Aldrich, St. Louis, MO) and a dose of 4.5 ng/animal (corresponding to 225 ng/kg) of STX2 (two times the LD50 dose) in a total volume of 150 $\mu$l. Saline injection was used for control.

Survival of the mice was monitored every 6 hours after dosing. Mice were culled as soon as they reached the lethargic stage of HUS pathology. After 36 hours, all mice were culled and both kidneys were removed for immunohistochemistry and scanning electron microscopy. Blood samples were taken at the end of the experiment by cardiac puncture. Serum was separated and kept frozen at -80°C for measuring BUN and serum Creatinine levels in both treated and control groups.

## Immunohistochemistry

One-third of each mouse kidney was fixed in 4% paraformaldehyde for 24 h, processed, and embedded in paraffin. Three-micron-thick sections were cut and placed onto charged slides for subsequent staining with H & E stain.

## Electron Microscopy

The middle section of the kidneys was cut into blocks of approximately 1 to 2 mm$^3$, and fixed overnight at 4°C in 2.5% glutaraldehyde in 1x PBS. The fixed tissue subsequently was processed by the University of Leicester Electron Microscopy Facility

## Cryostat sections

The other third of the kidneys was, cut into blocks approximately 1 to 2 mm$^3$ and snap frozen in liquid nitrogen and kept at -80°C for cryostat sections and mRNA analysis.

### Results:

**FIGURE 67** graphically illustrates the percent survival of saline-treated control mice (n=5) and MASP-2 antibody-treated mice (n=5) in the STX/LPS-induced model over time (hours). Notably, as shown in **FIGURE 67,** all of the control mice died by 42 hours. In sharp contrast, 100 % of the MASP-2 antibody-treated mice survived throughout the time course of the experiment. Consistent with the results shown in **FIGURE 67,** it was observed that all the untreated mice that either died or had to be culled with signs of severe disease had significant glomerular injuries, while the glomeruli of all MASP-2-antibody-treated mice looked normal (data not shown). These results demonstrate that MASP-2 inhibitors, such as MASP-2 antibodies, may be used to treat subjects suffering from, or at risk for developing a thrombotic microangiopathy (TMA), such as hemolytic uremic syndrome (HUS), atypical HUS (aHUS), or thrombotic thrombocytopenic purpura (TTP).

### EXAMPLE 34

This Example describes the effect of MASP-2 deficiency and MASP-2 inhibition in amurine FITC-dextran/light induced endothelial cell injury model of thrombosis.

Background/Rationale: As demonstrated in Examples 32 and 33, MASP-2 deficiency (MASP-2 KO) and MASP-2 inhibition (via administration of an inhibitory MASP-2 antibody) protects mice in a model of typical HUS, wherease all control mice exposed to STX and LPS developed severe HUS and became moribund or died within 48 hours. For example, as shown in FIGURE 67, all mice treated with a MASP-2 inhibitory antibody and then exposed to STX and LPS survived (Fisher's exact p<0.01; N=5). Thus, anti-MASP-2 therapy protects mice in this model of HUS.

The following experiments were carried out to analzye the effect of MASP-2 deficiency and MASP-2 inhibition in a fluorescein isothiocyanate (FITC)-dextran-induced endothelial cell injury model of thrombotic microangiopathy (TMA) in order to demonstrate further the benefit of MASP-2 inhibitors for the treatment of HUS, aHUS, TTP, and TMA's with other etiologies.

Methods:

### Intravital microscopy

Mice were prepared for intravital microscopy as described by Frommhold et al., BMC Immunology 12:56-68, 2011. Briefly, mice were anesthetized with intraperitoneal (i.p.) injection of ketamine (125 mg/kg bodyweight, Ketanest, Pfitzer GmbH, Karlsruhe, Germany) and xylazine (12.5 mg/kg body weight; Rompun, Bayer, Leverkusen, Germany) and placed on a heating pad to maintain body temperature at 37°C. Intravital microscopy was conducted on an upright microscope (Leica, Wetzlar, Germany) with a saline immersion objective (SW 40/0.75 numerical aperture, Zeiss, Jena, Germany). To ease breathing, mice were intubated using PE 90 tubing (Becton Dickson and Company, Sparks, MD, USA). The left carotid artery was cannuled with PE10 tubing (Becton Dickson and Company, Sparks, MD, USA) for blood sampling and systemic monoclonal antibody (mAb) administration.

### Cremaster muscle preparation

The surgical preparation of the cremaster muscle for intravital microscopy was performed as described by Sperandio et al., Blood, 97:3812-3819, 2001. Briefly, the scrotum was opened and the cremaster muscle mobilized. After longitudinal incision and spreading of the muscle over a cover glass, the epididymis and testis were moved and pinned to the side, giving full microscopic access to the cremaster muscle microcirculation. Cremaster muscle venules were recorded via a CCD camera (CF8/1; Kappa, Gleichen, Germany) on a Panasonic S-VHS recorder. The cremaster muscle was superfused with thermo-controlled (35°C bicarbonate-buffered saline) as previously described by Frommhold et al., BMCImmunology 12:56-68, 20112011.

### Light excitation FITC dextran injury model

A controlled, light-dose-dependent vascular injury of the endothelium of cremaster muscle venules and arterioles was induced by light excitation of phototoxic (FITC)-dextran (Cat. No. FD150S, Sigma Aldrich, Poole, U.K.). This procedure initiates localized thrombosis. As a phototoxic reagent, 60 μL of a 10% w/v solution of FITC-dextran was injected through the left carotid artery access and allowed to spread homogenously throughout the circulating blood for 10 minutes. After selecting a well-perfused venule, halogen light of low to midrange intensity (800-1500) was focused on the vessel of interest to induce FITC-dextran fluorescence and mild to moderate phototoxicity to the endothelial surface in order to stimulate thrombosis in a reproducible, controlled manner. The necessary phototoxic light intensity for the excitation of FITC-dextran was generated using a halogen lamp (12V, 100W, Zeiss, Oberkochen, Germany). The phototoxicity resulting from light-induced excitation of the fluorochrome requires a threshold of light intensity and/or duration of illumination and is caused by either direct heating of the endothelial surface or by generation of reactive oxygen radicals as described by Steinbauer et al., Langenbecks Arch Surg 385:290-298, 2000.

The intensity of the light applied to each vessel was measured for adjustment by a wavelength-correcting diode detector for low power measurements (Labmaster LM-2, Coherent, Auburn, USA). Off-line analysis of video scans was performed by means of a computer assisted microcirculation analyzing system (CAMAS, Dr. Zeintl, Heidelberg) and red blood cell velocity was measured as described by Zeintl et al., Int JMicrocirc Clin Exp, 8(3):293-302, 2000.

**Application of monoclonal anti-human MASP-2 inhibitory antibody (mAbH6) and vehicle control prior to induction of thrombosis**

Using a blinded study design, 9-week-old male C57BL/6 WT littermate mice were given i.p. injections of either the recombinant monoclonal human MASP-2 antibody (mAbH6), an inhibitor of MASP-2 functional activity (given at a final concentration of 10mg/kg body weight), or the same quantity of an isotype control antibody (without MASP-2 inhibitory activity) 16 hours before the phototoxic induction of thrombosis in the cremaster model of intravital microscopy. One hour prior to thrombosis induction, a second dose of either mAbH6 or the control antibody was given. MASP-2 knockout (KO) mice were also evaluated in this model.

mAbH6 (established against recombinant human MASP-2) is a potent inhibitor of human MASP-2 functional activity, which cross-reacts with, binds to and inhibits mouse MASP-2 but with lower affinity due to its species specificity (data not shown). In order to compensate for the lower affinity of mAbH6 to mouse MASP-2, mAbH6 was given at a high concentration (10mg/kg body weight) to overcome the variation in species specificity, and the lesser affinity for mouse MASP-2, to provide effective blockade of murine MASP-2 functional activity under *in vivo* conditions.

In this blinded study, the time required for each individual venuole tested (selection criteria were by comparable diameters and blood flow velocity) to fully occlude was recorded.

The percentage of mice with microvascular occlusion, the time of onset, and the time to occlusion were evaluated over a 60-minute observation period using intravital microscopy video recordings.

Results:

FIGURE 68 graphically illustrates, as a function of time after injury induction, the percentage of mice with microvascular occlusion in the FITC/Dextran UV model after treatment with isotype control or human MASP-2 antibody mAbH6 (10mg/kg) dosed at 16 hours and 1 hour prior to injection of FITC/Dextran. As shown in FIGURE 68, 85% of the wild-type mice receiving the isotype control antibody occluded within 30 minutes or less, whereas only 19% of the wild-type mice pre-treated with the human MASP-2 antibody (mAbH6) occluded within the same time period, and the time to occlusion was delayed in the mice that did eventually occlude in the human MASP-2 antibody-treated group. It is further noted that three of the MASP-2 mAbH6 treated mice did not occlude at all within the 60-minute observation period (i.e., were protected from thrombotic occlusion).

FIGURE 69 graphically illustrates the occlusion time in minutes for mice treated with the human MASP-2 antibody (mAbH6) and the isotype control antibody. The data are reported as scatter-dots with mean values (horizontal bars) and standard error bars (vertical bars). This figure shows the occlusion time in the mice where occlusion was observable. Thus, the three MASP-2 antibody-treated mice that did not occlude during the 60 minute observation period were not included in this analysis (there was no control treated mouse that did not occlude). The statistical test used for analysis was the unpaired t test; wherein the symbol "*" indicates p=0.0129. As shown in FIGURE 69, in the four MASP-2 antibody (mAbH6)-treated mice that occluded, treatment with MASP-2 antibody significantly increased the venous occlusion time in the FITC-dextran/light-induced endothelial cell injury model of thrombosis with low light intensity (800-1500) as compared to the mice treated with the isotype control antibody. The average of the full occlusion time of the isotype control was 19.75 minutes, while the average of the full occlusion time for the MASP-2 antibody treated group was 32.5 minutes.

FIGURE 70 graphically illustrates the time until occlusion in minutes for wild-type mice, MASP-2 KO mice, and wild-type mice pre-treated with human MASP-2 antibody (mAbH6) administered i.p. at 10mg/kg 16 hours before, and then administered again i.v. 1 hour prior to the induction of thrombosis in the FITC-dextran/light-induced endothelial cell injury model of thrombosis with low light intensity (800-1500). Only the animals that occluded were included in FIGURE 70; n=2 for wild-type mice receiving isotype control antibody; n=2 for MASP-2 KO; and n=4 for wild-type mice receiving human MASP-2 antibody (mAbH6). The symbol "*" indicates p<0.01. As shown in FIGURE 70, MASP-2 deficiency and MASP-2 inhibition (mAbH6 at 10mg/kg) increased the venous occlusion time in the FITC-dextran/light-induced endothelial cell injury model of thrombosis with low light intensity (800-1500).

Concl usi ons:

The results in this Example further demonstrate that a MASP-2 inhibitory agent that blocks the lectin pathway (e.g., antibodies that block MASP-2 function), inhibits microvascular coagulation and thrombosis, the hallmarks of multiple microangiopathic disorders, in a mouse model of TMA. Therefore, it is expected that administration of a MASP-2 inhibitory agent, such as a MASP-2 inhibitory antibody, will be an effective therapy in patients suffering from HUS, aHUS, TTP, or other microangiopathic disorders and provide protection from microvascular coagulation and thrombosis.

**EXAMPLE 35**

This Example describes a study demonstrating that human MASP-2 inhibitory antibody (mAbH6) has no effect on platelet function in platelet-rich human plasma.

Background/Rationale: As described in Example 34, it was demonstrated that MASP-2 inhibition with human MASP-2 inhibitory antibody (mAbH6) increased the venous occlusion time in the FITC-dextran/light-induced endothelial cell injury model of thrombosis. The following experiment was carried out to determine whether the MASP-2 inhibitory antibody (mAbH6) has an effect on platelet function.

Methods: The effect of human mAbH6 MASP-2 antibody was tested on ADP-induced aggregation of platelets as follows. Human MASP-2 mAbH6 at a concentration of either 1 $\mu$g/ml or 0.1 $\mu$g/ml was added in a 40 $\mu$L solution to 360 $\mu$L of freshly prepared platelet-rich human plasma. An isotype control antibody was used as the negative control. After adding the antibodies to the plasma, platelet activation was induced by adding ADP at a final concentration of 2 $\mu$M. The assay was started by stirring the solutions with a small magnet in the 1 mL cuvette. Platelet aggregation was measured in a two-channel Chronolog Platelet Aggregometer Model 700 Whole Blood/Optical Lumi-Aggregometer.

Results:

The percent aggregation in the solutions was measured over a time period of five minutes. The results are shown below in TABLE 22.

TABLE 22: Platelet Aggregation over a time period of five minutes.

| Antibody | Amplitude (percent aggregation) | Slope (percent aggregation over time) |
|---|---|---|
| MASP-2 antibody (mAbH6) (1 $\mu$g/ml) | 46% | 59 |
| Isotype control antibody (1 $\mu$g/ml) | 49% | 64 |
| | | |
| MASP-2 antibody (mAbH6) (0.1 $\mu$g/ml) | 52% | 63 |
| Isotype control antibody (0.1 $\mu$g/ml) | 46% | 59 |

As shown above in TABLE 22, no significant difference was observed between the aggregation of the ADP-induced platelets treated with the control antibody or the MASP-2 mAbH6 antibody. These results demonstrate that the human MASP-2 antibody (mAbH6) has no effect on platelet function. Therefore, the results described in Example 34 demonstrating that MASP-2 inhibition with human MASP-2 inhibitory antibody (mAbH6) increased the venous occlusion time in the FITC-dextran/light-induced endothelial cell injury model of thrombosis, were not due to an effect of mAbH6 on platelet function. Thus, MASP-2 inhibition prevents thrombosis without directly impacting platelet function, revealing a therapeutic mechanism that is distinct from existing anti-thrombotic agents.

While the preferred embodiment of the invention has been illustrated and described, it will be appreciated that various changes can be made therein without departing from the spirit and scope of the invention.

The invention includes the following aspects:

1. A method of inhibiting MASP-3-dependent complement activation in a subject suffering from, or at risk for developing, a disease or disorder selected from the group consisting of age-related macular degeneration, arthritis, disseminated intravascular coagulation, thrombotic microangiopathy, asthma, dense deposit disease, pauci-immune necrotizing crescentic glomerulonephritis, traumatic brain injury, aspiration pneumonia, endophthalmitis, neuromyelitis optica and Behcet's disease comprising administering to the subject a composition comprising an amount of a MASP-3 inhibitory agent effectiveto inhibit MASP-3-dependent complement activation.

2. The method of Aspect 1, wherein the MASP-3 inhibitory agent is a MASP-3 monoclonal antibody, or fragment thereof that specifically binds to a portion of SEQ ID NO:8.

3. The method of Aspect 1, further comprising administering to the subject a composition comprising a MASP-1 inhibitory agent.

4. The method of Aspect 3, wherein the MASP-1 inhibitory agent is a MASP-1 monoclonal antibody, or fragment thereof, that specifically binds to a portion of SEQ ID NO:10.

5. The method of Aspect 1, further comprising administering to the subject a composition comprising a MASP-2 inhibitory agent.

6. The method of Aspect 5, wherein the MASP-2 inhibitory agent is a MASP-2 monoclonal antibody, or fragment thereof that specifically binds to a portion of SEQ ID NO:5.

7. The method of Aspect 1, further comprising administering to the subject a composition comprising a MASP-1 inhibitory agent and a MASP-2 inhibitory agent.

8. The method of Aspect 1, wherein the MASP-3 inhibitory agent inhibits alternative-pathway driven C3b deposition.

9. The method of Aspect 1, wherein the MASP-3 inhibitory agent inhibits factor D maturation.

10. The method of Aspect 3, wherein the MASP-1 inhibitory agent specifically binds to a portion of MASP-1 with an affinity of at least 10 times greater than it binds to MASP-3 (SEQ ID NO:8).

11. The method of Aspect 3, wherein the MASP-1 inhibitory agent specifically binds to the serine protease domain of MASP-1 (aa 449-694 of SEQ ID NO:10).

12. The method of Aspect 1, wherein the MASP-3 inhibitory agent also binds to a portion of MASP-1 (SEQ ID NO:10).

13. The method of Aspect 12, wherein the MASP-3 inhibitory agent is a dual MASP-1/MASP-3 inhibitor that binds to a consensus region within the CUBI-CCP2 domains.

14. The method of Aspect 12, wherein the MASP-3 inhibitory agent is a dual MASP-1/MASP-3 inhibitor that binds to a consensus region within the CCP2 domain.

15. The method of Aspect 12, wherein the MASP-3 inhibitory agent is a bispecific monoclonal antibody that binds to the serine protease domain of MASP-1 (aa 449-694 of SEQ ID NO:10) and also binds to the serine protease domain of MASP-3 (aa 450-711 of SEQ ID NO:8).

16. The method of Aspect 1, wherein the MASP-3 inhibitory agent also binds to a portion of MASP-2 (SEQ ID NO:5).

17. The method of Aspect 16, wherein the MASP-3 inhibitory agent is a dual MASP-3/MASP-2 inhibitor that binds to a conserved region within the serine protease domains of MASP-3 and MASP-2.

18. The method of Aspect 16, wherein the MASP-3 inhibitory agent is a bispecific monoclonal antibody that binds to the serine protease domain of MASP-3 (aa450-711 of SEQ ID NO:8) and also binds to at least one of the serine protease domain of MASP-2 (aa 445-682 of SEQ ID NO:5) or the CCP-1-CCP2 domain of MASP-2 (aa300-431 of SEQ ID NO:5).

19. The method of Aspect 1, wherein the MASP-3 inhibitory agent binds to a portion of MASP-3 (SEQ ID NO:8) and does not inhibit MASP-1 or MASP-2.

20. The method of Aspect 1, wherein the MASP-3 inhibitory agent specifically binds to a portion of MASP-3 with an affinity of at least 10 times greater than it binds to MASP-1 (SEQ ID NO:10).

21. The method of Aspect 1, wherein the MASP-3 inhibitory agent specifically binds to the serine protease domain of MASP-3 (aa 450-711 of SEQ ID NO:8).

22. The method of Aspect 1, wherein the MASP-3 inhibitory agent is a pan inhibitor of MASP-1, MASP-2 and MASP-3 and binds to a conserved region in the CUB 1-EGF-CUB2 domains.

23. The method of Aspect 1, wherein the MASP-3 inhibitory agent is a trispecific inhibitor of MASP-1, MASP-2 and MASP-3.

24. The method of Aspect 2, wherein the composition further comprises a MASP-2 antibody.

25. The method of Aspect 2, wherein the composition further comprises a MASP-1 antibody.

26. The method of Aspect 7, wherein the composition comprises a MASP-1 antibody, and MASP-2 antibody and a MASP-3 antibody.

27. The method of Aspect 7, wherein the method comprises co-administration of a composition comprising at least one of a MASP-2 antibody, a MASP-1 antibody, or a MASP-3 antibody.

28. The method of Aspect 12, wherein the composition further comprises a MASP-2 antibody.

29. The method of Aspect 16, wherein the composition further comprises a MASP-1 antibody.

30. The method of Aspect 1, wherein the antibody or fragment thereof is selected from the group consisting of a recombinant antibody, an antibody having reduced effector function, a chimeric, and a humanized or human antibody.

31. The method of Aspect 1, wherein the composition is administered systemically.

32. The method of Aspect 31, wherein the composition is administered subcutaneously, intra-muscularly, intravenously, intra-arterially or as an inhalant.

33. The method of Aspect 1, wherein the subject is suffering from, or at risk for developing age-related macular degeneration.

34. The method of Aspect 1, wherein the subject is suffering from, or at risk for developing arthritis.

35. The method of Aspect 34, wherein the arthritis is selected fronm the group consisting of osteoarthritis, rheumatoid arthritis, juvenile rheumatoid arthritis and psoriatic arthritis.

36. The method of Aspect 1, wherein the subject is suffering from, or at risk for developing disseminated intravascular coagulation.

37. The method of Aspect 36, wherein the disseminated intravascular coagulation is secondary to sepsis, trauma, infection (bacterial, viral, fungal, parasitic), malignancy, transplant rejection, transfusion reaction, obstetric complication, vascular aneurysm, hepatic failure, heat stroke, burn, radiation exposure, shock, or severe toxic reaction.

38. The method of Aspect 1, wherein the subject is suffering from, or at risk for developing a thrombotic microangiopathy.

39. The method of Aspect 38, wherein the thrombotic microangiopathy is selected from the group consisting of hemolytic uremic syndrome (HUS), atypical hemolytic uremic syndrome (aHUS) and thrombotic thrombocytopenic purpura (TTP).

40. The method of Aspect 1, wherein the subject is suffering from, or at risk for developing asthma.

41. The method of Aspect 1, wherein the subject is suffering from, or at risk for developing dense deposit disease.

42. The method of Aspect 1, wherein the sugject is suffering from, or at risk for developing pauci-immune necrotizing crescentic glomerulonephritis.

43. The method of Aspect 1, wherein the subject is suffering from, or at risk for developing traumatic brain injury.

44. The method of Aspect 1, wherein the subject is suffering from, or at risk for developing aspiration pneumonia.

45. The method of Aspect 1, wherein the subject is suffering from, or at risk for developing endophthalmitis.

46. The method of Aspect 1, wherein the subject is suffering from, or at risk for developing neuromyelitis optica.

47. The method of Aspect 1, wherein the subject is suffering from, or at risk for developing Behcet's disease.

48. A method of inhibiting MASP-2-dependent complement activation in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica and Behcet's disease, comprising administering to the subject a composition comprising an amount of a MASP-2 inhibitory agent effective to inhibit MASP-2 dependent complement activation.

49. The method of aspect 48, wherein the MASP-2 inhibitory agent is a MASP-2 antibody or fragment thereof.

50. The method of Aspect 48, wherein the MASP-2 inhibitory agent is a MASP-2 monoclonal antibody, or fragment thereof that specifically binds to a portion of SEQ ID NO:5.

51. The method of Aspect 46, wherein the MASP-2 antibody is a chimeric, humanized or human antibody.

52. A method of manufacturing a medicament for use in inhibiting the effects of MASP-3-dependent complement activation in a subject suffering from, or at risk for developing a disease or disorder selected from the group consisting of age-related macular degeneration, arthritis, disseminated intravascular coagulation, thrombotic microangiopathy, asthma, dense deposit disease, pauci-immune necrotizing crescentic glomerulonephritis, traumatic brain injury, aspiration pneumonia, endophthalmitis, neuromyelitis optica and Behcet's disease.

53. The method of aspect 52, further comprising combining a therapeutically effective amount of a MASP-2 inhibitory agent with the medicament comprising the MASP-3 inhibitor.

54. A method of manufacturing a medicament for use in inhibiting the effects of MASP-2-dependent complement activation in a subject suffering from, or at risk for developing a disease or disorder selected from the group consisting of dense deposit disease, pauci-immune necrotizing crescentic glomerulonephritis, traumatic brain injury, aspiration pneumonia, endophthalmitis, neuromyelitis optica and Behcet's disease, comprising combining a therapeutically effective amount of a MASP-2 inhibitory agent in a pharmaceutical carrier.

55. The method of aspect 54, further comprising combining a therapeutically effective amount of a MASP-3 inhibitory agent with the medicament comprising the MASP-2 inhibitor.

**Claims**

1. A composition comprising an amount of a MASP-2 inhibitory agent effective to inhibit MASP-2-dependent complement activation for use in the treatment of a subject suffering from, or at risk for developing, a disease or disorder selected from the group consisting of traumatic brain injury, aspiration pneumonia, endophthalmitis, neuromyelitis optica and Behcet's disease.

2. The composition for use according to claim 1, wherein the MASP-2 inhibitory agent is a MASP-2 antibody or fragment thereof.

3. The composition for use according to Claim 2, wherein the MASP-2 inhibitory agent is a MASP-2 monoclonal antibody, or fragment thereof that specifically binds to a portion of SEQ ID NO: 5.

**4.** The composition for use according to Claim 2, wherein the MASP-2 antibody is a chimeric, humanized or human antibody.

**5.** The composition for use according to Claim 1, further comprising combining a therapeutically effective amount of a MASP-3 inhibitory agent.

FIG. 1

MASP 2

FIG. 2

FIG. 3

1　　　　　　　　　　　　　　　　　　　　　　　　　　　　　　　　　　　90
MASP1　MRWLLLYYALCFSLSKASAHTVELNNMFGQIQSPGYPDSYPSDSEVTWNITVPDGFRIKLYFMHFNLESSYLCEYDYVKVETEDQVLATF
MASP3　..................
MASP2　..L.T.LGL..G.VATPLGPKWP-EPV..RLA...F.GE.AN.Q.RR.TL.A.P.Y.LR...T..D..L.H.....F..LSSGAK....L

91　　　　　　　　　　　　　　　　　　　　　　　　　　　　　　　　　　　180
MASP1　CGRETTDTEQTPGQEVVLSPGSFMSITFRSDFSNEERFTGFDAHYMAMDVDECKEREDEELSCDHYCHNYIGGYYCSCRFGYILHTDNRT
MASP3　...................
MASP2　..Q.S....RA..KDTFY.L..SLD......Y...KP....E.F.A.E.I...QVAPG.APT...H...HL..F.....A..V..RNK..

181　　　　　　　　　　　　　　　　　　　　　　　　　　　　　　　　　270
MASP1　CRVECSDNLFTQRTGVITSPDFPNPYPKSSECLYTIELEEGFMVNLQFEDIFDIEDHPEVPCPYDYIKIKVGPKVLGPFCGEKAPEPIST
MASP3　...................
MASP2　.SAL..GQV....S.ELS..EY.R....L.S.T.S.S.....S.I.D.VES..V.T...TL....FL..QTDREEH.....KTL.HR.E.

271　　　　　　　　　　　　　　　　　　　　　　　　　　　　　　　　360
MASP1　QSHSVLILFHSDNSGENRGWRLSYRAAGNECPELQPPVHGKIEPSQAKYFFKDQVLVSCDTGYKVLKDNVEMDTFQIECLKDGTWSNKIP
MASP3　...................
MASP2　K.NT.T.T.VT.E..DHT..KIH.TSTAQP..YPMA.PN.HVS.V....IL..SFSIF.E...EL.QGKLPLKS.TAV.Q...S.DRPM.

361　　　　　　　　　　　　　　　　　　　　　　　　　　　　　　　　448
MASP1　TCKIVDCRAPGELEHGLITFSTRNNLTTYKSEIKYSCQEPYYKMLNNNTGIYTCSAQGVWMNKVLGRSLPTCLPVCGLPKFSR-KLMA
MASP3　.................................................................E..Q.SR.LPS.VK
MASP2　A.S....GP.DD.PS.RVEYI.GPGV....AV.Q...E.TF.T.KV.D-.K.V.E.D.F.TSSKGEK...V.E.....SARTT---GG

449　　　　　　　　　　　　　　　　　　　　　　　　　　　　　　　　540
MASP1　RIFNGRPAQKGTTPWIAML-----SHL-NGQPFCGGSLLGSSWIVTAAHCLHQSLDPEDPTLRDSDLLSPSDFKIILGKHWRLRSDENEQHL
MASP3　RIIG..N.EP.LF..Q.LIVVEDT.RVP.DKW.GS.A.,SA...L...V.RSQR--R.T.VIP---V.KEHVTVY..L.-DV.DKSGAVNS
MASP2　RIYG.QK.KP.DF..QVLIL---------.GTTAA.A..YDN.VL...AVYEQK--H.ASAL.---IRMGTL.RLSPHYTQAW.EAVFI.E

541　　　　　　　　　　　　　　　　　　　　　　　　　　　　　　　　630
MASP1　GVKHTTLHPQYDPNTFENDVALVELLESPVLNAFVMPICLPEGPQQE-GA---MVIVSGWG------------KQFLQRFPETLMEIEIP
MASP3　SAARVV...DFNIQNYNH.-I...Q.Q.PVP.GPH...V...RLEPEG-P.PHML.GL.A...ISNPNVTVDEIISSGTR.TLSDV.QYVKL.
MASP2　.YT.DA--------G.D.II...IK.NNKV.I.SNIT.....RKEAESFMRTDDIGTA....L------------TQRGFLARN..YVD..

631　　　　　　　　　　　　　　　　　　　　　　　　　　　　　　　　720
MASP1　IVDHSTCOKAYA-PLKKK--VTRDMICAGEKEGGKDACAGDGIGGPMVTLNRERGQWYLVGTVSWG--DDCGKKDRYGVYYSYIHHNKDWIQ
MASP3　V.P.AE.KTS.E-SRSGNYS..EN.F...YY.....T.L....AF.IFDDLSQK.VVQ.L....GPEE..S.QV.....TKVSNYV..VW
MASP2　....QK.TA..EK.PYPRGS..AN.L...LES....S.R.II.AL.F.DS.TER.FVG.I....S-MN..EAGQ.....TKVINYIP..E

721　　　　736
MASP1　RVTGVRN
MASP3　EQM.LPQSVVEPQVER
MASP2　NISDF

CUB/EGF/
CCP domain
(A-Chain)

serine
protease
domain
(B-Chain)

FIG. 4

CUB1 and CUB2 domains
EGF domain
CCP1 and CCP2 domains
catalytic triad residues

Alignment of MASP A-Chains

```
                1                                                                       90
MASP1_Achain   MRWLLLYYALCFSLSKASAHTVELNNMFGQIQSPGYPDSYPSDSEVTWNITVPDGFRIKLYFMHFNLESSYLCEYDYVKVETEDQVLATF
MASP3_Achain   ..........................................................................................
MASP2_Achain   ..L.T.LGL..G.VATPLGPKWP-EPV..RLA...F.GE.AN.Q.RR.TL.A.P.Y.LR    T   D   L.H      F..LSSGAK    L

                91                                                                      180
MASP1_Achain   CGRETTDTEQTPGQEVVLSPGSFMSITFRSDFSNEERFTGFDAHYMAVDVDECKEREDEELSCDHYCHNYIGGYYCSCRFGYILHTDNRT
MASP3_Achain   ..........................................................................................
MASP2_Achain     Q.S    RA..KDTFY.L..SLD      Y   KP    E.F.A.E.I   QYAPG.APT   H   HL   F     A   V..RNK..

                181                                                                     270
MASP1_Achain   CRVECSDNLFTQRTGVITSPDFPNPYPKSSECLYTIELEEGFMVNLQFEDIFDIEDHPEVPCPYDYIKIKVGPKVLGPFCGEKAPEPIST
MASP3_Achain   ..........................................................................................
MASP2_Achain   .SAL..GQV....S.ELS..EY.R....L.S.T.S.S    S.I.D.VES..V.T   TL   FL..QTDREEH     KTL.HR.E.

                271                                                                     360
MASP1_Achain   QSHSVLILFHSDNSGENRGWRLSYRAAGNECPELQPPVHGKIEPSQAKYFFKDQVLVSCDTGYKVLKDNVEMDTFQIECLKDGTWSNKIP
MASP3_Achain   ..........................................................................................
MASP2_Achain   K.NT.T.T.VT.E..DHT..KIH.TSTAQP..YPMA.PN.HVS.V....IL..SFSIF.E...EL.QGHLPLKS.TAV.Q...S.DRPM.

                361                                                         448
MASP1_Achain   TCKIVDCRAPGELEHGLITFSTRNNLTTYKSEIKYSCQEPYYKMLNNNTGIYTCSAQGVWMNKVLGRSLPTCLPVCGLPKFSRKLMA
MASP3_Achain   .........................................................................E..Q.SR.LPSLVK
MASP2_Achain   A.S....GP.DD.PS.RVEYI.GPGV....AV.Q...E.TF.T.KV.D-.K.V.E.D.F.TSSKGEK...V.E.....SARTTGG
```

FIG. 5

EP 4 119 577 A2

## Alignment of MASP Serine Protease Domains (B-Chains)

```
              1                                                                                    90
MASP1_Bchain  RIFNGRPAQKGTTPWIAMLSHLNGQP------FCGGSLLGSSWIVTAAHCLHQSLDPEDPTLRDSDLLSPSDFKIILGKHWRLRSDENEQ
MASP2_Bchain  ..YG.QK.KP.DF..QVLILGGTTA---------A.A..YDN.VL....AVYEQ--KH.ASAL.---I-RMGTLKR.SP.Y-TQAWSEAV
MASP3_Bchain  ..IG..N.EP.LF..Q.LIVVEDTSRVPNDKW.GS.A..SA...L....V.RSQ--RR.T.VIP---V.KEHVTVY..L.D-V.DKSGAV

              91                                                                                   180
MASP1_Bchain  HLGVKHTTLHPQYDPNTFENDVALVELLESPVLNAFVMPICLP----EGPQQEGAMVIVSGWGKQFLQR-----------FPETLMEIE
MASP2_Bchain  ------FIHEGYTHDAG.D..I..IK.NNKV.I.SNIT.....RKEA.SFMRTDDIGTA....LTQRGF-----------LARN..YVD
MASP3_Bchain  NSSAARVV...DFNIQNYNH.I....Q.Q.PVP.GPH...V...RLEP..-PAPHMLGL.A...ISNPNVTVDEIISSGTRTLSDV.QYVK

              181                                                                                  270
MASP1_Bchain  IPIVDHSTCQKAYAPL---KKKVTRDMICAGEKEGGKDACAGDSGGPMVTLNRERGQWYLVGTVSWG--DDCGKKDRYGVYSYIHHNKDW
MASP2_Bchain  ......QK.TA..EKPPYPRGS..AN.L____LES____S.R____AL.F.DS.TER.FVG.I____SMN-__EAGQ____TKVINYIP.
MASP3_Bchain  L.V.P.AE.KTS.ESRS-GNYS..EN.F____YY____T.L____AF.IFDDLSQR.VVQ.L____GPEE__S.QV____TKVSNYV..

              271          288
MASP1_Bchain  IQRVTGVRN
MASP2_Bchain  .ENISDF
MASP3_Bchain  VWEQM.LPQSVVEPQVER
```

FIG. 6

EP 4 119 577 A2

## Pairwise Alignment of MASP Serine Protease Domains (B-Chains)

EP 4 119 577 A2

1                                                                                                          90
MASP1_Bchain  RIFNGRPAQKGT TP WI AML S H LNG QPFCGGSLLGSSW I VTAAHC LHQ S LDP EDPT LRDSDLLS P SDFKI I LGKHWRL RSDENEQH L G V KH
MASP2_Bchain  . . YG . QK . K P . DF .  . QV L I L - - - . GTTAA . A . . YDN . VL . . . . AVY E Q - - KH . ASAL . - - - I RMGTL . RLSPHY T QAW. EAVF I . E . Y T .

91                                                                                                         180
MASP1_Bchain  TTLHPQYDPNT F ENDV ALV ELLES PVLNAFVMP I CLP - - - - EGPQQEGAMV I VSGWGKQF LQRF P ETLME I E I P I V DHS TCQKAYAPL - -
MASP2_Bchain  DA - - - - - - - - G . D . . I . . I K . NNKV . I . SNI T⸬⸬⸬⸬RKEA . SFMR TDD I GTA⸬⸬⸬⸬L TQRG F LARN⸬⸬YVD⸬⸬⸬⸬⸬QK . TA . . EKPPY

181                                                                                                        260
MASP1_Bchain  - KKKVTRDM I CAGE KE GGKD A CAGDS GGPMVTLNRERGQWY LVGTVSWGD - DCGKKDRYGVYSY I HHNKDW I QR VTGVRN
MASP2_Bchain  PRGS . . AN . L . . . LES . . . . S . R . . . . . AL . F . DS . TER . FVG. I . . . . SMN . . EAGQ . . . . TKV INY I P . . EN I SDF

## FIG. 7A

EP 4 119 577 A2

Pairwise Alignment of MASP Serine Protease Domains (B-Chains)

```
             1                                                                          90
MASP1_Bchain  R I FNGRPAQKGTTPWI AML - - - - - SHL - NGQ PFCGGS LLGSSWI VTA AHCLHQS LDPEDPTL R DSDLLSPSDF K I I LGKHWRLRS D E N E Q
MASP2_Bchain  . . IG . . N . EP . LF . . Q . L IVVEDT . RVP . DKW. GS . A . . SA⸏⸏⸏L⸏⸏⸏⸏V . RSQR - - R . T . V I P - - - V. KEHVTVY . . L . - DV. DK S G AV


             91                                                                         180
MASP1_Bchain  HLGVKHTTLHPQYDP N T FENDVA LVE LLESP V LNAFVMP I CLPE GPQQEGA- - - MV IVSGWG - - - - - - - - - - -KQFLQRFPETLMEI E I
MASP2_Bchain  NSSAARVV. . . DFN I QNYNH . I . . . Q. Q. PVP . GPH . . . V . . . R LEPEGP. PHM LGL. A . . . I SN PNVTVDEI ISSGTRTLSDV. QYVK L


             181                                                                        270
MASP1_Bchain  P IVDHS TCQKAY A PLKKK - -VTRDMICAGEKEGGKDACAGDSGGPMVTLNRE R GQW Y LVGTVSWG - - DDCGKKDRYGVYSY I HHNKDWI Q
MASP2_Bchain  . V . P . AE . KTS . ESRSGNYS . . EN . F⸏⸏⸏YY⸏⸏⸏⸏T . L⸏⸏⸏⸏AF . IFDDLSQR . VVQ. L⸏⸏⸏⸏GPEE . . S . QV⸏⸏⸏⸏TKVSNYV . .VW


             271            286
MASP1_Bchain  RV TGVRN
MASP2_Bchain  EQM. LPQS VVEPQVER
```

## FIG. 7B

Pairwise Alignment of MASP Serine Protease Domains (B-Chains)

```
                 1                                                                                    90
MASP2_Bchain   RIYGGQKAKPGDFPWQVLILGCTTA--------- ---AGALLYDNWVLTAAHAVYEQKHDASALDI-RMGTLKRLSPHYTQAWSEAV------
MASP3_Bchain   ..I RN.E L      A  VVED.SRVPNDKWFGS     SAS I.....VLRS.RR.TTVIPVSKEHVTVY.GL.DVRDK.G..NSSAAR

                 91                                                                                   180
MASP2_Bchain   FIHEGYTHDAGFDNDIALIKLNNKVVINSNITPICLPRKEAESFMRTDDIGTASGWGLTQRGF-----------LARNLMYVDIPIVDH
MASP3_Bchain   VVLHPDFNIQNYNH     VQ.QEP.PLGPHVM.V    L.P.G-PAPHML.LVA...ISNPNVTVDEIISSGTRT.SDV.Q..KL.V.P.

                 181                                                                                  270
MASP2_Bchain   CKCTAAYEKPPYPRGSVTANMLCAGLESGGKDSCRGDSGGALVFLDSETERWVGGIVSWGSMN-CGEAGQYGVYTKVINYIPWIENISD
MASP3_Bchain   AE.KTS..SRS-GNY   E  F   YYE     T.L     F.IF.DLSQ...Q.L....GPEE..SKCV         S..VD.VWEQMVG

                 271        282
MASP2_Bchain   F
MASP3_Bchain   LPQSVVEPQVER
```

FIG. 7C

Survival after N. meningitidis Serogroup A Z2491 infection

FIG. 8

FIG. 9

FIG. 10

FIG. 11

FIG. 12

FIG. 13

Complement dependent killing of *Neisseria meningitidis* in human 20% v/v serum

Legend:
- --■-- A (NHS)
- ---○--- B (Heat Inactivated NHS)
- --▲-- C (MBL -/-)
- —▼— D (MASP-3 -/-)

FIG. 14

**FIG. 15**

MASP-1/3 deficient mice are not deficient of LP functional activity

**FIG. 16**

FIG. 17

FIG. 18

FIG. 19A

FIG. 19B

FIG. 19C

FIG. 20A

FIG. 20B

FIG. 20C

Haemolysis of mannan-coated Murine RBC by human serum

FIG. 21

Haemolysis of mannan-coated Murine RBC by human serum

FIG. 22

Haemolysis of WT murine RBC by human serum

FIG. 23

Haemolysis of CD55/59 -/- murine RBC by human serum

FIG. 24

Haemolytic assay using Rabbit RBCs with WT and MASP-1/3 $^{-/-}$ mouse sera.

FIG. 25

FIG. 26

FIG. 27

FIG. 28

FIG. 29A

FIG. 29B

FIG. 30A

FIG. 30B

Kd = 31 nM

FIG. 31

# FIG. 32A

```
       1                                                              65
VH     AVTLDESGGGLQTPGGALSLVCKASGFTFS SNAMG WVRQAPGKGLEWVA GID-DDGSGTRYAPAV
M3J5   ...............G.............. .Y... M........Y... R-S...F.L..T..
M3M1   ...............G..........D.. .YQ.N I.........F.. A.N-RF.NS.GHGA..
D14    ............................. .Y.H. ..........   .Y-KS.A..N....
1E10   ............................. .YD.V .........F.. ..SRN..RY.E.GS..

       66                                                             129
VH     KG RATISRDNGQSTLRLQLNNLRAEDTGTYYCTK CAYSS-GCDYEGG--YIDA WGHGTEVIVSS
M3J5   .. .............V.............A.F..R SG--------NV.--D... ..........
M3M1   .. .V.....D...V...S.....A....A GV.GYC.SYSCC.VDT... ..........
D14    .. .............V...........A TTG.GCSSG.RAE-- .... ..........
1E10   .. .............V...........A RD.GG.-AYWFDA.--Q... ..........
```

# FIG. 32B

```
       1                                                    54
VL     ALTQPASVSANLGGTVKITC SGGGS-YAGSYYYG WQQKSPGSAPVTVIY DNDK
M3J5   ..........P.E....... ..Y.G........ .......A.....L. .Y.N.
M3M1   ..........P.E....... .......-........ .......A.....L. .Y.N.
D14    ..........P.E....... .......-........ .......A.....L. .Y.N.
1E10   ..........P.E....... .......-........ .......A.....L. .Y.N.

       55                                                    108
VL     RPS DIPSRFSGSLSGSTNTLTITGVRADDEAVYFC GSADNSGAA FGAGTTLTVL
M3J5   ... ................................ ......... .........
M3M1   ... ................................ ......... .........
D14    ... ................................ ......... .........
1E10   ... ................................ ......... .........
```

FIG. 33

FIG. 34

C3b Deposition on *S. Aureus*

FIG. 35A

C3b Deposition on *S. Aureus*

FIG. 35B

Factor B cleavage in response to *S. aureus*

Complementation of 3MC serum with recombinant
MASP-3 does increase Factor B cleavage.

## FIG. 36

MASP-3 directly cleaves pro-Bf

| | 1 | 2 | 3 | 4 | 5 | 6 | 7 |
|---|---|---|---|---|---|---|---|
| C3 | + | - | + | + | + | + | - |
| pro-Bf | + | + | - | + | + | - | + |
| MASP-3 | + | + | + | + | - | - | - |
| pro-Df | + | + | + | - | + | - | - |

## FIG. 37

FIG. 38

MASP-1 and MASP-3 activate pro-Df

FIG. 39

Partial inhibition of MASP-3 dependent
Cleavage of Pro-Df with MASP-3 antibodies

FIG. 40

FIG. 41

FIG. 42A

Human
DF(25kDa)

FIG. 42B

FIG. 42C

FIG. 43

FIG. 44

FIG. 45

FIG. 46

FIG. 47

FIG. 48A

FIG. 48B

FIG. 49

FIG. 50

FIG. 51A

FIG. 51B

FIG. 52A

FIG. 52B

FIG. 52C

FIG. 52D

FIG. 53A

FIG. 53B

FIG. 54A

FIG. 54B

FIG. 55A

FIG. 55B

FIG. 55C

FIG. 55D

FIG. 56A

FIG. 56B

FIG. 57A

FIG. 57B

FIG. 57C

FIG. 57D

FIG. 58

WT (+/+)

FIG. 59A

MASP-2 (-/-)

FIG. 59B

FIG. 60

C3 deposition on Dust Mite

FIG. 61

FIG. 62A

FIG. 62B

FIG. 63A

FIG. 63B

FIG. 64

FIG. 65

FIG. 66

Survival of STX/LPS induced HUS

FIG. 67

mAbH6 inhibits thrombus formation in the
mouse model of FITC-Dextran induced
thrombotic microangiopathy

FIG. 68

FIG. 69

FIG. 70

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 61661167 A
- US 7919094 B
- WO 2004106384 A
- WO 9111465 A, Goldenberg
- US 4816567 A, Cabilly
- US 6355245 B
- US 5693762 A, Queen
- US 4331647 A, Goldenberg
- US 4946778 A, Ladner
- US 5223409 A, Lardner
- US 5403484 A, Lardner
- US 5571698 A, Lardner
- WO 9627011 A
- US 4676980 A
- WO 9413804 A
- WO 08024188 A
- WO 07024715 A
- US 5211657 A, Yamada
- WO 2004009664 A2
- US 20020019369 A1
- US 5741516 A, Webb
- US 5567434 A, Szoka
- US 5552157 A, Yagi
- US 5565213 A, Nakamori
- US 5738868 A, Shinkarenko
- US 5795587 A, Gao
- WO 9523161 A
- US 20070172483 A
- WO 2009029315 A
- US 2010093033 A

### Non-patent literature cited in the description

- **M.K. LISZEWSKI ; J.P. ATKINSON.** Fundamental Immunology. Raven Press, Ltd, 1993
- **K.R. KALLI et al.** *Springer Semin. Immunopathol.,* 1994, vol. 15, 417-431
- **B.P. MORGAN.** *Eur. J. Clinical Investig.,* 1994, vol. 24, 219-228
- **J. LU et al.** *Biochim. Biophys. Acta,* 2002, vol. 1572, 387-400
- **HOLMSKOV et al.** *Annu. Rev. Immunol.,* 2003, vol. 21, 547-578
- **TEH et al.** *Immunology,* 2000, vol. 101, 225-232
- **J. LU ET al.** *Biochim Biophys Acta,* 2002, vol. 1572, 387-400
- **HOLMSKOV et al.** *Annu Rev Immunol,* 2003, vol. 21, 547-578
- **HANSEN et al.** *J. Immunol,* 2010, vol. 185 (10), 6096-6104
- **IKEDA et al.** *J. Biol. Chem.,* 1987, vol. 262, 7451-7454
- **WEIS et al.** *Nature,* 1992, vol. 360, 127-134
- **LEE et al.** *Archiv. Biochem. Biophys.,* 1992, vol. 299, 129-136
- **MAYNARD et al.** *J. Biol. Chem.,* 1982, vol. 257, 3788-3794
- **PALANIYAR et al.** *Ann. N.Y. Acad. Sci.,* 2003, vol. 1010, 467-470
- **NAKAMURA et al.** *J. Leuk. Biol.,* 2009, vol. 86, 737-748
- **LYNCH et al.** *J. Immunol.,* 2004, vol. 172, 1198-1202
- **THOMSEN et al.** *Mol. Immunol.,* 2011, vol. 48 (4), 369-81
- **JACK et al.** *J Leukoc Biol.,* 2004, vol. 77 (3), 328-36
- **MATSUSHITA ; FUJITA.** *Immunobiology,* 2002, vol. 205 (4-5), 490-7
- **AOYAGI et al.** *J Immunol,* 2005, vol. 174 (1), 418-25
- **KUHLMAN et al.** *J. Exp. Med.,* 1989, vol. 169, 1733
- **MATSUSHITA et al.** *J. Biol. Chem.,* 1996, vol. 271, 2448-54
- **MATSUSHITA et al.** *J Exp Med,* 1992, vol. 176 (6), 1497-1502
- **JI et al.** *J. Immunol.,* 1993, vol. 150, 571-578
- **THIEL et al.** *Nature,* 1997, vol. 386, 506-510
- **VORUP-JENSEN et al.** *J. Immunol.,* 2000, vol. 165, 2093-2100
- **AMBRUS et al.** *J. Immunol.,* 2003, vol. 170, 1374-1382
- **DAHL, M.R. et al.** *Immunity,* 2001, vol. 15, 127-35
- **SIM et al.** *Biochem. Soc. Trans.,* 2000, vol. 28, 545
- **STOVER.** *J. Immunol.,* 1999, vol. 162, 3481-90

- **TAKAHASHI et al.** *Int. Immunol.,* 1999, vol. 11, 859-863
- **DEGN et al.** *J Immunol. Methods,* 2011
- **SCHWAEBLE et al.** *Immunobiology,* 2002, vol. 205, 455-466
- **THIEL et al.** *J. Immunol.,* 2000, vol. 165, 878-887
- **DAHL et al.** *Immunity,* 2001, vol. 15, 127-35
- **MATSUSHITA et al.** *J. Immunol.,* 2002, vol. 168, 3502-3506
- **KILPATRICK.** *Biochim. Biophys. Acta,* 2002, vol. 1572, 401-413
- **COLLARD et al.** *Am. J. Pathol.,* 2000, vol. 156, 1549-1556
- **JORDAN et al.** *Circulation,* 2001, vol. 104, 1413-1418
- **COLLARD et al.** *Am. J. Pathol.,* 2001, vol. 159, 1045-1054
- **RIEDERMANN, N.C. et al.** *Am. J. Pathol.,* 2003, vol. 162, 363-367
- **TAKAHASHI M. et al.** *J Exp Med,* 2010, vol. 207 (1), 29-37
- **IWAKI et al.** *J. Immunol.,* 2011, vol. 187, 3751-58
- **LACHMANN, P.J. et al.** *Springer Semin. Immunopathol.,* 1984, vol. 7, 143-162
- **PANGBURN, M.K. et al.** *J. Exp. Med.,* 1981, vol. 154, 856-867
- **SCHWAEBLE W.J. ; REID K.B.** *Immunol Today,* 1999, vol. 20 (1), 17-21
- **SCHWAEBLE et al.** *Immunobiol,* 2002, vol. 205, 455-466
- **YONGQING et al.** *BBA,* 2012, vol. 1824, 253
- **IWAKI D. et al.** *J Immunol,* 2011, vol. 187 (7), 3751-8
- **STANTON et al.** Evidence That the HTRA1 Interactome Influences Susceptibility to Age-Related Macular Degeneration. *The Association for Research in Vision and Ophthalmology 2011 conference,* 04 May 2011
- **ROORYCK C et al.** *Nat Genet.,* 2011, vol. 43 (3), 197-203
- **ZUNDEL S et al.** *J Immunol.,* 2004, vol. 172 (7), 4342-50
- **SCHWAEBLE et al.** *PNAS,* 2011
- **KAWASAKI et al.** *J. Biochem,* 1989, vol. 106, 483-489
- **MATSUSHITA ; FUJITA.** *J. Exp Med.,* 1992, vol. 176, 1497-1502
- **JI et al.** *J. Immunol,* 1993, vol. 150, 571-578
- **DAHL et al.** *Immunity,* 2001, vol. 15, 127-135
- **STOVER et al.** *J. Immunol,* 1999, vol. 162, 3481-3490
- **STOVER et al.** *J. Immunol,* 1999, vol. 163, 6848-6859
- **TAKAHASHI et al.** *Int. Immunol,* 1999, vol. 11, 859-63
- **DEGN et al.** *J. Immunol,* 2009, vol. 183 (11), 7371-8
- **SKJOEDT et al.** *J Biol Chem,* 2010, vol. 285, 8234-43
- **MATSUSHITA et al.** *Collectins and Innate Immunity,* 1996
- **JI et al.** *J Immunol,* 1993, vol. 150, 571-578
- **THIEL et al.** *J Immunol,* 2000, vol. 165, 878-887
- **LE et al.** *FEBS Lett,* 1998, vol. 425, 367
- **SUGIMOTO et al.** *J. Biol Chem,* 1998, vol. 273, 20721
- **MATSUSHITA et al.** *J Immunol,* 2000, vol. 164, 2281-2284
- **MATSUSHITA et al.** *J Immunol,* 2002, vol. 168, 3502-3506
- **HANSEN et al.** *J Immunol,* 2010, vol. 185, 6096-6104
- **SCHWAEBLE et al.** *PNAS,* 2011, vol. 108, 7523-7528
- **WALLIS et al.** *J. Biol Chem,* 2004, vol. 279, 14065-14073
- **SATO et al.** *Int Immunol,* 1994, vol. 6, 665-669
- **TAKADA et al.** *Biochem Biophys Res Commun,* 1993, vol. 196, 1003-1009
- **TAKAYAMA et al.** *J. Immunol,* 1994, vol. 152, 2308-2316
- **ENDO et al.** *J Immunol,* 1998, vol. 161, 4924-30
- **TAKADA et al.** *Genomics,* 1995, vol. 25, 757-759
- **STOVER et al.** *Cytogenet and Cell Genet,* 1999, vol. 84, 148-149
- **STOVER et al.** *Genes and Immunity,* 2001, vol. 2, 119-127
- **NONAKA ; MIYAZAWA.** *Genome Biology,* 2001, vol. 3, 1001.1-1001.5
- **VORUP-JENSEN et al.** *J. Immunol,* 2000, vol. 165, 2093-2100
- **ROSSI et al.** *J Biol Chem,* 2001, vol. 276, 40880-40887
- **AMBRUS et al.** *J Immunol,* 2003, vol. 170, 1374-1382
- **GÁL et al.** *J Biol Chem,* 2005, vol. 280, 33435-33444
- **STENGAARD-PEDERSEN et al.** *New Eng. J. Med.,* 2003, vol. 349, 554-560
- **TAKAHASHI et al.** *J. Immunol,* 2008, vol. 180, 6132-6138
- **HÉJA et al.** *PNAS,* 2011, vol. 106, 10498-503
- **MEGYERI et al.** *J. Biol. Chem.,* 2013, vol. 288 (13), 8922-34
- **OGATA et al.** *J. Immunol,* 1995, vol. 154, 2351-2357
- **MATSUSHITA et al.** *J Immunol,* 2000, vol. 165, 2637-2642
- **ZUNDEL et al.** *J Immunol,* 2004, vol. 172, 4342-4350

- **TAKAHASHI et al.** *J Exp Med,* 2010, vol. 207, 29-37
- **TAKAHASHI et al.** *J Immunol,* 2008, vol. 180, 6132-6138
- **RAWAL et al.** *J Biol Chem,* 2008, vol. 283 (12), 7853-63
- **MEGYERI M. et al.** *J Biol Chem.,* 29 March 2013, vol. 288 (13), 8922-34
- **KOCSIS et al.** *J Immunol,* 2010, vol. 185 (7), 4169-78
- **DEGN et al.** *J. Immunol.,* 2012, vol. 189 (8), 3957-69
- **MEGYERI et al.** *J. Biol. Chem.,* 2013, vol. 288, 8922-8934
- **HÉJA et al.** *J. Biol. Chem.,* 2012, vol. 287 (24), 20290-300
- **HÉJA et al.** *PNAS,* 2012, vol. 109, 10498-503
- **HAJELA K. et al.** *Immunobiology,* 2002, vol. 205 (4-5), 467-75
- **KRARUP et al.** *Biochim Biophys Acta,* 2008, vol. 1784 (9), 1294-1300
- **MEGYERI et al.** *J Immunol,* 2009, vol. 183 (5), 3409-16
- **DOBÓ et al.** *J Immunol,* 2009, vol. 183, 1207-1214
- **KRARUP A. et al.** *PLoS One,* 2007, vol. 2 (7), e623
- **ZUNDEL S. et al.** *J Immunol,* 2004, vol. 172, 4342-4350
- **TAKAHASHI et al.** *JEM,* 2010, vol. 207 (1), 29-37
- **ROORYCK et al.** *Nat Genet.,* 2011, vol. 43 (3), 197-203
- **DOBÓ et al.** *J. Immunol,* 2009, vol. 183, 1207-1214
- **HARMAT et al.** *J Mol Biol,* 2004, vol. 342, 1533-1546
- **STOVER et al.** *Genes Immunity,* 2003, vol. 4, 374-84
- **LYNCH N. J. et al.** *J Immunol,* 2005, vol. 174, 4998-5006
- **STENGAARD-PEDERSEN et al.** *N Engl J Med,* 2003, vol. 349, 554-560
- **SUPER et al.** *Lancet,* 1989, vol. 2, 1236-1239
- **GARRED et al.** *Lancet,* 1995, vol. 346, 941-943
- **NIELSEN et al.** *Clin Exp Immunol,* 1995, vol. 100, 219-222
- **GARRED et al.** *Mol Immunol,* 1996, vol. 33 (1), 8
- **EGLI et al.** *PLoS One,* 2013, vol. 8 (1), e51983
- **RUSKAMP et al.** *J Infect Dis.,* 2008, vol. 198 (11), 1707-13
- **ISRAËLS et al.** *Arch Dis Child Fetal Neonatal Ed.,* 2010, vol. 95 (6), F452-61
- **TAKAHASHI M. et al.** *JEM,* 2010, vol. 207, 29-37
- **BANDA et al.** *Mol Imunol,* 2011, vol. 49 (1-2), 281-9
- **ROORYCK C et al.** *Nat Genet.,* vol. 43 (3), 197-203
- **IWAKI D. et al.** *J. Immunol.,* 2011, vol. 187 (7), 3751-3758
- **MATHIESON PW et al.** *J Exp Med,* 1993, vol. 177 (6), 1827-3
- **LINDORFER, M.A. et al.** *Blood,* 2010, vol. 115 (11), 2283-91
- **RISITANO et al.** *Mini-Reviews in Medicinal Chemistry,* 2011, vol. 11, 528-535
- **RISITANO et al.** *Mini Reviews in Med Chem,* 2011, vol. 11, 528-535
- **HILLMEN P. et al.** *N. Engl. J. Med.,* 2004, vol. 350 (6), 552-559
- **RISITANO et al.** *Mini-Reviews in Medicinal Chemistry,* 2011, vol. 11 (6
- **RISITANO A.M. et al.** *Blood,* 2009, vol. 113, 4094-100
- **LINDORFER et al.** *Blood,* 2010, vol. 115, 2283-91
- **RISITANO.** *Biologics,* 2008, vol. 2, 205-222
- *PLOS Pathog.,* 2012, vol. 8, e1002793
- *Nat Genet,* 2011, vol. 43 (3), 197-203
- **SELANDER et al.** *J Clin Invest,* 2006, vol. 116 (5), 1425-1434
- **SKJOEDT et al.** *Immunobiol.,* 2010, vol. 215, 921-931
- **RISITANO et al.** *Blood,* 2009, vol. 113, 4094-4100
- **FRIEDMAN, D.S. et al.** *Arch. Ophthalmol.,* 2004, vol. 122, 564-572
- **KHANDHADIA, S. et al.** *Immunobiol.,* 2012, vol. 217, 127-146
- **ISSA, P.C. et al.** *Graefes. Arch. Clin. Exp. Ophthalmol.,* 2011, vol. 249, 163-174
- **MULLINS et al.** *FASEB J.,* 2000, vol. 14, 835-846
- **JOHNSON et al.** *Exp. Eye Res.,* 2000, vol. 70, 441-449
- **MONTES, T. et al.** *Proc. Natl. Acad. Sci. U.S.A.,* 2009, vol. 106, 4366-4371
- **CAI et al.** *Front Biosci.,* 2012, vol. 17, 1976-95
- *J Biol Chem.,* 2009, vol. 284 (25), 16939-47
- **COLLARD CD et al.** *Mol Immunol.,* 1999, vol. 36 (13-14), 941-8
- **COLLARD C.D. et al.** *Am J Pathol.,* 2000, vol. 156 (5), 1549-56
- **ROHRER, B. et al.** *Invest. Ophthalmol. Vis. Sci.,* 2007, vol. 48, 5282-5289
- **ROHRER, B. et al.** *Invest. Ophthalmol. Vis. Sci.,* 2009, vol. 50, 3056-3064
- **ROHRER, B. et al.** *J. Ocul. Pharmacol. Ther.,* 2012, vol. 28, 402-409
- **ROHRER et al.** *Mol Immunol.,* 2011, vol. 48, e1-8

- **RYAN et al.** *Tr Am Opth Soc,* 1979, vol. LXXVII, 707-745
- **ELTZSCHIG, H.K. ; TOBIAS, E.** *Nat. Med.,* 2011, vol. 17, 1391-1401
- **DIEPENHORST, G.M.P. et al.** *Ann. Surg.,* 2009, vol. 249, 889-899
- **STAHL, G.L. et al.** *Am. J. Pathol.,* 2003, vol. 162, 449-455
- **HART, M.L. et al.** *J. Immunol.,* 2005, vol. 174, 6373-6380
- **LEE, H. et al.** *Mol. Immunol.,* 2010, vol. 47, 972-981
- **SCHWAEBLE, W.J. et al.** *Proc. Natl. Acad. Sci.,* 2011, vol. 108, 7523-7528
- **WALSH, M.C. et al.** *J. Immunol.,* 2005, vol. 175, 541-546
- **JORDAN, J.E. et al.** *Circulation,* 2001, vol. 104, 1413-18
- **M TRENDELENBURG et al.** *Eur Heart J.,* 2010, vol. 31, 1181
- **HAAHR-PEDERSEN S. et al.** *J Inv Cardiology,* 2009, vol. 21, 13
- **COLLARD, C.D. et al.** *Am. J. Pathol.,* 2000, vol. 156, 1549-56
- **ATKINSON, C. et al.** *J. Immunol.,* 2010, vol. 185, 7007-7013
- **THURMAN, J.M. et al.** *J. Immunol.,* 2003, vol. 170, 1517-1523
- **THURMAN, J.M. et al.** *J. Am. Soc. Nephrol.,* 2006, vol. 17, 707-715
- **MOLLER-KRISTENSEN, M. et al.** *Scand. J. Immunol.,* 2005, vol. 61, 426-434
- **VAN DER POL, P. et al.** *Am. J. Transplant.,* 2010, vol. 12, 877-887
- **CASTELLANO G. et al.** *Am J Pathol,* 2010, vol. 176 (4), 1648-59
- **LEINHASE, I. et al.** *BMC Neurosci.,* 2006, vol. 7, 55-67
- **PEDERSEN, E.D. et al.** *Scand. J. Immunol.,* 2009, vol. 69, 555-562
- **ARUMUGAM, T.V. et al.** *Neuroscience,* 2009, vol. 158, 1074-1089
- **CERVERA A et al.** *PLoS One,* 2010, vol. 5 (2), e8433
- **OSTHOFF M. et al.** *PLoS One,* 2011, vol. 6 (6), e21338
- **UNDAR, A. et al.** *Ann. Thorac. Surg.,* 2002, vol. 74, 355-362
- **MCINNES, I.B. ; SCHETT, G.** *New Engl. J. Med.,* 2011, vol. 365, 2205-2219
- **BRODEUR, J.P. et al.** *Arthritis Rheum.,* 1991, vol. 34, 1531-1537
- **BANDA, N.K. et al.** *J. Immunol.,* 2006, vol. 177, 1904-1912
- **BANDA, N.K. et al.** *Clin. Exp. Immunol.,* 2010, vol. 159, 100-108
- **TAKAHASHI, M. et al.** *J. Exp. Med.,* 2010, vol. 207, 29-37
- **BANDA, N.K. et al.** *J. Immunol.,* 2010, vol. 185, 5598-5606
- **HIETALA, M.A. et al.** *Eur. J. Immunol.,* 2004, vol. 34, 1208-1216
- **JI H. et al.** *Immunity,* 2002, vol. 16, 157-168
- **MALHOTRA et al.** *Nat. Med.,* 1995, vol. 1, 237-243
- **CUCHACOVICH et al.** *J. Rheumatol.,* 1996, vol. 23, 44-51
- **RUDD et al.** *Trends Biotechnology,* 2004, vol. 22, 524-30
- **GARRED et al.** *J. Rheumatol.,* 2000, vol. 27, 26-34
- **MOLLNES, T.E. et al.** *Arthritis Rheum.,* 1986, vol. 29, 1359-64
- **EL-GHOBAREY, A.F. et al.** *J. Rheumatology,* 1980, vol. 7, 453-460
- **AGARWAL, A. et al.** *Rheumatology,* 2000, vol. 39, 189-192
- **TRIOLO.** *Clin Exp Rheumatol.,* 2003, vol. 21 (2), 225-8
- **CHIMENTI.** *Clin Exp Rheumatol.,* 2012, vol. 30 (1), 23-30
- **BALLANTI et al.** *Autoimmun Rev.,* 2011, vol. 10 (10), 617-23
- **WANG, Q. et al.** *Nat. Med.,* 2011, vol. 17, 1674-1679
- **MORRISON, T.E. et al.** *J. Virol.,* 2007, vol. 81, 5132-5143
- **GUNN, B.M. et al.** *PLoS Pathog.,* 2012, vol. 8, e1002586
- **LEVI, M. ; OPAL, S.M.** *Crit. Care,* 2006, vol. 10, 222-231
- **SPRONG, T. et al.** *Clin. Infect. Dis.,* 2009, vol. 49, 1380-1386
- **LA BONTE, L.R. et al.** *J. Immunol.,* 2012, vol. 188, 885-891
- **KRARUP et al.** *PLoS One,* 2007, vol. 2 (7), e623
- **GULLA et al.** *Immunology,* 2010, vol. 129 (4), 482-95
- **PRESANIS J.S. et al.** *Mol Immunol,* 2004, vol. 40 (13), 921-9
- **GULLA K. C.** *Immunology,* 2010, vol. 129 (4), 482-95
- **ZHENG, X.L. ; SADLER, J.E.** *Annu. Rev. Pathol.,* 2008, vol. 3, 249-277
- **THURMAN, J.M. et al.** *Clin. J. Am. Soc. Nephrol.,* 2009, vol. 4, 1920-1924
- **ORTH, D. et al.** *J. Immunol.,* 2009, vol. 182, 6394-6400
- **MORIGI, M. et al.** *J. Immunol.,* 2011, vol. 187, 172-180
- **LOIRAT, C. ; FREMEAUX-BACCHI, V.** *Orphanet J. Rare Dis.,* 2011, vol. 6, 60-90
- **ATKINSON, J.P. ; GOODSHIP, T.H.J.** *J. Exp. Med.,* 2007, vol. 6, 1245-1248

- **PICKERING, M.C. et al.** *J. Exp. Med.,* 2007, vol. 204, 1249-1256
- **SULLIVAN M. et al.** *Ann Hum Genet,* 2010, vol. 74, 17-26
- **COLLARD et al.** *Am J. Pathol,* 2000, vol. 156 (5), 1549-56
- **MOLLER-KRISTENSEN et al.** *Scand J Immunol,* 2005, vol. 61 (5), 426-34
- **SCHWAEBLE.** *PNAS,* 2011
- **LEE, C.S. et al.** *Nephrology,* 2012, vol. 17 (1), 48-52
- **BANERJEE R. et al.** *Pediatr Infect Dis J.,* 2011, vol. 30 (9), 736-9
- **GEORGE, JN.** *N Engl J Med,* 2006, vol. 354, 1927-35
- **TSAI, H.** *J Am Soc Nephrol,* 2003, vol. 14, 1072-1081
- **GEORGE JN.** *Oncology (Williston Park),* 2011, vol. 25, 908-14
- **GEORGE JN.** *Curr Opin Hematol,* 2003, vol. 10, 339-344
- **HAMASAKI K et al.** *Clin Rheumatol.,* 2003, vol. 22, 355-8
- **AZARM, T. et al.** *J Res Med Sci.,* 2011, vol. 16, 353-357
- **MOAKE JL.** *N Engl JMed.,* 2002, vol. 347, 589-600
- *Pediatr Nephrol,* 2011, vol. 26, 631-5
- **ROCK GA et al.** *N Engl J Med,* 1991, vol. 325, 393-397
- **TSAI, H-M.** *Hematol Oncol Clin North Am.,* 2007, vol. 21 (4), 609-v
- **GALBUSERA, M. et al.** *Haematologica,* 2009, vol. 94, 166-170
- **RUIZ-TORRES MP et al.** *Thromb Haemost,* 2005, vol. 93, 443-52
- **M. RÉTI et al.** *J Thromb Haemost.,* 2012, vol. 10 (5), 791-798
- **PEERSCHKE E. et al.** *Mol Immunol,* 2010, vol. 47, 2170-5
- **ZHANG, X. ; KOHL, J.** *Expert. Rev. Clin. Immunol.,* 2010, vol. 6, 269-277
- **VARGA et al.** *Mol Immunol.,* 2003, vol. 39 (14), 839-46
- **ROBBINS, R.A. et al.** *Am. J. Physiol.,* 1991, vol. 260, L254-L259
- **KANEMITSU, H. et al.** *Biol. Pharm. Bull.,* 1998, vol. 21, 129-132
- **TAUBE et al.** *Am J Respir Crit Care Med.,* 2003, vol. 168 (11), 1333-41
- **TAUBE, C. et al.** *Proc. Natl. Acad. Sci. USA,* 2006, vol. 103, 8084-8089
- **TAKEDA, K. et al.** *J. Immunol.,* 2012, vol. 188, 661-667
- **HOGABOAM et al.** *J. Leukocyte Biol.,* 2004, vol. 75, 805-814
- **ALCHI, B. ; JAYNE, D.** *Pediatr. Nephrol.,* 2010, vol. 25, 1409-1418
- **SETHI et al.** *Clin J Am Soc Nephrol.,* 2011, vol. 6 (5), 1009-17
- **SMITH, R.J.H. et al.** *Mol. Immunol.,* 2011, vol. 48, 1604-1610
- **DAINA, E. et al.** *New Engl. J. Med.,* 2012, vol. 366, 1161-1163
- **VIVARELLI, M. et al.** *New Engl. J. Med.,* 2012, vol. 366, 1163-1165
- **LHOTTA et al.** *Nephrol Dial Transplant.,* 1999, vol. 14 (4), 881-6
- **PICKERING et al.** *Nat Genet.,* 2002, vol. 31 (4), 424
- **HEDGER, N. et al.** *Nephrol. Dial. Transplant.,* 2000, vol. 15, 1593-1599
- **CHEN, M. ; KALLENBERG, C.G.M.** *Nat. Rev. Rheumatol.,* 2010, vol. 6, 653-664
- **XING, G.Q. et al.** *J. Clin. Immunol.,* 2009, vol. 29, 282-291
- **XIAO, H. et al.** *J. Clin. Invest.,* 2002, vol. 110, 955-963
- **XIAO, H. et al.** *Am. J. Pathol.,* 2007, vol. 170, 52-64
- **CHEN et al.** *JASN,* 2007, vol. 18 (2), 599-605
- **CHEN et al.** *Nat Rev Nephrol.,* 2009, vol. 5 (6), 313-8
- **XING et al.** *J Clin Immunol.,* 2010, vol. 30 (1), 144-56
- **LANGLOIS, J.A. et al.** *J. Head Trauma Rehabil.,* 2006, vol. 21, 375-378
- **RUTLAND-BROWN, W. et al.** *J. Head Trauma Rehabil.,* 2006, vol. 21, 544-548
- **MCALLISTER, T.W.** *Dialogues Clin. Neurosci.,* 2011, vol. 13, 287-300
- **WAGNER, E. et al.** *Nature Rev Drug Disc.,* 2010, vol. 9, 43-56
- **STEVENS, B. et al.** *Cell,* 2007, vol. 131, 1164-1178
- **KURAYA, M. et al.** *Int Immunol.,* 2003, vol. 15, 109-17
- **LEINHASE I et al.** *BMC Neurosci.,* 2006, vol. 7, 55
- **LEINHASE I et al.** *J Neuroinflammation,* 2007, vol. 4, 13
- **IWAKI, D. et al.** *J Immunol.,* 2011, vol. 187, 3751-8
- **MARIK, P.E.** *New Engl. J. Med.,* 2001, vol. 344, 665-671
- **SOBORG et al.** *Journal of Infectious Diseases,* 2003, vol. 188, 777-82
- **WEISER MR et al.** *J. Appl. Physiol.,* 1997, vol. 83 (4), 1090-1095
- **TABAN, M. et al.** *Arch. Ophthalmol.,* 2005, vol. 123, 613-620
- **DINU V et al.** *Genet Epidemiol,* 2007, vol. 31, 224-37
- **CHOW SP et al.** *Clin Experiment Ophthalmol.,* 2011, vol. 39, 871-7
- **WANG Y. et al.** *Mol Vis,* 2007, vol. 13, 1226-33

- **PAPADOPOULOS ; VERKMAN.** *Lancet Neurol.,* 2013, vol. 11 (6), 535-44
- **JARIUS S ; WILDEMANN B.** *Nat Rev Neurol.,* July 2010, vol. 6 (7), 383-92
- **MISU et al.** *Acta Neuropathol,* 2013, vol. 125 (6), 815-27
- **JARIUS et al.** *Nat Clin Pract Neurol.,* 2008, vol. 4 (4), 202-14
- **KURODA et al.** *J Neuroimmunol.,* 2013, vol. 254 (1-2), 178-82
- **SAADOUN et al.** *Brain,* 2010, vol. 133 (2), 349-61
- **PHUAN et al.** *Acta Neuropathol,* 2013, vol. 125 (6), 829-40
- **HARBOE et al.** *Clin Exp Immunol,* 2004, vol. 138 (3), 439-46
- **TÜZÜN E et al.** *J Neuroimmunol.,* 2011, vol. 233 (1-2), 211-5
- **AL-ARAJI A et al.** *Lancet Neurol.,* 2009, vol. 8 (2), 192-204
- **KIRINO et al.** *Nat Genet,* 2013, vol. 45 (2), 202-7
- **BARDAK ; ARIDOGAN.** *Ocul Immunol Inflamm,* 2004, vol. 12 (1), 53-8
- **JONGEN et al.** *Arch Neurol,* 1992, vol. 49 (10), 1075-8
- **TÜZÜN E et al.** *J Neuroimmunol,* 2011, vol. 233 (1-2), 211-5
- **HARBOE M et al.** *Clin Exp Immunol,* 2004, vol. 138 (3), 439-46
- **ZUNDEL, S. et al.** *J.Immunol.,* 2004, vol. 172, 4342-4350
- **MA Y. et al.** *J Innate Immun.,* 04 December 2012
- **IKEDA, K. et al.** *J. Biol. Chem.,* 1987, vol. 262, 7451-7454
- **MATSUSHITA, M. et al.** *J. Exp. Med.,* 2000, vol. 176, 1497-2284
- **MATSUSHITA, M. et al.** *J. Immunol.,* 2002, vol. 168, 3502-3506
- **THIELENS, N.M. et al.** *J. Immunol.,* 2001, vol. 166, 5068
- **WALLIS, R. et al.** *J. Biol. Chem.,* 2000, vol. 275, 30962-30969
- **PETERSON, S.V. et al.** *Mol. Immunol.,* 2000, vol. 37, 803-811
- **MOLLER-KRISTENSEN, M. et al.** *J. of Immunol. Methods,* 2003, vol. 282, 159-167
- **PETERSON, S.V. et al.** *Mol. Immunol.,* April 1998, vol. 35, 409
- **TERAI I. et al.** *Clin Exp Immunol,* 1997, vol. 110, 317-323
- **ENDO M. et al.** *Nephrol Dial Transplant,* 1998, vol. 13, 1984-1990
- **SKJOEDT et al.** *Immunobiology,* 2010, vol. 215 (11), 921-31
- **MOLLER-KRISTENSEN et al.** *Int Immunol,* 2007, vol. 19, 141
- **DUNCAN, A.R. et al.** *Nature,* 1988, vol. 332, 738-740
- **HARLOW.** Antibodies: A Laboratory Manual. Cold Spring Harbor Laboratory, 1988
- **JOLLIFFE et al.** *Int'l Rev. Immunol.,* 1993, vol. 10, 241-250
- **RODRIGUES et al.** *J. Immunol.,* 1998, vol. 151, 6954-6961
- **RAVETCH, J.V. et al.** *Annu. Rev. Immunol.,* 1991, vol. 9, 457-492
- **ISAACS, J.D. et al.** *J. Immunol.,* 1992, vol. 148, 3062-3071
- **VAN DE WINKEL, J.G. et al.** *Immunol. Today,* 1993, vol. 14, 215-221
- **VAUGHAN, T.J. et al.** *Nature Biotechnical,* 1998, vol. 16, 535-539
- Production of Polyclonal Antisera. **GREEN et al.** Immunochemical Protocols
- **LOSMAN, M.J. et al.** *Int. J. Cancer,* 1990, vol. 46, 310
- **KOHLER, G. et al.** *Nature,* 1975, vol. 256, 495
- **CLACKSON, T. et al.** *Nature,* 1991, vol. 352, 624-628
- **MARKS, J.D. et al.** *J. Mol. Biol.,* 1991, vol. 222, 581-597
- Current Protocols in Immunology. John Wiley & Sons, 1991, vol. 1, 2.5.1-2.6.7
- **GREEN, L.L. et al.** *Nature Genet.,* 1994, vol. 7, 13
- **LONBERG, N. et al.** *Nature,* 1994, vol. 368, 856
- **TAYLOR, L.D. et al.** *Int. Immun.,* 1994, vol. 6, 579
- Purification of Immunoglobulin G (IgG). **BAINES et al.** Methods in Molecular Biology. The Humana Press, Inc, 1992, vol. 10, 79-104
- **HARLOW ; LANE.** Antibodies: A Laboratory Manual. Cold Spring Harbor Laboratory Press, 1988
- **BENNY K. C. LO.** Antibody Engineering: Methods and Protocols. Humana Press, 2004
- **BORREBAEK.** Antibody Engineering, A Practical Guide. W.H. Freeman and Co., NY, 1992
- **BORREBAEK.** Antibody Engineering. Oxford University Press, 1995
- **JOHNE et al.** *Immunol. Meth.,* 1993, vol. 160, 191-198
- **JONSSON et al.** *Ann. Biol. Clin.,* 1993, vol. 51, 19-26
- **JONSSON et al.** *Biotechniques,* 1991, vol. 11, 620-627
- **SCATCHARD, A.** *NY Acad. Sci.,* 1949, vol. 51, 660-672
- **MORRISON, S.L. et al.** *Proc. Nat'l Acad. Sci. USA,* 1984, vol. 81, 6851-6855
- **JONES, P.T. et al.** *Nature,* 1986, vol. 321, 522-525
- **REICHMANN, L. et al.** *Nature,* 1988, vol. 332, 323-329

- **PRESTA.** *Curr. Op. Struct. Biol.,* 1992, vol. 2, 593-596
- **JONES, P.T. et al.** *Nature,* 1986, vol. 321, 522
- **CARTER, P. et al.** *Proc. Nat'l. Acad. Sci. USA,* 1992, vol. 89, 4285
- **SANDHU, J.S.** *Crit. Rev. Biotech.,* 1992, vol. 12, 437
- **SINGER, I.I. et al.** *J. Immun.,* 1993, vol. 150, 2844
- Antibody Engineering Protocols. Humana Press, Inc, 1995
- Engineering Therapeutic Antibodies. **KELLEY et al.** Protein Engineering: Principles and Practice. John Wiley & Sons, Inc, 1996, 399-434
- **GREENSPAN, N.S. et al.** *FASEBJ,* 1993, vol. 7, 437
- **CLARK, W.R.** The Experimental Foundations of Modern Immunology. Wiley & Sons, Inc, 1986
- **NISONOFF, A. et al.** *Arch. Biochem. Biophys.,* 1960, vol. 89, 230
- **PORTER, R.R.** *Biochem. J.,* 1959, vol. 73, 119
- **EDELMAN et al.** Methods in Enzymology. Academic Press, 1967, vol. 1, 422
- **MARIANI, M. et al.** *Mol. Immunol.,* 1991, vol. 28, 69-71
- **WHITLOW et al.** *Methods: A Companion to Methods in Enzymology,* 1991, vol. 2, 97
- **BIRD et al.** *Science,* 1988, vol. 242, 423
- **PACK, P. et al.** *Bio/Technology,* 1993, vol. 11, 1271
- **KAY et al.** Phage Display of Peptides and Proteins. Academic Press, Inc, 1996
- **LARRICK et al.** Methods: A Companion to Methods. *Enzymology,* 1991, vol. 2, 106
- Genetic Manipulation of Monoclonal Antibodies. **COURTENAY-LUCK et al.** Monoclonal Antibodies: Production, Engineering and Clinical Application. Cambridge University Press, 1995, 166
- Genetic Manipulation and Expression of Antibodies. **WARD et al.** Monoclonal Antibodies: Principles and Applications. Wiley-Liss, Inc, 1995, 137
- **MILSTEIN ; CUELLO.** *Nature,* 1983, vol. 305, 537-539
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J. Immunol.,* 1992, vol. 148 (5), 1547-1553
- **HOLLINGER et al.** *Proc. Natl. Acad. Sci USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- **KOSTELNY et al.** *J.Immunol.,* 1992, vol. 148 (5), 1547-1553
- **GRUBER et al.** *J. Immunol,* 1994, vol. 152, 5368
- **ZAPATA et al.** *Protein Eng.,* 1995, vol. 8 (10), 1057-1062
- **WU et al.** *Nat Biotechnol,* 2007, vol. 25, 1290-1297
- **THEIL et al.** *Clin. Exp. Immunol.,* 2012, vol. 169, 38
- **SKJOEDT M et al.** *Immunobiology,* 2010, vol. 215 (11), 921-31
- **DEGN et al.** *J. Immunol Methods,* 2010, 361-37
- **CSUKA et al.** *Mol. Immunol.,* 2013, vol. 54, 271
- **MOLLER-KRISTENSEN M. et al.** *J. Immunol. Methods,* 2003, vol. 282, 159-167
- **LEE, V.H.L.** *Crit. Rev. Ther. Drug Carrier Sys.,* 1988, vol. 5, 69
- **LEE, V.H.L.** *J. Controlled Release,* 1990, vol. 13, 213
- Peptide and Protein Drug Delivery. Marcel Dekker, 1991
- **DEBOER, A.G. et al.** *J. Controlled Release,* 1990, vol. 13, 241
- **LEE, W.A.** *Biopharm.,* November 1990, vol. 22
- **FUERTGES, F. et al.** *J. Controlled Release,* 1990, vol. 11, 139
- **BAE, Y.H. et al.** *J. Controlled Release,* 1989, vol. 9, 271
- **HORI, R. et al.** *Pharm. Res.,* 1989, vol. 6, 813
- **YAMAKAWA, I. et al.** *J. Pharm. Sci.,* 1990, vol. 79, 505
- **YOSHIHIRO, I. et al.** *J. Controlled Release,* 1989, vol. 10, 195
- **ASANO, M. et al.** *J. Controlled Release,* 1989, vol. 9, 111
- **ROSENBLATT, J. et al.** *J. Controlled Release,* 1989, vol. 9, 195
- **MAKINO, K.** *J. Controlled Release,* 1990, vol. 12, 235
- **TAKAKURA, Y. et al.** *J. Pharm. Sci.,* 1989, vol. 78, 117
- **TAKAKURA, Y. et al.** *J. Pharm. Sci.,* 1989, vol. 78, 219
- **FLUGELMAN, M.Y. et al.** *Circulation,* 1992, vol. 85, 1110-1117
- **KILPATRICK et al.** *Biochim Biophys Acta,* 2002, vol. 1572, 401-413
- **KNAPPIK, A. et al.** *J. Mol. Biol.,* 2000, vol. 296, 57-86
- **CHEN, C.B. ; WALLIS.** *J. Biol. Chem.,* 2001, vol. 276, 25894-25902
- **SCHWAEBLE W. et al.** *PNAS,* 2011, vol. 108 (18), 7523-7528
- **TAKAHASHI M. et al.** *J Immunol,* 2008, vol. 180, 6132-6138

- **ROORYCK et al.** *Nature Genetics,* 2011, vol. 43, 197-203
- **BITTER-SUERMANN et al.** *Eur. J. Immunol,* 1981, vol. 11, 291-295
- **LINDORFER, M.A. et al.** *Blood,* 2010, vol. 115 (11
- **RISITANO, A.M.** *Mini-Reviews in Medicinal Chemistry,* 2011, vol. 11, 528-535
- **HILLMEN P. et al.** *N. Engl. J. Med.,* 2004, vol. 350 (6), 552-9
- **HILLMEN P et al.** *Mini-Reviews in Medicinal Chemistry,* 2011, vol. 11 (6
- **YONGQING et al.** *Biochemica et Biophysica Acta,* 2012, vol. 1824, 253-262
- **TEILLET et al.** *J. Biol. Chem.,* 2008, vol. 283, 25715-25724
- **MARCHLER-BAUER et al.** *Nucleic Acids Res.,* 2011, vol. 39, D225-229
- **YABUKI et al.** *PLoS ONE,* 2012, vol. 7 (4), e36032
- **HOLMES.** *Nature Reviews, Drug Discovery,* 2011, vol. 10, 798-800
- **MÜLLER ; KONTERMANN.** *Biodrugs,* 2010, vol. 24, 89-98
- **KIPRIYANOV et al.** *J. Mol. Biol.,* 1999, vol. 293, 41
- **PETERSEN, S.V. et al.** *J. Immunol. Methods,* 2001, vol. 257, 107
- **DODDS, A.W.** *Methods Enzymol.,* 1993, vol. 223, 46
- **FEINBERG et al.** *EMBO J,* 2003, vol. 22, 2348-59
- **GAL et al.** *J. Biol. Chem.,* 2005, vol. 280, 33435-44
- **PETERSEN et al.** *J. Immunol Methods,* 2001, vol. 257, 107-16
- **CHEN et al.** *J. Biol. Chem.,* 2001, vol. 276, 25894-02
- **STOVER et al.** *J Immunol,* 1999, vol. 163, 6848-59
- **KAUFMAN R.J. et al.** *Nucleic Acids Research,* 1991, vol. 19, 4485-90
- **KAUFMAN.** *Methods in Enzymology,* 1991, vol. 185, 537-66
- **CUMBERS, S.J. et al.** *Nat Biotechnol,* 2002, vol. 20, 1129-1134
- **SEO, H. et al.** *Nat Biotechnol,* 2005, vol. 23, 731-735
- **BUERSTEDDE, J.M. et al.** *Embo J,* 1990, vol. 9, 921-927
- **MAIZELS, N.** *Annu Rev Genet,* 2005, vol. 39, 23-46
- **CUMMINGS, W.J. et al.** *PLoS Biol,* 2007, vol. 5, e246
- **SEO, H. et al.** *Nat,* 2005, vol. 23, 731-735
- **HEJA et al.** *J Biol Chem,* 2012, vol. 287, 20290
- **HEJA et al.** *PNAS,* 2012, vol. 109, 10498
- **HEJA et al.** *J. Biol. Chem.,* 2012, vol. 287, 20290
- **HEJA et al.** *Proc. Natl. Acad. Sci.,* 2012, vol. 109, 10498
- **SCHWAEBLE et al.** *PNAS,* 2011, vol. 108, 7523
- **DOBO et al.** *J. Immunol,* 2009, vol. 183, 1207
- **NG et al.** *Nat Rev. Drug Discov,* 2006, vol. 5, 123-32
- **RYAN, S.J.** *Tr. Am. Opth. Soc.,* 1979, vol. LXXVII, 707-745
- **TOBE et al.** *Am. J. Pathol.,* 1998, vol. 153, 1641-46
- **AMBATI et al.** *Survey Ophthalmology,* 2003, vol. 48, 257-293
- **NOZAKI et al.** *Proc. Natl. Acad. Sci. USA,* 2006, vol. 103, 2328-33
- **BORA P.S. et al.** *J Immunol,* 2005, vol. 174, 491-497
- **WALSH M.C. et al.** *Jour of Immunol.,* 2005, vol. 175, 541-546
- **MARBER et al.** *J. Clin Invest.,* 1995, vol. 95, 1446-1456
- **ECKLE et al.** *Am J Physiol Heart Circ Physiol,* 2006, vol. 291, H2533-H2540
- **F.J. SUTHERLAND et al.** *Pharmacol Res,* 2000, vol. 41, 613
- **A.M. KABIR et al.** *Am J Physiol Heart Circ Physiol,* 2006, vol. 291, H1893
- **Y. NISHINO et al.** *Circ Res,* 2008, vol. 103, 307
- **I.G. WEBB et al.** *Cardiovasc Res,* 2010
- **WEISMAN, H.F. et al.** *Science,* 1990, vol. 249, 146-151
- **SHANDELYA, S. et al.** *Circulation,* 1993, vol. 87, 536-546
- **COLLARD, C.D. et al.** *Am. J. Pathol.,* 2000, vol. 156, 1549-1556
- **WALPORT, M.J.** *N. Engl. J. Med.,* 2001, vol. 344, 1058-1066
- **SCHWAEBLE, W. et al.** *Immunobiology,* 2002, vol. 205, 455-466
- **FUJITA, T.** *Nat. Rev. Immunol.,* 2002, vol. 2, 346-353
- **THIEL, S. et al.** *Nature,* 1997, vol. 386, 506-510
- **STOVER, C.M. et al.** *J. Immunol.,* 1999, vol. 162, 3481-3490
- **TAKAHASHI, M. et al.** *Int. Immunol.,* 1999, vol. 11, 859-863
- **STOVER, C.M. et al.** *J. Immunol.,* 1999, vol. 163, 6848-6859
- **IWAKI, D. et al.** *J. Immunol.,* 2006, vol. 177, 8626-8632
- **VORUP-JENSEN, T. et al.** *J. Immunol.,* 2000, vol. 165, 2093-2100
- **THIEL, S. et al.** *J. Immunol.,* 2000, vol. 165, 878-887

- **ROSSI, V. et al.** *J. Biol. Chem.,* 2001, vol. 276, 40880-40887
- **TAKAHASHI, M. et al.** *J. Immunol.,* 2008, vol. 180, 6132-6138
- **TAKAHASHI, K. et al.** *Microbes Infect.,* 2002, vol. 4, 773-784
- **MAY, J.E. ; M. FRANK.** *J. Immunol.,* 1973, vol. 111, 1671-1677
- **YANG, Y. et al.** *J. Immunol.,* 2004, vol. 173, 2803-2814
- **MOLLER-KRISTENSEN et al.** *Int. Immunol.,* 2007, vol. 19, 141-149
- **BROWN, J. S. et al.** *Proc. Natl. Acad. Sci. U. S. A.,* 2002, vol. 99, 16969-16974
- **BOOS, L.A. et al.** *Glia,* 2005, vol. 49, 158-160
- **CLARK, A. et al.** *Mol. Immunol.,* 2008, vol. 45, 3125-3132
- **LIN, T. et al.** *Am. J. Pathol.,* 2006, vol. 168, 1241-1248
- **FARRAR, C.A. et al.** *Mol. Immunol.,* 2009, vol. 46, 2832
- **ZHANG, M. et al.** *Proc. Natl. Acad. Sci. U. S. A.,* 2004, vol. 101, 3886-3891
- **ZHANG, M. et al.** *J. Exp. Med.,* 2006, vol. 203, 141-152
- **ZHANG, M. et al.** *Proc. Natl. Acad. Sci. U.S.A.,* 2004, vol. 101, 3886-3891
- **PETERSEN et al.** *J. Immunol. Methods,* 2001, vol. 257, 107-116
- **ZHANG, M. et al.** *Proc. Natl. Acad. Sci. U.S.A.,* 2004, vol. 101, I3886-3891
- **ZHANG, M. et al.** *J. Immunol.,* 2006, vol. 177, 4727-4734
- **MCMULLEN, M.E. et al.** *Immunobiology,* 2006, vol. 211, 759-766
- **NORWOOD, M.G. et al.** *Eur. J. Vasc. Endovasc. Surg.,* 2006, vol. 31, 239-243
- **MOLLER-KRISTENSEN, M. et al.** *J. Immunol. Methods,* 2003, vol. 282, 159-167
- **ENDO, Y. et al.** *Int. Immunol.,* 2002, vol. 14, 1193-1201
- **YANAMOTO et al.** *Exp Neurology,* 2003, vol. 182 (2), 261-274
- **BEDERSON, J.B. et al.** *Stroke,* 1986, vol. 17, 1304-1308
- **LIN T.N. et al.** *Stroke,* 1993, vol. 24, 117-121
- **LINTON ; MORGAN.** *Mol. Immunol.,* 1999, vol. 36, 905-14
- **KAGARI et al.** *J. Immunol.,* 2002, vol. 169, 1459-66
- **KATO et al.** *J. Rheumatol.,* 2003, vol. 30, 247-55
- **BANDA et al.** *J. Immunol.,* 2006, vol. 177, 1904-12
- **X. ZHANG et al.** *J. of Immunol.,* 2009, vol. 182, 5123-5130
- **POLAK, L. et al.** *Nature,* 1969, vol. 223, 738-739
- **FONG J.S. et al.** *J Exp Med,* 1971, vol. 134, 642-655
- *J. Immunol,* 2011, vol. 187 (1), 172-80
- **FROMMHOLD et al.** *BMC Immunology,* 2011, vol. 12, 56-68
- **SPERANDIO et al.** *Blood,* 2001, vol. 97, 3812-3819
- **FROMMHOLD et al.** *BMC Immunology,* 2011, vol. 12, 56-68
- **STEINBAUER et al.** *Langenbecks Arch Surg,* 2000, vol. 385, 290-298
- **ZEINTL et al.** *Int J Microcirc Clin Exp,* 2000, vol. 8 (3), 293-302